(19) Europäisches Patentamt
European Patent Office
Office européen des brevets

(11) EP 2 647 702 A2

(12) EUROPEAN PATENT APPLICATION

(43) Date of publication:
**09.10.2013 Bulletin 2013/41**

(51) Int Cl.:
***C12N 9/54*** *(2006.01)* ***A23K 1/165*** *(2006.01)*

(21) Application number: **13171131.9**

(22) Date of filing: **03.06.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **06.06.2008 US 59695 P**

(62) Document number(s) of the earlier application(s) in accordance with Art. 76 EPC:
**12183921.1 / 2 578 680**
**09759304.0 / 2 297 315**

(71) Applicant: **Danisco US Inc.**
**Palo Alto, CA 94304-1013 (US)**

(72) Inventors:
- **Yuryevich Alekseyev, Viktor**
  **Palo Alto, CA 94304 (US)**
- **Cascao-Pereira, Luis G.**
  **Palo Alto, CA 94304 (US)**
- **Pisarchik, Alexander**
  **Palo Alto, CA 94304 (US)**
- **Kellis, Jr., James T.**
  **Palo Alto, CA 94304 (US)**

(74) Representative: **Forrest, Graham Robert et al**
**Mewburn Ellis LLP**
**33 Gutter Lane**
**London**
**EC2V 8AS (GB)**

Remarks:
This application was filed on 07-06-2013 as a divisional application to the application mentioned under INID code 62.

# (54) Compositions and methods comprising variant microbial proteases

(57) The present invention provides variant subtilisins and compositions comprising at least one variant subtilisin set forth herein, as well as methods for using these variants and compositions. In some embodiments, the present invention provides variant proteases suitable for laundry cleaning applications.

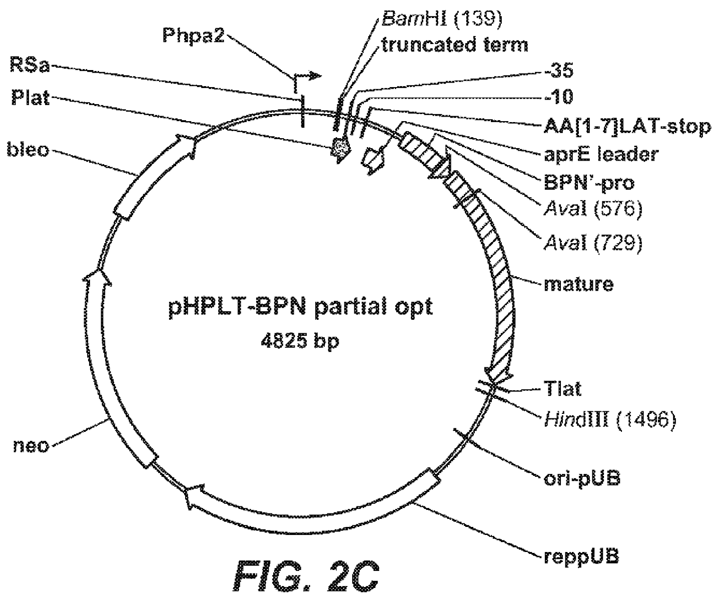


FIG. 2C

EP 2 647 702 A2

## Description

### FIELD OF THE INVENTION

The present invention provides variant subtilisins and compositions comprising at least one variant subtilisin set forth herein, as well as methods for using these variants and compositions.

### BACKGROUND OF THE INVENTION

Serine proteases are a subgroup of carbonyl hydrolases comprising a diverse class of enzymes having a wide range of specificities and biological functions. Much research has been conducted on the subtilisins, due largely to their usefulness in cleaning and feed applications. Additional work has been focused on the adverse environmental conditions (e.g., exposure to oxidative agents, chelating agents, extremes of temperature and/or pH) which can adversely impact the functionality of these enzymes in various applications. Nonetheless, there remains a need in the art for enzyme systems that arc able to resist these adverse conditions and retain or have improved activity over those currently known in the art.

### SUMMARY OF THE INVENTION

The present invention provides variant subtilisins and compositions comprising at least one variant subtilisin set forth herein, as well as methods for using these variants and compositions. In particular, the present invention provides variant proteases suitable for laundry cleaning applications.

The present invention provides subtilisin variants comprising at least one modification, wherein the at least one modification is selected from G100C, S101D, S101H, Q103D, M124G. L126G, S132H, S161N, Q275Z, and/or at least one set of modifications selected from A001T/L096V/G096S/A098D/D099I, A001V/G097S/A098R/D099G, V004M/A098D/D099G, V004M/D099R/G100S/Z100.01L, V004M/G127C/G128S/P129R, V004M/M1119C/D120S, V008I/A098G/D099R, V008I/A098N, V008I/M119C/D120G, Q010L/G097R/A098C/D099S, K012N/A098R/D099V, K012R/G097D/A098H/D099G, A013T/S101R/Q103S, A013V/G097N/A098H/D099G/G160S, A013V/G097S/Z097.01R/A098R, A013V/G100R/S101G, A013V/N118G/M119I, A013V/P129Z/S130H/S132G, P014L/N062D/S101R/Q103S, P014S/A098S/D099G, A015T/V068N/A069G, L016M/G128R/P129R/Z129.01G, H017Q/G127R/P129G, H017Y/A098R/D099I, H017Y/A098R/D099Z/S101G, H017Y/P129R, H017Y/S130Y/G131S/S132L, Q019H/G128R/Z128.01R/P129D, Q0191H/Z128.01G/Z128.202G, G020D/G097R/A098G/D099C, G020D/P129Z/S130G/G131R/S132C, N025Y/S101L/Q103N, A029V/G097R/A098S, S033I/T066G/H067R/V068D/A216E, S038/A098R/D099S, V044I/G097C/A098D/D099G, V044I/G097R/A098R, V044I/G100S/S101G, A045V/G097C/A098R/D099S, A048V/G0097L/A098G/D099S, A048V/G100R/S101G, A048V/G128S/P129L/Z129.01G, M050I/A098G, S053F/S130I/S132G, S053T/D099H/S101Z, E054D/A098R/D099G, T055I/S101R/Q103R, T055S/T066I/Z066.011G/Z066.02G, Q059H/D099S/S101G, Q059H/M119S/D120R, Q059K/M119V/V121C, Q059L/P129G, N061D/G128R/P129V/Z129.01G, S063T/G097D/A098R/D099S, G065R/Z101.01R/Z101.02C, T066S/H067S, V06C/A069G, V068C/A069G/H238Q, V068G/A069G, V068G/A069G/V150I, V068G/A069S, V068I/A069G/A144V, V068I/G097D/A098G/D099Z/G100R, V068L/A069G, V068L/A069S, V068S/A069G, V068S/A069G/A116T, A069G/T071G, A069G/T071I, L075G/N076G/N077Z, L075R/N076G/N077Z, N076D/D099R/Z100.01G, I079F/A098G, V081A/S101R/Q103V, V081I/G097R/A098G/D099C, V084D/G097S/A098G/D099N, V084I/G127C/P129G, V084I/G127L, A085V/A098G, A085V/G097R/A098S/D099N, A085V/G128S/P129R/Z129.01G, A085V/N218I, A085V/S101G, A085V/S204P, A085V/Z099.01H/S101R, P086S/G131/S132G, P086T/S101D/Q103S/V147I, A088S/S101R/Q103V, A088T/A098R/D099R, A088T/D099G/Z100.01G, A088T/G127N/P129R, A088T/G127S/P129R/Q206R/A273S, A088T/P129S/G146D, A088T/S101G, A088T/S130R/G131H/S132N, A088V/G100Z/S101C/Q103R, A088V/P129Z, A092G/S249R, A092G/V093C, A092G/V093I, A092G/V093L, A092L/V093G/K094Z, A092S/K094V, A092S/V093C, A092S/V093G, A092S/V093S, A092V/V093L, V093I/G128D/P129R, V093I/P129Z, V093R/Z093.01G/K094R, K094E/V095D, K094S/V148I, L096I/N118R/M119C/D120G/A151V, Z096.01D/A098R, Z096.01H/A098G, Z096.01N/A098H, Z096.01R/A098C, Z096.01R/A098G, Z096.01R/A098G/V192I, Z096.01R/A098R, Z096.01R/A098R/V192A, Z096.01R/A098S, Z096.01R/A098S/G157S, Z096.01R/Z096.02G, Z096.01R/Z096.02G/V147I, Z096.01S/A098G, Z096.01S/A098N, G097C/A098D/D099S, G097C/A098G/D099G, G097C/A098G/D099N, G097C/A098G/D099S, G097C/A098G/D099S/G100Z, G097C/A098G/D099V, G097C/A098N/D099S, C097C/A098R, G097C/A098R/D099G, G097C/A098R/D99H, G097C/A098R/D099H, G097C/A098R/D099N, G097C/A098R/D099R, G097C/A098R/D099R/N109D/A114S, G097C/A098S/D099H/A151 V, G097C/A098V/D099S, G097C/Z097.01G/A098H/T253I, G097C/Z097.01S/A098G, G097D/A098C, G097D/A098G/D099G, G097D/A098G/D099R, G097D/A098H/D099R, G097D/A098H/D099V, G097D/A098R/D099G, G097D/A098R/D099R,

G097D/A098R/D099S, G0970D/A098R/D099Z, G097D/G100C/S101R, G097D/G100R/S101G, G097H/A098C/D099G, G097H/A098D/D099N, G097H/A098G, G097H/A098G/D099G, G097H/A098G/D099R, G097H/A098G/D099Z, G097H/A098G/D099Z/A151V, G097H/A098G/D099Z/G 100R, G097H/A098G/D099Z/G100V, G097H/A098G/D099Z/S101N/G166S, G097H/A098R, G097H/A098R/D099G, G097H/A098R/D099L, G097H/A098R/D099N, G097H/A098S/D099G, G097H/A098S/D099I, G097H /A098S/D099L, G097H/A098S/D099S/K237E, G097L/A098C/D099S, G097L/A098G/D099S, G097L/A098G/D099S/G100Z, G097L/A098G/D099Z, G097L/A098H, G097L/A098R/D099N, G097L/A098V, G097N/A098G/D099C, G097N/A098G/D099R, G097N/A098G/D099R/V270I, G097N/A098G/D099S, G097N/A098G/D099V, G097N/A098G/D099V/A151V, G097N/A098G/D099V/S101F, G097N/A098H/D099N, G097N/A098R/D099R, G097N/A098R/D099S, G097N/A098R/D099V, G097N/A098S/D099S, G097R/A098C, G097R/A098C/D099S, G097R/A098F/A200V, G097R/A098G, G097R/A098G/D099C, G097R/A098G/D099G, G097R/A098G/D099G/A273T, G097R/A098G/D099S, G097R/A09HG/D099S/A133D, G097R/A098G/D099V, G097R/A098H/D099C/S182N, G097R/A098H/D99G, G097R/A098N/D099G, G097R/A098N/D099Z, G097R/A098R, G097R/A098R/D099C, G097R/A098R/D099G, G097R/A098R/D099S, G097R/A098S/D099C, G097R/A098S/D099G. G097R/A098S/D099L, G097R/A098S/D099R, G097R/A098S/D099S, G097R/A098S/D099V, G097R/A098V/D099G, G097R/Z097.01H/A098C, G097R/Z097.01R/A098G, G097R/Z097.01R/A098G/V147I, G097R/Z097.01R/A098S, G097R/Z097.01R, G097R/Z097.01S/A098G, G097R/Z097.01S/A098R, G097S/A098C, G097S/A098C/D099G, G097S/A098C/D099H, G097S/A098C/D099L, G097S/A098C/D099R, G097S/A098C/D099S, G097S/A098C/D099Z, G097S/A098G/D099F, G097S/A09SG/D099G, G097S/A098G/D099G, G097S/A098G/D099S, G097S/A098G/D099Z, G097S/A098G/D099Z/G100R, G097S/A098H/D099C, G097S/A098H/D099G, G097S/A098N/D099G, G097S/A098R, G097S/A098R/D099C, G097S/A098R/D099G, G097S/A098R/D099L, G097S/A098R/D099V, G097S/A098R/D099Y, G097S/A098S/D099G, G097S/A098S/D099G/A133V/A153V/S236Y, G097S/A098S/D099G/A153V, G097S/A098S/D099Z, G097S/Z097.01D/A098G/A144T/Q271H, G097S/Z097.01G/A098V, G097S/Z097.01S/A098G, G097S/Z097.01S/A098G/A273T, G097S/Z097.01Y, G097V/A098R, G097V/A098R/D099G, G097V/A098S/D099S, G097Y/A098H/D099N, G097Z/A098S/D099H Z097.01D/A098R/D099G, Z097.01D/A098S/D099L, Z097.01D/A098V/D099G, Z097.01G. Z097.01G/A098G, Z097.01G/A098G/D099S, Z097.01G/A098R, Z097.01G/A098R/D099C, Z097.01G/A098R/D099G, Z097.01G/A098R/D099S, A098C/D099G, A098C/D099G/A116T, A098C/D099H, A098C/D099L, A098C/D099L/K213R, A098C/D99L, A098C/D099N, A098C/D99R, A098C/D099S, A098C/D099S/P194L, A098D/D099C, A098D/D099G, A098D/D099G/G100L, A098D/D099H/G100S, A098D/D099H/H238Y, A098D/D099R, A098D/D099R/G100D, A098D/D099R/G160S, A098F/D099S/G100L, A098G/D099C, A098G/D099G, A098G/D099G/A228T, A098G/D099G/G100H, A098G/D099G/N243D, A098G/D099G/S101P, A098G/D099G/T244S, A098G/D099G, A098G/D099H, A098G/D099L, A098G/D099R, A098G/D099R/A116T, A098G/D099R/G100D, A098G/D099R/V148I, A098G/D099S, A098G/D099S/L267M, A098G/D099V, A098G/D099Z, A098H/D099C, A09HH/D099G, A098H/D99G/A216T, A098H/D099G/G100D, A098H/D099G/G100N/L267M, A098H/099G/G100S, A098H/D099G/V143I, A098H/H099R, A098H/D099R/G100S, A098H/D099S/G100C, A098H/D099S/G100N, A098H/D099Y, A098I/D099G, A098I/D099S, A098L/D099G, A098L/D099S, A098N/D099G, A098N/D099L, A098N/D099S, A098R/A144T, A098R/A274V, A098R/D099C, A098R/D099C, A098R/D099G/G100C, A098R/D099G/G100D, A098R/D099G/G100L/S145T, A098R/D099G/G100N, A098R/D099G/G100R, A098R/D099G/G100S, A098R/D099G/V150I,A098R/D099H, A098R/D099I, A098R/D099L, A098R/D099N, A098R/D099N/G100L, A098R/D099R, A098R/D099R/G100D, A098R/D099R/S101Z, A098R/D099R/130P, A098R/D099R, A098R/D099S, A098R/D099S/G100L/A153V, A098R/D099S/, A098R/D099V, A098R/D099Z/S101G, A098R/G100C/A137T, A098R/G100S, A098S/D099C, A098S/D099G, A098S/D099G/G100C, A098S/D099G/G100D, A098S/D099G/G100R/L250I, A098S/D099G/G100S, A098S/D099G/Z099.01R, A098S/D099H, A098S/D099H/M124V, A098S/D099H/Z099.01R/G169A/V180A, A098S/D099R, A098S/D099R/G100L, A098S/D099R/G154S, A098S/D099R/G166S, A098S/D099R/Z100.01G, A098S/D099S, A098S/D099S/Z099.01R, A098S/D099V, A098T/P129Z/S130R/S132G, A098T/S101G, A098V/D099G, A098V/D099G/G100D, A098V/D099G/G100D/A153V, A098V/D099G/G100S, A098Y/D099R/G100L, A098Y/D099R/G100Z/S101Z/G102D, A098Z/S101R, Z098.01N, Z098.01R, Z098.01R/G100R, D099C/K136N, D099C/S101D, D099C/S101R, D099C/S101Z, D099C/Z099.01S, D099C/Z100.01G, D099F/S101Z, D099G/G100C, D099G/G100D/S101R, D099G/G100H, D099G/G100R/S101V, D099G/G100S/S101R, D099G/S101G, D099G/S101H, D099G/S101V, D099G/S101Z, D099G/S101Z/N269D, D099G/Z099.01N, D099G/Z099.01R/G100S, D099G/Z099.01S, D099G/Z100.01G, D099G/Z100.01G/S183N/Q275K, D099H/S101Z, D099H/S101Z/V150I, D099H/Z100.01G/A273V, D099H/Z100.01G/Z100.02G, D099I/Z099.01S, D099L/N118D, D099L/S101G, D099L/S101V, D099L/Z100.01G, D099N/A116T, D099N/G100R/S101V, D099N/S101G, D099N/S101H, D099N/S101Z, D099N/Z099.01S, D099N/Z100.01G, D099R/S101D, D0099R/S101G, D099R/S101V, D099R/S101Z, D099R/S101Z/A133T, D099R/Z099.01N, D099R/Z099.01S, D099R/Z100.01G, D099R/Z100.01N, D099S/S101G, D099S/S101I, M099S/S101Z, D099S/Z099.01H, D099S/Z099-01L, D099S/Z099.01V, D099S/Z100.01G, D099S/Z100.01G/Z100.02G, D099V/S101D, D099V/S101V, D099V/S101Z, Z099.01H/S101L, Z099.01H/Z099.02R,

Z099.01N/S101R, Z099.01S, Z099.01S/S101V, Z099.01S/Z099.02G/A228T, G100D/S101G, G100D/S101R, G100H/G102V, G100H/S101 G, G100H/S101R, G100L/S101G, G100L/S 101R, G100N/S101D, G100N/S101L, G 100N/S101R, G 100R/L267R/I268V, G100R/S101G, G100R/S101G/G160S/S 182N, G100R/S101G/Q103Z/Y104Z/S161N, G100R/S101G/S163N, G100R/S101N, G100R/S101R, G I 00R/S 101 R/Q103Z/V270I, G100R/S101Z/G102C/Q103D, G100S/S101C, G100S/S101G, G100S/S101R, G100S/S101R, G100S/S101V, G100S/S101V/A231T, Z100.01G, Z100.01L, Z100.01N, Z100.01R, Z100.01R/S101G, S101C/Q103I, S101C/Q103S, S101D/Q103C, S101D/Q103D, S101D/Q103F, S101D/Q103H, S101D/Q103H, S101D/Q103R, S101D/Q103R/H238Y, S101D/Q103S, S101D/Q103V, S10F/Q103G, S101G/A151V, S101G/A179V, S101G/G102S, S101G/Q103G, S101G/Q103L, S101G/Q103N, S101G/Q103R, S101G/Q103R/A179T, S101G/Q103R/G157D, S101G/Q103S, S101G/Q103S/L233S, S101G/Q103S/L233S, S101G/Q245H, S101G/S249N, S101G/V139I, S101G/Z101.01R, S101G/Z101.01R/A200T, S101G/Z102.01G, S101H/G102S, S101H/Q103D, S101H/Q103G, S101H/Q103G/E251Q, S101H/Q103H, S101H/Q103I, S101H/Q103R, S101H/Q103R/A274Z/Q275Z, S101H/Q103S, S101H/Q103V, S101I/Q103R, S101L/G102S/Q103R, S101L/Q103D, S101L/Q103G, S101L/Q103R, S101L/Q103R/A138T, S101L/Q103R/V121I/K213E, S101L/Q103S, S101L/Q103V, S101L/Z101.01R, S101N/Q103R, S101N/Z102.01G, S101R/A151V, S101R/A273V, S101R/E156G, S101R/G102L/Q103G, S101R/G102S, S101R/G102S/Q103R, S101R/G102S/Q103V, S101R/G131D, S101R/Q103C, S101R/Q103D, S101R/Q103D/S248R, S101R/Q103C, S101R/Q103G/A116T, S101R/Q103G/N147I/A153V, S101R/Q103G, S101R/Q103L, S101R/Q103N, S101R/Q103R, S101R/Q103R/S161N, S101R/Q103R/V148I, S101R/Q103S, S101R/Q103S/L12F, S101R/Q103V, S101V/Q103G, S101V/Q103H, S101V/Q103N/Z275.01K, S101V/Q103R, S101V/Q103V, S101Y/G102S/Q103R, S101Y/Q103L S101Y/Q103S, S101Y/Q103V, S101Z/G101I/Q103H/Y104G/L135F, S101Z/G102V/Q103R/Y104L, S101Z/Q103D, S101Z/Q103F, S101Z/Q103L, S101Z/Q103R, G102C/Q103L/Y104S, G102R/Q103C/Y104C/V192I, G102R/Q103G/Y104R, G102R/Q103R/Z103.01G, G102Z/Q103S, Z102.01G/Q103R, Z102.01G/Q103R/L267V, Z102.01G/Z102.02R/Z102.03G, Q103H/Y104N/Q275Z, Q103N/E156D, Q103R/I122L/N123S/M124V, Q103R/Y104F, Q103Y/S182N, S105G/P129S/S130H/G131H, W106C/A116D/N117S/N118C, N109S/M119C/D120G/V121L, A116G/N117Z/N118R/M119C, A116H/N117G/N118R, N117C/N118G/M119S, N117C/N118G/M119S/A216V, N117G/N118G/M119C, N117G/N118R/M119C, N117H/N118C/M119C, N117H/N118D/M119S, N117H/N118G/M119C, N117H/N118G/M119L, N117H/N118G/M119S, N117H/N118G/M119V, N117H/N118H/M119V, N117H/N118R/M119C, N117H/N118R/M119S, N117H/N118S/M119C, N117H/N118S/M119S, N117H/N118S/M119V, N117I/N118G/M119V, N117L/N118G/M119V, N117R/N118G/M119C, N117R/N118G/M119I/V270I, N117R/N118G/M119S, N117R/N118H/M119V, N117R/N118R/M119C, N117R/N118R/M119I, N117R/N118R/M119S, N117R/N118R/M119V, N117R/N118S/M119I, N117R/N118S/M119L, N117S/N118G/M119C, N117S/N118G/M119S, N117S/N118H/M119V, N117S/N118R/M119V, N117V/N118G/M119C, N117V/N118H/M119C, N117Y/N118G/M119S, N117Y/N118G/M119V, Z117.01G/N118R/M119C, N118C/M119V/D120S, N118C/Z118.01G/M119C, N118D/M119C/D120L, N118D/M119S/D120R, N118G/M119C/D120G, N118G/M119C/D120L, N118G/M119I/D120R, N118G/M119N, N118G/M119S/D120G, N118G/M119S/D120R, N118C/M119V/D120G, N118G/M119V/D120S/S182N, N118H/M119C/D120R, N118H/M119I/D120G, N118H/M119N/V148I, N118H/M119S/D120G, N118H/M119V/D120G, N118R/M119C, N118R/M119C/D120G, N118R/M119C/D120R, N118R/M119S, N118R/M119S/D120G, N1118R/M119S/D120R, N118R/M119S/D120S, N118R/M119V/D120C, N118R/M119V/D120G, N118R/M119V/D120G/A232T, N118S/M119C/D120R, M119C/D120G, M119C/D120G/V121I, M119C/D120G/VN121L, M119C/D120H, M119C/D120H/A187V, M119C/D120R, M119H/V121I, M119I/D120C, M119I/D120G, M 119I/D120R, M119L/D120G, M 119S/D120R, M119S/D120S/V121I, M119S/V121I, M119V/D120C, M119V/D120G, M119V/D20R, M119V/D120S/V198L, D120G/I122C, D120G/I122C/L217S, D120G/I122G, D120G/I122V, D120H/I122L, D120H/V121C/I122V, D120N/V121L/I122V, D120R/A151V, D120R/I122V, D120R/V121I, D120R/V121I/I122C, D120R/V121I/I122C/A228T, D120S/I122C/A133S, V121C/I122C/N123C, V121C/I122G/N123V, V121C/I122S/N123V, V121C/I122V/N123S, V121I/I12C/N123C, V121I/I122C/V150I, V121I/I122G/N123C, V121I/I122S/N123C, V121I/I122S/N123I, V121I/I122V/N123S, V121L/I122S/N123V, V121L/N123C, I122C/N123C, I122C/N123S/M124L, I122D/N123V/M124L, I122G/N123C/M124L, I122G/N123G/Z1123.01C, I122H/N123V/M124LI122S/N123S, I122V/N123G/M124C/A138V, I122V/N123S/M124I, I122V/S183G, N 123C/M124S, N 123C/M124V, N123S/M124L, N 123S/M124S, M 124C/L126S, M124C/L126S/T244I, M124C/L126V, M124I/L126H, L126F/P129Z, L126F/P129Z/S182N, L126I/P129Z, L126R/G127R, Z126.01S, G127C/G128S/P129R, G127C/P129G, G127C/P129G/A200T, G127C/P129R, G127C/P129R/A153T, G127D/P129G, G127D/P129S, G127H/P129G, G127H/P129R, G127L//P129R, G127N/P129C, G127N/P129G, G127N/P129G/A133T, G127N/P129G/A151V/K213N, G127N/P129R, G127N/P129S/A273V, G127N/P129V, G127R/A230V, G127R/P129D, G127R/P129D/L217S, G127R/P129G, G127R/P129R, G127R/P129S, G127S/P129D, G127S/P129F, G127S/P129G, G127S/P129G/G166D, G127S/P129G/I175T, G127S/P129R, G127S/P129S, G127S/P129V, G127V/P129G, G127V/P129R, Z127.01G/Z127.02H/P129D, Z127.01G/Z127.02H/P129G, Z127.01G/Z127.02H/P129R, Z127.01H/G128C/P129C/A230V, Z127.01H/P129G, Z127.01H/P129G/V148I, Z127.01H/P129R, Z127.01N/G128C/P129V, Z127.01N/G128S/P129S, Z127.01N/G128S/P129V, Z127.01N/P129R,

Z127.01R/G128S/P129S, Z127.01R/P129G, Z127.01S/P129D, Z127.01S/P129G, Z127.01S/P129R, G128D/P129G, G128D/P129R/Z129.01R, G128D/P129S/G154S, G128H/P129C, G128H/P129H/Z129.01G, G28H/P129H/Z129.01R, G128H/P129R, G128H/P129R/Z129.01G, G128H/P129R/Z129.01R, G128H/P129S/Z129.01G, G128H/P129S/Z129.01R, G128H/P129S/Z129.01R/S248N, G128H/P129Y, G128H/Z128.01R/P129D, G128H/Z128.01R/P129S, G128H/Z128.01R/P129S/A144T, G128H/Z129.01R, 128N/P129R/Z129.01G, G128N/P129R/Z129.01R, G128N/P129S/Z129.01D, G128N/Z128.01R/P129S, G128R/P129G, G128R/P129H/Z129.01G, G128R/P129L/Z130.01S, G128R/P129R, G128R/P129R/Z129.01G, G128R/P129R/Z129.01G/A216V, G128R/P129S, G128R/P129S/Z129.01G, 128R/P129Z/S130R/S132G, G128R/Z128.01R/P129D, G128R/Z128.01R/P129F, G128S/P129C, G128S/P129C/Z129.01R, G128S/P129D, G128S/P129D/S248R, G128S/P129D/T244N, G128S/P129G, G128S/P129G/A187V, G128S/P129H/Z129.01R, G128S/P129R/L209F, G128S/P129R/Z129.01G, G128S/P129S/Z129.01D, G128S/P129S/Z129.01R, G128S/P129V, G128Y/P129R/Z129.01G, Z128.01G, Z128.01G/P129D, Z128.01G/P129R, Z128.01G/P129S/A134T, Z128.01G/Z128.02G, Z128.01R/P129D, Z128.01R/Z128.02G/P129S, Z128.01R/Z128.02H/P129G/G160S, Z128.01R/Z128.02R/P129D, Z128.01Y/Z128.02H/P129R, P129C/S130C/S132Z, P129D/Z129.01G/Z129.02D/G160D, P129D/Z129.01G/Z129.02G, P129G/G131Z, P129G/S130G/Z131.01G, P129G/S130H/S132Z, P129G/S130R/S132Z, P129G/V270I, P129G/Z129.01G, P129G/Z129.01R/Z129.02G, P129H/G131Z, P129H/G131Z/S132C, P129H/G131Z/S132H, P129H/S132Z, P129H/Z130.01D/Z130.02S, P129R/G131Z/S132H, P129R/S130D/S132Z, P129R/S130N/G131Z, P129R/S132T, P129R/S132Z, P129R/Z129.01G, P129R/Z129.01G/Z129.02G, P129R/Z129.01G/Z129.02G/A144T, P129R/Z129.01G/Z129.02G/G146D, P129R/Z129.01G/Z129.02R, P129R/Z129.01G, P129R/Z129.01R/Z129.02G, P129S/G131ZP129S/S130H/S132Z, P129S/Z129.01C, P129S/Z129.01R/Z129.02S, P129S/Z130.01N/Z130.02S, P129V/S132Z, P129Z, P129Z/G131R/S132C, P129Z/S130G, P 129Z/S 130G/G 131 H/S 132H, P 129Z/S 130G/G 131 V, P 129Z/S 130H, P 129Z/S 130H/S132G, P129Z/S130H/S132N, P129Z/S130L, P129Z/S130R, P129Z/S130R/G131C. P129Z/S130R/G131C, P129Z/S130R/G131D/S132C, P129Z/S130R/G131H/A144E. P129Z/S130R/G131H/S132D, P129Z/S130R/G131R/S132D, P129Z/S130R/L267M, P129Z/S 130R/S132C, P129Z/S130R/S132G, P129Z/S130V/S132G, P129Z/S130Z/G131N/S132G, S130D/G131C/S132D, S130D/G131R, S130D/G131R/S132D, S130D/G131Z, S130D/G131Z/S132H, S130F/G131N/S132V, S 130F/G131S/S132V, S130F/S132V, S 130G/G131D/S132C, S130G/G131H/S132D, S130G/G131S/S132I, S130G/S132G, S130H/G131C/S132G, S130H/G131H/S132R, S 130H/G131R/S132C, S130H/G131S, S130H/G131S/S132C, S130H/S132Z, S130I/G131S/S132H, S130I/S132G, S130N/G131S/S132C, S130R/G131R/S132C, S130R/G131S/S132C/S161N, S130R/S132G, S130R/S132G/S183N, S130R/S132Z, S 130V/G131D/S132I, S130V/G131H/S132C, S130V/S132N, S130V/S132Z, S130Y/G131H/S132C, S130Y/S132G, S130Z/G131H/S132H, G131H/S132L/W241R, G131N/S132C, G131S/S132G, G131Y/S132G, and A142V/G211V, wherein the positions correspond to the positions of BPN' subtilisin of SEQ ID NO:2.

The present invention also provides subtilisin variants comprising at least one set of modifications, wherein said set of modifications is selected from: V072G/G097A/G128A/Y217Q, G097A/G128S/Y217Q, G097A/G128S/P129D/Y217Q, G097A/G128A/A134T/Y217Q, M124V/L26A/Y217Q, G097A/G128A/P129D/Y217Q/A232P, G097A/G128A/P129D/Y217Q, G097A/G128S/P129V/A134T/Y217Q, G097A/G128S/P129V/Y217Q, V068I/G097A/G128S/P129D/Y217Q, G097A/S101D/G128S/S204F/Y217Q/S236F/T254P, G097A/S101D/G128S/Y217Q, G097A/G128A/P129V/N217Q, G097A/S101D/G128A/Y217Q, G097A/S101D/G128S/P129D/Y217Q, G097A/S101D/G128A/A134T/Y217Q, G097A/Z099.01S/A114S/G128S/Y217Q, G097A/S101D/G128A/P194L/Y217Q, G097A/Z099.01S/G128S/Y217Q, G097A/S101D/G128A/PI29D/Y217Q, Q019R/G097A/Z099.01S/G128S/P129D/Y217Q, G097A/G100S/G128S/Y217Q, G097A/S101D/G128S/P129V/Y217Q, G097A/Z099.01S/G128A/A134T/Y217Q, G097A/G100S/I107V/G128S/P129D/Y217Q, G097A/G100S/G128S/Y217Q/A230V, G097A/G100S/G128A/Y217Q/K237N, G097A/Z099.01S/G128S/P129D/Y217Q, G097A/G100S/G128S/P129D/Y217Q, Z096.01D/G097A/A098R/G128A/Y217Q, G097A/Z099.01S/I122S/N123G/G128A/Y217Q, G097A/Z099.01S/I122S/N123G/G128A/Y217Q/T220A/A232T, P005S/G097A/Z099.01S/S101/G128A/Y217Q, G097A/S101D/G128A/P129V/Y217Q, V093Z/K094S/V095C/L096S/G097A/G128A/Y217Q, G097A/G100S/S101D/G128A/Y217Q/A232P, Z096.01D/G097A/A098R/S101D/G128A/Y217Q, and G097A/Z099.01S/G100S/G128A/Y217Q, wherein the positions correspond to the positions of BPN' su btilisin of SEQ ID NO:2.

The present invention further provides subtilisin variants comprising at one set of modifications, wherein said set of modifications is selected from: G097A/G128S/P129T/Y217Q, G097A/G128A/P129S/Y217Q, G097A/G128S/P129C/Y217Q, G097A/G128S/P129Q/Y217Q, G097A/G128S/P129E/Y217Q, G097A/P129S/Y217Q, G097A/G128S/P129A/Y217Q, G097A/G128S/P129K/Y217Q, G097A/P129N/Y217Q, G097A/P129E/Y217Q, G097A/G128S/P129K/G131S/Y217Q, G097A/G128S/P129R/Y217Q, G097A/G128S/P129V/Y217Q, G097A/G128S/P129Y/Y217Q, G097A/G128A/P129R/Y217Q, G097A/G128H/P129S/Y217Q,

G097A/G128N/P129S/Y217Q/V270A, G097A/G128S/P129L/Y217Q, G097A/P129R/Y217Q, G097A/P129W/Y217Q, G097A/P129Z/S130A/Y217Q, G097A/G128H/P129R/Y217Q, G097A/G128H/P129T/Y217Q, G097A/P129G/Y217Q, G097A/G128N/P129A/Y217Q, G097A/G128H/Y217Q, G097A/G128S/P129W/Y217Q, G097A/P129Z/Y217Q, G097A/G128N/Z128.01A/P129S/Y217Q, G097A/G128N/Y217Q, G097A/G128A/P129L/Y217Q, G097A/G128T/P129N/Y217Q, G097A/G128H/P129A/Y217Q, G097A/G28M/P129R/Y217Q, G097A/G128Q/P129K/Y217Q, G097A/G128T/P129Q/Y217Q, G097A/G128T/P129H/Y217Q, G097A/G128T/P129R/Y217Q, G097A/G128D/P129H/Y217Q, G097A/G128T/P129T/Y217Q, G097A/G128T/P129A/Y217Q, G097A/G128Z/P129Z/Y217Q, .G097A/G128S/Z128.01A/P129S/Y217Q, G097A/G128N/P129M/Y217Q, G097A/G128N/P129V/Y217Q, G097A/G128E/P129K/Y217Q, G097A/G128N/Z128.01A/P129F/Y217Q, G097A/G128C/P129R/Y217Q, G097A/G128T/7P129M/Y217Q, G097A/G128R/Z128.01A/P129S/Y217Q, G097A/G128S/Z128.01A/P129A/Y217Q, G097A/G128D/Y217Q, G097A/G128T/P129Y/Y217Q, G097A/G128R/P129S/Y217Q, G097A/G128R/Z128.01A/P129T/Y217Q, G097A/G128R/Z128.01A/P129A/Y217Q, G097A/G128T/P129W/Y217Q, G097A/G128Y/Z128.0A/P129R/Y217Q, G097A/G128C/P129D/Y217Q, V084/G097A/P129Z/JY217Q, G097A/G128R/P129D/Y217Q, G097A/G128A/Z128.01A/P129H/E195A/Y217Q, G097A/G128S/Z128.01A/P129Y/Y217Q, G097A/G128N/P129L/Y217Q, G097A/G128C/P129H/Y217Q, G097A/G128T/Z128.01A/P129T/Y217Q, G097A/G128C/Z128.01A/P129A/Y217Q, G097A/G128S/Z128.01A/P1291/Y217Q, G097A/G128S/Z128.01A/P129E/Y217Q/Q271H, G097A/G128S/Z128.01A/P129L/Y217Q, G097A/G128Y/Z128.01A/P129S/Y217Q, G097A/G128S/Z128.01A/P129M/R186H/Y217Q, G097A/G128E/Z128.01A/P129D/Y217Q, G097A/G128T/P129L/Y217Q, G097A/G128F/P129D/Y217Q, G097A/G128V/Z128.01A/P129R/Y217Q, G097A/G128L/Z128.01A/P129S/Y217Q, G097A/G128Y/P29T/Y217Q, G097A/G128C/P129Y/Y217Q, G097A/G128C/P129T/Y217Q, wherein the positions correspond to the positions of BPN' subtilisin of SEQ ID NO:2.

The present invention also provides subtilisin variants comprising at least one set of modifications, wherein said set of modifications is selected from: G097A/G128A/Y217Q/M222Q, M124V/L126A/P129V/Y217Q, G128A/P129V/Y217Q, G097A/G128A/P129V/Y217Q, N123G/P129V/Y217Q, V301I/Z096.01D/G097A/A098R/G128A/Y217Q, Z096.01D/A098R/G128A/Y217Q, Z096.01D/A098R/M124V/L126A/Y217Q, G097A/098.01S/G128A/Y217Q, Z096.01D/A098R/N123G/Y217Q, Z096.01D/G097A/A098R/G128A/Y217Q/S236F, Z098.01S/M124V/L126A/Y217Q, Z098.01S/G128A/Y217Q, Z096.01D/0097A/A098R/0128A/Y217Q, V093I/Z098.01S/M124V/L126A/Y217Q, A073V/Z098.01S/N123G/Y217Q, G065D/Z098.01S/G128A/Y217Q, I011L/Z098.01S/G128A/Y217Q, L082F/Z096.01D/A098R/N123G/Y217Q, Z098.01S/N123G/A138V/Y217Q, and Z098.01S/N123G/Y217Q, wherein the positions correspond to the positions of BPN' subtilisin of SEQ ID NO:2.

The present invention also provides subtilisin variants comprising the substitution Y217L and further at least one set of any of the modification sets provided above, wherein the positions correspond to the positions of BPN' subtilisin of SEQ ID NO:2. The present invention further provides subtilisin variants comprising the substitutions G97A/G128A/Y217Q and further comprising at least one modification of any of the modifications sets provided herein, wherein the positions correspond to the positions of BPN' subtilisin of SEQ ID NO:2.

The present invention also provides cleaning compositions comprising at least one subtilisin variant as provided herein. In some preferred embodiments, the cleaning composition is a laundry detergent. In some particularly preferred embodiments, the laundry detergent is a heavy duty liquid laundry detergent. In some alternative embodiments, the cleaning composition is a dish detergent. In some further embodiments, the cleaning compositions further comprise one or more additional enzymes or enzyme derivatives selected from the group consisting of hemicellulases, peroxidases, proteases, cellulases, xylanases, lipases, phospholipases, esterases, cutinases, pectinases, keratinases, reductases, oxidases, phenol oxidases, lipoxygenases, ligninases, pullulanases, tannases, pentosanases, malanases, β-glucanases, arabinosidases, hyaluronidase, chondroitinase, laccase, and amylases, or mixtures thereof. In some further embodiments, the cleaning compositions further comprise at least one stabilizing agent. In still some additional embodiments, the cleaning compositions comprise at least 0.0001 weight percent of at least one of any of the subtilisin variants provided herein, and optionally, at least one suitable adjunct ingredient.

In some embodiments, the present invention provides methods of cleaning, the method comprising the steps of: contacting a surface and/or an article comprising a fabric with at least one of the cleaning compositions set forth herein and optionally washing and/or rinsing the surface or article.

The present invention also provides animal feeds comprising at least one subtilisin variant provided herein, as well as food processing compositions comprising at least one subtilisin variant provided herein.

## BRIEF DESCRIPTION OF THE DRAWINGS

FIG. 1 shows a diagram illustrating the method used for creating in-frame deletions and insertions. Library was designed to have 33% of clones with substitutions; 33% of clones with insertions and 33% of clones with deletions.
FIG. 2A Plasmid map of pAC-FNA10. The plasmid elements are as follows: pUB110 = DNA fragment from plasmid pUB110 (McKenzie et al., Plasmid 15:93-103 [1986], pHR322 = DNA fragment from plasmid pBR322 (Bolivar et al., Gene 2:95-113 [1977]), pC194 = DNA fragment from plasmid pC194 (Horinouchi and Weisblum, J. Bacteriol 150:815-825 [1982]).
FIG. 2B provides a map of pHPLT-FNA.
IG. 2C provides a map of pHPLT-BPN. Vector pHPLT contains the LAT promoter (PLAT), a sequence encoding the LAT signal peptide (preLAT). The origin of replication and ncoR arc derived from plasmid pUB110.

## DESCRIPTION OF THE INVENTION

The present invention provides variant sublilisins and compositions comprising at least one variant subtilisin set forth herein, as well as methods for using these variants and compositions. The modification of the precursor proteases of the invention to produce these variant subtilisins includes at least one substitution, at least one deletion, or at least one insertion. In some embodiments, the modification comprises a combination of mutations. For example, in some embodiments, the modification includes a combination of at least one substitution and at least one deletion. In some other embodiments, the modification includes a combination of at least one substitution and at least one insertion. In some further embodiments, the modification includes a combination of at least one deletion and at least one insertion. In yet other embodiments, the modification includes a combination of at least one substitution, at least one deletion, and at least one insertion.

Several suitable methods are known in the art for generating modified polynucleotide sequences of the present invention, including but not limited to site- saturation mutagenesis, scanning mutagenesis, insertional mutagenesis, deletion mutagenesis, random mutagenesis, site- directed mutagenesis, and directed- evolution, as well as various other recombinatorial approaches. The commonly used methods include DNA shuffling (See, Stemmer, Proc Natl Acad Sci U S A. 25: 10747- 51 [1994] ), methods based on non- homologous recombination of genes (*e.g.* ITCHY) (Ostermeier et al., Bioorg Med Chem. 7: 2139- 44 [1999] ), SCRACHY (Lutz et al. Proc Natl Acad Sci USA. 98: 11248- 53 [2001] ), SHIPREC (Sieber et al., Nat Biotechnol., 19: 456- 60 [2001] ), and NRR (Bittker et al., Nat Biotechnol., 20: 1024- 9 [2001] ; Bittker et al., Proc Natl Acad Sci. USA. 101: 7011- 6 [2004] ), and methods that rely on the use of oligonucleotides to insert random and targeted mutations, deletions and/or insertions (Ness et al., Nat Biotechnol. 20: 1251- 5 [2002] ; Coco et al., Nat Biotechnol., 20: 1246- 50 [2002] ; Zha et al., Chembiochem. 3: 34- 9 [2003], Glaser et al., J Immunol., 149: 3903- 13 [1992], Sondek and Shortle, Proc Natl Acad Sci USA 89: 3581- 5 [1992], Yáñez et al., Nucleic Acids Res., 32: e158 [2004], Osuna et al., Nucleic Acids Res., 32: e136 [2004], Gaytán et al., Nucleic Acids Res., 29: E9 [2001], and Gaytán et al., Nucleic Acids Res., 30: e84 [2002] ) .

In some embodiments, the full-length parent polynucleotide is ligated into an appropriate expression plasmid, and the following mutagenesis method is used to facilitate the construction of the modified protease of the present invention, although other methods may be used. The method is based on that described by Pisarchik *et al.* (Pisarchik et al., Prot. Eng. Des. Select., 20:257-265 [2007]). In some embodiments, an added advantage is provided, in that the restriction enzyme used herein cuts outside its recognition sequence, which allows digestion of practically any nucleotide sequence and precludes formation of a restriction site scar.. First, as described herein, a naturally-occurring gene encoding the full-length protease is obtained and sequenced and scanned for one or more points at which it is desired to make a mutation (deletion, insertion, substitution, or a combination thereof) at one or more amino acids. Mutation of the gene in order to change its sequence to conform to the desired sequence is accomplished by primer extension in accord with generally known methods. Fragments to the left and to the right of the desired point(s) of mutation are amplilied by PCR and to include the *Eam*1104I restriction site. The left and right fragments are digested with *Eam*1104I to generate a plurality of fragments having complementary three base overhangs, which are then pooled and ligated to generate a library of modified sequences containing one or more mutations. This method avoids the occurrence of frame-shift mutations. In addition, this method simplifies the mutagenesis process because all of the oligonucleotides can be synthesized so as to have the same restriction site, and no synthetic linkers are necessary to create the restriction sites as is required by some other methods

### Compositions

The present invention provides variant proteases and compositions comprising at least one of the variant subtilisins set forth herein. In some embodiments, the present invention provides means to produce protease with various commercial applications where degradation or synthesis of polypeptides are desired, including cleaning compositions, as well as feed components, textile processing, leather finishing, grain processing, meat processing, food processing, preparation of protein hydrolysates, digestive aids, microbicidal compositions, bacteriostatic compositions, fungistatic

compositions, and personal care products (*e.g.*, oral care, hair care, and/or skin care).

The present invention further provides enzyme compositions have comparable or improved wash performance, as compared to presently used subtilisin proteases.

## Cleaning Compositions

The cleaning composition of the present invention are advantageously employed for example, in laundry applications. Indeed, due to the unique advantages of increased effectiveness in lower temperature solutions, the enzyme of the present invention are ideally suited for laundry applications. Furthermore, the enzymes of the present invention may be employed in both granular and liquid compositions.

The variant proteases of the present invention also find use laundry cleaning additive products. In some embodiments, low temperature solution cleaning applications find use. The additive product may be, in its simplest form, one or more proteases. In some embodiments, the additive is packaged in dosage form for addition to a cleaning process. Any suitable single dosage form also finds use with the present invention, including but not limited to pills, tablets, gelcaps, or other single dosage units such as pre-measured powders or liquids. In some embodiments, filler(s) or carrier material(s) arc included to increase the volume of such composition. Suitable filler or carrier materials include, but are not limited to, various salts of sulfate, carbonate and silicate as well as talc, clay and the like. Suitable filler or carrier materials for liquid compositions include, but are not limited to water or low molecular weight primary and secondary alcohols including polyols and diols. Examples of such alcohols include, but are not limited to, methanol, ethanol, propanol and isopropanol. In some embodiments, the compositions contain from about 5% to about 90% of such materials. Acidic fillers find use to reduce pH. Alternatively, the cleaning additive include adjunct ingredients as more fully described below.

The present cleaning compositions and cleaning additives require an effective amount of at least one of the protease variants provided herein, alone or in combination with other proteases and/or additional enzymes. The required level of enzyme is achieved by the addition of one or more protease variants of the present invention. Typically the present cleaning compositions comprise at least about 0.0001 weight percent, from about 0.0001 to about 1, from about 0.001 to about 0.5, or even from about 0.01 to about 0.1 weight percent of at least one of the variant proteases of the present invention.

The cleaning compositions herein are typically formulated such that, during use in aqueous cleaning operations, the wash water will have a pH of from about 5.0 to about 11.5 or even from about 7.5 to about 10.5. Liquid product formulations are typically formulated to have a neat pH from about 3.0 to about 9.0 or even from about 3 to about 5. Granular laundry products are typically formulated to have a pH from about 9 to about 11. Techniques for controlling pH at recommended usage levels include the use of buffers, alkalis, acids, etc., and are well known to those skilled in the art.

Suitable low pH cleaning compositions typically have a neat pH of from about 3 to about 5, and are typically free of surfactants that hydrolyze in such a pH environment. Such surfactants include sodium alkyl sulfate surfactants that comprise at least one ethylene oxide moiety or even from about 1 to about 16 moles of ethylene oxide. Such cleaning compositions typically comprise a sufficient amount of a pH modifier, such as sodium hydroxide, monoethanolamine or hydrochloric acid, to provide such cleaning composition with a neat pH of from about 3 to about 5. Such compositions typically comprise at least one acid stable enzyme. In some embodiments, the compositions are liquids, while in other embodiments, they are solids. The pH of such liquid compositions is typically measured as a neat pH. The pH of such solid compositions is measured as a 10% solids solution of said composition wherein the solvent is distilled water. In these embodiments, all pH measurements are taken at 20°C.

In some embodiments, when the variant protease(s) is/are employed in a granular composition or liquid, it is desirable for the variant protease to be in the form of an encapsulated particle to protect the variant protease from other components of the granular composition during storage. In addition, encapsulation is also a means of controlling the availability of the variant protease during the cleaning process. In some embodiments, encapsulation enhances the performance of the variant protease(s) and/or additional enzymes. In this regard, the variant proteases of the present invention are encapsulated with any suitable encapsulating material known in the art. In some embodiments, the encapsulating material typically encapsulates at least part of the catalyst for the variant protease(s) of the present invention. Typically, the encapsulating material is water-soluble and/or water-dispersible. In some embodiments, the encapsulating material has a glass transition temperature (Tg) of 0°C or higher. Glass transition temperature is described in more detail in WO 97/11151. The encapsulating material is selected from consisting of carbohydrates, natural or synthetic gums, chitin, chitosan, cellulose and cellulose derivatives, silicates, phosphates, borates, polyvinyl alcohol, polyethylene glycol, paraffin waxes, and combinations thereof. When the encapsulating material is a carbohydrate, it is typically selected from monosaccharides, oligosaccharides, polysaccharides, and combinations thereof. Typically, the encapsulating material is a starch. Suitable starches are described in EP 0 922 499; US 4,977,252; US 5,354,559, and US 5,935,826. In some embodiments, the encapsulating material is a microsphere made from plastic such as thermoplastics, acrylonitrile, methacrylonitrile, polyacrylonitrile, polymethacrylonitrile and mixtures thereof; commercially available microspheres that find use include those supplied by EXPANCEL® (Stockviksverken, Sweden), and PM 6545, PM 6550, PM 7220, PM

7228, EXTENDOSPHERES®, LUXSIL®, Q-CEL®, and SPHERICEL® (PQ Corp., Valley Forge, PA)>

As described herein, the variant proteases of the present invention find particular use in laundry detergents. These applications place enzymes under various environmental stresses. The variant proteases of the present invention provide advantages over many currently used enzymes, due to their stability under various conditions.

Indeed, there are a variety of wash conditions including varying detergent formulations, wash water volumes, wash water temperatures, and lengths of wash time, to which proteases involved in washing are exposed. In addition, detergent formulations used in different geographical areas have different concentrations of their relevant components present in the wash water. For example, a European detergent typically has about 4500- 5000 ppm of detergent components in the wash water, while a Japanese detergent typically has approximately 667 ppm of detergent components in the wash water. In North America, particularly the United States, detergents typically have about 975 ppm of detergent components present in the wash water.

A low detergent concentration system includes detergents where less than about 800 ppm of detergent components are present in the wash water. Japanese detergents are typically considered low detergent concentration system as they have approximately 667 ppm of detergent components present in the wash water.

A medium detergent concentration includes detergents where between about 800 ppm and about 2000ppm of detergent components are present in the wash water. North American detergents are generally considered to be medium detergent concentration systems as they have approximately 975 ppm of detergent components present in the wash water. Brazil typically has approximately 1500 ppm of detergent components present in the wash water.

A high detergent concentration system includes detergents where greater than about 2000 ppm of detergent components are present in the wash water. European detergents are generally considered to be high detergent concentration systems as they have approximately 4500-5000 ppm of detergent components in the wash water.

Latin American detergents are generally high suds phosphate builder detergents and the range of detergents used in Latin America can fall in both the medium and high detergent concentrations as they range from 1500 ppm to 6000 ppm of detergent components in the wash water. As mentioned above, Brazil typically has approximately 1500 ppm of detergent components present in the wash water. However, other high suds phosphate builder detergent geographies, not limited to other Latin American countries, may have high detergent concentration systems up to about 6000 ppm of detergent components present in the wash water.

In light of the foregoing, it is evident that concentrations of detergent compositions in typical wash solutions throughout the world varies from less than about 800 ppm of detergent composition ("low detergent concentration geographies"), for example about 667 ppm in Japan, to between about 800 ppm to about 2000 ppm ("medium detergent concentration geographies"), for example about 975 ppm in U.S. and about 1500 ppm in Brazil, to greater than about 2000 ppm ("high detergent concentration geographies"), for example about 4500 ppm to about 5000 ppm in Europe and about 6000 ppm in high suds phosphate builder geographies.

The concentrations of the typical wash solutions are determined empirically. For example, in the U.S., a typical washing machine holds a volume of about 64.4 L of wash solution. Accordingly, in order to obtain a concentration of about 975 ppm of detergent within the wash solution about 62.79 g of detergent composition must be added to the 64.4 L of wash solution. This amount is the typical amount measured into the wash water by the consumer using the measuring cup provided with the detergent.

As a further example, different geographies use different wash temperatures. The temperature of the wash water in Japan is typically less than that used in Europe. For example, the temperature of the wash water in North America and Japan is typically between 10 and 30°C (*e.g.*, about 20°C), whereas the temperature of wash water in Europe is typically between 30 and 60°C (*e.g.*, about 40°C).

As a further example, different geographies typically have different water hardness. Water hardness is usually described in terms of the grains per gallon mixed $Ca^{2+}/Mg^{2+}$. Hardness is a measure of the amount of calcium ($Ca^{2+}$) and magnesium ($Mg^{2+}$) in the water. Most water in the United States is hard, but the degree of hardness varies. Moderately hard (60-120 ppm) to hard (121-181 ppm) water has 60 to 181 parts per million (parts per million converted to grains per U.S. gallon is ppm # divided by 17.1 equals grains per gallon) of hardness minerals.

| Water | Grains per gallon | Parts per million |
|---|---|---|
| Soft | less than 1.0 | less than 17 |
| Slightly hard | 1.0 to 3.5 | 17 to 60 |
| Moderately hard | 3.5 to 7.0 | 60 to 120 |
| Hard | 7.0 to 10.5 | 120 to 180 |
| Very hard | Greater than 10.5 | greater than 180 |

European water hardness is typically greater than 10.5 (for example 10.5- 20.0) grains per gallon mixed $Ca^{2+}/Mg^{2+}$ (*e.g.*, about 15 grains per gallon mixed $Ca^{2+}/Mg^{2+}$) . North American water hardness is typically greater than Japanese water hardness, but less than European water hardness. For example, North American water hardness can be between 3 to 10 grains, 3- 8 grains or about 6 grains. Japanese water hardness is typically lower than North American water hardness, usually less than 4, for example 3 grains per gallon mixed $Ca^{2+}/Mg^{2+}$.

Accordingly, in some embodiments, the present invention provides variant proteases that show surprising wash performance in at least one set of wash conditions (*e.g.*, water temperature, water hardness, and/or detergent concentration) . In some embodiments, the variant proteases of the present invention are comparable in wash performance to other subtilisin proteases. In some embodiments, the variant proteases of the present invention exhibit enhanced wash performance as compared to subtilisin proteases currently commercially available. Thus, in some preferred embodiments of the present invention, the variant proteases provided herein exhibit enhanced oxidative stability, enhanced thermal stability, and/or enhanced chelator stability. In addition, the variant proteases of the present invention find use in cleaning compositions that do not include detergents, again either alone or in combination with builders and stabilizers.

In some embodiments of the present invention, the cleaning compositions comprise at least one variant protease of the present invention at a level from about 0.00001 % to about 10% by weight of the composition and the balance (*e.g.*, about 99.999% to about 90.0%) comprising cleaning adjunct materials by weight of composition. In other aspects of the present invention, the cleaning compositions of the present invention comprises at least one variant protease at a level of about 0.0001 % to about 10%, about 0.001% to about 5%, about 0.001% to about 2%, about 0.005% to about 0.5% by weight of the composition and the balance of the cleaning composition (*e.g.*. about 99.9999% to about 90.0%, about 99.999 % to about 98%, about 99.995% to about 99.5% by weight) comprising cleaning adjunct materials.

In some embodiments, preferred cleaning compositions comprise one or more additional enzymes or enzyme derivatives which provide cleaning performance and/or fabric care benefits, in addition to one or more of the variant proteases provided herein. Such enzymes include, but are not limited to other proteases, lipases, cutinases, amylases, cellulases, peroxidases, oxidases (*e.g.*, laccases), and/or mannanases.

Any other suitable protease finds use in the compositions of the present invention. Suitable proteases include those of animal, vegetable or microbial origin. In some particularly preferred embodiments, microbial proteases are used. In some embodiments, chemically or genetically modified mutants are included. In some embodiments, the protease is a serine protease, preferably an alkaline microbial protease or a trypsin-like protease. Examples of alkaline proteases include subtilisins, especially those derived from *Bacillus* (*e.g.,* subtilisin, *lentus, amyloliquefaciens,* subtilisin Carlsberg, subtilisin 309, subtilisin 147 and subtilisin 168). Additional examples include those mutant proteases described in U.S. Pat. Nos. RE 34,606, 5,955,340, 5,700,676, 6,312,936, and 6,482,628, all of which are incorporated herein by reference. Additional protease examples include, but are not limited to trypsin (*e.g.*, of porcine or bovine origin), and the *Fusarium* protease described in WO 89/06270. Preferred commercially available protease enzymes include MAXATASE®, MAXACAL™, MAXAPEM™, OPTICLEAN®, OPTIMASE®, PROPERASE®, PURAFECT® and PURAFECT® OXP (Genencor); ALCALASE®, SAVINASE®, PRIMASE®, DURAZYM™, RELASE® and ESPERASE® (Novozymes); and BLAP™ (Henkel Kommanditgesellschaft auf Aktien, Duesseldorf, Germany. Various proteases are described in WO95/23221, WO 92/21760, and U.S. Pat. Nos. 5,801,039, 5,340,735, 5,500,364, 5,855,625, US RE 34,606, 5,955,340, 5,700,676, 6,312,936, and 6,482,628, and various other patents.

In addition, any suitable lipase finds use in the present invention. Suitable lipases include, but arc not limited to those of bacterial or fungal origin. Chemically or genetically modified mutants are encompassed by the present invention. Examples of useful lipases include *Humicola lanuginosa* lipase (*See e.g.,* EP 258 068, and EP 305 216), *Rhizomucor miehei* lipase *(See e.g.,* EP 238 023), *Candida* lipase, such as *C. antarctica* lipase (*e.g.,* the *C. antarctica* lipase A or B; *See e.g.,* EP 214 761), a *Pseudomonas* lipase such as *P. alcaligenes* and *P. pseudoalcaligenes,* lipase *(See e.g.,* EP 218 272), *P. cepacia* lipase *(See e.g.,* EP 331 376), *P. stutzeri* lipase *(See e.g.,* GB 1, 372, 034), *P. fluorescens* lipase, *Bacillus* lipase (*e.g., B. subtilis* lipase [Dartois et al., Biochem. Biophys. Acta 1131: 253- 260 [1993] ) ; *B. stearothermophilus* lipase [*See e.g.,* JP 64/744992] ; and B. *pumilus* lipase [*See e.g.,* WO 91/16422] ) .

Furthermore, a number or cloned lipases find use in some embodiments of the present invention, including but not limited to *Penicillium camembertii* lipase (*See*, Yamaguchi et al., Gene 103: 61- 67 [1991] ), *Geotricum candidum* lipase *(See,* Schimada et al., J. Biochem., 106: 383- 388 [1989] ), and various *Rhizopus* lipases such as *R. delemar* lipase *(See,* Hass et al., Gene 109: 117- 113 [1991] ), a *R. niveus* lipase (Kugimiya et al., Biosci. Biotech. Biochem. 56: 716- 719 [1992] ) and *R. oryzae* lipase.

Other types of lipolytic enzymes such as cutinases also find use in some embodiments of the present invention, including but not limited to the cutinase derived from *Pseudomonas mendocina* (*See,* WO 88/09367), and the cutinase derived from *Fusarium solani pisi* (*See,* WO 90/09446).

Additional suitable lipases include commercially available lipases such as M1 LIPASE™, LUMA FAST™, and LIPOMAX™ (Genencor); LIPOLASE® and LIPOLASE® ULTRA (Novozymes); and LIPASEP™ "Amano" (Amano Pharmaceutical Co. Ltd., Japan).

In some embodiments of the present invention, the cleaning compositions of the present invention further

comprise lipases at a level from about 0.00001 % to about 10% of additional lipase by weight of the composition and the balance of cleaning adjunct materials by weight of composition. In other aspects of the present invention, the cleaning compositions of the present invention also comprise, lipases at a level of about 0.0001 % to about 10%, about 0-001% to about 5%, about 0.001% to about 2%, about 0.005% to about 0.5% lipase by weight of the composition.

Any amylase (alpha and/or beta) suitable for use in alkaline solutions also find use in some embodiments of the present invention. Suitable amylases include, but are not limited to those of bacterial or fungal origin. Chemically or genetically modified mutants are included in some embodiments. Amylases that find use in the present invention, include, but are not limited to α- amylases obtained from *B. licheniformis (See e.g.,* GB 1, 296, 839) . Commercially available amylases that find use in the present invention include, but arc not limited to DURAMYL®, TERMAMYL®, FUNGAMYL® and BAN™ (Novozymes) and RAPIDASE® and MAXAMYL® P (Genencor) .

In some embodiments of the present invention, the cleaning compositions of the present invention further comprise amylases at a level from about 0.00001 % to about 10% of additional amylase by weight of the composition and the balance of cleaning adjunct materials by weight of composition, In other aspects of the present invention, the cleaning compositions of the present invention also comprise, amylase at a level of about 0.0001 % to about 10%, about 0.001 % to about 5%, about 0.001% to about 2%, about 0-005% to about 0.5% amylase by weight of the composition.

In some further embodiments, any suitable cellulase finds used in the cleaning compositions of the present invention. Suitable cellulases include, but are not limited to those of bacterial or fungal origin. Chemically or genetically modified mutants are included in some embodiments. Suitable cellulases include, but are not limited to *Humicola insolens* cellulases (*See e.g.,* U.S. Pat. No. 4, 435, 307) . Especially suitable cellulases are the cellulases having color care benefits (*See e.g.,* EP 0 495 257) . Commercially available cellulases that find use in the present include, but are not limited to CELLUZYME® (Novozymes), and KAC- 500 (B) ™ (Kao Corporation) . In some embodiments, cellulases are incorporated as portions or fragments of mature wild- type or variant cellulases, wherein a portion of the N- terminus is deleted (*See e.g.,* U.S. Pat. No. 5, 874, 276) . In some embodiments, the cleaning compositions of the present invention further comprise cellulases at a level from about 0.00001 % to about 10% of additional cellulase by weight of the composition and the balance of cleaning adjunct materials by weight of composition. In other aspects of the present invention, the cleaning compositions of the present invention also comprise cellulases at a level of about 0.0001% to about 10%, about 0.001% to about 5%, about 0.001% to about 2%, about 0.005% to about 0.5% cellulase by weight of the composition.

Any mannanase suitable for use in detergent compositions also finds use in the present invention. Suitable mannanases include, but are not limited to those of bacterial or fungal origin. Chemically or genetically modified mutants are included in some embodiments. Various mannanases are known which find use in the present invention (*See e.g.,* U.S. Pat. No. 6, 566, 114, U.S. Pat. No. 6, 602, 842, and US Patent No. 6, 440, 991, all of which are incorporated herein by reference), In some embodiments, the cleaning compositions of the present invention further comprise mannanases at a level from about 0.00001 % to about 10% of additional mannanase by weight of the composition and the balance of cleaning adjunct materials by weight of composition. In other aspects of the present invention, the cleaning compositions of the present invention also comprise, mannanases at a level of about 0.0001 % to about 10%, about 0.001% to about 5%, about 0.001% to about 2%, about 0.005% to about 0.5% mannanase by weight of the composition.

In some embodiments, peroxidases are used in combination with hydrogen peroxide or a source thereof (*e.g.,* a percarbonate, perborate or persulfate) in the compositions of the present invention. In some alternative embodiments, oxidases are used in combination with oxygen. Both types of enzymes arc used for "solution bleaching" (*i.e.*, to prevent transfer of a textile dye from a dyed fabric to another fabric when the fabrics arc washed together in a wash liquor), preferably together with an enhancing agent (*See e.g.,* WO 94/12621 and WO 95/01426). Suitable peroxidases/oxidases include, but are not limited to those of plant, bacterial or fungal origin. Chemically or genetically modified mutants are included in some embodiments. In some embodiments, the cleaning compositions of the present invention further comprise peroxidase and/or oxidase enzymes at a level from about 0.00001% to about 10% of additional peroxidase and/or oxidase by weight of the composition and the balance of cleaning adjunct materials by weight of composition. In other aspects of the present invention, the cleaning compositions of the present invention also comprise peroxidase and/or oxidase enzymes at a level of about 0.0001 % to about 10%, about 0.001 % to about 5%, about 0.001% to about 2%, about 0.005% to about 0.5% peroxidase and/or oxidase enzymes by weight of the composition.

In some embodiments, additional enzymes find use, including but not limited to perhydrolases (*See e.g.,* WO 05/056782). In addition, in some particularly preferred embodiments, mixtures of the above mentioned enzymes are encompassed herein, in particular one or more additional protease, amylase, lipase, mannanase, and/or at least one cellulase. Indeed, it is contemplated that various mixtures of these enzymes will find use in the present invention. It is also contemplated that the varying levels of the variant protease(s) and one or more additional enzymes may both independently range to about 10%, the balance of the cleaning composition being cleaning adjunct materials. The specific selection of cleaning adjunct materials arc readily made by considering the surface, item, or fabric to be cleaned, and the desired form of the composition for the cleaning conditions during use (*e.g.*, through the wash detergent use).

Examples of suitable cleaning adjunct materials include, but are not limited to, surfactants, builders, bleaches,

bleach activators, bleach catalysts, other enzymes, enzyme stabilising systems, chelants, optical brighteners, soil release polymers, dye transfer agents, dispersants, suds suppressors, dyes, perfumes, colorants, filler salts, hydrotropes, photoactivators, fluorescers, fabric conditioners, hydrolyzable surfactants, preservatives, anti- oxidants, anti- shrinkage agents, anti- wrinkle agents, germicides, fungicides, color speckles, silvercare, anti- tarnish and/or anti- corrosion agents, alkalinity sources, solubilizing agents, carriers, processing aids, pigments, and pH control agents (*See e.g.,* U.S. Pat. Nos. 6, 610, 642, 6, 605, 458, 5, 705, 464, 5, 710, 115, 5, 698, 504, 5, 695, 679, 5, 686, 014 and 5, 646, 101, all of which are incorporated herein by reference) . Embodiments of specific cleaning composition materials are exemplified in detail below. In embodiments in which the cleaning adjunct materials are not compatible with the variant proteases of the present invention in the cleaning compositions, then suitable methods of keeping the cleaning adjunct materials and the protease (s) separated (*i.e.,* not in contact with each other) until combination of the two components is appropriate are used. Such separation methods include any suitable method known in the art (*e.g.,* gelcaps, encapulation, tablets, physical separation, etc.) .

By way of example, several cleaning compositions wherein the variant proteases of the present invention find use are described in greater detail below. In embodiments in which the cleaning compositions of the present invention are formulated as compositions suitable for use in laundry machine washing method(s), the compositions of the present invention preferably contain at least one surfactant and at least one builder compound, as well as one or more cleaning adjunct materials preferably selected from organic polymeric compounds, bleaching agents, additional enzymes, suds suppressors, dispersants, lime-soap dispersants, soil suspension and anti-redeposition agents and corrosion inhibitors. In some embodiments, laundry compositions also contain softening agents (*i.e.*, as additional cleaning adjunct materials). The compositions of the present invention also find use detergent additive products in solid or liquid form. Such additive products arc intended to supplement and/or boost the performance of conventional detergent compositions and can be added at any stage of the cleaning process. In some embodiments, the density of the laundry detergent compositions herein ranges from about 400 to about 1200 g/liter, while in other embodiments, it ranges from about 500 to about 950 g/liter of composition measured at 20°C.

In some embodiments, various cleaning compositions such as those provided in U.S, Pat. No. 6, 605, 458 find use with the variant proteases of the present invention. Thus, in some embodiments, the compositions comprising at least one variant protease of the present invention is a compact granular fabric cleaning composition, while in other embodiments, the composition is a granular fabric cleaning composition useful in the laundering of colored fabrics, in further embodiments, the composition is a granular fabric cleaning composition which provides softening through the wash capacity, in additional embodiments, the composition is a heavy duty liquid fabric cleaning composition. In some embodiments, the compositions comprising at least one variant protease of the present invention are fabric cleaning compositions such as those described in U.S. Pat. Nos. 6, 610, 642 and 6, 376, 450. In addition, the variant proteases of the present invention find use in granular laundry detergent compositions of particular utility under European or Japanese washing conditions (*See e.g.,* U.S. Pat. No. 6, 610, 642) .

The cleaning compositions of the present invention are formulated into any suitable form and prepared by any process chosen by the formulator, non-limiting examples of which are described in U.S. Pat. Nos. 5,879,584, 5,691,297, 5,574,005, 5,569,645, 5,565,422, 5,516,448, 5,489,392, and 5,486,303, all of which are incorporated herein by reference. When a low pH cleaning composition is desired, the pH of such composition is adjusted via the addition of a material such as monoethanolamine or an acidic material such as HCl.

While not essential for the purposes of the present invention, the non-limiting list of adjuncts illustrated hereinafter are suitable for use in the instant cleaning compositions. In some embodiments, these adjuncts are incorporated for example, to assist or enhance cleaning performance, for treatment of the substrate to be cleaned, or to modify the aesthetics of the cleaning composition as is the case with perfumes, colorants, dyes or the like. It is understood that such adjuncts are in addition to the variant proteases of the present invention. The precise nature of these additional components, and levels of incorporation thereof, will depend on the physical form of the composition and the nature of the cleaning operation for which it is to be used. Suitable adjunct materials include, but are not limited to, surfactants, builders, chelating agents, dye transfer inhibiting agents, deposition aids, dispersants, additional enzymes, and enzyme stabilizers, catalytic materials, bleach activators, bleach boosters, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, processing aids and/or pigments. In addition to the disclosure below, suitable examples of such other adjuncts and levels of use are found in U.S. Patent Nos. 5,576,282, 6,306,812, and 6,326,348, that are incorporated by reference. The aforementioned adjunct ingredients may constitute the balance of the cleaning compositions of the present invention.

In some embodiments, the cleaning compositions according to the present invention comprise a surfactant or surfactant system wherein the surfactant is selected from nonionic surfactants, anionic surfactants, cationic surfactants, ampholytic surfactants, zwitterionic surfactants, semi-polar nonionic surfactants and mixtures thereof.

In some additional embodiments, the cleaning compositions of the present invention comprise one or more detergent builders or builder systems. Builders include, but are not limited to, the alkali metal, ammonium and alkanolammonium salts of polyphosphates, alkali metal silicates, alkaline earth and alkali metal carbonates, aluminosilicate builders polycarboxylate compounds, ether hydroxypolycarboxylates, copolymers of maleic anhydride with ethylene or vinyl methyl ether, 1, 3, 5- trihydroxy benzene- 2, 4, 6- trisulphonic acid, and carboxymethyloxysuccinic acid, the various alkali metal, ammonium and substituted ammonium salts of polyacetic acids such as ethylenediamine tetraacetic acid and nitrilotriacetic acid, as well as polycarboxylates such as mellitic acid, succinic acid, citric acid, oxydisuccinic acid, polymaleic acid, benzene 1, 3, 5- tricarboxylic acid, carboxymethyloxysuccinic acid, and soluble salts thereof.

In some further embodiments, the cleaning compositions herein contain a chelating agent. Suitable chelating agents include copper, iron and/or manganese chelating agents and mixtures thereof.

In some still further embodiments, the cleaning compositions provided herein contain a deposition aid. Suitable deposition aids include, polyethylene glycol, polypropylene glycol, polycarboxylate, soil release polymers such as polytelephthatic acid, clays such as Kaolinite, montmorillonite, atapulgite, illite, bentonite, halloysite, and mixtures thereof.

In some additional embodiments, the cleaning compositions of the present invention also include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof.

In some still additional embodiments, the cleaning compositions of the present invention also contain dispersants. Suitable water-soluble organic materials include the homo- or co-polymeric acids or their salts, in which the polycarboxylic acid comprises at least two carboxyl radicals separated from each other by not more than two carbon atoms.

In some particularly preferred embodiments, the cleaning compositions comprise one or more detergent enzymes which provide cleaning performance and/or fabric care benefits. Examples of suitable enzymes include, but are not limited to, hemicellulases, peroxidases, proteases, cellulases, xylanases, lipases, phospholipases, esterases, cutinases, pectinases, keratinases, reductases, oxidases, phenol oxidases, lipoxygenases, ligninases, pullulanases, tannases, pentosanases, malanases, ß-glucanases, arabinosidases, hyaluronidase, chondroitinase, laccase, and amylases, or mixtures thereof. A typical combination is cocktail of conventional applicable enzymes including at least one protease, at least one lipase, at least one cutinase, and/or at least one cellulase in conjunction with at least one amylase.

In some further embodiments, the enzymes used in the cleaning compositions are stabilized any suitable technique. In some embodiments, the enzymes employed herein are stabilized by the presence of water-soluble sources of calcium and/or magnesium ions in the finished compositions that provide such ions to the enzymes.

In some still further embodiments, the cleaning compositions of the present invention include catalytic metal complexes. One type of metal-containing bleach catalyst is a catalyst system comprising a transition metal cation of defined bleach catalytic activity, such as copper, iron, titanium, ruthenium, tungsten, molybdenum, or manganese cations, an auxiliary metal cation having little or no bleach catalytic activity, such as zinc or aluminum cations, and a sequestrate having defined stability constants for the catalytic and auxiliary metal cations, particularly ethylenediaminetetraacetic acid, ethylenediaminetetra (methylenephosphonic acid) and water-soluble salts thereof. Such catalysts are disclosed in U.S. Pat. No. 4,430,243.

In some embodiments, the compositions herein arc catalyzed by means of a manganese compound. Such compounds and levels of use are well known in the art and include, for example, the manganese- based catalysts disclosed in U.S. Pat. No. 5, 576, 282. In addition, cobalt bleach catalysts useful herein are known, and are described, for example, in U.s. Pat. Nos. 5, 597, 936, and 5, 595, 967. Such cobalt catalysts are readily prepared by known procedures, such as taught for example in U.S. Pat. Nos. 5, 597, 936, and 5, 595, 967. In some embodiments, the compositions provided herein also suitably include a transition metal complex of a macropolycyclic rigid ligand (*i.e.*, "MRL") . As a practical malter, and not by way of limitation, the compositions and cleaning processes herein are adjustable, to provide on the order of at least one part per hundred million of the active MRL species in the aqueous washing medium, and will preferably provide from about 0.005 ppm to about 25 ppm, more preferably from about 0.05 ppm to about 10 ppm, and most preferably from about 0.1 ppm to about 5 ppm, of the MRL in the wash liquor. Preferred transition- metals in the instant transition- metal bleach catalyst include manganese, iron and chromium. Preferred MRLs herein are a special type of ultra- rigid ligand that is cross- bridged such as 5, 12- diethyl- 1, 5, 8, 12- tetraazabicyclo [6.6.2] hexadecane. Suitable transition metal MRLs are readily prepared by known procedures, such as taught for example in WO 00/332601, and U.S. Pat. No. 6, 225, 464.

As indicated above, the cleaning compositions of the present invention are formulated into any suitable form and prepared by any process chosen by the formulator, non-limiting examples of which are described in U.S. Pat. Nos. 5,879,584, 5,691,297, 5,574,005, 5,569,645, 5,516,448, 5,489,392, and 5,486,303, all of which arc incorporated herein by reference.

The cleaning compositions disclosed herein of find use in cleaning a situs (*e.g.,* a surface, dishware, or fabric). Typically, at least a portion of the situs is contacted with an embodiment of the present cleaning composition, in neat form or diluted in a wash liquor, and then the situs is optionally washed and/or rinsed. For purposes of the present invention, "washing" includes but is not limited to, scrubbing, and mechanical agitation. In some embodiments, the cleaning compositions are typically employed at concentrations of from about 500 ppm to about 15,000 ppm in solution.

When the wash solvent is water, the water temperature typically ranges from about 5°C to about 90°C and, when the situs comprises a fabric, the water to fabric mass ratio is typically from about 1:1 to about 30:1.

**Definitions**

Unless otherwise indicated, the practice of the present invention involves conventional techniques commonly used in molecular biology, protein engineering, microbiology, and recombinant DNA, which are within the skill of the art. Such techniques are known to those of skill in the art and arc described in numerous texts and reference works (*See e.g.,* Sambrook et al., "Molecular Cloning: A Laboratory Manual", Second Edition (Cold Spring Harbor), [1989]); and Ausubel et al., "Current Protocols in Molecular Biology" [1987]). All patents, patent applications, articles and publications mentioned herein, both *supra* and *infra,* are hereby expressly incorporated herein by reference.

Unless defined otherwise herein, all technical and scientific terms used herein have the same meaning as commonly understood by one of ordinary skill in the art to which this invention pertains. There are various dictionaries available and known to those in the art that provide definitions of these terms. Although any methods and materials similar or equivalent to those described herein find use in the practice of the present invention, the preferred methods and materials are described herein. Accordingly, the terms defined immediately below arc more fully described by reference to the Specification as a whole. Also, as used herein, the singular "a", "an" and "the" includes the plural reference unless the context clearly indicates otherwise. Numeric ranges are inclusive of the numbers defining the range. Unless otherwise indicated, nucleic acids are written left to right in 5' to 3' orientation; amino acid sequences are written left to right in amino to carboxy orientation, respectively. It is to be understood that this invention is not limited to the particular methodology, protocols, and reagents described, as these may vary, depending upon the context they are used by those of skill in the art.

The practice of the present invention employs, unless otherwise indicated, conventional techniques of protein purification, molecular biology, microbiology, recombinant DNA techniques and protein sequencing, all of which are within the skill of those in the art.

Furthermore, the headings provided herein are not limitations of the various aspects or embodiments of the invention which can be had by reference to the specification as a whole. Accordingly, the terms defined immediately below are more fully defined by reference to the specification as a whole. Nonetheless, in order to facilitate understanding of the invention, a number of terms are defined below.

As used herein, the terms "protease, " and "proteolytic activity" refer to a protein or peptide exhibiting the ability to hydrolyze peptides or substrates having peptide linkages. Many well known procedures exist for measuring proteolytic activity (Kalisz, "microbial Proteinases, " In: Fiechter (ed.), Advances in Biochemical Engincering/ Biotechnology, [1988] ) . For example, proteolytic activity may be ascertained by comparative assays which analyze the respective protease's ability to hydrolyze a commercial substrate. Exemplary substrates useful in the analysis of protease or proteolytic activity, include, but are not limited to di- methyl casein (Sigma C- 9801), bovine collagen (Sigma C- 9879), bovine elastin (Sigma E- 1625), and bovine keratin (ICN Biomedical 902111) . Colorimetric assays utilizing these substrates are well known in the art *(See e.g.,* WO 99/34011; and U.S. Pat. No. 6, 376, 450, both of which are incorporated herein by reference) . The pNA assay (*See e.g.,* Del Mar et al., Anal. Biochem., 99: 316- 320 [1979] ) also finds use in determining the active enzyme concentration for fractions collected during gradient elution. This assay measures the rate at which *p*- nitroaniline is released as the enzyme hydrolyzes the soluble synthetic substrate, succinyl- alanine- alanine- proline- phenylalanine- *p*- nitroanilide (sAAPF- *p*NA) . The rate of production of yellow color from the hydrolysis reaction is measured at 410 nm on a spectrophotometer and is proportional to the active enzyme concentration. In addition, absorbance measurements at 280 nm can be used to determine the total protein concentration. The active enzyme/ total- protein ratio gives the enzyme purity.

As used herein, "the genus *Bacillus"* includes all species within the genus *"Bacillus,"* as known to those of skill in the art, including but not limited to *B. subtilis, B. licheniformis. B. lentus. B. brevis, B. stearothermophilus. B. alkalophilus, B. amyloliquefaciens, B. clausii, B. halodurans, B. megaterium B. coagulans, B. circulans, B. lautus,* and *B. thuringiensis.* It is recognized that the genus *Bacillus* continues to undergo taxonomical reorganization. Thus, it is intended that the genus include species that have been reclassified, including but not limited to such organisms as *B. stearothermophilus,* which is now named *"Geobacillus stearothermophilus."* The production of resistant endospores in the presence of oxygen is considered the defining feature of the genus *Bacillus,* although this characteristic also applies to the recently named *Alicyclobacillus, Amphibacillus, Aneurinibacillus, Anoxybacillus, Brevibacillus, Filobacillus, Gracilibacillus, Halobacillus, Paenibacillus, Salibacillus, Thermobacillus, Ureibacillus,* and *Virgibacillus.*

The terms "polynucleotide" and "nucleic acid", used interchangeably herein, refer to a polymeric form of nucleotides of any length, either ribonucleotides or deoxyribonucleotides. These terms include, but are not limited to, a single-, double- or triple-stranded DNA, genomic DNA, cDNA, RNA, DNA-RNA hybrid, or a polymer comprising purine and pyrimidine bases, or other natural, chemically, biochemically modified, non-natural or derivatized nucleotide bases. The following are non-limiting examples of polynucleotides: genes, gene fragments, chromosomal fragments, ESTs, exons,

introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes, and primers. In some embodiments, polynucleotide comprise modified nucleotides, such as methylated nucleotides and nucleotide analogs, uracyl, other sugars and linking groups such as fluororibose and thioate, and nucleotide branches. In alternative embodiments, the sequence of nucleotides is interrupted by non-nucleotide components.

As used herein, the terms "DNA construct" and "transforming DNA" are used interchangeably to refer to DNA used to introduce sequences into a host cell or organism. The DNA may be generated *in vitro* by PCR or any other suitable technique(s) known to those in the art. In particularly preferred embodiments, the DNA construct comprises a sequence of interest (*e.g.*, as an incoming sequence). In some embodiments, the sequence is operably linked to additional elements such as control elements (*e.g.*, promoters, etc.). The DNA construct may further comprise a selectable marker. It may further comprise an incoming sequence flanked by homology boxes. In a further embodiment, the transforming DNA comprises other non-homologous sequences, added to the ends (*e.g.*, stuffer sequences or flanks). In some embodiments, the ends of the incoming sequence are closed such that the transforming DNA forms a closed circle. The transforming sequences may be wild-type, mutant or modified. In some embodiments, the DNA construct comprises sequences homologous to the host cell chromosome. In other embodiments, the DNA construct comprises non-homologous sequences. Once the DNA construct is assembled *in vitro* it may be used to: 1) insert heterologous sequences into a desired target sequence of a host cell, and/or 2) mutagenize a region of the host cell chromosome (*i.e.*, replace an endogenous sequence with a heterologous sequence), 3) delete target genes, and/or 4) introduce a replicating plasmid into the host.

As used herein, the terms "expression cassette" and "expression vector" refer to nucleic acid constructs generated recombinantly or synthetically, with a series of specified nucleic acid elements that permit transcription of a particular nucleic acid in a target cell. The recombinant expression cassette can be incorporated into a plasmid, chromosome, mitochondrial DNA, plastid DNA, virus, or nucleic acid fragment. Typically, the recombinant expression cassette portion of an expression vector includes, among other sequences, a nucleic acid sequence to be transcribed and a promoter. In preferred embodiments, expression vectors have the ability to incorporate and express heterologous DNA fragments in a host cell. Many prokaryotic and eukaryotic expression vectors are commercially available. Selection of appropriate expression vectors is within the knowledge of those of skill in the art. The term "expression cassette" is used interchangeably herein with "DNA construct," and their grammatical equivalents. Selection of appropriate expression vectors is within the knowledge of those of skill in the art.

As used herein, the term "vector" refers to a polynucleotide construct designed to introduce nucleic acids into one or more cell types. Vectors include cloning vectors, expression vectors, shuttle vectors, plasmids, cassettes and the like. In some embodiments, the polynucleotide construct comprises a DNA sequence encoding the protease (*e.g.*, precursor or mature protease) that is operably linked to a suitable prosequence (*e.g.*, secretory, etc.) capable of effecting the expression of the DNA in a suitable host.

As used herein, the term "plasmid" refers to a circular double-stranded (ds) DNA construct used as a cloning vector, and which forms an extrachromosomal self-replicating genetic element in some eukaryotes or prokaryotes, or integrates into the host chromosome.

As used herein in the context of introducing a nucleic acid sequence into a cell, the term "introduced" refers to any method suitable for transferring the nucleic acid sequence into the cell. Such methods for introduction include but are not limited to protoplast fusion, transfection, transformation, conjugation, and transduction (*See e.g.,* Ferrari et al., "Genetics, " in Hardwood et al, (eds.), Bacillus, Plenum Publishing Corp., pages 57-72, [1989]).

As used herein, the terms "transformed" and "stably transformed" refers to a cell that has a non-native (heterologous) polynucleotide sequence integrated into its genome or as an episomal plasmid that is maintained for at least two generations.

A nucleic acid is "operably linked" when it is placed into a functional relationship with another nucleic acid sequence. For example, DNA encoding a secretory leader *(i.e.,* a signal peptide), is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

As used herein the term "gene" refers to a polynucleotide (*e.g.*, a DNA segment), that encodes a polypeptide and includes regions preceding and following the coding regions as well as intervening sequences (introns) between individual coding segments (exons).

As used herein, "homologous genes" refers to a pair of genes from different, but usually rotated species, which correspond to each other and which are identical or very similar to each other. The term encompasses genes that are

separated by speciation (*i.e.,* the development of new species) (*e.g.*, orthologous genes), as well as genes that have been separated by genetic duplication (*e.g.*, paralogous genes).

As used herein, "ortholog" and "orthologous genes" refer to genes in different species that have evolved from a common ancestral gene (*i.e.,* a homologous gene) by speciation. Typically, orthologs retain the same function during the course of evolution. Identification of orthologs finds use in the reliable prediction of gene function in newly sequenced genomes.

As used herein, "paralog" and "paralogous genes" refer to genes that are related by duplication within a genome. While orthologs retain the same function through the course of evolution, paralogs evolve new functions, even though some functions are often related to the original one. Examples of paralogous genes include, but are not limited to genes encoding trypsin, chymotrypsin, elastase, and thrombin, which are all serine proteinases and occur together within the same species.

As used herein, proteins are defined as having a common "fold" if they have the same major secondary structures in the same arrangement and with the same topological connections. Different proteins with the same fold often have peripheral elements of secondary structure and turn regions that differ in size and conformation. In some cases, these differing peripheral regions may comprise half the structure. Proteins placed together in the same fold category do not necessarily have a common evolutionary origin (*e.g.*, structural similarities arising from the physics and chemistry of proteins favoring certain packing arrangements and chain topologies).

As used herein, "homology" refers to sequence similarity or identity, with identity being preferred. This homology is determined using standard techniques known in the art (*See e.g.,* Smith and Waterman, Adv. Appl. Math., 2:482 [1981]; Needleman and Wunsch, J. Mol. Biol., 48:443 [1970]; Pearson and Lipman, Proc. Natl. Acad. Sci. USA 85:2444 [1988]; programs such as GAP, BESTFIT, FASTA, and TFASTA in the Wisconsin Genetics Software Package (Genetics Computer Group, Madison, WI); and Devereux et al., Nucl. Acid Res., 12:387-395 [1984]).

As used herein, an "analogous sequence" is one wherein the function of the gene is essentially the same as the gene based on the wild-type subtilisin protease. Additionally, analogous genes include at least about 45%, about 50%, about 55%, about 60%, about 65%, about 70%, about 75%, about 80%, about 85%, about 90%, about 95%, about 97%, about 98%, about 99%, or about 100% sequence identity with the sequence of the wild-type subtilisin protease. Alternately, analogous sequences have an alignment of between about 70 to about 100% of the genes found in the *B. amyloliquefaciens* subtilisin protease region. In additional embodiments more than one of the above properties applies to the sequence. Analogous sequences are determined by known methods of sequence alignment. A commonly used alignment method is BLAST, although as indicated above and below, there are other methods that also find use in aligning sequences.

One example of a useful algorithm is PILEUP. PILEUP creates a multiple sequence alignment from a group of related sequences using progressive, pair-wise alignments. It can also plot a tree showing the clustering relationships used to create the alignment. PILEUP uses a simplification of the progressive alignment method of Feng and Doolittle (Feng and Doolittle, J. Mol. Evol., 35:351-360 [1987]). The method is similar to that described by Higgins and Sharp (Higgins and Sharp, CABIOS 5:151-153 [1989]). Useful PILEUP parameters including a default gap weight of 3.00, a default gap length weight of 0.10, and weighted end gaps.

Another example of a useful algorithm is the BLAST algorithm, described by Altschul *et al.*, (Altschul et al., J. Mol. Biol., 215:403-410, [1990]; and Karlin et al., Proc. Natl. Acad. Sci. USA 90:5873-5787 [1993]). A particularly useful BLAST program is the WU-BLAST-2 program (*See*, Altschul et al., Meth. Enzymol., 266:460-480 [1996]). WU-BLAST-2 uses several search parameters, most of which are set to the default values. The adjustable parameters are set with the following values: overlap span =1, overlap fraction = 0.125, word threshold (T) = 11. The 11SP S and 11SP S2 parameters are dynamic values and are established by the program itself depending upon the composition of the particular sequence and composition of the particular database against which the sequence of interest is being searched. However, the values may be adjusted to increase sensitivity. A % amino acid sequence identity value is determined by the number of matching identical residues divided by the total number of residues of the "longer" sequence in the aligned region. The "longer" sequence is the one having the most actual residues in the aligned region (gaps introduced by WU-Blast-2 to maximize the alignment score are ignored).

Thus, "percent (%) nucleic acid sequence identity" is defined as the percentage of nucleotide residues in a candidate sequence that are identical with the nucleotide residues of the starting sequence (*i.e.*, the sequence of interest) . A preferred method utilizes the BLASTN module of WU- BLAST- 2 set to the default parameters, with overlap span and overlap fraction set to I and 0.125, respectively.

As used herein, "recombinant" includes reference to a cell or vector, that has been modified by the introduction of a heterologous nucleic acid sequence or that the cell is derived from a cell so modified. Thus, for example, recombinant cells express genes that are not found in identical form within the native (non- recombinant) form of the cell or express native genes that are otherwise abnormally expressed, under expressed or not expressed at all as a result of deliberate human intervention. "Recombination, " "recombining, " and generating a "recombined" nucleic acid arc generally the assembly of two or more nucleic acid fragments wherein the assembly gives rise to a chimeric gene.

In some preferred embodiments, mutant DNA sequences are generated with site saturation mutagenesis in at least one codon. In another preferred embodiment, site saturation mutagenesis is performed for two or more codons. In a further embodiment, mutant DNA sequences have more than about 50%, more than about 55%, more than about 60%, more than about 65%, more than about 70%, more than about 75%, more than about 80%, more than about 85%, more than about 90%, more than about 95%, or more than about 98% homology with the wild-type sequence. In alternative embodiments, mutant DNA is generated *in vivo* using any known mutagenic procedure such as, for example, radiation, nitrosoguanidine and the like. The desired DNA sequence is then isolated and used in the methods provided herein.

As used herein, the terms "amplification" and "gene amplification" refer to a process by which specific DNA sequences are disproportionately replicated such that the amplified gene becomes present in a higher copy number than was initially present in the genome. In some embodiments, selection of cells by growth in the presence of a drug (*e.g.*, an inhibitor of an inhibitable enzyme) results in the amplification of either the endogenous gene encoding the gene product required for growth in the presence of the drug or by amplification of exogenous (*i.e.,* input) sequences encoding this gene product, or both.

"Amplification" is a special case of nucleic acid replication involving template specificity. It is to be contrasted with non-specific template replication (*i.e.*, replication that is template-dependent but not dependent on a specific template). Template specificity is here distinguished from fidelity of replication (*i.e.,* synthesis of the proper polynucleotide sequence) and nucleotide (ribo- or deoxyribo-) specificity. Template specificity is frequently described in terms of "target" specificity. Target sequences are "targets" in the sense that they are sought to be sorted out from other nucleic acid. Amplification techniques have been designed primarily for this sorting out.

As used herein, the term "primer" refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, (*i.e.,* in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer and die use of the method.

As used herein, the term "probe" refers to an oligonucleotide (*i.e.,* a sequence of nucleotides), whether occurring naturally as in a purified restriction digest or produced synthetically, recombinantly or by PCR amplification, which is capable of hybridizing to another oligonucleotide of interest. A probe may be single-stranded or double-stranded. Probes are useful in the detection, identification and isolation of particular gene sequences. It is contemplated that any probe used in the present invention will he labeled with any "reporter molecule," so that is detectable in any detection system, including, but not limited to enzyme (*e.g.*, ELISA, as well as enzyme-based histochemical assays), fluorescent, radioactive, and luminescent systems. It is not intended that the present invention be limited to any particular detection system or label.

As used herein, the term "target," when used in reference to the polymerase chain reaction, refers to the region of nucleic acid bounded by the primers used for polymerase chain reaction. Thus, the "target" is sought to be sorted out from other nucleic acid sequences. A "segment" is defined as a region of nucleic acid within the target sequence.

As used herein, the term "polymerase chain reaction" ("PCR") refers to the methods of U.S. Patent Nos. 4,683,195 4,683,202, and 4,965,188, hereby incorporated by reference, which include methods for increasing the concentration of a segment of a target sequence in a mixture of genomic DNA without cloning or purification. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing and polymerase extension can be repeated many times (*i.e.,* denaturation, annealing and extension constitute one "cycle"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified".

As used herein, the term "amplification reagents" refers to those reagents (deoxyribonucleotide triphosphates, buffer, etc.), needed for amplification except for primers, nucleic acid template and the amplification enzyme. Typically, amplification reagents along with other reaction components are placed and contained in a reaction vessel (test tube, microwell, etc.).

As used herein, the term "RT-PCR" refers to the replication and amplification of RNA sequences. In this method, reverse transcription is coupled to PCR, most often using a one enzyme procedure in which a thermostable polymerase is employed, as described in U.S. Patent No. 5,322,770, herein incorporated by reference. In RT-PCR, the RNA template is converted to cDNA due to the reverse transcriptase activity of the polymerase, and then amplified using the polymerizing activity of the polymerase (*i.e.,* as in other PCR methods).

As used herein, the terms "restriction endonucleases" and "restriction enzymes" refer to bacterial enzymes, each of which cut double-stranded DNA at or near a specific nucleotide sequence.

A "restriction site" refers to a nucleotide sequence recognized and cleaved by a given restriction endonuclease and is frequently the site for insertion of DNA fragments. In certain embodiments of the invention restriction sites are engineered into the selective marker and into 5' and 3' ends of the DNA construct.

"Homologous recombination" means the exchange of DNA fragments between two DNA molecules or paired chromosomes at the site of identical or nearly identical nucleotide sequences. In a preferred embodiment, chromosomal integration is homologous recombination.

As used herein "amino acid" refers to peptide or protein sequences or portions thereof. The terms "protein," "peptide," and "polypeptide" are used interchangeably.

As used herein, "protein of interest" and "polypeptide of interest" refer to a protein/polypeptide that is desired and/or being assessed. In some embodiments, the protein of interest is expressed intracellularly, while in other embodiments, it is a secreted polypeptide. In particularly preferred embodiments, these enzymes include the serine proteases of the present invention. In some embodiments, the protein of interest is a secreted polypeptide which is fused to a signal peptide (*i.e.*, an amino-terminal extension on a protein to be secreted). Nearly all secreted proteins use an amino-terminal protein extension which plays a crucial role in the targeting to and translocation of precursor proteins across the membrane. This extension is proteolytically removed by a signal peptidase during or immediately following membrane transfer.

A polynucleotide is said to "encode" an RNA or a polypeptide if, in its native state or when manipulated by methods known to those of skill in the art, it can be transcribed and/or translated to produce the RNA, the polypeptide or a fragment thereof. The anti-sense strand of such a nucleic acid is also said to encode the sequences. As is known in the art, a DNA can be transcribed by an RNA polymerase to produce RNA, but an RNA can be reverse transcribed by reverse transcriptase to produce a DNA. Thus a DNA can encode a RNA and vice versa.

"Host strain" or "host cell" refers to a suitable host for an expression vector comprising DNA according to the present invention.

An enzyme is "overexpressed" in a host cell if the enzyme is expressed in the cell at a higher level that the level at which it is expressed in a corresponding wild-type cell.

The terms "protein" and "polypeptide" are used interchangeability herein. The 3-letter code for amino acids as defined in conformity with the IUPAC-IUB Joint Commission on Biochemical Nomenclature (JCBN) is used through out this disclosure. It is also understood that a polypeptide may be coded for by more than one nucleotide sequence due to the degeneracy of the genetic code.

A "prosequence" is an amino acid sequence between the signal sequence and mature protease that is necessary for the secretion of the protease. Cleavage of the pro sequence results in a mature active protease.

The term "signal sequence" or "signal peptide" refers to any sequence of nucleotides and/or amino acids which may participate in the secretion of the mature or precursor forms of the protein. This definition of signal sequence is a functional one, meant to include all those amino acid sequences encoded by the N-terminal portion of the protein gene, which participate in the effectuation of the secretion of protein. They are often, but not universally, bound to the N-terminal portion of a protein or to the N-terminal portion of a precursor protein. The signal sequence may be endogenous or exogenous. The signal sequence may be that normally associated with the protein (*e.g.*, protease), or may be from a gene encoding another secreted protein. One exemplary exogenous signal sequence comprises the first seven amino acid residues of the signal sequence from *Bacillus subtilis* subtilisin fused to the remainder of the signal sequence of the subtilisin from *Bacillus lentus* (ATCC 21536).

The term "hybrid signal sequence" refers to signal sequences in which part of sequence is obtained from the expression host fused to the signal sequence of the gene to be expressed. In some embodiments, synthetic sequences are utilized.

The term "mature" form of a protein or peptide refers to the final functional form of the protein or peptide. For example, the mature form of the BPN' subtilisin protease of the present invention includes at least the amino acid sequence identical to residue positions 1-275 of SEQ ID NO:2, as set forth below:

AQSVPYGVSQIKAPALHSQGYTGSNVKVAVIDSGIDSSHPDLKVAGGASMVPSETNP FQDNNSHGTHVAGTVAALNNSIGVLGVAPSASLYAVKVLGADGSGQYSWIINGIEW AIANNMDVINMSLGGPSGSAALKAAVDKAVASGVVVVAAAGNEGTSGSSSTVGYP GKYPSVIAVGAVDSSNQRASFSSVGPELDVMAPGVSIQSTLPGNKYGAYNGTSMASP HVAGAAALILSKHPNWTNTQVRSSLENTTTKLGDSFYYGKGLINVQAAAQ (SEQ ID NO:2)

The term "precursor" form of a protein or peptide refers to a mature form of the protein having a prosequence operably linked to the amino or carbonyl terminus of the protein. The precursor may also have a "signal" sequence operably linked, to the amino terminus of the prosequence. The precursor may also have additional polynucleotides that are involved in post-translational activity (*e.g.,* polynucleotides cleaved therefrom to leave the mature form of a protein or peptide).

"Naturally occurring enzyme" refers to an enzyme having the unmodified amino acid sequence identical to that found in nature. Naturally occurring enzymes include native enzymes, those enzymes naturally expressed or found in the particular microorganism.

The terms "derived from" and "obtained from" refer to not only a protease produced or producible by a strain of the organism in question, but also a protease encoded by a DNA sequence isolated from such strain and produced in a host organism containing such DNA sequence. Additionally, the term refers to a protease which is encoded by a DNA sequence of synthetic and/or cDNA origin and which has the identifying characteristics of the protease in question..

A "derivative" within the scope of this definition generally retains the characteristic proteolytic activity observed in the wild-type, native or parent form to the extent that the derivative is useful for similar purposes as the wild-type, native or parent form. Functional derivatives of serine protease encompass naturally occurring, synthetically or recombinantly produced peptides or peptide fragments which have the general characteristics of the serine protease of the present invention.

The term "functional derivative" refers to a derivative of a nucleic acid which has the functional characteristics of a nucleic acid which encodes serine protease. Functional derivatives of a nucleic acid which encode serine protease of the present invention encompass naturally occurring, synthetically or recombinantly produced nucleic acids or fragments and encode serine protease characteristic of the present invention. Wild type nucleic acid encoding serine proteases according to the invention include naturally occurring alleles and homologues based on the degeneracy of the genetic code known in the art.

The term "identical" in the context of two nucleic acids or polypeptide sequences refers to the residues in the two sequences that are the same when aligned for maximum correspondence, as measured using one of the following sequence comparison or analysis algorithms.

The term "optimal alignment" refers to the alignment giving the highest percent identity score.

"Percent sequence identity," "percent amino acid sequence identity," "percent gene sequence identity," and/or "percent nucleic acid/polynucloctide sequence identity," with respect to two amino acid, polynucleotide and/or gene sequences (as appropriate), refer to the percentage of residues that are identical in the two sequences when the sequences are optimally aligned. Thus, 80% amino acid sequence identity means that 80% of the amino acids in two optimally aligned polypeptide sequences are identical.

The phrase "substantially identical" in the context of two nucleic acids or polypeptides thus refers to a polynucleotide or polypeptide that comprising at least about 70% sequence identity, preferably at least about 75%, preferably at least about 80%, preferably at least about 85%, preferably at least about 90%, preferably at least about 95% , preferably at least about 97% , preferably at least about 98%, and preferably at least about 99% sequence identity as compared to a reference sequence using the programs or algorithms (*e.g.*, BLAST, ALIGN, CLUSTAL) using standard parameters. One indication that two polypeptides are substantially identical is that the first polypeptide is immunologically cross-reactive with the second polypeptide. Typically, polypeptides that differ by conservative amino acid substitutions are immunologically cross-reactive. Thus, a polypeptide is substantially identical to a second polypeptide, for example, where the two peptides differ only by a conservative substitution. Another indication that two nucleic acid sequences are substantially identical is that the two molecules hybridize to each other under stringent conditions (*e.g.*, within a range of medium to high stringency).

The phrase "equivalent," in this context, refers to scrine proteases enzymes that arc encoded by a polynucleotide capable of hybridizing to the polynucleotide having the sequence as shown in SEQ ID NO:1, under conditions of medium to maximum stringency. For example, being equivalent means that an equivalent mature serine protease comprises at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, and/or at least about 99% sequence identity to the mature subtilisin protease having the amino acid sequence of SEQ ID NO:2.

The term "isolated" or "purified" refers to a material that is removed from its original environment (*e.g.*, the natural environment if it is naturally occurring). For example, the material is said to be "purified" when it is present in a particular composition in a higher or lower concentration than exists in a naturally occurring or wild type organism or in combination with components not normally present upon expression from a naturally occurring or wild type organism. For example, a naturally-occurring polynucleotide or polypeptide present in a living animal is not isolated, but the same polynucleotide or polypeptide, separated from some or all of the coexisting materials in the natural system, is isolated. In some embodiments, such polynucleotides are part of a vector, and/or such polynucleotide or polypeptides are part of a composition, and still be isolated in that such vector or composition is not part of its natural environment. In preferred embodiments, a nucleic acid or protein is said to be purified, for example, if it gives rise to essentially one hand in an electrophoretic gel or blot.

The term "isolated", when used in reference to a DNA sequence, refers to a DNA sequence that has been removed from its natural genetic milieu and is thus free of other extraneous or unwanted coding sequences, and is in a form suitable for use within genetically engineered protein production systems. Such isolated molecules are those that arc separated from their natural environment and include cDNA and genomic clones. Isolated DNA molecules of the present invention are free of other genes with which they are ordinarily associated, but may include naturally occurring 5' and 3' untranslated regions such as promoters and terminators. The identification of associated regions will be evident to one of ordinary skill in the art (*See e.g.,* Dynan and Tijan, Nature 316:774-78 [1985]). The term "an isolated DNA sequence" is alternatively referred to as "a cloned DNA sequence".

The term "isolated," when used in reference to a protein, refers to a protein that is found in a condition other than its native environment. In a preferred form, the isolated protein is substantially free of other proteins, particularly other homologous proteins. An isolated protein is more than about 10% pure, preferably more than about 20% pure, and even more preferably more than about 30% pure, as determined by SDS-PAGE. Further aspects of the invention encompass the protein in a highly purified form (*i.e.,* more than about 40% pure, more than about 60% pure, more than about 80% pure, more than about 90% pure, more than about 95% pure, more than about 97% pure, and even more than about 99% pure), as determined by SDS-PAGE.

As used herein, the term, "combinatorial mutagenesis" refers to methods in which libraries of variants of a starting sequence are generated. In these libraries, the variants contain one or several mutations chosen from a predefined set of mutations. In addition, the methods provide means to introduce random mutations which were not members of the predefined set of mutations. In some embodiments, the methods include those set forth in U.S. Patent Appln. Ser. No. 09/699.250, filed October 26, 2000, hereby incorporated by reference. In alternative embodiments, Combinatorial mutagenesis methods encompass commercially available kits (*e.g.,* QuikChange® Multisite, Stratagene, San Diego, CA).

As used herein, the term "library of mutants" refers to a population of cells which are identical in most of their genome but include different homologues of one or more genes. Such libraries can be used, for example, to identify genes or operons with improved traits.

As used herein, the term "starting gene" refers to a gene of interest that encodes a protein of interest that is to be improved and/or changed using the present invention.

As used herein, the term "multiple sequence alignment" ("MSA") refers to the sequences of multiple homologs of a starting gene that arc aligned using an algorithm (*e.g.*, Clustal W).

As used herein, the terms "consensus sequence" and "canonical sequence" refer to an archetypical amino acid sequence against which all variants of a particular protein or sequence of interest are compared. The terms also refer to a sequence that sets forth the nucleotides that are most often present in a DNA sequence of interest. For each position of a gene, the consensus sequence gives the amino acid that is most abundant in that position in the MSA.

As used herein, the term "consensus mutation" refers to a difference in the sequence of a starting gene and a consensus sequence. Consensus mutations are identified by comparing the sequences of the starting gene and the consensus sequence resulting from an MSA. In some embodiments, consensus mutations are introduced into the starting gene such that it becomes more similar to the consensus sequence. Consensus mutations also include amino acid changes that change an amino acid in a starting gene to an amino acid that is more frequently found in an MSA at that position relative to the frequency of that amino acid in the starting gene. Thus, the term consensus mutation comprises all single amino acid changes that replace an amino acid of the starting gene with an amino acid that is more abundant than the amino acid in the MSA.

As used herein, the term "enhanced Combinatorial consensus mutagenesis library" refers to a CCM library that is designed and constructed based on screening and/or sequencing results from an earlier round of CCM mutagenesis and screening. In some embodiments, the enhanced CCM library is based on the sequence of an initial hit resulting from an earlier round of CCM. In additional embodiments, the enhanced CCM is designed such that mutations that were frequently observed in initial hits from earlier rounds of mutagenesis and screening are favored. In some preferred embodiments, this is accomplished by omitting primers that encode performance-reducing mutations or by increasing the concentration of primers that encode performance-enhancing mutations relative to other primers that were used in earlier CCM libraries.

As used herein, the term "performance-reducing mutations" refer to mutations in the combinatorial consensus mutagenesis library that are less frequently found in hits resulting from screening as compared to an unscreened combinatorial consensus mutagenesis library. In preferred embodiments, the screening process removes and/or reduces the abundance of variants that contain "performance-reducing mutations."

As used herein, the term "functional assay" refers to an assay that provides an indication of a protein's activity. In particularly preferred embodiments, the term refers to assay systems in which a protein is analyzed for its ability to function in its usual capacity. For example, in the case of enzymes, a functional assay involves determining the effectiveness of the enzyme in catalyzing a reaction.

As used herein, the term "target property" refers to the property of the starting gene that is to be altered. It is not intended that the present invention be limited to any particular target property. I lowever, in some preferred embodiments, the target property is the stability of a gene product (*e.g.*, resistance to denaturation, proteolysis or other degradative factors), while in other embodiments, the level of production in a production host is altered. Indeed, it is contemplated that any property of a starting gene will find use in the present invention.

The term "property" or grammatical equivalents thereof in the context of a nucleic acid, as used herein, refer to any characteristic or attribute of a nucleic acid that can be selected or detected. These properties include, but are not limited to, a property affecting binding to a polypeptide, a property conferred on a cell comprising a particular nucleic acid, a property affecting gene transcription (*e.g.*, promoter strength, promoter recognition, promoter regulation, enhancer function), a property affecting RNA processing (*e.g.,* RNA splicing, RNA stability, RNA conformation, and post-transcriptional modification), a property affecting translation (*e.g.*, level, regulation, binding of mRNA to ribosomal proteins, post-translational modification). For example, a binding site for a transcription factor, polymerase, regulatory factor, etc., of a nucleic acid may be altered to produce desired characteristics or to identify undesirable characteristics.

The term "property" or grammatical equivalents thereof in the context of a polypeptide (including proteins), as used herein, refer to any characteristic or attribute of a polypeptide that can be selected or detected. These properties include, but are not limited to oxidative stability, substrate specificity, catalytic activity, thermal stability, alkaline stability, pH activity profile, resistance to proteolytic degradation, $K_M$, $k_{cat}$, $k_{cat}/k_M$ ratio, protein folding, inducing an immune response, ability to bind to a ligand, ability to bind to a receptor, ability to be secreted, ability to be displayed on the surface of a cell, ability to oligomerize, ability to signal, ability to stimulate cell proliferation, ability to inhibit cell proliferation, ability to induce apoptosis, ability to be modified by phosphorylation or glycosylation, and/or ability to treat disease, etc.

The terms "modified sequence" and "modified genes" are used interchangeably herein to refer to a sequence that includes a deletion, insertion or interruption of naturally occurring nucleic acid sequence. In some preferred embodiments, the expression product of the modified sequence is a truncated protein (*e.g.*, if the modification is a deletion or interruption of the sequence). In some particularly preferred embodiments, the truncated protein retains biological activity. In alternative embodiments, the expression product of the modified sequence is an elongated protein (*e.g.,* modifications comprising an insertion into the nucleic acid sequence). In some embodiments, an insertion leads to a truncated protein (*e.g.,* when the insertion results in the formation of a stop codon). Thus, an insertion may result in either a truncated protein or an elongated protein as an expression product.

As used herein, the terms "mutant sequence" and "mutant gene" arc used interchangeably and refer to a sequence that has an alteration in at least one codon occurring in a host cell's wild-type sequence. The expression product of the mutant sequence is a protein with an altered amino acid sequence relative to the wild-type. The expression product may have an altered functional capacity (e.g., enhanced enzymatic activity).

The terms "mutagenic primer" or "mutagenic oligonucleotide" (used interchangeably herein) are intended to refer to oligonucleotide compositions which correspond to a portion of the template sequence and which are capable of hybridizing thereto. With respect to mutagenic primers, the primer will not precisely match the template nucleic acid, the mismatch or mismatches in the primer being used to introduce the desired mutation into the nucleic acid library. As used herein, "non-mutagenic primer" or "non-mutagenic oligonucleotide" refers to oligonucleotide compositions which will match precisely to the template nucleic acid. In one embodiment of the invention, only mutagenic primers are used. In another preferred embodiment of the invention, the primers are designed so that for at least one region at which a mutagenic primer has been included, there is also non-mutagenic primer included in the oligonucleotide mixture. By adding a mixture of mutagenic primers and non-mutagenic primers corresponding to at least one of the mutagenic primers, it is possible to produce a resulting nucleic acid library in which a variety of combinatorial mutational patterns are presented. For example, if it is desired that some of the members of the mutant nucleic acid library retain their precursor sequence at certain positions while other members arc mutant at such sites, the non-mutagenic primers provide the ability to obtain a specific level of non-mutant members within the nucleic acid library for a given residue. The methods of the invention employ mutagenic and non-mutagenic oligonucleotides which are generally between 10-50 bases in length, more preferably about 15-45 bases in length. However, it may he necessary to use primers that are either shorter than 10 bases or longer than 50 bases to obtain the mutagenesis result desired. With respect to corresponding mutagenic and non-mutagenic primers, it is not necessary that the corresponding oligonucleotides be of identical length, but only that there is overlap in the region corresponding to the mutation to be added. Primers may be

added in a pre-defined ratio according to the present invention. For example, if it is desired that the resulting library have a significant level of a certain specific mutation and a lesser amount of a different mutation at the same or different site, by adjusting the amount of primer added, it is possible to produce the desired biased library. Alternatively, by adding lesser or greater amounts of non-mutagenic primers, it is possible to adjust the frequency with which the corresponding mutation(s) are produced in the mutant nucleic acid library.

As used herein, the phrase "contiguous mutations" refers to mutations which are presented within the same oligonucleotide primer. For example, contiguous mutations may be adjacent or nearby each other, however, they will be introduced into the resulting mutant template nucleic acids by the same primer.

The terms "wild-type sequence," or "wild-type gene" are used interchangeably herein, to refer to a sequence that is native or naturally occurring in a host cell. In some embodiments, the wild-type sequence refers to a sequence of interest that is the starting point of a protein engineering project (*i.e.*, the "parent" sequence). The wild-type sequence may encode either a homologous or heterologous protein. A homologous protein is one the host cell would produce without intervention. A heterologous protein is one that the host cell would not produce but for the intervention.

As used herein, the terms "protease variant," "subtilisin variant," "subtilisin protease variant," are used in reference to proteases that arc similar to a wild-type subtilisin or a parent protease (*e.g.,* subtilisin) used as a starting point in protein engineering. These variants are similar to the wild-type or other parent in their function, but have mutations in their amino acid sequence that make them different in sequence from the wild-type or parent protease (*e.g.,* subtilisin).

Unless otherwise indicated, the amino acid position numbers refer to those assigned to the mature *Bacillus amyloliquefaciens* subtilisin sequence of SEQ ID NO:2. The invention, however, is not limited to the mutation of this particular subtilisin but extends to precursor proteases containing amino acid residues at positions which are "equivalent" to the particular identified residues in the *Bacillus amyloliquefaciens* subtilisin.

As used herein, the terms "modification" and "mutation" refers to any change or alteration in an amino acid sequence. It is intended that the term encompass substitutions, deletions, insertions, and/or replacement of amino acid side chains in an amino acid sequence of interest (*e.g.*, a subtilisin sequence). It is also intended that the term encompass chemical modification of an amino acid sequence of interest (*e.g.*, a subtilisin sequence).

The term "oxidation stable" refers to proteases of the present invention that retain a specified amount of enzymatic activity over a given period of time under conditions prevailing during the proteolytic, hydrolyzing, cleaning or other process of the invention, for example while exposed to or contacted with bleaching agents or oxidizing agents. In some embodiments, the proteases retain at least about 50%, about 60%, about 70%, about 75%, about 80%, about 85%, about 90%, about 92%, about 95%, about 96%, about 97%, about 98%,or about 99% proteolytic activity after contact with a bleaching or oxidizing agent over a given time period, for example, at least about 1 minute, about 3 minutes, about 5 minutes, about 8 minutes, about 12 minutes, about 16 minutes, about 20 minutes, etc. In some embodiments, the stability is measured as described in the Examples.

The term "chelator stable" refers to proteases of the present invention that retain a specified amount of enzymatic activity over a given period of time under conditions prevailing during the proteolytic, hydrolyzing, cleaning or other process of the invention, for example while exposed to or contacted with chelating agents. In some embodiments, the proteases retain at least about 50%, about 60%, about 70%, about 75%, about 80%, about 85%, about 90%, about 92%, about 95%, about 96%, about 97%, about 98%,or about 99% proteolytic activity after contact with a chelating agent over a given time period, for example, at least about 10 minutes, about 20 minutes, about 40 minutes, about 60 minutes, about 100 minutes, etc. In some embodiments, the chelator stability is measured as described in the Examples.

The terms "thermally stable" and "thermostable" refer to proteases of the present invention that retain a specified amount of enzymatic activity after exposure to identified temperatures over a given period of time under conditions prevailing during the proteolytic, hydrolyzing, cleaning or other process of the invention, for example while exposed altered temperatures. Altered temperatures includes increased or decreased temperatures. In some embodiments, the proteases retain at least about 50%, about 60%, about 70%, about 75%, about 80%, about 85%, about 90%, about 92%, about 95%, about 96%, about 97%, about 98%,or about 99% proteolytic activity after exposure to altered temperatures over a given time period, for example, at least about 60 minutes, about 120 minutes, about 180 minutes, about 240 minutes, about 300 minutes, etc. In some embodiments, the thermostability is determined as described in the Examples.

The term "enhanced stability" in the context of an oxidation, chelator, thermal and/or pH stable protease refers to a higher retained proteolytic activity over time as compared to other serine proteases (*e.g.*, subtilisin proteases) and/or wild-type enzymes.

The term "diminished stability" in the context of an oxidation, chelator, thermal and/or pH stable protease refers to a lower retained proteolytic activity over time as compared to other serine proteases (*e.g.*, subtilisin proteases) and/or wild-type enzymes.

The term "cleaning activity" refers to the cleaning performance achieved by the protease under conditions prevailing during the proteolytic, hydrolyzing, cleaning or other process of the invention. In some embodiments, cleaning performance is determined by the application of various cleaning assays concerning enzyme sensitive stains, for example

grass, blood, milk, or egg protein as determined by various chromatographic, spectrophotometric or other quantitative methodologies after subjection of the stains to standard wash conditions. Exemplary assays include, but are not limited to those described in WO 99/34011, and U.S. Pat. 6,605,458 (both of which are herein incorporated by reference), as well as those methods included in the Examples.

The term "cleaning effective amount" of a protease refers to the quantity of protease described hereinbefore that achieves a desired level of enzymatic activity in a specific cleaning composition. Such effective amounts are readily ascertained by one of ordinary skill in the art and are based on many factors, such as the particular protease used, the cleaning application, the specific composition of the cleaning composition, and whether a liquid or dry (*e.g.*, granular, bar) composition is required, etc.

The term "cleaning adjunct materials," as used herein, means any liquid, solid or gaseous material selected for the particular type of cleaning composition desired and the form of the product (*e.g.*, liquid, granule, powder, bar, paste, spray, tablet, gel; or foam composition), which materials are also preferably compatible with the protease enzyme used in the composition. In some embodiments, granular compositions are in "compact" form, while in other embodiments, the liquid compositions are in a "concentrated" form.

The term "enhanced performance" in the context of cleaning activity refers to an increased or greater cleaning activity of certain enzyme sensitive stains such as egg, milk, grass or blood, as determined by usual evaluation after a standard wash cycle and/or multiple wash cycles.

The term "diminished performance" in the context of cleaning activity refers to an decreased or lesser cleaning activity of certain enzyme sensitive stains such as egg, milk, grass or blood, as determined by usual evaluation after a standard wash cycle.

The term "comparative performance" in the context of cleaning activity refers to at least about 60%, at least about 70%, at least about 80% at least about 90%, or at least about 95% of the cleaning activity of a comparative subtilisin protease (*e.g.,* commercially available proteases), including but not limited to OPTIMASE™ protease (Genencor), PURAFECT™ protease products (Genencor), SAVINASE™ protease (Novozymes), BPN'- variants (*See e.g.,* U.S. Pat. No. Re 34, 606), RELASE™ DURAZYME™, EVERLASE™, KANNASE™ protease (Novozymes), MAXACAL™, MAXAPEM™, PROPERASE™ proteases (Genencor; *See also,* U.S. Pat. No. Re 34, 606, and U.S. Pat. Nos. 5, 700, 676; 5, 955, 340; 6, 312, 936; and 6, 482, 628), and *B. lentus* variant protease products (*e.g.,* those described in WO 92/21760, WO 95/23221 and/or WO 97/07770) .

Cleaning performance can be determined by comparing the proteases of the present invention with those subtilisin proteases in various cleaning assays concerning enzyme sensitive stains such as grass, blood or milk as determined by usual spectrophotometric or analytical methodologies after standard wash cycle conditions.

As used herein, "cleaning compositions" and "cleaning formulations" refer to compositions that find use in the removal of undesired compounds from items to be cleaned, such as fabric, dishes, contact lenses, other solid substrates, hair (shampoos), skin (soaps and creams), teeth (mouthwashes, toothpastes) etc. The term encompasses any materials/ compounds selected for the particular type of cleaning composition desired and the form of the product (*e.g.*, liquid, gel, granule, or spray composition), as long as the composition is compatible with the perhydrolase and other enzyme (s) used in the composition. The specific selection of cleaning composition materials are readily made by considering the surface, item or fabric to be cleaned, and the desired form of the composition for the cleaning conditions during use.

The terms further refer to any composition that is suited for cleaning, bleaching, disinfecting, and/or sterilizing any object and/or surface. It is intended that the terms include, but are not limited to detergent compositions (*e.g.,* liquid and/or solid laundry detergents and fine fabric detergents; hard surface cleaning formulations, such as for glass, wood, ceramic and metal counter tops and windows; carpet cleaners; oven cleaners; fabric fresheners; fabric softeners; and textile and laundry pre-spotters, as well as dish detergents).

Indeed, the term "cleaning composition" as used herein, includes unless otherwise indicated, granular or powder-form all- purpose or heavy- duty washing agents, especially cleaning detergents; liquid, gel or paste- form all- purpose washing agents, especially the so- called heavy- duty liquid (HDL) types; liquid fine- fabric detergents; hand dishwashing agents or light duty dishwashing agents, especially those of the high- foaming type; machine dishwashing agents, including the various tablet, granular, liquid and rinse- aid types for household and institutional use; liquid cleaning and disinfecting agents, including antibacterial hand- wash types, cleaning bars, mouthwashes, denture cleaners, car or carpet shampoos, bathroom cleaners; hair shampoos and hair- rinses; shower gels and foam baths and metal cleaners; as well as cleaning auxiliaries such as bleach additives and "stain- stick" or pre- treat types.

As used herein, the terms "detergent composition" and "detergent formulation" are used in reference to mixtures which are intended for use in a wash medium for the cleaning of soiled objects. In some preferred embodiments, the term is used in reference to laundering fabrics and/or garments (*e.g.,* "laundry detergents") . In alternative embodiments, the term refers to other detergents, such as those used to clean dishes, cutlery, etc. (*e.g.*, "dishwashing detergents") . It is not intended that the present invention be limited to any particular detergent formulation or composition. Indeed, it is intended that in addition to perhydrolase, the term encompasses detergents that contain surfactants, transferase (s), hydrolytic enzymes, oxido reductases, builders, bleaching agents, bleach activators, bluing agents and fluorescent dyes,

caking inhihitors, masking agents, enzyme activators, antioxidants, and solubilizers.

As used herein, "fabric cleaning compositions" include hand and machine laundry detergent compositions including laundry additive compositions and compositions suitable for use in the soaking and/or pretreatment of stained fabrics (*e.g.*, clothes, linens, and other textile materials).

As used herein, "non-fabric cleaning compositions" include non-textile (*i.e.*, fabric) surface cleaning compositions, including but not limited to dishwashing detergent compositions, oral cleaning compositions, denture cleaning compositions, and personal cleansing compositions.

The "compact" form of the cleaning compositions herein is best reflected by density and, in terms of composition, by the amount of inorganic filler salt. Inorganic filler salts are conventional ingredients of detergent compositions in powder form. In conventional detergent compositions, the filler salts are present in substantial amounts, typically about 17 to about 35% by weight of the total composition. In contrast, in compact compositions, the filler salt is present in amounts not exceeding about 15% of the total composition. In some embodiments, the filler salt is present in amounts that do not exceed about 10%, or more preferably, about 5%, by weight of the composition. In some embodiments, the inorganic filler salts are selected from the alkali and alkaline-earth-metal salts of sulfates and chlorides. A preferred filler salt is sodium sulfate.

As used herein, the term "surfactant" refers to any compound generally recognized in the art as having surface active qualities. Surfactants generally include anionic, cationic, nonionic, and zwitterionic compounds, which are further described, herein.

## EXPERIMENTAL

The present invention is described in further detail in the following Examples which arc not in any way intended to limit the scope of the invention as claimed. The attached Figure are meant to be considered as integral parts of the specification and description of the invention. The following Examples are offered to illustrate, but not to limit the claimed invention

In the experimental disclosure which follows, the following abbreviations apply: PI or Pi (Performance Index), ppm (parts per million) ; M (molar) ; mM (millimolar) ; μM (micromolar) ; nM (nanomolar) ; mol (moles) ; mmol (millimoles) ; μmol (micromoles) ; nmol (nanomoles) ; gm (grams) ; mg (milligrams) ; μg (micrograms) ; pg (picograms) ; L (liters) ; ml and mL (milliliters) ; μl and μL (microliters) ; cm (centimeters) ; mm (millimeters) ; μm (micrometers) ; nm (nanometers) ; U (units) ; V (volts) ; MW (molecular weight) ; sec (seconds) ; min (s) (minute/ minutes) ; h (s) and hr (s) (hour/ hours) ; °C. (degrees Centigrade) ; QS (quantity sufficient) ; ND (not done) ; NA (not applicable) ; rpm (revolutions per minute) ; $H_2O$ (water) ; $dH_2O$ (deionized water) ; (HCl (hydrochloric acid) ; aa (amino acid) ; bp (base pair) ; kb (kilobase pair) ; kD (kilodaltons) ; cDNA (copy or complementary DNA) ; DNA (deoxyribonucleic acid) ; ssDNA (single stranded DNA) ; dsDNA (double stranded DNA) ; dNTP (deoxyribonucleotide triphosphate) ; RNA (ribonucleic acid) ; $MgCl_2$ (magnesium chloride) ; NaCl (sodium chloride) ; w/v (weight to volume) ; v/v (volume to volume) ; *g* (gravity) ; OD (optical density) ; Dulbecco's phosphate buffered solution (DPBS) ; SOC (2% Bacto- Tryptone, 0.5% Bacto Yeast Extract, 10 mM NaCl, 2.5 mM KCl) ; Terrific Broth (TB; 12 g/l Bacto Tryptone, 24 g/l glycerol, 2.31 g/l $KH_2PO_4$, and 12.54 g/l $K_2HPO_4$) ; $OD_{280}$ (optical density at 280 nm) ; $OD_{600}$ (optical density at 600 nm) ; $A_{405}$ (absorbance at 405 nm) ; Vmax (the maximum initial velocity of an enzyme catalyzed reaction) ; PAGE (polyacrylamide gel electrophoresis) ; PBS (phosphate buffered saline [150 mM NaCl, 10 mM sodium phosphate buffer, pH 7.2] ) ; PBST (PBS+0.25% TWEEN® 20) ; PEG (polyethylene glycol) ; PCR (polymerase chain reaction) ; RT- PCR (reverse transcription PCR) ; SDS (sodium dodecyl sulfate) ; Tris (tris (hydroxymethyl) aminomethane) ; HEPES (N- [2- Hydroxyethyl] piperazine- N- [2- ethanesulfonic acid] ) ; HBS (HEPES buffered saline) ; Tris- HCl (tris [Hydroxymethyl] aminomethane- hydrochloride) ; Tricine (N- [tris- (hydroxymethyl)- methyl]- glycine) ; CHES (2- (N- cyclo- hexylamino) ethane- sulfonic acid) ; TAPS (3- { [tris- (hydroxymethyl)- methyl]- amino}- propanesulfonic acid) ; CAPS (3- (cyclo- hexylamino)- propane- sulfonic acid; DMSO (dimethyl sulfoxide) ; DTT (1, 4- dithio- DL- threitol) ; SA (sinapinic acid (s, 5- dimethoxy- 4- hydroxy cinnamic acid) ; TCA (trichloroacetic acid) ; Glut and GSH (reduced gtutathione) ; GSSG (oxidized glutathione) ; TCEP (Tris [2- carboxyethyl] phosphine) ; Ci (Curies) ; mCi (milliCuries) ; μCi (microCuries) ; HPLC (high pressure liquid chromatography) ; RP- HPLC (reverse phase high pressure liquid chromatography) ; TLC (thin layer chromatography) ; MALDI- TOF (matrix- assisted laser desorption/ ionization-- time of flight) ; Ts (tosyl) ; Bn (benzyl) ; Ph (phenyl) ; Ms (mesyl) ; Et (ethyl), Me (methyl) ; *Taq* (*Thermus aquaticus* DNA polymerase) ; Klenow (DNA polymerase I large (Klenow) fragment) ; EGTA (ethylene glycol-bis (ß- aminoethyl ether) N, N, N', N'- tetraacetic acid) ; EDTA (ethylenediaminetetracetic acid) ; bla (β- lactamase or ampicillin- resistance gene) ; HDL (high density liquid) ; MJ Research (MJ Research, Reno, NV) ; Baseclear (Baseclear BV, Inc., Leiden, the Netherlands) ; PerSeptive (PerSeptive Biosystems, Framingham, MA) ; ThermoFinnigan (ThermoFinnigan, San Jose, CA) ; Argo (Argo BioAnalytica, Morris Plains, NJ) ; Seitz EKS (SeitzSchenk Filtersystems GmbH, Bad Kreuznach, Germany) ; Pall (Pall Corp., East Hills, NY) ; Spectrum (Spectrum Laboratories, Dominguez Rancho, CA) ; Molecular Structure (Molecular Structure Corp., Woodlands, TX) ; Accelrys (Accelrys, Inc., San Diego, CA) ; Chemical Computing (Chemical Computing Corp., Montreal, Canada) ; New Brunswick (New Brunswick Scientific, Co.,

Edison, NJ) ; CFT (Center for Test Materials, Vlaardingen, the Netherlands) ; Test Fabrics (Test Fabrics, Inc., West Pittiston, PA), Procter & Gamble (Procter & Gamble, Inc., Cincinnati, OH) ; Epicentre (Epicentre Biotechnologies, Madison, WI) ; GE Healthcare (GE Healthcare, Chalfont St. Giles, United Kingdom) ; OXOID (Oxoid, Basingstoke, Hampshire, UK) ; Megazyme (Megazyme International Ireland Ltd., Bray Business Park, Bray, Co., Wicklow, Ireland) ; Finnzymes (Finnzymes Oy, Espoo, Finland) ; Kelco (CP Kelco, Wilmington, DE) ; Coming (Corning Life Sciences, Corning, NY) ; (NEN (NEN Life Science Products, Boston, MA) ; Pharma AS (Pharma AS, Oslo, Norway) ; Dynal (Dynal, Oslo, Norway) ; Bio- Synthesis (Bio- Synthesis, Lewisville, TX) ; ATCC (American Type Culture Collection, Rockville, MD) ; Gibco/BRL (Gibco/BRL, Grand Island , NY) ; Sigma (Sigma Chemical Co., St. Louis, MO) ; Pharmacia (Pharmacia Biotech, Piscataway, NJ) ; NCBI (National Center for Biotechnology Information) ; Applied Biosystems (Applied Biosystems, Foster City, CA) ; BD Biosciences and/or Clontech (BD Biosciences CLONTECH Laboratories, Palo Alto, CA) ; Operon Technologies (Operon Technologies, Inc., Alamcda, CA) ; MWG Biotech (MWG Biotech, High Point, NC) ; Oligos Etc (Oligos Etc. Inc, Wilsonville, OR) ; Bachem (Bachem Bioscience, Inc., King of Prussia, PA) ; Difco (Difco Laboratories, Detroit, MI) ; Mediatech (Mediatech, Herndon, VA; Santa Cruz (Santa Cruz Biotechnology, Inc., Santa Cruz, CA) ; Oxoid (Oxoid Inc., Ogdensburg, NY) ; Worthington (Worthington Biochemical Corp., Freehold, NJ) ; GIBCO BRL or Gibco BRL (Life Technologies, Inc., Gaithersburg, MD) ; Millipore (Millipore, Billerica, MA) ; Bio- Rad (Bio- Rad, Hercules, CA) ; Invitrogen (Invitrogen Corp., San Diego, CA) ; New England Biolabs and NEB (New England Biolabs, Ipswich, MA) ; Sigma (Sigma Chemical Co., St. Louis, MO) ; Pierce (Pierce Biotechnology, Rockford, IL) ; Takara (Takara Bio Inc. Otsu, Japan) ; Roche (Hoffmann- La Roche, Basel, Switzerland) ; EM Science (EM Science, Gibbstown, NJ) ; Qiagen (Qiagen, Inc., Valencia, CA) ; Biodesign (Biodesign Intl., Saco, Maine) ; Aptagen (Aptagen, Inc., Herndon, VA) ; Sorvall (Sorvall brand, from Kendro Laboratory Products, Asheville, NC) ; United States Testing (United States Testing Co., Hoboken, NJ) ; Molecular Devices (Molecular Devices, Corp., Sunnyvale, CA) ; R&D Systems (R&D Systems, Minneapolis, MN) ; Stratagene (Stratagene Cloning Systems, La Jolla, CA) ; Marsh (Marsh Biosciences, Rochester, NY) ; Geneart (Geneart GmbH, Regensburg, Germany) ; DNA2.0 (DNA2.0, Menlo Park, CA) ; Gene Oracle (Gene Oracle, Mountain View, CA) ; Zymo Research (Zymo Research, Orange, CA) ; Bio- Tek (Bio- Tek Instruments, Winooski, VT) ; Biacore (Biacore, Inc., Piscataway, NJ) ; PeproTech (PeproTech, Rocky Hill, NJ) ; SynPep (SynPep, Dublin, CA) ; New Objective (New Objective brand; Scientific Instrument Services, Inc., Ringoes, NJ) ; Waters (Waters, Inc., Milford, MA) ; Matrix Science (Matrix Science, Boston, MA) ; Dionex (Dionex, Corp., Sunnyvale, CA) ; Monsanto (Monsanto Co., St. Louis, MO) ; Wintershall (Wintershall AG, Kassel, Germany) ; BASF (BASF Co., Florham Park, NJ) ; Huntsman (Huntsman Petrochemical Corp., Salt Lake City, UT) ; Enichem (Enichem Iberica, Barcelona, Spain) ; Fluka Chemie AG (Fluka Chemie AG, Buchs, Switzertand) ; Gist- Brocades (Gist- Brocades, NV, Delft, the Netherlands) ; Dow Corning (Dow Corning Corp., Midland, MI) ; and Microsoft (Microsoft, Inc., Recimond, WA) .

**EXAMPLE 1**

**Assays**

In the following Example, various assays were used as set forth below for ease in reading. Any deviations from the protocols provided below are indicated in the Examples. In the present invention, the subtilisin numbering corresponds to that of the mature BPN' sequence, as set forth below:

AQSVPYGVSQIKAPALHSQGYTGSNVKVAVIDSGIDSSHPDLKVAGGASMVPSETNPFQDNN
SHGTHVAGTVAALNNSIGVLGVAPSASLYAVKVLGADGSGQYSWIINGIEWAIANNMDVIN
MSLGGPSGSAALKAAVDKAVASGVVVVAAAGNEGTSGSSSTVGYPGKYPSVIAVGAVDSSN
QRASFSSVGPELDVMAPGVSIQSTLPGNKYGAYNGTSMASPHVAGAAALILSKHPNWTNTQ
VRSSLENTTTKLGDSFYYGKGLINVQAAAQ (SEQ ID NO:1)

**<u>A. TCA Assay for Protein Content Determination in 96-well Microtiter Plates</u>**

For BPN' (*e.g.,* reference protease) and BPN' variants, this assay was started using filtered culture supernatant from microliter plates grown 3-4 days at 33°C with shaking at 230 rpm and humidified aeration. A fresh 96-well flat bottom microtiter plate (MTP) was used for the assay. First, 100 $\mu$L/well of 0.25 N HCl was placed in each well. Then, 50 $\mu$L of filtered culture broth was added. The light scattering/absorbance at 405 nm (use 5 sec mixing mode in the plate reader) was then determined, in order to provide the "blank" reading. For the test, 100 $\mu$L/well of 15% (w/v) trichloroacetic acid (TCA) was placed in the plates and incubated between 5 and 30 min at room temperature. The light scattering/absorbance at 405 nm (use 5 sec mixing mode in the plate reader) was then determined.

The equipment used was a Biomek FX Robot (Beckman Coulter) and a SpectraMAX (type 340; Molecular Devices) MTP Reader; the MTP's were from Costar (type 9017). The equipment used was a Biomek FX Robot (Beckman

Coulter) and a SpectraMAX type 340 (Molecular Devices) MTP Reader; and the MTPs were type 9017 (Costar).

The calculations were performed by subtracting the blank (no TCA) from the test reading with TCA to provide a relative measure of the protein content in the samples. If desired, a standard curve can be created by calibrating the TCA readings with AAPF assays of clones with known conversion factors. However, the TCA results are linear with respect to protein concentration from 50 to 500 protein per ml (ppm) and can thus be plotted directly against enzyme performance for the purpose of choosing good-performing variants. The turbidity/light scatter increase in the samples correlates to the total amount of precipitable protein in the culture supernatant.

B. **AAPF Protease Assay in 96-well Microtiter Plates**

In order to determine the protease activity of the proteases and variants thereof of the present invention, the hydrolysis of N- succinyl- L- alanyl- L- alanyl- L- prolyl- L- phenyl- p- nitroanilide (suc- AAPF- pNA) was measured. The reagent solutions used were: 100 mM Tris/HCl, pH 8.6, containing 0.005% TWEEN®- 80 (Tris dilution buffer) ; 100 mM Tris buffer, pH 8.6, containing 10 mM $CaCl_2$ and 0.005% TWEEN®- 80 (Tris/Ca buffer) ; and 160 mM suc- AAPF- pNA in DMSO (suc- AAPF- pNA stock solution) (Sigma: S- 7388) . To prepare a suc- AAPF- pNA working solution, 1 ml suc- AAPF- pNA stock solution was added to 100 ml Tris/Ca buffer and mixed well for at least 10 seconds. The assay was performed by adding 10 μl of diluted protease solution to each well, immediately followed by the addition of 190 μl 1 mg/ml suc- AAPF- pNA working solution. The solutions were mixed for 5 sec., and the absorbance change in kinetic mode (20 readings in 5 minutes) was read at 410 nm in an MTP reader, at 25°C. The protease activity was expressed as AU (activity = $\Delta OD \cdot min^{-1}\ ml^{-1}$) .

**C. Surfactant and Chelant Stability Assays**

LAS and LAS/EDTA stability was measured after incubation of the test protease in the presence of LAS and LAS/EDTA respectively, as a function of residual activity determined using the AAPF assay.

**LAS Stability Method**

**Reagents:**

Dodecylbenzenesulfonate, Sodium salt (=LAS) : Sigma D- 2525
TWEEN®- 80: Sigma P- 8074
TRIS buffer (free acid) : Sigma T- 1378) ; 6.35 g is dissolved in about 960 ml water; pH is adjusted to 8.2 with 4N HCl. Final concentration of TRIS is 52.5 mM.
LAS stock solution: Prepare a 10.5 % LAS solution in MQ water (=10.5 g per 100 ml MQ)
TRIS buffer- 100 mM /pH 8- 6 (100mM Tris/ 0.005% Tween®R- 80)
TRIS- Ca buffer, pH 8.6 (100mM Tris/ 10mM CaCl2/0.005% Tween- ®80)

**Hardware:**

Flat bottom MTPs (Costar No. 9017)
Biomek FX
ASYS Multipipettor
Spectramax MTP Reader
iEMS Incubator/Shaker
Innova 4330 Incubator/Shaker
Biohit multichannel pipette
BMG Thermostar Shaker

A 0.063% LAS solution was prepared in 52.5 mM Tris buffer pH 8.2. The suc-AAPF-pNA working solution was prepared by adding 1 ml of 100 mg/ml suc-AAPF-pNA stock solution (in DMSO) to 100 ml (100 mM) TRIS buffer, pH 8.6. To dilute the supernatants, flat-bottomed plates were filled with dilution buffer and an aliquot of the supernatant was added and mixed well. The dilution ratio depended on the concentration of the protease controls in the growth plates (AAPF activity). The desired protein concentration was 80 ppm.

Ten μl of the diluted supernatant were added to 190 μl 0.063% LAS buffer/well. The MTP was covered with tape, shaken for a few seconds and placed in an incubator (Innova 4230) at 45°C for for 60 minutes at 200 rpm agitation. The initial activity (*t*=10 minutes) was determined after 10 minutes of incubation by transferring 10 μl of the mixture in each well to a fresh MTP containing 190μl suc-AAPF-pNA work solution. These solutions were mixed well and the AAPF activity was measured using a MTP Reader (20 readings in 5 minutes and 25 "C).

The final activity (*t*=60 minutes) was determined by removing another 10 μl of solution from the incubating plate after 60 minutes of incubation. The AAPF activity was then determined as described above. The stability of the samples was determined by calculating the ration of the residual and initial AAPF activity as follows:

$$\text{Residual Activity (\%)} = [t\text{-60 value}]*100 / [t\text{-10 value}].$$

**LAS/EDTA Stability Method**

The stability of protease variants in the presence of a representative anionic surfactant (LAS=linear alkylbene sulfonate, sodium dodecylbenzenesulfonate- DOBS) and di- sodium EDTA was measured after incubation under defined conditions and the residual activity was determined using the AAPF assay. The reagents used were dodecyllbenzene sulfonate, sodium salt (DOBS, Sigma No. D- 2525), TWEEN®- 80 (Sigma No. P- 8074), di- sodium EDTA (Siegfried Handel No. 164599- 02), HEPES (Sigma No. H- 7523), unstress buffer: 50 mM HEPES (11.9 g/l) + 0.005% TWEEN®- 80, pH 8.0, Stress buffer: 50 mM HEPES (11.9 g/l), 0.1% (w/v) DOBS (1 g/l), 10 mM EDTA (3.36 g/l), pH 8.0, reference protease and protease variant culture supernatants, containing 200- 400 μg/ml protein. The equipment used was V- or U- bottom MTP as dilution plates (Greiner 651101 and 650161 respectively), F- bottom MTP (Coming 9017) for unstress and LAS/ EDTA buffer as well as for suc- AAPF- pNA plates, Biomek FX (Beckman Coulter), Spectramax Plus 384 MTP Reader (Molecular Devices), iEMS Incubator/ Shaker (1 mm amplitude) from Thermo Electron Corporation, sealing tape: Nunc (236366)

The iEMS incubator/shaker (Thermol/Labsystems) was set at 29°C. Culture supernatants were diluted into plates containing unstress buffer to a concentration of ~ 25 ppm (master dilution plate). 20 μl of sample from the master dilution plate was added to plates containing 180 μl unstress buffer to give a final incubation concentration of 2.5 ppm. The contents were mixed and kept at room temperature and a AAPF assay was performed on this plate. 20 μl of sample from the master dilution plate was also added to plates containing 180 μl stress buffer (50 mM HEPES (11.9 g/l), 0.1% (w/v) DOBS (1 g/l), 10 mM EDTA (3.36 g/l), pH 8.0). The solutions were mixed and immediately placed in 29°C iEMS shaker for 30 min at 400 rpm. Following 30 minutes of incubation, a AAPF assay was performed on the stress plate. The stability of the samples was determined by calculating the ration of the residual and initial AAPF activity as follows: Residual Activity (%) - [mOD.min-1 stressed]*100 / [mOD. min-1 unstressed].

**D. Cleaning Performance Assays**

The stain removal performance of the protease variants was determined in commercially available detergents. Heat inactivation of commercial detergent formulas serves to destroy the enzymatic activity of any protein components while retaining the properties of non-enzymatic components. Thus this method was suitable for preparing commercially purchased detergents for use in testing the enzyme variants of the present invention.

**Microswatches:**

Microswatches of ¼" circular diameter were obtained from CFT. Single microswatches or two microswatches were placed vertically in each well of a 96-well MTP to expose the whole surface area (*i.e.*, not flat on the bottom of the well).

**BMI Microswatch Assay**

Microswatches containing blood milk and ink (BMI) of 0.25 inch circular diameter were obtained from CFT. Before cutting of the swatches, the fabric (EMPA 116) was washed with water. One microswatch was vertically placed in each well of a 96-well microtiter plate in order to expose the whole surface area (*i.e.*, not flat on the bottom of the well). The desired detergent solution was prepared as described herein. After equilibrating the Thermomixer at 25°C, 190 μl of detergent solution was added to each well of the MTP, containing microswatches. To this mixture. 10 μl of the diluted enzyme solution was added so that the final enzyme concentration was 1 μg/ml (determined from BCA assay). The MTP was sealed with tape and placed in the incubator for 30 minutes, with agitation at 1400 rpm. Following incubation under the appropriate conditions, 100 μl of the solution from each well was transferred into a fresh MTP. The new MTP containing 100 μl of solution/well was read at 405 nm using a MTP SpectraMax reader. Blank controls, as well as a control containing two microswatches and detergent but no enzyme were also included.

**"Pre-washed" Swatch**

This type of microswatch was pre-washed in deionised water for 20 minutes at ambient temperature. After the pre-washing step, the swatches were put on top of paper towels to dry. The airdried swatches were then punched using a ¼" circular die on an expulsion press. Finally two microswatches were put into each well of a 96-well MTP vertically to expose the whole surface area (*i.e.* not flat on the bottom of the well).

**Detergents**

For North American (NA) and Western European (WE) heavy duty liquid laundry (HDL) detergents, heat inactivation was performed by placing pre-weighed liquid detergent (in a glass bottle) in a water bath at 95°C for 2 hours. The detergents were purchased from local supermarket stores. Both un-heated and heated detergents were assayed within 5 minutes of dissolving the detergent to accurately determine percentage deactivated. Enzyme activity was tested by the AAPF assay.

For testing of enzyme activity in heat-inactivated detergents, working solutions of detergents were made from the heat inactivated stocks. Appropriate amounts of water hardness (6 gpg or 12 gpg) and buffer were added to the detergent solutions to match the desired conditions. The solutions were mixed by vortexing or inverting the bottles.

**Enzymes and Equipment**

Samples of reference serine proteases variants thereof were obtained from filtered culture broth of cultures grown in MTP plates. The equipment used was a Biomek FX Robot (Beckman Coulter), a SpectraMAX MTP Reader (type 340; Molecular Devices), an iEMS incubator/shaker (Thermo/Labsystems); F-bottom MTPs (Costar type 9017 used for reading reaction plates after incubation); and V-bottom MTPs (Greiner 651101 used for pre-dilution of supernatant. In this assay, the proteases hydrolyze the substrate and liberate pigment and insoluble particles from the substrate. Thus the rate of turbidity is a measure of enzyme activity.

The stain removal performance of reference serine proteases and variants therefrom on microswatches was determined on a MTP scale in commercially available heat-inactivated detergent. The reagents used were: 5 mM HEPES, pH 8.0 or 5 mM MOPS, pH 7 buffer, 3:1 Ca: Mg for medium water hardness. ($CaCl_2$: $MgCl_2 \cdot 6H2O$); 15000 grains per gallon (gpg) stock diluted to 6 gpg, 2 BMI (blood/milk/ink) swatches per plate: EMPA-116 BMI cotton swatches processed by CFT: pre-rinsed and punched 2 swatches per well, and heat inactivated TIDE® 2X Cold off-the-shelf detergent in which lack of protease activity was confirmed.

| Table 1-2 Working Detergent Solutions | | | | | | |
|---|---|---|---|---|---|---|
| **Detergent** | **Temp (C)** | **Detergent g/L** | **pH** | **Buffer** | **Gpg** | **Protease** |
| TIDE® 2X Cold | 16 | 0.98 | 8 | 5mM HEPES | 6 | BPN’ |
| TIDE® 2X Cold | 32 | 0.98 | 8 | 5mM HEPES | 6 | BPN’ |
| TIDE® 2X Cold | 16 | 0.98 | 7 | 5mM MOPS | 6 | BPN’ |

The incubator was set at the desired temperature (16°C or 32°C). 10 $\mu$L samples from the master dilution plate of ~10 ppm enzyme was added to BMI 2-swatch plates with 190 $\mu$L working detergent solutions listed above. The volume was adjusted to give final concentration of 0.5 ppm for variants in the assay plates. The plates were immediately transferred to iEMS incubators and incubated for 30 minutes with 1400 rpm shaking at given temperature. Following incubation, 100 $\mu$L of supernatant was transferred into a new 96-well plate and the absorbance was measured in MTP Reader at 405nm and/or 600nm. Control wells, containing 1 or 2 microswatches and detergent without the addition of protease samples were also included in the test. The measurement at 405 nm provides a higher value and tracks pigment removal, while the measurement at 600 nm tracks turbidity and cleaning.

**Calculation of the Stain Removal Activity for All Microswatch Assay Methods:**

The absorbance value obtained was corrected for the blank value (substrate without enzyme), providing a measure of hydrolytic activity. For each sample (variant) the performance index was calculated. The performance index compares the performance of the variant (actual value) and the standard enzyme (theoretical value) at the same protein concentration. In addition, the theoretical values can be calculated, using the parameters of the Langmuir equation of the standard enzyme.

## E. Relative Specific Activity of Proteases and Variants Thereof

In order to discriminate the protease variants, the relative specific activity was calculated using suc-AAPF-pNA as a substrate, which enabled the comparison and ranking of the variants versus the wild-type or standard protease. The specific activity on the suc-AAPF-pNA substrate was determined by dividing the proteolytic activity by the measured TCA-values of each sample, using the assays described above. Using these values, the relative specific activity was calculated (specific activity of variant/specific activity of reference protease).

## F. Performance Index

The performance index compares the performance of the variant (actual value) and the standard or reference protease (theoretical value) at the same protein concentration. In addition, the theoretical values can be calculated, using the parameters of the Langmuir equation of the standard protease. A performance index (PI) that is greater than 1 (PI>1) identifies a better variant as compared to the standard (*e.g.*, wild- type), while a PI of 1 (PI=1) identifies a variant that performs the same as the standard, and a PI that is less than 1 (PI<1) identifies a variant that performs worse than the standard. Thus, the PI identifies winners, as well as variants that are less desirable for use under certain circumstances.

## EXAMPLE 2

### Targeted ISD (Insertion Substitution Deletion) Library Construction

A PCR-based method was used to create a library of modified *B. amyloliquefaciens* subtilisin BPN'-Y217L (commercially available as PURAFECT® PRIME subtilisin) polynucleotides containing in-frame insertions, deletions and/or substitutions in two regions of BPN'-Y217L (positions 63-77 and 92-132 of the mature region) as shown in Figure 1 (*See,* Pisarchik et al., Prot. Eng. Des. Select., 20:257-265 [2007]). Two sets of oligonucleotides that evenly cover the targeted regions of the BPN'-Y217L gene of a full-length protein of 392 amino acids, in both forward and reverse direction were used to amplify the 5' and 3' segments of the portion of the BPN'-Y217L gene. The coding region of the BPN'-Y217L mature protease contains the *Kpn*I and *Xho*I restriction sites for cloning purposes:

*gaattcatctcaaaaaaatgggtctactaaaatattattccatctattataataaattcacagaatagtcttttaagtaagtctactctgaatttttta*
*aaaggagagggtaaagagtgagaagcaaaaaatt*gtgagaagcaaaaaattgtggatcagtttgctgtttgctttagcgttaatctttacgatggc
gttcggcagcacatccagcgcgcaggctgcagggaaatcaaacggggaaaagaaatatattgtcgggtttaaacagacaatgagcacgatgagc
gccgctaagaagaaagacgtcatttctgaaaaaggcgggaaagtgcaaaagcaattcaaatatgtagacgcagctagcgctacattaaacgaaaa
agctgtaaaagaattgaaaaaagacccgagcgtcgcttacgttgaagaagatcacgtagcacacgcgtac**gcgcagtccgtgccatatggcgta**
**tcacaaattaaagcccctgctctgcactctcaaggctacaccggttcaaatgttaaagtagcggttatcgacagcggtatcgattcttctcat**
**ccagatcttaaagtagcaggcggagccagcatggttccttctgaaacaaatcctttccaagacaacaactctcacggaacacacgttgctg**
**gtaccgttgcggctcttaataactcaatcggtgtattaggcgttgcgccaagcgcatcactttacgctgtaaaagttctcggcgccgacggtt**
**ccggccaatacagctggatcattaacggaatcgagtgggcgatcgcaaacaatatggacgttattaacatgagcctcggcggaccgtccg**
**gttctgctgctttaaaagcggcagttgataaagccgttgcatccggcgtcgtagtcgttgcggcagccggcaacgaaggcacttccggcag**
**ctcaagcacagtgggctaccctggtaaatacccttctgtcattgcagtaggcgctgtcgacagcagcaaccaaagagcatctttctcaagc**
**gtaggacctgagctcgatgtcatggcacctggcgtatctatccaaagcacgcttcctggaaacaaatacggcgcgttgaacggtacatcaa**
**tggcatctccgcacgttgccggagccgcggctttgattctttctaagcacccgaactggacaaacactcaagtccgcagctctctagaaaac**
**accactacaaaacttggtgattctttctactatggaaaagggctgatcaatgtacaggcggcagctcagtaa***aactcgagataaaaaaccg*
*gccttggccccgccggttttttat* (SEQ ID NO:3).

The amino acid sequence of the BPN'-Y217L precursor protein is provided below. In this sequence, bold indicates the mature BPN'-Y217L protease:

MRSKKLWISLLFALALIFTMAFGSTSSAQAAGKSNGEKKYIVGFKQTMSTMSAAKKKDVISE
KGGKVQKQFKYVDAASATLNEKAVKELKKDPSVAYVEEDHVAHAY**AQSVPYGVSQIKAP**
**ALHSQGYTGSNVKVAVIDSGIDSSHPDLKVAGGASMVPSETNPFQDNNSHGTHVAGTVA**
**ALNNSIGVLGVAPSASLYAVKVLGADGSGQYSWIINGIEWAIANNMDVINMSLGGPSGS**
**AALKAAVDKAVASGVVVVAAAGNEGTSGSSSTVGYPGKYPSVIAVGAVDSSNQRASFSS**
**VGPELDVMAPGVSIQSTLPGNKYGALNGTSMASPHVAGAAALILSKHPNWTNTQVRSSL**
**ENTTTKLGDSFYYGKGLINVQAAAQ** (SEQ ID NO:4).

The amino acid sequence of the mature BPN'-Y217L protease was used as the basis for making the variant libraries described herein:

**AQSVPYGVSQIKAPALHSQGYTGSNVKVAVIDSGIDSSHPDLKVAGGASMVPSETNPFQD NNSHGTHVAGTVAALNNSIGVLGVAPSASLYAVKVLGADGSGQYSWIINGIEWAIANNM DVINMSLGGPSGSAALKAAVDKAVASGVVVVAAAGNEGTSGSSSTVGYPGKYPSVIAVG AVDSSNQRASFSSVGPELDVMAPGVSIQSTLPGNKYGALNGTSMASPHVAGAAALILSK HPNWTNTQVRSSLENTTTKLGDSFYYGKGLINVQAAAQ** (SEQ ID NO:5).

Each oligonucleotide contained about 20-24 nucleotides necessary for annealing to the template, targeted codons (with or without degeneracy) and the Eam1 104I recognition sequence. Every following primer was moved 3 bp downstream until the end of the targeted region was reached. Two PCR reactions (5' and 3' segments) contained either the 5' forward or the 3' reverse gene sequence flanking oligonucleotides each in combination with the corresponding oppositely priming oligonucleotides. The 5' fragments were amplified from pAC-FNA10 plasmid (*See,* Figure 2A) using a single forward primer (P3203, TGGATCAGTTTGCTGTTTGCT; SEQ ID NO:6) and reverse primers P4385-P4441 (*See,* Table 2-1) each containing the *Eam*1 104I restriction site. The 3' fragments were amplified using a single reverse primer (P3435, ATGTATCAAGATAAGAAAGAACAAG; SEQ ID NO:7) and forward primers P4328-P4384 (*See,* Table 2-1) each containing an *Eam*1 104I restriction site.

| Table 2-1. Primers Used for BPN'-Y217L Library Construction | | |
|---|---|---|
| SEQ ID NO. | PRIMER NAME | PRIMER SEQUENCE |
| 8 | P4328 | AAACTCTTCANDTNDTCACGGAACTCACGTTGCCGGCACA |
| 9 | P4329 | AAACTCTTCANDTNDTGGAACTCACGTTGCCGGCACAGTT |
| 10 | P4330 | AAACTCTTCANDTNDTNACTCACGTTCCCUCCACACTTG |
| 11 | P4331 | AAACTCTTCANDTNDTCACGTTGCCGGCACAGTTGCGGCT |
| 12 | P4332 | AAACTCTTCANDTNDTGTTGCCGGCACAGTTGCGGCTCTT |
| 13 | P4333 | AAACTCTTCANDTNDTCCCUCCACAGTTGCCCCTCTTAAT |
| 14 | P4334 | AAACTCTTCANDTNDTCCGCACAGTTCGCCTCTTAATAAC |
| 15 | P4335 | AAACTCTTCANDTNDTACAGTTGCGGGCTCTTAACTCA |
| 16 | P4336 | AAACTCTTCANDTNDTGTTGCGGCTCTTAATAACTCAATC |
| 17 | P4337 | AAACTCTTCANDTNDTGCGGCTCTTAATAACTCAATCGGT |
| 18 | P4338 | AAACTCTTCANDTNDTGCTCTTAATAACTCAATCGGTGTA |
| 19 | P4339 | AAACTCTTCANDTNDTCTTAATAACTCAATCUGTGTATTA |
| 20 | P4340 | AAACTCTTCANDTNDTAATAACTCAATCCCTGTATTAG |
| 21 | P4341 | AAACTCTTCANDTNDTAACTCAATCGGTGTATTAGGCGTT |
| 22 | P4342 | AAACTCTTCANDTNDTTCAATCGGTGTATTAGGCGTTG |
| 23 | P4343 | AAACTCTTCANDTTCAATCGGTGTATTAGGCGTTG |
| 24 | P4344 | AAACTCTTCANDTNDTAAAGTTCTCGGTGCTGACGGTTG |
| 25 | P4345 | AAACTCTTCANDTNDTGTTCTCGGTGCTGACGGTTCC |
| 26 | P4346 | AAACTCTTCANTTCNDTCTCGGTGCTGACGGTTCCGGCCAA |
| 27 | P4347 | AAACTCTTCANDTNDTGGTGCTGACGGTTCCGGCCAATAC |
| 28 | P4348 | AAACTCTTCANDTNDTGCTGACGGTTCCGGCCAATACA |
| 29 | P4349 | AAACTCTTCANDTNDTGACGGTTCCGGCCAATACAGCTG |
| 30 | P4350 | AAACTCTTCANDTNDTCGTCCGGCCAATACACCTCCATC |
| 31 | P4351 | AAACTCTTCANDTNDTTCCGGCCAATACAGCTGGATCATT |

(continued)

| Table 2-1. Primers Used for BPN'-Y217L Library Construction | | |
|---|---|---|
| SEQ ID NO. | PRIMER NAME | PRIMER SEQUENCE |
| 32 | P4352 | AAACTCTTCANDTNDTGGCCAATACAGCTGGATCATTAAC |
| 33 | P4353 | AAACTCTTCANTTNDTCAATACAGCTGGATCATTAACGGA |
| 34 | P4354 | AAACTCTTCANDTNDTTACAGCTGGATCATTAACGGAATC |
| 35 | P4355 | AAACTCTTCANDTNDTAGCTGGATCATTAACGGAATCGAG |
| 36 | P4356 | AAACTCTTCANDTNDTTGGATCATTAACGGAATCGAGTG |
| 37 | P4357 | AAACTCTTCANDTNDTATCATTAACGGAATCGAGTG |
| 38 | P4358 | AAACTCTTCANDTNDTATTAACGGAATCGAGTGGGCGATC |
| 39 | P4359 | AAACTCTTCANDTNDTAACGGAATCGAGTGGGCGATCGCA |
| 40 | P4360 | AAACTCTTCANDTNDTGGAATCGAGTGGGCGATCGCAAAC |
| 41 | P4361 | AAACTCTTCANDTNDTATCCAGTCGCCGATCCCAAAGAAT |
| 42 | P4362 | AAACTCTTCANDTNDTGAGTGGGCGATCGCAAACAATATG |
| 43 | P4363 | AAACTCTTCANDTNDTTGGGCGATCGCAAACAATATGGAC |
| 44 | P4364 | AAACTCTTCANDTNDTGCGATCGCAAACAATATGGACGTT |
| 45 | P4365 | AAACTCTANDTNDTATCGCAAACAATATGGACGTTATT |
| 46 | P4366 | AAACTCTTCANDTNDTGCAAACAATATGGACGTTATTAAC |
| 47 | P4367 | AAACTCTTCANDTNDTAACAATATGGACGTTATTAACATG |
| 48 | P4368 | AAACTCTTCANDTNDTAATATGGACGTTATTAACATGAG |
| 49 | P4369 | AAACTCTTCANDTNDTATGGACGTTATTAACATGAGCCTC |
| 50 | P4370 | AAACTCTTCANDTNDTGACGTTATTAACATGAGCCTC |
| 51 | Y4371 | AAACTCTTCANDTNDTGTTATTAACATGAGCCTCGGCGGA |
| 52 | P4372 | AAACTCTTCANDTATTAACATGAGCCTCGGCGGACCT |
| 53 | P4373 | AAACTCTTCANDTNDTAACATGAGCCTCGGCGGACCTTC |
| 54 | P4374 | AAACTCTTCANDTNDTATGAGCCTCGGCGGACCTTCTGGT |
| 55 | P4375 | AAACTCTTCANDTNDTAGCCTCGGCGGACCTTCTGGTCT |
| 56 | P4376 | AAACTCTTCANDTNDTCTCGGCGGACCTTCTGGTTCTGCT |
| 57 | Y4377 | AAACTCTTCANDTNDTGGCGGACCTTCTGGTCTGCTGCT |
| 58 | P4378 | AAACTCTTCANDTNDTGGACCTTCTGGTTCTGCTGCTTTA |
| 59 | P4379 | AAACTCTTCANDTNDTCCTTCTGGTTCTGCTGCTTTAAAA |
| 60 | P4380 | AAACTCTTCANDTNDTTCTGGTTCTGCTGCTTTAAAAG |
| 61 | P4381 | AAACTCTTCANDTNDTGGTTCTCCTCCTTTAAAAGCGGCA |
| 62 | P4382 | AAACTCTTCANDTNDTTCTGCTGCTTTAAAAGCGGCAGTT |
| 63 | P4383 | AAACTCTTCANDTNDTGCTGCTTTAAAAGCGGCAGTTGAT |
| 64 | P4384 | AAACTCTTCANDTGCTGCTTTAAAAGCGGCAGTTGAT |
| 65 | P4385 | AAACTCTTCAAHNGTTGTGTTGGAAAGGATTTGTTTC |
| 66 | P4386 | AAACTCTTCAAHNAHNGTTGTCTTGGAAAGGATTTGTTTC |
| 67 | P4387 | AAACTCTTCAAHNAHNGTTGTTGTCTTGGAAAGGATTTGT |
| 68 | P4388 | AAACTCTTCAAHNAHNAGAGTTGTTGTCTTGGAAAGGATT |

(continued)

| Table 2-1. Primers Used for BPN'-Y217L Library Construction | | |
|---|---|---|
| SEQ ID NO. | PRIMER NAME | PRIMER SEQUENCE |
| 69 | P4389 | AAACTCTTCAAHNAHNGTGAGAGTTGTTGTCTTGGAAAGG |
| 70 | P4390 | AAACTCTTCAAHNAHNTCCGTGAGAGTTGTTGTCTTGGAA |
| 71 | P4391 | AAACTCTTCAAHNAHNAGTTCCGTGAGAGTTGTTGTCTTG |
| 72 | P4392 | AAACTCTTCAAHNAHNGTGAGTTCCGTGAGAGTTGTTGTC |
| 73 | P4393 | AAACTCTTCAAHNAHNAACGTGAGTTCCGTGAGAGTTGTT |
| 74 | P4394 | AAACTCTTCAAHNAHNGGCAACGTGAGTTCCGTGAGAGTT |
| 75 | P4395 | AAACTCTTCAAHNAHNGCCGGCAACGTGAGTTCCGTGAGA |
| 76 | P4396 | AAACTCTTCAAHNAHNTGTGCCGGCAACGTGAGTTCCGTG |
| 77 | P4397 | AAACTCTTCAAHNAHNAACTGTGCCGGCAACGTGAGTTCC |
| 78 | P4398 | AAACTCTTCAAHNAHNCGCAACTGTGCCGCCAACGTGAGT |
| 79 | P4399 | AAACTCTTCAAHNAHNAGCCGCAACTGTGCCGGCAACGTG |
| 80 | P4400 | AAACTCTTCAAHNAHNAAGAGCCGCAACTGTGCCGGCAAC |
| 81 | P4401 | AAACTCTTCAAHNCTAAAGTGATGCGCTTGGCGCAAC |
| 82 | P4402 | AAACTCTTCAAHNAHNGTAAAGTGATGCGCTTGUCGCAAC |
| 83 | P4403 | AAACTCTTCAAHNAHNAGCGTAAAGTGATGCGCTTG |
| 84 | P4404 | AAACTCTTCAAHNAHNTACAGCGTAAAGTGATGCGCTTG |
| 85 | P4405 | AAACTCTTCAAHNAHNTTTTACAGCGTAAAGTGATGCGCT |
| 86 | P4406 | AAACTCTTCAAHNAHNAACTTTTACAGCGTAAAGTGATG |
| 87 | P4407 | AAACTCTTCAAHNAHNGAGAACTTTTACAGCGTAAAGTGA |
| 88 | P4408 | AAACTCTTCAAHNAHNACCGAGAACTTTTACAGCGTAAAG |
| 89 | P4409 | AAACTCTTCAAHNAHNAGCACCGAGAACTTTTACAGCGTA |
| 90 | P4410 | AAACTCTTCAAHNAHNGTCAGCACCGAGAACTTTTACAG |
| 91 | P4411 | AAACTCTTCAAHNAHNACCGTCAGCACCGAGAACTTTTAC |
| 92 | P4412 | AAACTCTTCAAHNAHNGGAACCGTCAGCACCGAGAACTTT |
| 93 | P4413 | AAACTCTTCAAHNAHNGCCGGAACCGTCAGCACCGAGAAC |
| 94 | P4414 | AAACTCTTCAAHNAHNTTGGCCGGAACCGTCAGCACCGAG |
| 95 | P4415 | AAACTCTTCAAHNAHNGTATTGGCCCGAACCGTCAGCAC |
| 96 | P4416 | AAACTCTTCAAHNAHNGCTGTATTGGCCGGAACCGTCAG |
| 97 | P4417 | AAACTCTTCAAHNAHNCCAGCTGTATTGGCCGGAACCGTC |
| 98 | P4418 | AAACTCTTCAAHNAHNGATCCAGCTGTATTGGCCGGAAC |
| 99 | P4419 | AAACTCTTCAAHNAHNAATGATCCAGCTGTATTGGCCGGA |
| 100 | P4420 | AAACTCTTCAAHNAHNGTTAATGATCCAGCTGTATTG |
| 101 | P4421 | AAACTCTTCAAHNAHNTCCGTTAATGATCCAGCTGTATTG |
| 102 | P4422 | AAACTCTTCAAHNAHNGATTCCGTTAATGATCCAGCTGTA |
| 103 | P4423 | AAACTCTTCAAHNAHNCTCGATTCCGTTAATGATCCAGCT |
| 104 | P4424 | AAACTCTTCAAHNAHNCCACTCGATTCCGTTAATGATCCA |
| 105 | P4425 | AAACTCTTCAAHNAHNCGCCCACTCGATTCCGTTAATGAT |

(continued)

| Table 2-1. Primers Used for BPN'-Y217L Library Construction | | |
|---|---|---|
| SEQ ID NO. | PRIMER NAME | PRIMER SEQUENCE |
| 106 | P4426 | AAACTCTTCAAHNAHNGATCGCCCACTCGATTCCGTTAAT |
| 107 | P4427 | AAACTCTTCAAHNAHNTGCGATCGCCCACTCGATTCCGTT |
| 108 | P4428 | AAACTCTTCAAHNAHNGTTTGCGATCGCCCACTCGATTCC |
| 109 | P4429 | AAACTCTTCAAHNAHNATTGTTTGCGATCGCCCACTCGAT |
| 110 | P4430 | AAACTCTTCAAHNAHNCATATTGTTTGCGATCGCCCACTC |
| 111 | P4431 | AAACTCTTCAAHNAHNGTCCATATTGTTTGCGATCGCCCA |
| 112 | P4432 | AAACTCTTCAAHNAHNAACGTCCATATTGTTTGCGATCGC |
| 113 | P4433 | AAACTCTTCAAHNAHNAATAACGTCCATATTGTTTGCGAT |
| 114 | P4434 | AAACTCTTCAAHNAHNGTTAATAACGTCCATATTGTTTGC |
| 115 | P4435 | AAACTCTTCAAHNAHNCATGTTAATAACGTCCATATTGTT |
| 116 | P4436 | AAACTCTTCAAHNAHNGCTCATGTTAATAACGTCCATATT |
| 117 | P4437 | AAACTCTTCAAHNAHNGAGGCTCATGTTAATAACCTCCAT |
| 118 | P4438 | AAACTCTTCAAHNAHNGCCGAGGCTCATCTTAATAACCTC |
| 119 | P4439 | AAACTCTTCAAHNAHNTCCGCCGAGGCTCATGTTAATAAC |
| 120 | P4440 | AAACTCTTCAAHNAHNAGGTCCGCCGAGGCTCATGTTAAT |
| 121 | P4441 | AAACTCTTCAAHNAHNAGAAGGTCCGCCGAGGCTCATGTT |

Each amplification reaction contained 30pmol of each oligonucleotide and 100 ng of pAC-FNA10 template DNA. Amplifications were carried out using Vent DNA polymerase (New England Biolabs). The PCR mix (20 ul) was initially heated at 95°C for 2.5 min followed by 30 cycles of denaturation at 94°C for 15s, annealing at 55°C for 15s and extension at 72°C for 40s. Following amplification, left and right fragments generated by the PCR reactions were gel-purified and mixed (about 200 ng of each fragment). Every mix contained three left fragments targeting three adjacent codons and three right fragments targeting same codons. These mixes were digested with *Eam*1104I, ligated with T4 DNA ligase and amplified by flanking primers (P4299 CGTTAAGAAGATCACGTAGCA; SEQ ID NO:122, and P3246 TTTATTTTATAAACTCATTCCCTGAT; SEQ ID NO:123). The resulting fragments were digested with *Mlu*I and *Xho*I, and cloned into the *Miu*I/*Xho*I sites in the *Bacillus subtilis* expression plasmid pHPLT- FNA (Figure 2B). The BPN-Y217L expression cassette from the pHPLT vector used has the polynucleotide sequence shown below.

The polynucleotide sequence of the expression cassette from the pHPLT vector (Plat promoter- pre- pro- BPN'-Y217L- terminator) (SEQ ID NO. 124) is shown below:

GCTTTTCTTTTGGAAGAAAATATAGGGAAAATGGTACTTGTTAAAAATTCGGAATATTTA
TACAATATCATATGTTTCACATTGAAAGGGGAGGAAAATCGTGAAACAACAAAAACGGC
TTTAGTCTAGCAAAAGGAGAGGGTAAAGAGTGAGAAGCAAAAAATTGTGGATCAGTTTG
CTGTTTGCTTTAGCGTTAATCTTTACGATGGCGTTCGGCAGCACATCCTCTGCCCAGGCGG
CAGGGAAATCAAACGGGGAAAAGAAATATATTGTCGGGTTTAAACAGACAATGAGCAC
GATGAGCGCCGCTAAGAAGAAAGATGTCATTTCTGAAAAAGGCGGGAAAGTGCAAAAG
CAATTCAAATATGTAGACGCAGCTTCAGCTACATTAAACGAAAAAGCTGTAAAAGAATT
GAAAAAAGACCCGAGCGTCGCTTACGTTGAAGAAGATCACGTAGCACACGCGTACGCGC
AGTCCGTGCCTTACGGCGTATCACAAATTAAAGCCCCTGCTCTGCACTCTCAAGGCTACA
CTGGATCAAATGTTAAAGTAGCGGTTATCGACAGCGGTATCGATTCTTCTCATCCTGATT
TAAAGGTAGCAGGCGGAGCCAGCATGGTTCCTTCTGAAACAAATCCTTTCCAAGACAAC
AACTCTCACGGAACTCACGTTGCCGGCACAGTTGCGGCTCTTAATAACTCAATCGGTGTA
TTAGGCGTTGCGCCAAGCGCATCACTTTACGCTGTAAAAGTTCTCGGTGCTGACGGTTCC
GGCCAATACAGCTGGATCATTAACGGAATCGAGTGGGCGATCGCAAACAATATGGACGT
TATTAACATGAGCCTCGGCGGACCTTCTGGTTCTGCTGCTTTAAAAGCGGCAGTTGATAA
AGCCGTTGCATCCGGCGTCGTAGTCGTTGCGGCAGCCGGTAACGAAGGCACTTCCGGCA

GCTCAAGCACAGTGGGCTACCCTGGTAAATACCCTTCTGTCATTGCAGTAGGCGCTGTTG
ACAGCAGCAACCAAAGAGCATCTTTCTCAAGCGTAGGACCTGAGCTTGATGTCATGGCA
CCTGGCGTATCTATCCAAAGCACGCTTCCTGGAAACAAATACGGCGCGTTGAACGGTAC
ATCAATGGCATCTCCGCACGTTGCCGGAGCGGCTGCTTTGATTCTTTCTAAGCACCCGAA
CTGGACAAACACTCAAGTCCGCAGCAGTTTAGAAAACACCACTACAAAACTTGGTGATT
CTTTCTACTATGGAAAAGGGCTGATCAACGTACAGGCGGCAGCTCAGTAAACTCGAGAG
AGGACGGATTTCCTGAAGGAAATCCGTTTTTTTATTTTAAGCTTG (SEQ ID NO:124)

Ligation mixtures were amplified using rolling circle amplification according to manufacturer's recommendation (Epicentre). One ul of the ligation mixture was mixed with 5 ul of the sample buffer, heated to 95°C for 3 min and cooled on ice. Next, 5 ul of the reaction buffer and 0.2 ul of the enzyme were added to each tube, followed by incubation at 30°C for 10 hours. Products of the rolling circle amplification were diluted 100 times and used to transform *Bacillus subtilis. Bacillus subtilis* cells (genotype: *ΔaprE, ΔnprE, ΔspoHE, amyE::xylRPxylAcomK-phleo)* made competent by the induction of the *comK* gene under control of a xylose inducible promoter (*See e.g.,* Hahn et al., Mol Microbiol, 21:713-775 [1996]) were transformed with 45 libraries containing DNA sequences encoding BPN'-Y217L protease with mutated regions. The cells were grown in 1 ml of Luria Broth (LB) at 37°C for 1 hour and then plated on LB plates containing 1.6% skim milk and 10 mg/l neomycin, and were incubated overnight at 37°C. At least 2000 clones from each of the 45 libraries were screened for producing halos. When plated on skim milk plates, 0.05% to 20% of clones generated halos. A limited number of the clones generating halos were sequenced. These clones varied in the ratio of insertions and deletions, but none of them had frame shift mutations. Clones generating halos were further screened for AAPF activity using a 96-well plate assay as described in Example 1. The BPN-Y217L variant proteins were produced by growing the *B. subtilis* transformants in 96 well microtiter plates as described earlier. Protein concentration of culture supernatants was determined by TCA precipitation as described in Example 1.

## EXAMPLE 3

### Stain Removal Performance of BPN'-Y217L Variants Generated by Targeted ISD

Results of experiments conducted to determine stain removal activity (microswatch assay to determine stain removal performance in laundry applications using EMPA 116 swatches (BMI stain, CFT) at pH 8/16°C and protein determination by TCA precipitation (tests of properties of interest) of BPN'-Y217L variants generated as described above are shown in Table 3-1. The results were obtained using the methods described in Example 1, with the following modifications for the stain removal performance assay. The test detergent used was heat inactivated Tide 2X detergent (Procter & Gamble).

Heat inactivation of commercial detergent formulas serves to destroy the endogenous enzymatic activity of any protein components while retaining the properties of nonenzymatic components. Heat inactivation of the detergents was performed by placing pre-weighed amounts of liquid detergent (in a glass bottle) in a water bath at 95°C for 2 hours. The detergent was purchased from local supermarket stores. Both unheated and heated detergents were assayed within 5 minutes of dissolving the detergent to accurately determine percentage deactivated. Enzyme activity was tested

by AAPF assay. As described throughout functionality of BPN-Y217L variants was quantified as a performance index ("*Pi*" or "PI"), which is the ratio of performance of a variant to parent protein BPN'-Y217L. BPN-Y217L variants showing a PI value greater or equal than 0.5 for BMI stain removal performance and/or TCA precipitation showed improved cleaning benefits and/or expression.

Mutations are named by the one letter code for the parent amino acid, followed by a three digit position number and then the one letter code for the variant amino acid. For example, mutating glycine (G) at position 87 to serine (S) is represented as "G087S." Multiple mutations are indicated by inserting a "/" between the mutations. Mutations at positions 87 and 90 arc represented as "G087S/A090Y." For deletions, the one letter code "Z" is used. For an insertion relative to the parent sequence, the one letter code "Z" is on the left side of the position number. For a deletion, the one letter code "Z" is on the right side of the position number. For insertions, the position number is the position number before the inserted amino acid(s), plus 0.01 for each amino acid. For example, an insertion of three amino acids alanine (A), serine (S) and tyrosine (Y) between position 87 and 88 is shown as "Z087.01A/Z087.02S/Z087.03Y." Thus, combining all the mutations above plus a deletion at position 100 is: "G087S/Z087.01A/Z087.02S/Z087.03Y/A090Y/A100Z."

**Table 3-1. Mutations and Performance Index for TCA protein and BMI Activity for BPN'-Y217L Variants**

| Mutations | TCA PI | PI on BMI at pH 8 16°C |
|---|---|---|
| A001T/L096V/G097S/A098D/D099I | 0.45 | 1.1 |
| Λ001V/G097S/A098R/D099G | 0.66 | 0.74 |
| S003F | 1.08 | 0.89 |
| V004M/A098D/D099G | 0.69 | 0.97 |
| V004M/D099R/G100S/Z100.01L | 0.8 | 0.75 |
| V004M/G127C/G128S/P129R | 0.92 | 0.52 |
| V004M/MH9C/D120S | 0.68 | 1 |
| V008I | 0.97 | 0.985 |
| V008I/A098G/D099R | 0.67 | 0.66 |
| V008I/A098N | 0.52 | 1.14 |
| V008I/M119C/D120G | 0.53 | 0.99 |
| Q010I/G097R/A098C/D099S | 0.27 | 0.52 |
| Q010R | 0.91 | 0.86 |
| K012N/A098R/D099V | 0.46 | 0.8 |
| K012R/G097D/A098H/D099G | 0.86 | 0.96 |
| Λ013T/S101R/Q103S | 0.52 | 0.68 |
| Λ013V/G097N/Λ098H/D099G/G160S | 0.46 | 0.74 |
| Λ013V/G097S/Z097.01R/A098R | 0.46 | 0.64 |
| A013V/G100R/S101G | 0.43 | 0.79 |
| A013V/N118G/M119I | 0.5 | 0.85 |
| A013V/P129Z/S130H/S132G | 0.47 | 0.58 |
| P0141/N062D/S101R/Q103S | 0.41 | 1.02 |
| P014S/A098S/D099G | 0.56 | 0.99 |
| A015T/V068N/A069G | 0.52 | 0.38 |
| L016M/G128R/P129R/Z129.01G | 0.5 | 0.28 |
| H017Q/G127K/P129G | 0.54 | 0.43 |
| H017R | 0.5 | 1.025 |
| E1017Y/A098R/D099I | 0.42 | 0.56 |

(continued)

| Table 3-1. Mutations and Performance Index for TCA protein and BMI Activity for BPN'-Y217L Variants | | |
|---|---|---|
| **Mutations** | **TCA PI** | **PI on BMI at pH 8 16°C** |
| H017Y/A098R/D099Z/S101G | 0.6 | 0.47 |
| H017Y/P129R | 0.685 | 0.85 |
| H017Y/S130Y/G131S/S132L | 0.38 | 0.58 |
| Q019H/G128R/Z128.01R/P129D | 0.4 | 0.78 |
| Q019H/Z128.0G/Z128.02G | 0.56 | 0.29 |
| G020D/G097R/A098G/D099C | 0.39 | 0.8 |
| G020D/P129Z/S130G/G131R/S132C | 0.37 | 0.51 |
| N025Y/S101L/Q103N | 1.05 | 1.13 |
| A029V/G097R/A098S | 0.44 | 0.94 |
| S033G | 0.9 | 1.05 |
| S0331/T066G/H067R/V068D/A216E | 0.88 | 1.01 |
| S038F/A098R/D099S | 0.98 | 0.6 |
| V044I/G097C/A098D/D099G | 0.42 | 0.9 |
| V044I/G097R/A098R | 0.83 | 0.87 |
| V044I/G100S/S101G | 0.36 | 1.09 |
| A045V/G097C/A098K/D099S | 0.41 | 0.88 |
| A048V/G097L/A098G/D099S | 0.42 | 1 |
| A048V/G100R/S101G | 0.65 | 1.085 |
| A048V/G128S/P129L/Z129.01G | 0.45 | 0.68 |
| M050I/A098G | 0.2 | 0.54 |
| S053F/S130I/S132G | 0.65 | 0.54 |
| S053T/D099H/S101Z | 0.66 | 0.74 |
| E054D/A098R/D099G | 1.05 | 0.76 |
| T055I/S101R/Q103R | 0.58 | 0.51 |
| T055S/T066I/X066.01G/Z066.02G | 0.33 | 0.7 |
| Q059H/D099S/S101G | 0.36 | 0.79 |
| Q059H/M119S/D120R | 0.37 | 0.52 |
| Q059K/M119V/V121C | 0.23 | 0.58 |
| Q059L/P129G | 0.69 | 0.87 |
| D060N | 0.47 | 0.9 |
| N061D/G128R/P129V/Z129.0G | 0.59 | 0.43 |
| S063T/097D/A098R/D099S | 0.97 | 0.95 |
| G065R/Z101.01R/Z101.02G | 0.57 | 1.035 |
| T066S/H067S | 0.51 | 0.61 |
| V068C/A069G | 0.42 | 1.22 |
| V06HC/A069G/H238Q | 0.32 | 0.62 |
| V068G/A069G | 0.36 | 0.71 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| V068G/Λ069G/V150I | 0.33 | 0.59 |
| V068G/Λ069S | 0.34 | 0.63 |
| V068I/Λ069G/A144V | 0.46 | 0.98 |
| V068I/G097D/A098G/D099Z/G100R | 0.41 | 0.58 |
| V068L/A069G | 0.445 | 0.905 |
| V068L/Λ069S | 0.47 | 0.83 |
| V068S/A069G | 0.48 | 1.04 |
| V068S/A116T | 0.4 | 1.05 |
| A069G | 0.7 | 1.02 |
| A069G/T071G | 0.23 | 0.84 |
| A009G/T071I | 0.79 | 1 |
| L075G/N076G/N077Z | 0.29 | 0.77 |
| L075I | 0.78 | 0.94 |
| L075R/N076G/N077Z | 0.34 | 0.99 |
| N076D/D099R/Z100.01G | 0.28 | 0.63 |
| I079F/A098G | 1.16 | 1.16 |
| V081A/S101R/Q103V | 0.4 | 0.81 |
| V081I/G097R/A098G/D099C | 0.36 | 0.54 |
| V084D/G097S/A098G/D099N | 0.42 | 0.63 |
| V084I/G127C/P129G | 1.28 | 1.01 |
| V084I/G127L | 0.88 | 0.72 |
| A085V/A098G | 0.75 | 1.09 |
| A085V/G097R/A098S/D099N | 0.65 | 0.75 |
| A085V/G128S/P129R/Z129.01G | 0.61 | 0.47 |
| Λ085V/N218I | 0.435 | 0.845 |
| A085V/S101G | 0.59 | 0.84 |
| A085V/S204P | 0.43 | 0.78 |
| Λ085V/Z099.01H/S101R | 0.46 | 0.6 |
| P086S/G131D/S132G | 0.25 | 0.64 |
| P086T/S101D/Q103S/V147I | 0.35 | 1.27 |
| A088S/S101R/Q103V | 0.63 | 0.88 |
| A088T | 0.855 | 1.01 |
| Λ088T/A098R/D099R | 0.73 | 0.36 |
| Λ088T/D099G/Z100.01G | 0.21 | 0.75 |
| A088T/G127N/P129R | 0.99 | 0.8 |
| A088T/G127S/T129R/Q206R/A273S | 1.22 | 0.67 |
| A088T/P1295/G146 | 0.74 | 1.16 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| A088T/S101G | 1.13 | 1.08 |
| A088T/S130R/G131H/S132N | 1.24 | 0.78 |
| A088V/G100Z/S101C/Q103R | 0.52 | 0.46 |
| A088V/P129Z | 0.61 | 1.05 |
| A092G | 0.61 | 1.15 |
| Λ092G/S249R | 0.69 | 1.07 |
| A092G/V093C | 0.2 | 0.855 |
| A092G/V093I | 0.5 | 1.15 |
| Λ092G/V093L | 0.275 | 0.75 |
| Λ0921L/V093G/K094Z | 0.54 | 0.96 |
| Λ092S/K094V | 0.47 | 1.13 |
| A092S/V093C | 0.72 | 1.06 |
| A092S/V093G | 0.21 | 0.79 |
| Λ092S/V093S | 0.33 | 0.67 |
| A092V/V093L | 0.34 | 0.695 |
| V093I/G128D/P129R | 0.6 | 1.22 |
| V093I/T129Z | 0.6 | 0.92 |
| V093R/Z093.01G/K094R | 0.28 | 0.88 |
| K094E/V095D | 0.87 | 0.95 |
| K094G | 0.33 | 0.56 |
| K094S/V148I | 0.3 | 0.85 |
| L096I/N118R/M119C/D120G/A151V | 0.29 | 0.58 |
| Z096.01H/A098R | 0.66 | 1.2 |
| Z096.01H/A098G | 0.32 | 1.16 |
| Z096.01N/A098H | 0.65 | 1.05 |
| Z096.01R/A098C | 0.47 | 0.95 |
| Z096.01R/A098G | 0.39 | 0.82 |
| Z096.01R/A098G/V192I | 0.4 | 0.77 |
| Z096.0R/A098R | 0.60 | 0.70 |
| Z096.01R/A098R/V192A | 0.61 | 0.68 |
| Z096.01R/A098S | 0.47 | 0.92 |
| Z096.01R/A0985/G157S | 0.3 | 0.88 |
| Z096.01R/Z096.02G | 0.52 | 0.67 |
| Z096.01R/Z096.02G/V147I | 0.41 | 0.96 |
| Z096.01S/A098G | 0.44 | 1.12 |
| Z096.01S/A098N | 0.47 | 1.04 |
| G097C/A098D/D099S | 0.675 | 1.09 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| G097C/A098G/D099G | 0.42 | 0.86 |
| G097C/A098G/D099N | 0.82 | 1.07 |
| G097C/A098G/D099S | 0.45 | 0.95 |
| G097C/A098G/D099S/G100Z | 0.32 | 0.64 |
| G097C/A098G/D099V | 0.43 | 0.88 |
| G097C/A098N/D099S | 0.45 | 0.94 |
| G097C/A098R | 1.24 | 1.11 |
| G097C/A098R/D099G | 0.40 | 0.84 |
| G097C/A098R/D099H | 0.45 | 0.64 |
| G097C/A098R/D099H | 0.46 | 0.78 |
| G097C/A098R/D099N | 0.79 | 0.87 |
| G097C/A098R/D099R | 0.4 | 0.55 |
| G097C/A098R/D099R/N109D/A114S | 0.56 | 0.5 |
| G097C/A098S/099H/A151V | 0.32 | 0.65 |
| G097C/A098V/D099S | 0.2 | 0.81 |
| G097C/Z097.01G/A098H/T253I | 0.32 | 0.58 |
| G097C/Z097.01S/A098G | 0.45 | 1.04 |
| G097D/A098C | 1 | 1.03 |
| G097D/A098G/D099G | 0.89 | 0.985 |
| G097D/A09HG/D099R | 1.00 | 0.83 |
| G097D/A098H/D099R | 1.24 | 0.92 |
| G097D/A098H/D099V | 0.95 | 0.86 |
| G097D/A098R/D099G | 0.955 | 0.96 |
| G097D/A098R/D099R | 1.29 | 0.79 |
| G097D/A098R/D099S | 1.11 | 0.87 |
| G097D/A098R/D099Z | 0.255 | 1.09 |
| G097D/G100C/S101R | 1.15 | 0.86 |
| G097D/G100R/S101G | 0.47 | 0.85 |
| G0197H/A098C/D099G | 0.34 | 0.76 |
| G097H/A098D/D099N | 0.5 | 1.15 |
| G097H/A098G | 0.67 | 1.1 |
| 0097H/A098G/D099G | 0.38 | 0.78 |
| G097H/A098G/D099R | 0.42 | 0.73 |
| G097H/A098G/D099Z | 0.39 | 1.02 |
| G097H/A098G/D099Z/A151V | 0.37 | 0.52 |
| G097H/A098G/D099//G100R | 0.55 | 0.49 |
| G097H/A098G/D0997/G100V | 0.2 | 0.87 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| G097H/A098G/D099Z/S101N/G166S | 0.41 | 0.68 |
| G097H/A098R | 0.60 | 0.97 |
| G097H/A098R/D099G | 0.46 | 0.67 |
| G097H/A098R/D099L | 0.31 | 0.5 |
| G097H/A098R/D099N | 0.48 | 0.74 |
| G097H/A09HS/D099G | 0.76 | 0.84 |
| G097H/A098S/D099I | 0.35 | 0.55 |
| G097H/A098S/D099L | 0.36 | 0.83 |
| G097H/A098S/D099S/K237E | 0.53 | 0.88 |
| G097L/A098/D099S | 0.26 | 0.68 |
| G097L/A098G/D099S | 0.33 | 0.77 |
| G097L/7A098G/099S/G100Z | 0.28 | 0.53 |
| G097L/A098G/D099Z | 0.29 | 0.56 |
| G097L/A098H | 0.71 | 1.1 |
| G097L/A098R/D099N | 0.5 | 0.78 |
| G097L/A098V | 0.42 | 1.11 |
| G097N/A098G/D099C | 0.74 | 0.99 |
| C097N/A098G/D099R | 0.82 | 0.88 |
| G097N/A098G/D099R/V270I | 0.74 | 0.88 |
| G097N/A098G/D099S | 0.45 | 0.7 |
| G097N/A098G/D099V | 0.47 | 0.9 |
| G097N/A098G/D099V/A151V | 0.3 | 1.07 |
| G097N/A098G/D099V/S101F | 0.54 | 0.82 |
| G097N/A098H/D099N | 0.79 | 0.75 |
| G097N/A098R/D099R | 0.69 | 0.25 |
| G097N/A098R/D099S | 0.94 | 0.73 |
| G097N/A098R/D099V | 0.59 | 0.675 |
| G097N/A098S/D099S | 0.72 | 0.91 |
| G097R/A098C | 1.04 | 0.98 |
| G097R/A098C/D099S | 0.28 | 0.735 |
| G097R/A098F/A200V | 0.305 | 0.74 |
| G097R/A098G | 0.97 | 0.985 |
| G097R/A098C/D099C | 0.31 | 0.68 |
| G097R/A098G/D099G | 0.33 | 0.60 |
| G097R/A098G/D099G/A273T | 0.48 | 0.85 |
| G097R/A098G/D099N | 0.64 | 0.82 |
| G097R/A098G/D099S | 0.31 | 0.66 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| G097R/A098G/D099S/A133D | 0.26 | 0.61 |
| G097R/A098G/D099V | 0.275 | 0.55 |
| G097R/A098H/D99C/S182N | 0.38 | 0.75 |
| G097R/A098H/D099G | 0.47 | 0.695 |
| G097R/A098N/D099G | 0.32 | 1.05 |
| G097R/A098N/D099Z | 0.52 | 0.72 |
| G097R/A098R | 1.06 | 0.83 |
| G097R/A098R/D099C | 0.50 | 0.53 |
| G097R/A098R/D099G | 0.54 | 0.51 |
| G097R/A098R/D099S | 0.45 | 0.55 |
| G097R/A098S/D099C | 0.39 | 0.66 |
| G097R/A098S/D099G | 0.51 | 0.59 |
| G097R/A098S/D099L | 0.2 | 0.51 |
| G097R/A098S/D099R | 0.45 | 0.52 |
| G097R/A098S/D099S | 0.33 | 0.81 |
| G097R/A098S/D099V | 0.32 | 0.61 |
| G097R/A098V/D099G | 0.45 | 0.68 |
| G097R/Z097.01H/A098C | 0.56 | 0.81 |
| G097R/Z097.01R/A098G | 0.5 | 0.74 |
| G097R/Z097.01R/A098G/V147I | 0.56 | 0.74 |
| G097R/Z097.01R/A098S | 0.4 | 0.59 |
| G097R/Z097.01R | 0.39 | 0.6 |
| G097R/Z097.01S/A098G | 0.57 | 0.95 |
| G097R/Z097.01S/A098R | 0.53 | 0.7 |
| G097S/A098C | 0.87 | 1.06 |
| G097S/A098C/D099G | 0.47 | 1.09 |
| G097S/A098C/D099H | 0.62 | 1.02 |
| G097S/A098C/D099L | 0.45 | 0.96 |
| G097S/Λ098C/D099R | 0.575 | 0.79 |
| G097S/A098C/D099S | 0.71 | 1.15 |
| G097S/A098C/D099Z | 0.2 | 0.81 |
| G097S/A098G/D099F | 0.28 | 0.63 |
| G097S/A098G/D099G | 0.43 | 0.90 |
| G097S/A098G/D099G | 0.43 | 0.91 |
| G097S/A098G/D099S | 0.56 | 0.95 |
| G097S/A098G/D099Z | 0.3 | 0.94 |
| G097S/A098G/D099Z/G100R | 0.3 | 0.52 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| G097S/A098H/D099C | 0.46 | 0.84 |
| G097S/A098H/D099G | 0.42 | 0.97 |
| G097S/A098N/D099G | 0.53 | 1.14 |
| G097S/A098R | 1.06 | 0.94 |
| G097S/A098R/D099C | 0.45 | 0.7 |
| G097S/A098R/D099G | 0.63 | 0.76 |
| G097S/A098R/D099L | 0.55 | 0.715 |
| G097S/A098R/D099V | 0.27 | 0.63 |
| G097S/A098R/D099Y | 0.44 | 0.74 |
| G097S/A098S/D099G | 0.65 | 0.945 |
| G097S/A098S/D099G/A133V/A153V/S236Y | 0.48 | 0.69 |
| G097S/A098S/D099G/A153V | 0.46 | 0.82 |
| G097S/A098S/D099Z | 0.875 | 0.76 |
| G097S/Z097.01D/A098G/A144T/Q271H | 0.46 | 1.04 |
| G097S/Z097.01G/A098V | 0.6 | 1.02 |
| G097S/Z097.01S/A098G | 0.6 | 1.12 |
| G097S/Z097.01S/A098G/A273T | 0.41 | 1.2 |
| G097S/Z097.01Y | 0.33 | 1.04 |
| G097V/A098R | 0.41 | 0.74 |
| G097V/A098R/D099G | 0.46 | 0.56 |
| G097V/A098S/D099S | 0.24 | 0.84 |
| G097Y/A098H/D099N | 0.3 | 0.56 |
| G097Z/A098S/D099H | 0.31 | 0.77 |
| Z097.01D/A098R/D099G | 0.39 | l |
| Z097.01D/A098S/D099L | 0.36 | 0.59 |
| Z097.01D/A098V/D099G | 0.345 | 0.805 |
| Z097.01G | 0.54 | 1.12 |
| Z097.01G/A098G | 0.47 | 1.02 |
| Z097.01G/A098G/D099S | 0.21 | 1.03 |
| Z097.01G/A098R | 0.73 | 0.86 |
| Z097.01G/A098R/D099C | 0.43 | 0.73 |
| Z097.01G/A098R/D099G | 0.39 | 0.64 |
| Z097.01G/A098R/D099S | 0.48 | 0.63 |
| A098C | 0.97 | 0.91 |
| A098C/D099G | 0.57 | 0.98 |
| A098C/D099G/A116T | 0.48 | 1.03 |
| A098C/D099H | 0.68 | 0.78 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| A098C/D099L | 0.4 | 0.64 |
| A098C/D099L/K213R | 0.36 | 0.85 |
| A098C/D099L | 0.39 | 0.95 |
| A098C/D099N | 0.66 | 0.96 |
| A098C/D099R | 0.62 | 0.80 |
| A098C/D099S | 0.69 | 0.98 |
| A098C/D099S/P194L | 0.47 | 0.79 |
| A098D/D099C | 0.59 | 0.91 |
| A098D/D099G | 0.59 | 1.09 |
| A098D/D099G/G100L | 0.65 | 1.06 |
| A098D/D099H/G100S | 0.3 | 0.91 |
| A098D/D099H/H238Y | 0.38 | 1.07 |
| A098D/D099R | 0.69 | 0.72 |
| A098D/D099R/G100D | 0.28 | 0.91 |
| A098D/D099R/G160S | 0.6 | 0.78 |
| A098F/D099S/G100L | 0.3 | 0.52 |
| A098G | 0.91 | 1.01 |
| A098G/D099C | 0.67 | 0.77 |
| A098G/D099G | 0.55 | 0.85 |
| A098G/D099G/A228T | 0.58 | 0.9 |
| A98G/D099G/G100H | 0.58 | 0.38 |
| A098G/D099G/N243D | 0.27 | 0.71 |
| A098G/D099G/S101P | 0.55 | 0.57 |
| A098G/D099G/T244S | 0.51 | 0.895 |
| A098G/D099G | 0.49 | 0.85 |
| A098G/D099H | 0.44 | 0.89 |
| A098G/D099L | 0.4 | 0.75 |
| A098G/D099R | 0.56 | 0.70 |
| A098G/D099R/A116T | 0.6 | 0.68 |
| A098G/D099R/G100D | 0.28 | 0.62 |
| A098G/D099R/V148I | 0.79 | 0.61 |
| A098G/D099S | 0.58 | 1.01 |
| A098G/D099S/L267M | 0.48 | 0.89 |
| A098G/D099V | 0.32 | 0.93 |
| A098G/D099Z | 0.25 | 0.69 |
| A098H | 1.04 | 0.96 |
| A098H/D099C | 0.46 | 0.79 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| A098H/D099G | 0.82 | 0.89 |
| A098H/D099G/A216T | 0.72 | 0.76 |
| A098H/D099G/G100D | 0.52 | 1.09 |
| A098H/D099G/G100N/L267M | 0.35 | 1.02 |
| A098H/D099G/G100S | 0.355 | 0.9 |
| A098H/D099G/V143I | 0.41 | 0.84 |
| A098H/D099R | 1.07 | 0.62 |
| A098H/D099R/G100S | 0.37 | 0.54 |
| A098H/D099S/G100C | 0.33 | 0.67 |
| A098H/D099S/G100N | 0.43 | 1.05 |
| A098H/D099Y | 0.32 | 0.81 |
| A098I/D099G | 0.54 | 0.97 |
| A098I/D099S | 0.47 | 0.91 |
| A098L/D099G | 0.44 | 1.15 |
| A098L/D099S | 0.5 | 0.91 |
| A098N | 1.05 | 1 |
| A098N/D099G | 1.14 | 1.01 |
| A098N/D099L | 0.32 | 0.71 |
| A098N/D099S | 0.74 | 1 |
| A098R/A144T | 0.81 | 0.91 |
| A098R/A274V | 1.11 | 0.88 |
| A098R/D099C | 0.56 | 0.82 |
| A098R/D099G | 0.94 | 0.70 |
| A098R/D099G/G100C | 0.35 | 0.68 |
| A098R/D099G/G100D | 0.83 | 0.92 |
| A098R/D099G/G100L/S145T | 0.68 | 0.84 |
| A098R/D099G/G100N | 0.66 | 0.74 |
| A098R/D099G/G100R | 0.57 | 0.25 |
| A098R/D099G/G100S | 0.55 | 0.82 |
| A098R/D099G/V150I | 0.84 | 0.68 |
| A098R/D099H | 0.85 | 0.59 |
| A098R/D099I | 0.39 | 0.665 |
| A098R/D099L | 0.54 | 0.62 |
| A098R/D099N | 1 | 0.895 |
| A098R/D099N/G100L | 0.57 | 0.79 |
| A098R/D099R | 0.72 | 0.32 |
| A098R/D099R/G100D | 0.41 | 0.53 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| A098R/D099R/S101Z | 0.61 | 0.23 |
| A098R/D099R/S130F | 0.23 | 0.51 |
| A098R/D099R | 0.64 | 0.51 |
| A098R/D099S | 0.75 | 0.80 |
| A098R/D099S/G100L/A153V | 0.55 | 0.79 |
| A098R/D099S | 0.87 | 0.81 |
| A098R/D099V | 0.505 | 0.775 |
| A098R/D099Z/S101G | 0.395 | 0.61 |
| A098R/G100C/A137T | 0.64 | 0.96 |
| A098R/G100S | 0.95 | 1 |
| A098S/D099C | 0.655 | 0.865 |
| A098S/D099G | 0.89 | 0.91 |
| A098S/D099G/G100C | 0.35 | 0.86 |
| A098S/D099G/G100D | 0.26 | 1.26 |
| A098S/D099G/G100R/L250I | 0.58 | 0.55 |
| A098S/D099G/G100S | 0.48 | 0.98 |
| A098S/D099G/Z099.01R | 0.25 | 0.52 |
| A098S/D099H | 0.6 | 0.95 |
| A098S/D099H/M124V | 0.53 | 0.95 |
| A098S/D099H/Z099.01R/G169A/V180A | 0.63 | 0.62 |
| A098S/D099R | 0.80 | 0.78 |
| A098S/D099R/G100L | 0.71 | 0.67 |
| A098S/D099R/G154S | 0.41 | 0.61 |
| A098S/D099R/G166S | 1.16 | 0.75 |
| A098S/D099R/Z100.01G | 0.47 | 0.66 |
| A098S/D099S | 0.82 | 0.97 |
| A098S/D099S/Z099.01R | 0.38 | 0.66 |
| A098S/D099V | 0.41 | 1.03 |
| A098T/P129Z/S130R/S132G | 0.33 | 0.53 |
| A098T/S101G | 0.785 | 0.925 |
| A098V/D099G | 0.965 | 0.96 |
| A098V/D099G/G100D | 0.5 | 0.98 |
| A098V/D099G/G100D/A153V | 0.41 | 0.62 |
| A098V/D099G/G100S | 0.66 | 0.83 |
| A098Y/D099R/G100L | 0.31 | 0.54 |
| A098Y/D099R/G100Z/S101Z/G102D | 0.98 | 0.66 |
| A098Z/S101R | 0.56 | 0.62 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| Z098.01N | 0.5 | 1.31 |
| Z098.01R | 0.54 | 0.97 |
| Z098.01R/G100R | 0.55 | 0.51 |
| D099C/K136N | 0.3 | 0.8 |
| D099C/S101D | 0.73 | 1.08 |
| D099C/S101R | 0.655 | 0.67 |
| D099C/S101Z | 0.40 | 0.61 |
| D099C/Z099.01S | 0.3 | 0.88 |
| D099C/Z100.01G | 0.36 | 0.56 |
| D099F/S101Z | 0.35 | 0.51 |
| D099G/G100C | 0.5 | 0.58 |
| D099G/G100D/S101R | 0.54 | 0.93 |
| D099G/G100H | 0.62 | 0.71 |
| D099G/G100R/S101V | 0.45 | 0.58 |
| D099G/G100S/S101 R | 0.55 | 0.79 |
| D099G/S101G | 0.67 | 0.83 |
| D099G/S101H | 0.79 | 0.95 |
| D099G/S101V | 0.85 | 0.95 |
| D099G/S101Z | 0.56 | 0.81 |
| D099G/S101Z/N269D | 0.45 | 0.81 |
| D099G/Z099.01N | 0.33 | 1.21 |
| D099G/Z099.01R/G100S | 0.26 | 0.59 |
| D099G/Z099.01S | 0.21 | 1.17 |
| D099G/Z100.01G | 0.26 | 0.71 |
| D099G/Z100.01G/S183N/Q275K | 0.35 | 0.71 |
| D099H/S101Z | 0.49 | 0.74 |
| D099H/S101Z/V150I | 0.56 | 0.59 |
| D099H/Z100.01G/A273V | 1.91 | 0.38 |
| D099H/Z100.01G/Z100.02G | 0.2 | 0.84 |
| D099I/Z099.01S | 0.48 | 0.805 |
| D099L/N118D | 0.5 | 0.82 |
| D099L/S101G | 0.23 | 0.91 |
| D099L/S101V | 0.46 | 0.88 |
| D099L/Z100.01G | 0.26 | 0.51 |
| D099N/A116T | 1 | 0.92 |
| D099N/G100R/S101V | 0.525 | 0.725 |
| D099N/S101G | 0.57 | 0.95 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| D099N/S101H | 0.98 | 0.89 |
| D099N/S101Z | 0.48 | 0.94 |
| D099N/Z099.01S | 0.69 | 1 |
| D099N/Z100.01G | 0.42 | 0.94 |
| D099R | 0.7 | 0.71 |
| D099R/S101D | 0.78 | 1 |
| D099R/S101G | 0.595 | 0.58 |
| D099R/S101V | 0.75 | 0.66 |
| D099R/S101Z | 0.48 | 0.52 |
| D099R/S101Z/A133T | 0.35 | 0.52 |
| D099R/Z099.01N | 0.28 | 1.06 |
| D099R/Z099.01S | 0.42 | 0.71 |
| D099R/Z100.01G | 0.43 | 0.66 |
| D099R/Z100.01N | 0.44 | 0.5 |
| D099S/S101G | 0.53 | 0.98 |
| D099S/S101I | 0.69 | 0.925 |
| D099S/S101Z | 0.71 | 0.77 |
| D099S/Z099.01H | 0.62 | 0.67 |
| D099S/Z099.01L | 0.35 | 0.59 |
| D099S/Z099-01V | 0.5 | 0.35 |
| D099S/Z100.01G | 0.47 | 0.85 |
| D099S/Z100.01G/Z100.02G | 0.21 | 1.07 |
| D099V/S101D | 0.54 | 1.24 |
| D099V/S101V | 0.33 | 0.825 |
| D099V/S101Z | 0.36 | 0.51 |
| Z099.01H/S101L | 0.56 | 0.58 |
| Z099.01H/Z099.02R | 0.5 | 0.44 |
| Z099.01N/S101R | 0.46 | 1.09 |
| Z099.01S | 0.48 | 1.17 |
| Z099.01S/S101V | 0.51 | 0.91 |
| Z099.01S/Z099.02G/A228T | 0.61 | 0.24 |
| G100C | 0.85 | 0.98 |
| G100D | 0.54 | 1.18 |
| G100D/S101G | 0.54 | 0.68 |
| G100D/S101R | 0.74 | 1.13 |
| G100H/G102V | 0.26 | 1.04 |
| G100H/S101G | 0.35 | 0.91 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| G100H/S101R | 0.74 | 0.945 |
| G100L/S101G | 0.775 | 1.135 |
| G100L/S101R | 1.24 | 1.01 |
| G100N/S101D | 0.5 | 0.86 |
| G100N/S101L | 0.93 | 1.075 |
| G100N/S101R | 1.27 | 0.99 |
| G100R | 0.64 | 0.84 |
| G100R/L267R/I268V | 0.7 | 1.01 |
| G100R/S101G | 0.46 | 0.78 |
| G100R/S101G/G160S/S182N | 0.5 | 0.69 |
| G100R/S101G/Q103Z/Y104Z/S161N | 0.33 | 0.73 |
| G100R/S101G/S163N | 0.55 | 0.54 |
| G100R/S101N | 0.78 | 0.89 |
| G100R/S101R | 0.60 | 0.87 |
| G100R/S101R/Q103Z/V270I | 0.58 | 1 |
| G100R/S101Z/G102C/Q103D | 0.5 | 0.26 |
| G100S | 0.59 | 1.14 |
| G100S/S101C | 0.69 | 0.96 |
| G100S/S101G | 0.47 | 1.02 |
| G100S/S101R | 0.75 | 0.99 |
| G100S/S101R | 0.94 | 1.01 |
| G100S/S101V | 0.53 | 1.13 |
| G100S/S101V/A231T | 0.51 | 0.46 |
| Z100.01G | 0.43 | 0.76 |
| Z100.01L | 0.43 | 1.05 |
| Z100.01N | 0.43 | 0.67 |
| Z100.01R | 0.47 | 0.76 |
| Z100.01R/S101G | 0.45 | 0.95 |
| S101C | 1.48 | 0.89 |
| S101C/Q103I | 0.68 | 0.91 |
| 5101C/Q103S | 1.34 | 0.97 |
| S101D | 1.055 | 1.125 |
| S101D/Q103C | 0.85 | 1.15 |
| S101D/Q103D | 0.94 | 1.1 |
| S101D/Q103F | 1.04 | 1.1 |
| S101D/Q103H | 0.89 | 1.24 |
| S101D/Q103H | 0.68 | 1.09 |

(continued)

Table 3-1. Mutations and Performance Index for TCA protein and BMI Activity for BPN'-Y217L Variants

| Mutations | TCA PI | PI on BMI at pH 8 16°C |
|---|---|---|
| S101D/Q103R | 1.06 | 0.87 |
| S101D/Q103R/H238Y | 0.69 | 1.11 |
| S101D/Q103S | 0.63 | 1.14 |
| S101D/Q103V | 0.41 | 1.09 |
| S101F/Q103G | 0.32 | 0.75 |
| S101G | 0.83 | 0.99 |
| S101G/A151V | 0.41 | 1.23 |
| S101G/A179V | 0.29 | 0.58 |
| S101G/G102S | 0.42 | 1.13 |
| S101G/Q103G | 0.38 | 0.59 |
| S101G/Q103L | 0.74 | 1.01 |
| S101G/Q103N | 0.75 | 1.14 |
| S101G/Q103R | 0.71 | 0.83 |
| S101G/Q103R/A179T | 0.32 | 0.52 |
| S101G/Q103R/G157D | 0.59 | 0.81 |
| S101G/Q103S | 0.57 | 1.12 |
| S101G/Q103S/L233S | 0.8 | 0.97 |
| S101G/Q103S/L233S | 0.45 | 1.13 |
| S101G/Q245H | 0.56 | 0.93 |
| S101G/S249N | 0.77 | 1.115 |
| S101G/V139I | 0.85 | 1.01 |
| S101G/Z101.01R | 0.435 | 0.965 |
| S101G/Z101.01R/A200T | 0.52 | 0.26 |
| S101G/Z102.01G | 0.465 | 0.69 |
| S101H | 0.92 | 1.03 |
| S101H/G102S | 0.68 | 1.23 |
| S101H/Q103D | 0.985 | 1.185 |
| S101H/Q103G | 0.38 | 0.84 |
| S101H/Q103G/E251Q | 0.37 | 0.7 |
| S101H/Q103H | 1.07 | 0.93 |
| S101H/Q103I | 0.715 | 1.035 |
| S101H/Q103R | 0.73 | 0.82 |
| S101H/Q103R/A274Z/Q275Z | 0.67 | 0.68 |
| S101H/Q103S | 0.76 | 1.07 |
| S101H/Q103V | 0.67 | 1.09 |
| S101I | 0.78 | 0.95 |
| S101I/Q103R | 0.81 | 0.91 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| S101L | 1.09 | 1.04 |
| S101L/G102S/Q103R | 0.87 | 1.07 |
| S101L/Q103D | 1.03 | 1.07 |
| S101L/Q103G | 0.33 | 0.9 |
| S101L/Q103R | 0.61 | 0.75 |
| S101L/Q103R/A138T | 0.43 | 0.73 |
| S101L/Q103R/V121I/K213E | 0.81 | 0.95 |
| S101L/Q103S | 1.04 | 1.04 |
| S101L/Q103V | 0.66 | 1.05 |
| S101L/Z101.01R | 0.5 | 0.99 |
| S101N/Q103R | 0.94 | 0.925 |
| S101N/Z102.01G | 0.49 | 0.65 |
| S101R | 0.85 | 0.90 |
| S101R/A151V | 0.4 | 0.66 |
| S101R/A273V | 0.78 | 0.71 |
| S101R/E156G | 1.1 | 0.66 |
| S101R/G102L/Q103G | 0.95 | 1.04 |
| S101R/G102S | 1.58 | 1.1 |
| S101R/G102S/Q103R | 0.81 | 0.91 |
| S101R/G102S/Q103V | 0.54 | 0.89 |
| S101R/G131D | 1.45 | 0.79 |
| S101R/Q103G | 1.05 | 0.89 |
| S101R/Q103D | 0.75 | 1.01 |
| S101R/Q103D/S248R | 0.89 | 0.75 |
| S101R/Q103G | 0.49 | 0.84 |
| S101R/Q103G/A116T | 0.52 | 0.97 |
| S101R/Q103G/V147I/A153V | 0.445 | 1.075 |
| S101R/Q103G | 0.49 | 0.7 |
| S101R/Q103L | 0.80 | 0.63 |
| S101R/Q103N | 1.00 | 0.89 |
| S101R/Q103R | 0.74 | 0.56 |
| S101R/Q103R/S161N | 1.35 | 0.63 |
| S101R/Q103R/V148I | 0.58 | 0.44 |
| S101R/Q103S | 0.93 | 0.85 |
| S101R/Q103S/L126F | 1.23 | 0.84 |
| S101R/Q103V | 0.74 | 0.83 |
| S101V | 0.915 | 1.05 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| S101V/Q103G | 0.46 | 0.99 |
| S101V/Q103H | 0.75 | 0.98 |
| S101V/Q103N/Z275.01K | 1.17 | 1.13 |
| S101V/Q103R | 0.62 | 1.03 |
| S101V/Q103V | 0.58 | 1.09 |
| S101Y/G102S/Q103R | 0.83 | 0.94 |
| S101Y/Q103L | 0.54 | 0.75 |
| S101Y/Q103S | 0.54 | 0.93 |
| S101Y/Q103V | 0.58 | 0.95 |
| S101Z | 0.34 | 0.95 |
| S101Z/G102I/Q103H/Y104G/L135F | 0.32 | 0.7 |
| S101Z/G102V/Q103R/Y104L | 0.37 | 0.61 |
| S101Z/Q103D | 0.52 | 0.29 |
| S101Z/Q103F | 0.45 | 1.07 |
| S101Z/Q103L | 0.41 | 1 |
| S101Z/Q103R | 0.49 | 0.76 |
| G102C/Q103L/Y104S | 0.96 | 1.15 |
| G102R/Q103C/Y104C/V192I | 0.33 | 1.26 |
| G102R/Q103G/Y104R | 0.25 | 0.69 |
| G102R/Q103R/Z103.01G | 0.655 | 0.885 |
| G102Z/Q103S | 0.33 | 0.61 |
| Z102.01G/Q103R | 0.41 | 1.02 |
| Z102.01G/Q103R/L267V | 0.31 | 0.64 |
| Z102.01G/Z102.02R/Z102.03G | 0.53 | 0.67 |
| Q103D | 1.04 | 1.21 |
| Q103G | 0.335 | 1.04 |
| Q103H/Y104N/Q275Z | 0.56 | 0.81 |
| Q103N/E156D | 0.92 | 1.07 |
| Q103R | 0.75 | 0.82 |
| Q103R/I122L/N123S/M124V | 0.68 | 0.65 |
| Q103R/Y104F | 0.7 | 0.795 |
| Q103S | 1.35 | 0.86 |
| Q103V | 0.61 | 0.955 |
| Q103Y/S182N | 0.87 | 0.77 |
| S105G/P129S/S130H/G131H | 1.09 | 0.7H |
| W106C/A116D/N117S/N118C | 0.47 | 0.73 |
| N109S/M119C/D120G/V121L | 0.28 | 0.97 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| A116G/N117Z/N118R/M119C | 0.37 | 0.88 |
| A116H/N117G/N118R | 0.39 | 0.94 |
| N117C/N118G/M119S | 0.44 | 1.05 |
| N117C/N118G/M119S/A216V | 0.33 | 0.87 |
| N117G/N118G/M119C | 0.43 | 0.94 |
| N117G/N118R/M119C | 0.29 | 1.03 |
| N117H/N118C/M119C | 0.45 | 0.57 |
| N117H/N118D/M119S | 0.63 | 0.88 |
| N117H/N118G/M119C | 0.97 | 0.83 |
| N117H/N118G/M119L | 0.59 | 0.86 |
| N117H/N118G/M119S | 0.72 | 0.89 |
| N117H/N118G/M119V | 0.62 | 0.95 |
| N117H/N118H/M119V | 1.07 | 0.96 |
| N117H/N118R/M119C | 0.98 | 0.75 |
| N117H/N118R/M119S | 0.53 | 0.77 |
| N117H/N118S/M119C | 0.71 | 0.84 |
| N117H/N118S/M119S | 0.4 | 0.985 |
| N117H/N118S/M119V | 0.64 | 0.89 |
| N117I/N118G/M119V | 0.38 | 1.01 |
| N117L/N118G/M119V | 0.38 | 0.77 |
| N117R/N118G/M119C | 0.63 | 0.80 |
| N117R/N118G/M119I/V270I | 0.46 | 0.88 |
| N117R/N118G/M119S | 0.375 | 0.79 |
| N117R/N118H/M119V | 0.66 | 0.91 |
| N117R/N118R/M119C | 0.755 | 0.755 |
| N117R/N118R/M119I | 0.67 | 0.71 |
| N117R/N118R/M119S | 0.28 | 0.605 |
| N117R/N118R/M119V | 0.80 | 0.73 |
| N117R/N118S/M119I | 0.38 | 0.75 |
| N117R/N118S/M119L | 0.23 | 0.72 |
| N117S/N118G/M119C | 0.595 | 1.085 |
| N117S/N118G/M119S | 0.3 | 1.1 |
| N117S/N118H/M119V | 0.49 | 1.01 |
| N117S/N118R/M119V | 0.58 | 0.93 |
| N117V/N118G/M119C | 0.47 | 0.99 |
| N117V/N118H/M119C | 0.39 | 0.71 |
| N117Y/N118G/M119S | 0.26 | 0.74 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| N117Y/N118G/M119V | 0.475 | 1 |
| Z117.01G/N118R/M119C | 0.29 | 0.65 |
| N118C/M119V/D120S | 0.27 | 0.82 |
| N118C/Z118.01G/M119C | 0.485 | 0.86 |
| N118D/M119C/D120L | 0.43 | 0.98 |
| N118D/M119S/D120R | 0.24 | 0.88 |
| N118G/M119C/D120G | 0.63 | 1.02 |
| N118G/M119C/D120L | 0.5 | 0.99 |
| N118G/M119I/120R | 0.65 | 0.94 |
| N118G/M119N | 0.28 | 0.73 |
| N118G/M119S/D120G | 0.29 | 0.69 |
| N118G/M119S/D120R | 0.38 | 0.83 |
| N118G/M119V/D120G | 0.49 | 0.94 |
| N118G/M119V/D120S/S182N | 0.52 | 0.99 |
| N118H/M119C/D120R | 0.88 | 0.87 |
| N118H/M119I/D120G | 0.54 | 1.05 |
| N118H/M119N/V148I | 0.38 | 0.63 |
| N118II/M119S/D120G | 0.28 | 0.62 |
| N118H/M119V/D120G | 0.54 | 1 |
| N118R/M119C | 0.97 | 0.99 |
| N118R/M119C/D120G | 0.63 | 0.85 |
| N118R/M119C/D120R | 0.8 | 0.85 |
| N118R/M119S | 0.45 | 0.99 |
| N118R/M119S/D120G | 0.32 | 0.53 |
| N118R/M119S/D120R | 0.29 | 0.57 |
| N118R/M119S/D120S | 0.28 | 0.82 |
| N118R/M119V/D120C | 0.27 | 0.97 |
| N118R/M119V/D120G | 0.56 | 0.76 |
| N118R/M119V/D120G/A232T | 0.32 | 0.825 |
| N118S/M119C/D120R | 0.56 | 0.91 |
| M119C/D120G | 0.58 | 0.98 |
| M119C/D120G/V121I | 0.48 | 0.95 |
| M119C/D120G/V121L | 0.39 | 0.52 |
| M119C/D120H | 0.91 | 1.01 |
| M119C/D120H/A187V | 0.44 | 0.93 |
| M119C/D120R | 0.76 | 0.95 |
| M119H/V121I | 0.42 | 0.58 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| M119I/D120C | 0.44 | 0.88 |
| M119I/D120G | 0.47 | 0.96 |
| M119I/D120R | 0.67 | 0.86 |
| M119L/D120G | 0.39 | 0.51 |
| M119S/D120R | 0.28 | 0.97 |
| M119S/D120S/V121I | 0.46 | 0.72 |
| M119S/V121I | 0.44 | 1.15 |
| M119V/D120C | 0.27 | 0.93 |
| M119V/D120G | 0.43 | 1.06 |
| M119V/D120R | 0.63 | 0.86 |
| M119V/D120S/V198L | 0.5 | 0.93 |
| D120G/I122C | 0.27 | 0.83 |
| D120G/I122C/L217S | 0.34 | 0.56 |
| D120G/I122G | 0.54 | 0.23 |
| D120G/I122V | 0.5 | 1 |
| D120H/I122L | 0.66 | 1.03 |
| D120H/V121C/I122V | 0.31 | 0.74 |
| D120N/V121L/I122V | 0.32 | 0.93 |
| D120R/A151V | 0.37 | 0.71 |
| D120R/I122V | 0.63 | 0.97 |
| D120R/V121I | 0.72 | 1 |
| D120R/V121I/I122C | 0.39 | 0.74 |
| D120R/V121I/I122C/A228T | 0.29 | 0.98 |
| D120S/I122C/A133S | 0.31 | 1.1 |
| V121C | 0.63 | 0.86 |
| V121C/I122C/N123C | 0.54 | 0.2 |
| V121C/I122G/N123V | 0.56 | 0.6 |
| V121C/I122S/N123V | 0.56 | 0.57 |
| V121C/I122V/N123S | 0.52 | 0.59 |
| V121I/I122C/N123C | 0.53 | 0.6 |
| V121I/I122C/V150I | 0.8 | 1.13 |
| V121I/I122G/N123C | 0.65 | 1 |
| V121I/I122S/N123C | 0.64 | 1.36 |
| V121I/I122S/N123I | 0.95 | 0.83 |
| V121I/I122V/N123S | 0.82 | 0.98 |
| V121L/I122S/N123V | 0.53 | 0.9 |
| V121L/N123C | 1.02 | 1.16 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| I122C/N123C | 0.6 | 0.6 |
| I122C/N123S/M124L | 0.98 | 1.015 |
| I122D/N123V/M124L | 0.55 | 0.63 |
| I122G/N123C/M124L | 0.96 | 0.96 |
| I122G/N123G/Z123.01C | 0.63 | 0.45 |
| I122H/N123V/M124L | 0.65 | 0.76 |
| I122S/N123S | 0.63 | 1.27 |
| I122V/N123G/M124C/A138V | 0.51 | 0.25 |
| I122V/N123S/M124I | 0.81 | 0.75 |
| I122V/S183G | 0.91 | 0.97 |
| N123C/M124S | 0.98 | 0.785 |
| N123C/M124V | 1.875 | 0.745 |
| N123D | 0.7 | 0.89 |
| N123S/M124L | 1.55 | 1.13 |
| N123S/M124S | 0.71 | 0.26 |
| M124C | 0.92 | 1.05 |
| M124C/L126S | 0.7 | 0.51 |
| M124C/L126S/T244I | 0.72 | 0.455 |
| M124C/L126V | 1.535 | 1.085 |
| M124G | 0.53 | 0.3 |
| M124I | 1.72 | 1.16 |
| M124I/L126H | 0.87 | 0.54 |
| M124S | 0.6 | 0.58 |
| M124V | 1.08 | 1.20 |
| L126C | 1.8 | 0.35 |
| L126F/P129Z | 1.07 | 1 |
| L126F/P129Z/S182N | 1.47 | 1.24 |
| L126G | 0.67 | 0.58 |
| L126I/P129Z | 0.53 | 0.71 |
| L126R/G127R | 0.54 | 0.47 |
| L126S | 1.08 | 0.94 |
| L126Y | 1.04 | 1.33 |
| Z126.01S | 0.67 | 0.59 |
| G127C/G128S/P129R | 0.77 | 0.48 |
| G127C/P129G | 1.53 | 0.72 |
| G127C/P129G/A200T | 0.56 | 0.4 |
| G127C/P129R | 1.785 | 0.825 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| G127C/P129R/A153T | 0.76 | 0.48 |
| G127D/P129G | 0.91 | 0.73 |
| G127D/P129S | 0.71 | 0.69 |
| G127H/P129G | 1.65 | 0.71 |
| G127H/P129R | 1.1 | 0.77 |
| G127L | 1.14 | 0.83 |
| G127L/P129R | 1.02 | 0.80 |
| G127N/P129C | 1.05 | 0.88 |
| G127N/P129G | 1.1 | 0.91 |
| G127N/P129G/A133T | 0.87 | 1.04 |
| G127N/P129G/A151V/K213N | 0.37 | 0.72 |
| G127N/P129R | 1.10 | 0.77 |
| G127N/P129S/A273V | 1.07 | 0.9 |
| G127N/P129V | 1.16 | 0.92 |
| G127R/A230V | 0.5 | 0.58 |
| G127R/P129D | 1.01 | 0.8 |
| G127R/P129D/L217S | 0.76 | 0.78 |
| G127R/P129G | 0.57 | 0.48 |
| G127R/P129R | 0.85 | 0.56 |
| G127R/P129S | 0.725 | 0.725 |
| G127S/P129D | 0.67 | 1.255 |
| G127S/P129F | 0.94 | 0.93 |
| G127S/P129G | 1.29 | 0.99 |
| G127S/P129G/G166D | 0.81 | 0.98 |
| G127S/P129G/I175T | 0.33 | 0.77 |
| G127S/P129R | 1.47 | 0.86 |
| G127S/P129S | 1.06 | 1.09 |
| G127S/P129V | 1.16 | 1.15 |
| G127V/P129G | 1.59 | 0.84 |
| G127V/P129R | 1.01 | 0.89 |
| Z127.01G/Z127.02H/P129D | 0.58 | 0.65 |
| Z127.01G/Z127.02H/P129G | 0.55 | 0.57 |
| Z17.01G/7127.02H/P129R | 0.52 | 0.61 |
| Z127.01H/G128C/P129C/A230V | 0.57 | 0.57 |
| Z127.01H/P129G | 0.59 | 0.36 |
| Z127.01H/P129G/V148I | 0.61 | 0.27 |
| Z127.01H/P129R | 0.57 | 0.44 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| Z127.01N/G128C/P129V | 0.5 | 0.2 |
| Z127.01N/G128S/P129S | 0.585 | 1.19 |
| Z127.01N/G128S/P129V | 0.57 | 0.67 |
| Z127.01N/P129R | 0.59 | 1.04 |
| Z127.01R/G128S/P129S | 0.67 | 0.5 |
| Z127.01R/P129G | 0.485 | 0.475 |
| Z127.01S/P129D | 0.58 | 0.5 |
| Z127.01S/P129G | 0.65 | 0.67 |
| Z127.01S/P129R | 0.42 | 0.94 |
| G128D/P129G | 0.53 | 1.00 |
| G128D/P129R/Z129.01R | 0.7 | 0.33 |
| G128D/P129S/G154S | 0.5 | 0.54 |
| G128H/P129C | 0.58 | 0.89 |
| G128H/P129H/Z129.01G | 0.65 | 0.53 |
| G128H/P129H/Z129.01R | 0.595 | 0.44 |
| G128H/P129R | 0.78 | 0.97 |
| G128H/P129R/Z129.01G | 0.52 | 0.54 |
| G128H/P129R/Z129.01R | 0.57 | 0.36 |
| G128H/P129S/Z129.01G | 0.66 | 0.54 |
| G128H/P129S/Z129.01R | 0.47 | 0.88 |
| G128H/P129S/Z129.01R/S248N | 0.54 | 0.62 |
| G128H/P129Y | 0.44 | 1.22 |
| G28H/Z128.01R/P129D | 0.66 | 0.63 |
| G128H/Z128.01R/P129S | 0.46 | 0.755 |
| G128H/Z128.01R/P129S/A144T | 0.555 | 0.705 |
| G128H/Z129.01R | 0.46 | 0.76 |
| G128N/P129R/Z129.01G | 0.55 | 0.83 |
| G128N/P129R/Z129.01R | 0.5 | 0.93 |
| G128N/P129S/Z129.01D | 0.67 | 0.93 |
| G128N/Z128.01R/P129S | 0.58 | 1.01 |
| G128R/P129G | 0.54 | 0.57 |
| G128R/P129H/Z129.01G | 0.495 | 0.755 |
| G128R/P129L/Z130.01S | 0.58 | 0.25 |
| G128R/P129R | 0.45 | 0.53 |
| G128R/P129R/Z129.01G | 0.55 | 0.32 |
| G128R/P129R/Z129.01G/A216V | 0.64 | 0.26 |
| G128R/P129S | 0.46 | 0.78 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| G128R/P129S/Z129.01G | 0.605 | 0.7 |
| G128R/P129Z/S130R/S132G | 0.43 | 0.51 |
| G128R/Z128.01R/P1291D | 0.46 | 0.57 |
| G128R/Z128.01R/P129F | 0.52 | 0.28 |
| G128S/P129C | 0.845 | 1.09 |
| G128S/P129C/Z129.01R | 0.575 | 0.435 |
| G128S/P129D | 0.87 | 1.37 |
| G128S/P129D/S248R | 0.9 | 1.28 |
| G128S/P129D/T244N | 1.3 | 1.13 |
| G128S/P129G | 1.05 | 1.38 |
| G128S/P129G/A187V | 0.75 | 0.89 |
| G128S/P129H/Z129.01R | 0.44 | 0.92 |
| G128S/P129R/L209F | 1.2 | 1.01 |
| G128S/P129R/Z129.01G | 0.8 | 0.76 |
| G128S/P129S/Z129.01D | 0.6 | 0.55 |
| G128S/P129S/Z129.01R | 0.59 | 0.84 |
| G128S/P129V | 0.735 | 1.145 |
| G128Y/P129R/Z129.01G | 0.55 | 0.4 |
| Z128.01G | 0.57 | 0.515 |
| Z128.01G/P129D | 0.39 | 1.04 |
| Z128.01G/P129R | 0.46 | 0.87 |
| Z128.01G/P129S/A134T | 0.55 | 0.62 |
| Z128.01G/Z128.02G | 0.59 | 0.27 |
| Z128.01R/P129D | 0.35 | 0.6 |
| Z128.01R/Z128.02G/P129S | 0.5 | 0.58 |
| Z128.01R/Z128.02H/P129G/G160S | 0.59 | 0.44 |
| Z128.01R/Z128.02R/P129D | 0.58 | 0.74 |
| Z128.01Y/Z128.02H/P129R | 0.57 | 0.34 |
| P129C/S130C/S132Z | 0.42 | 0.6 |
| P129D/Z129.01G/Z129.02D/G160D | 0.54 | 0.61 |
| P129D/Z129.01G/Z129.02G | 0.69 | 0.3 |
| P129G | 0.64 | 0.90 |
| P129G/G131Z | 0.57 | 1.19 |
| P129G/S130G/Z131.01G | 0.51 | 1.02 |
| P129G/S130H/S132Z | 0.37 | 1.17 |
| P129G/S130R/S132Z | 0.71 | 0.3 |
| P129G/V270I | 0.7 | 0.9 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| P129G/Z129.01G | 0.45 | 0.84 |
| P129G/Z129.01R/Z129.02G | 0.69 | 0.82 |
| P129H | 0.79 | 1 |
| P129H/G131Z | 0.6 | 1.16 |
| P129H/G131Z/S132C | 0.52 | 0.59 |
| P129H/131Z/S132H | 0.43 | 1.04 |
| P129H/S132Z | 0.41 | 0.51 |
| P129H/Z130.01D/Z130.02S | 0.44 | 1.02 |
| P129L | 1 | 1.22 |
| P129R | 0.80 | 0.87 |
| P129R/G131Z/S132H | 0.58 | 0.78 |
| P129R/S130D/S132Z | 0.745 | 1.04 |
| P129R/S130N/G131Z | 1.23 | 0.95 |
| P129R/S132T | 0.37 | 0.69 |
| P129R/S132Z | 0.395 | 0.745 |
| P129R/Z129.01G | 0.42 | 0.60 |
| P129R/Z129.01G/Z129.02G | 0.49 | 0.57 |
| P129R/Z129.01G/Z129.02G/A144T | 0.64 | 0.49 |
| P129R/Z129.01G/Z129.02G/G146D | 0.53 | 0.31 |
| P129R/Z129.01G/Z129.02R | 0.52 | 0.42 |
| P129R/Z129.01G | 0.46 | 0.65 |
| P129R/Z129.01R/Z129.02G | 0.58 | 0.28 |
| P129S | 0.92 | 1.00 |
| P129S/G131Z | 0.75 | 1.15 |
| P129S/S130H/S132Z | 0.52 | 1.19 |
| P129S/Z129.01C | 0.55 | 0.45 |
| P129S/Z129.01R/Z129.02S | 0.59 | 0.82 |
| P129S/Z130.01N/Z130.02S | 0.45 | 1.1 |
| P129V | 1.32 | 1.09 |
| P129V/S132Z | 0.37 | 0.59 |
| P129Z | 0.57 | 1.16 |
| P129Z/G131R/S132C | 0.38 | 0.51 |
| P129Z/S130G | 0.47 | 1.29 |
| P129Z/S130G/G131H/S132H | 1.08 | 1.18 |
| P129Z/S130G/G131V | 0.52 | 0.82 |
| P129Z/S130H | 0.56 | 1.17 |
| P129Z/S130H/S132G | 0.42 | 0.61 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| P129Z/S130H/S132N | 1.91 | 0.62 |
| P129Z/S130L | 0.49 | 1.14 |
| P129Z/S130R | 0.93 | 1.00 |
| P129Z/S130R/G131C | 0.53 | 0.75 |
| P129Z/S130R/G131C | 0.61 | 0.89 |
| P129Z/S130R/G131D/S132C | 0.7 | 0.75 |
| P129Z/S130R/G131H/A144E | 0.86 | 0.88 |
| P129Z/S130R/G131H/S132D | 0.33 | 0.52 |
| P129Z/S130R/G131R/S132D | 0.37 | 0.64 |
| P129Z/S130R/L267M | 0.67 | 0.99 |
| P129Z/S130R/S132C | 0.72 | 0.46 |
| P129Z/S130R/S132G | 0.42 | 0.64 |
| P129Z/S130V/S132G | 0.37 | 0.81 |
| P129Z/S130Z/G131N/S132G | 0.5 | 0.9 |
| S130C | 1.25 | 0.86 |
| S130D/G131C/S132D | 0.71 | 0.81 |
| S130D/G131R | 0.77 | 0.87 |
| S130D/G131R/S132D | 0.52 | 0.9 |
| S130D/G131Z | 1.53 | 0.91 |
| S130D/G131Z/S132H | 0.68 | 0.82 |
| S130F/G131N/S132V | 0.59 | 0.64 |
| S130F/G131S/S132V | 0.48 | 0.59 |
| S130F/S132V | 0.29 | 0.53 |
| S130G | 0.8 | 1.1 |
| S130G/G131D/S132C | 1.13 | 0.81 |
| S130G/G131H/S132D | 0.51 | 0.88 |
| S130G/G131S/S132I | 0.4 | 0.67 |
| S130G/S132G | 0.635 | 0.845 |
| S130H | 1.01 | 0.92 |
| S130H/G131C/S132G | 0.94 | 0.98 |
| S130H/G131H/S132R | 0.53 | 0.41 |
| S130H/G131R/S132C | 0.74 | 0.86 |
| S130H/G131S | 1.08 | 0.75 |
| S130H/G131S/S132C | 1.11 | 0.78 |
| S130H/S132Z | 0.81 | 0.94 |
| S130I/G131S/S132H | 0.69 | 0.77 |
| S130I/S132G | 0.94 | 0.76 |

(continued)

| Table 3-1. Mutations and Performance Index for TCA protein and BMI Activity for BPN'-Y217L Variants | | |
|---|---|---|
| Mutations | TCA PI | PI on BMI at pH 8 16°C |
| S130L | 1.06 | 0.87 |
| S130N/G131S/S132C | 1.04 | 0.91 |
| S130R/G131R/S132C | 0.7 | 0.625 |
| S130R/G131S/S132C/S161N | 1.29 | 0.84 |
| S130R/S132G | 1.05 | 0.36 |
| S130R/S132G/S183N | 0.98 | 0.36 |
| S130R/S132Z | 0.48 | 0.69 |
| S130V/G131D/S132I | 0.98 | 1.23 |
| S130V/G131H/S132C | 1.34 | 0.91 |
| S130V/S132N | 0.74 | 0.91 |
| S130V/S132Z | 0.35 | 0.52 |
| S130Y/G131H/S132C | 1.14 | 0.87 |
| S130Y/S132G | 0.97 | 0.62 |
| S130Z/G131H/S132H | 0.78 | 0.88 |
| G131H/S132L/W241R | 0.35 | 0.61 |
| G131N/S132C | 0.94 | 0.93 |
| G131S/S132G | 0.42 | 0.63 |
| G131Y/S132G | 0.33 | 0.615 |
| S132H | 0.65 | 0.88 |
| S132Z | 0.795 | 1.045 |
| A134T | 0.66 | 1.1 |
| A142V | 0.55 | 1.05 |
| A142V/G211V | 0.33 | 1.15 |
| A144T | 0.89 | 0.96 |
| S161N | 0.92 | 1.04 |
| A200T | 0.5 | 0.9 |
| L217Y | 1.14 | 0.95 |
| A231T | 0.69 | 0.95 |
| W241R | 0.54 | 0.95 |
| S248G | 0.96 | 0.95 |
| S260F | 0.81 | 1.04 |
| F261L | 0.58 | 0.86 |
| L267G | 1.06 | 1.08 |
| A272S | 1.21 | 0.92 |
| Q275Z | 0.79 | 0.98 |

Table 3-2 provides a list of the insertions that make up the combinations.

| Table 3-2. Mutations | |
|---|---|
| POS | Variant |
| 66.01 | Z066.01G |
| 93.01 | Z093.01G |
| 96.01 | Z096.01D |
| 96.01 | Z096.01H |
| 96.01 | Z096.01N |
| 96.01 | Z096.01R |
| 96.01 | Z096.01S |
| 96.02 | Z096.02G |
| 97.01 | Z097.01D |
| 97.01 | Z097.01G |
| 97.01 | Z097.01H |
| 97.01 | Z097.01R |
| 97.01 | Z097.01S |
| 97.01 | Z097.01Y |
| 98.01 | Z098.01N |
| 98.01 | Z098.01R |
| 99.01 | Z099.01H |
| 99.01 | Z099.01L |
| 99.01 | Z099.01N |
| 99.01 | Z099.01R |
| 99.01 | Z099.01S |
| 99.01 | Z099.01V |
| 99.02 | Z099.02G |
| 99.02 | Z099.02R |
| 100.01 | Z100.01G |
| 100.01 | Z100.01L |
| 100.01 | Z100.01N |
| 100.01 | Z100.01R |
| 100.02 | Z100.02G |
| 101.01 | Z101.01R |
| 101.02 | Z101.02G |
| 102.01 | Z102.01G |
| 102.02 | Z102.02R |
| 102.03 | Z102.03G |
| 103.01 | Z103.01G |
| 117.01 | Z117.01G |
| 118.01 | Z118.01G |
| 123.01 | Z123.01C |
| 126.01 | Z126.01S |
| 127.01 | Z127.01G |
| 127.01 | Z127.01H |
| 127.01 | Z127.01N |
| 127.01 | Z127.01R |
| 127.01 | Z127.01S |
| 127.02 | Z127.02H |
| 128.01 | Z128.01G |
| 128.01 | Z128.01R |
| 128.01 | Z128.01Y |
| 128.02 | Z128.02G |
| 128.02 | Z128.02H |
| 128.02 | Z128.02R |
| 129.01 | Z129.01C |
| 129.01 | Z129.01D |
| 129.01 | Z129.01G |
| 129.01 | Z129.01R |
| 129.02 | Z129.02D |
| 129.02 | Z129.02G |
| 129.02 | Z129.02R |
| 129.02 | Z129.02S |
| 130.01 | Z130.01D |
| 130.01 | Z130.01N |
| 130.01 | Z130.01S |
| 131.01 | Z131.01G |
| 275 | Q275Z |

## EXAMPLE 4

### Construction of Combinatorial Variants of BPN' 3

Using BPN'G97A-G128A-Y217Q ("BPN'3") as a parent, combinatorial variants were created by a strategy that involved extension (fusion) PCR using four fragments of the gene. Fragment 1 was amplified by primers P4974 and P4977 (*See,* Table 4-1). Fragment 4 was amplified by primers 4978 and P4976 (Table 4-1). Both fragment 1 and 4 did not contain any mutations. Fragments 2 and 3 each were created as a set of 12 and 16 sequences respectively that contained the desired mutations, using either BPN'3 as template or in the absence of template using partially overlapping forward and reverse primers.

The set of Fragment 2 sequences were amplified by the following primers:

01: P4979 and P4980 (BPN'3 as a template)
02: P4981 and P4982 (no template)
03: P4983 and P4984 (no template)
04: P4985 and P4986 (no template)
05: P4987 and P4988 (no template)
06: P4989 and P4990 (no template)
07: P4991 and P4992 (no template)
08: P4993 and P4994 (no template)
09: P4995 and P4996 (no template)
10: P4997 and P4998 (no template)
11: P4999 and P5000 (no template)
12: P5001 and P5002 (no template)

The set of Fragment 3 sequences were amplified by the following primers:

01: P5003 and P5004 (BPN'3 as a template)
02: P5005 and P5006 (no template)
03: P5007 and P5008 (no template)
04: P5009 and P5010 (no template)
05: P5011 and P5012 (no template)
06: P5013 and P5014 (no template)
07: P5015 and P5016 (no template)
08: P5017 and P5018 (no template)
09: P5019 and P5020 (no template)
10: P5021 and P5022 (no template)
11: P5023 and P5024 (no template)
12: P5025 and P5026 (no template)
13: P5027 and P5028 (no template)
14: P5029 and P5030 (no template)
15: P5031 and P5032 (no template)
16: P5033 and P5034 (no template)

The primer sequences used in the construction of the combinatorial variants are listed in Table 4-1.

**Table 4-1. Primer Sequences and Mutations Introduced in Combinatorial Variants of BPN'3**

| SEQ ID NO: | Primer Name | Sequence |
|---|---|---|
| 125 | P4948 | GTGTTTTTCTTCCAATTGTGCTC |
| 126 | P4974 | GCCTCACATTTGTGCCACCTA |
| 127 | P4975 | CATATGAGTTATGCAGTTTGTAG |
| 128 | P4976 | CCTCTCGGTTATGAGTTAGTTC |
| 129 | P4977 | GTAGAGCGAAGCACAGGGGGCTA |

(continued)

| Table 4-1. Primer Sequences and Mutations Introduced in Combinatorial Variants of BPN'3 | | |
|---|---|---|
| SEQ ID NO: | Primer Name | Sequence |
| 130 | P4978 | CTTAAAGCAGCAGTTGATAAAGCT |
| 131 | P4979 | CTTGGTGTAGCCCCGTCTGCTT |
| 132 | P4980 | ATCCATGTTATTCGCGATGGCCCATT |
| 133 | P4981 | CTTGGTGTAGCCCCGTCTGCTTCGCTCTACGCCTCTTGCTCTGC AGCAGACGGATCAGGCCAATACTCATGGATTATCAA |
| 134 | P4982 | ATCCATGTTATTCGCGATGGCCCATTCGATGCCGTTGATAATCC ATGAGTATTGGCCTGATCCGTCTGCTGCAGAGCAAG |
| 135 | P4983 | CTTGGTGTAGCCCCGTCTGCTTCGCTCTACGCCGTTAAAGTTCT TGATGCACGTGACGGATCAGGCCAATACTCATGGAT |
| 136 | P4984 | ATCCATGTTATTCGCGATGGCCCATTCGATGCCGTTGATAATCC ATGAGTATTGGCCTGATCCGTCACGTGCATCAAGAA |
| 137 | P4985 | CTTGGTGTAGCCCCGTCTGCTTCGCTCTACGCCGTTAAAGTTCT TGCAGCAGACTCTGGATCAGGCCAATACTCATGGAT |
| 138 | P4986 | ATCCATGTTATTCGCGATGGCCCATTCGATGCCGTTGATAATCC ATGAGTATTGGCCTGATCCAGAGTCTGCTGCAAGAA |
| 139 | P4987 | CTTGGTGTAGCCCCGTCTGCTTCGCTCTACGCCGTTAAAGTTCT TGCAGCAGACTCTTCAGGCCAATACTCATGGATTAT |

| Table 4-1. Primer Sequences and Mutations Introduced in Combinatorial Variants of BPN'3 | | |
|---|---|---|
| SEQ ID NO: | Primer Name | Sequence |
| 140 | P4988 | ATCCATGTTATTCGCGATGGCCCATTCGATGCCGTTGATAATCC ATGAGTATTGGCCTGAAGAGTCTGCTGCAAGAACTT |
| 141 | P4989 | CTTGGTGTAGCCCCGTCTGCTTCGCTCTACGCCGTTAAAGTTCT TGCAGCAGACGGAGATGGCCAATACTCATGGATTAT |
| 142 | P4990 | ATCCATGTTATTCGCGATGGCCCATTCGATGCCGTTGATAATCC ATGAGTATTGGCCATCTCCGTCTGCTGCAAGAACTT |
| 143 | P4991 | CTTGGTGTAGCCCCGTCTGCTTCGCTCTACGCCGTTAAAGTTCT TGATGCACGTGACTCTGGATCAGGCCAATACTCATG |
| 144 | P4992 | ATCCATGTTATTCGCGATGGCCCATTCGATGCCGTTGATAATCC ATGAGTATTGGCCTGATCCAGAGTCACGTGCATCAA |
| 145 | P4993 | CTTGGTGTAGCCCCGTCTGCTTCGCTCTACGCCGTTAAAGTTCT TGATGCACGTGACTCTTCAGGCCAATACTCATGGAT |
| 146 | P4994 | ATCCATGTTATTCGCGATGGCCCATTCGATGCCGTTGATAATCC ATGAGTATTGGCCTGAAGAGTCACGTGCATCAAGAA |
| 147 | P4995 | CTTGGTGTAGCCCCGTCTGCTTCGCTCTACGCCGTTAAAGTTCT TGATGCACGTGACGGAGATGGCCAATACTCATGGAT |

(continued)

| Table 4-1. Primer Sequences and Mutations Introduced in Combinatorial Variants of BPN'3 | | |
|---|---|---|
| SEQ ID NO: | Primer Name | Sequence |
| 148 | P4996 | ATCCATGTTATTCGCGATGGCCCATTCGATGCCGTTGATAATCC ATGAGTATTGGCCATCTCCGTCACGTGCATCAAGAA |
| 149 | P4997 | CTTGGTGTAGCCCCGTCTGCTTCGCTCTACGCCGTTAAAGTTCT TGCAGCAGACTCTTCTTCAGGCCAATACTCATGGAT |
| 150 | P4998 | ATCCATGTTATTCGCGATGGCCCATTCGATGCCGTTGATAATCC ATGAGTATTGGCCTGAAGAAGAGTCTGCTGCAAGAA |
| 151 | P4999 | CTTGGTGTAGCCCCGTCTGCTTCGCTCTACGCCGTTAAAGTTCT TGCAGCAGACTCTGGAGATGGCCAATACTCATGGAT |
| 152 | P5000 | ATCCATGTTATTCGCGATGGCCCATTCGATGCCGTTGATAATCC ATGAGTATTGGCCATCTCCAGAGTCTGCTGCAAGAA |
| 153 | P5001 | CTTGGTGTAGCCCCGTCTGCTTCGCTCTACGCCGTTAAAGTTCT TGCAGCAGACTCTGATGGCCAATACTCATGGATTAT |
| 154 | P5002 | ATCCATGTTATTCGCGATGGCCCATTCGATGCCGTTGATAATCC ATGAGTATTGGCCATCAGAGTCTGCTGCAAGAACTT |
| 155 | P5003 | ATCGAATGGGCCATCGCGAAT |
| 156 | P5004 | TGCAACAGCTTTATCAACTGCT |
| 157 | P5005 | ATCGAATGGGCCATCGCGAATAACATGGATGTATCTGGAATGA GCCTGGGAGCACCAAGCGGCAGT |
| 158 | P5006 | TGCAACAGCTTTATCAACTGCTGCTTTAAGTGCCGCACTGCCG CTTGGTGCTCCCAGGCTCATTCCAGAT |
| 159 | P5007 | ATCGAATGGGCCATCGCGAATAACATGGATGTAATCAACATGA GCCTGGGATCTCCAAGCGGCAGT |
| 160 | P5008 | TGCAACAGCTTTATCAACTGCTGCTTTAAGTGCCGCACTGCCG CTTGGAGATCCCAGGCTCATGTTGATT |

| Table 4-1. Primer Sequences and Mutations Introduced in Combinatorial Variants of BPN'3 | | |
|---|---|---|
| SEQ ID NO: | Primer Name | Sequence |
| 161 | P5009 | ATCGAATGGGCCATCGCGAATAACATGGATGTAATCAACATGA GCCTGGGAGCAGTTAGCGGCAGT |
| 162 | P5010 | TGCAACAGCTTTATCAACTGCTGCTTTAAGTGCCGCACTGCCG CTAACTGCTCCCAGGCTCATGTTGATT |
| 163 | P5011 | ATCGAATGGGCCATCGCGAATAACATGGATGTAATCAACATGA GCCTGGGAGCAGATAGCGGCAGT |
| 164 | P5012 | TGCAACAGCTTTATCAACTGCTGCTTTAAGTGCCGCACTGCCG CTATCTGCTCCCAGGCTCATGTTGATT |
| 165 | P5013 | ATCGAATGGGCCATCGCGAATAACATGGATGTAATCAACATGA GCCTGGGAGCAAGCGGCAGTGCGGCA |

(continued)

**Table 4-1. Primer Sequences and Mutations Introduced in Combinatorial Variants of BPN'3**

| SEQ ID NO: | Primer Name | Sequence |
|---|---|---|
| 166 | P5014 | TGCAACAGCTTTATCAACTGCTGCTTTAAGTGCCGCACTGCCGCTTGCTCCCAGGCTCATGTTGATTACA |
| 167 | P5015 | ATCGAATGGGCCATCGCGAATAACATGGATGTAATCAACATGAGCCTGGGAGCAGGAGGCAGTGCGGCA |
| 168 | P5016 | TGCAACAGCTTTATCAACTGCTGCTTTAAGTGCCGCACTGCCTCCTGCTCCCAGGCTCATGTTGATTACA |
| 169 | P5017 | ATCGAATGGGCCATCGCGAATAACATGGATGTAATCAACATGAGCCTGGGAGCACCAGTTGATAT |
| 170 | P5018 | TGCAACAGCTTTATCAACTGCTGCTTTAAGTGCCGCGATATCAACTGGTGCTCCCAGGCTCATGTTGATT |
| 171 | P5019 | ATCGAATGGGCCATCGCGAATAACATGGATGTAATCAACATGAGCCTGGGAGCACCAAGCGGCAGT |
| 172 | P5020 | TGCAACAGCTTTATCAACTGCTGCTTTAAGTGTCGCACTGCCGCTTGGTGCTCCCAGGCTCATGTTGATT |
| 173 | P5021 | ATCGAATGGGCCATCGCGAATAACATGGATGTATCTGGAATGAGCCTGGGATCTCCAAGCGGCAGT |
| 174 | P5022 | TGCAACAGCTTTATCAACTGCTGCTTTAAGTGCCGCACTGCCGCTTGGAGATCCCAGGCTCATTCCAGAT |
| 175 | P5023 | ATCGAATGGGCCATCGCGAATAACATGGATGTATCTGGAATGAGCCTGGGAGCAGTAAGCGGCAGT |
| 176 | P5024 | TGCAACAGCTTTATCAACTGCTGCTTTAAGTGCCGCACTGCCGCTTACTGCTCCCAGGCTCATTCCAGAT |
| 177 | P5025 | ATCGAATGGGCCATCGCGAATAACATGGATGTATCTGGAATGAGCCTGGGAGCAGATAGCGGCAGT |
| 178 | P5026 | TGCAACAGCTTTATCAACTGCTGCTTTAAGTGCCGCACTGCCGCTATCTGCTCCCAGGCTCATTCCAGAT |
| 179 | P5027 | ATCGAATGGGCCATCGCGAATAACATGGATGTATCTGGAATGAGCCTGGGAGCAAGCGGCAGTGCGGCA |
| 180 | P5028 | TGCAACAGCTTTATCAACTGCTGCTTTAAGTGCCGCACTGCCGCTTGCTCCCAGGCTCATTCCAGATACATCCATGTT |

**Table 4-1. Primer Sequences and Mutations Introduced in Combinatorial Variants of BPN'3**

| SEQ ID NO: | Primer Name | Sequence |
|---|---|---|
| 181 | P5029 | ATCGAATGGGCCATCGCGAATAACATGGATGTAATCAACATGAGCCTGGGATCTGTTAGCGGCAGT |
| 182 | P5030 | TGCAACAGCTTTATCAACTGCTGCTTTAAGTGCCGCACTGCCGCTAACAGATCCCAGGCTCATGTTGATT |

(continued)

| Table 4-1. Primer Sequences and Mutations Introduced in Combinatorial Variants of BPN'3 | | |
|---|---|---|
| SEQ ID NO: | Primer Name | Sequence |
| 183 | P5031 | ATCGAATGGGCCATCGCGAATAACATGGATGTAATCAACATGA GCCTGGGATCTGATAGCGGCAGT |
| 184 | P5032 | TGCAACAGCTTTATCAACTGCTGCTTTAAGTGCCGCACTGCCG CTATCAGATCCCAGGCTCATGTTGATT |
| 185 | P5033 | ATCGAATGGGCCATCGCGAATAACATGGATGTAATCAACATGA GCCTGGGATCTAGCGGCAGTGCGGCA |
| 186 | P5034 | TGCAACAGCTTTATCAACTGCTGCTTTAAGTGCCGCACTGCCG CTAGATCCCAGGCTCATGTTGATT |
| 187 | P5035 | TGATCCGTCTGAGCAGGGGCTTTAATTTGTGA |
| 188 | P5036 | ATTAAAGCCCCTGCTCAGACGGATCAGGCCAATACTC |

Each amplification reaction contained 30 pmol of each oligonucleotide and 100 ng of the template DNA. Amplifications were carried out using Vent DNA polymerase (New England Biolabs). The PCR mix (20 ul) was initially heated at 95°C for 2.5 min followed by 30 cycles of denaturation at 94°C for 15s, annealing at 55°C for 15s and extension at 72°C for 40s. Following amplification all the fragments were purified by the Gel Band Purification kit (Qiagen) and mixed to serve as templates for a fusion PCR using primers P4975 and P4948. The full-length DNA fragment from the fusion PCR reaction was gel purified by the QIAGEN gel-band purification kit, digested by the BamHI and HindIII restriction enzymes and ligated in the *Bacillus* expression plasmid *pHPLT-BPN* partial opt (Figure 2C), which was cut with the same restriction enzymes.

Ligation mixtures were amplified using rolling circle amplification according to manufacturer's recommendation (Epicentre). One ul of the ligation mixture was mixed with 5 ul of the sample buffer, heated to 95°C for 3 min and cooled on ice. Next, 5 ul of the reaction buffer and 0.2 ul of the enzyme was added to each tube, followed by incubation at 30°C for 10 hours. Products of the rolling circle amplification were diluted 100 times and 1 ul of this dilution was used to transform *Bacillus subtilis* cells (genotype: *ΔaprE, ΔnprE, ΔspoHE, amyE::xylRPxylAcomK-phleo*) as described in Example 2. Transformations were plated on LB plates containing 1.6% skim milk and 10 ppm Neomycin and incubated overnight at 37°C. Only colonies with halos were picked and grown in microtiter plates for further analysis as described in Example 2. Protein concentration of culture supernatants was determined by TCA precipitation as described in Example 1.

| Table 4.5: Combinatorial Libraries of BPN': G97A-G128A-Y217Q Parent | |
|---|---|
| Variant | Name |
| **1** | V093Z/K094S/V095C/L096S/C097A/G128A/Y217Q |
| 2 | Z096.01D/G097C/A098R/C128A/Y217Q |
| 3 | C097A/X099.01S/G128A/Y217Q |
| 4 | C1097A/G100S/G128A/Y217Q |
| 5 | G097A/S101D/G128A/Y217Q |
| 6 | Z096.01D/G097G/A098R/Z099.01S/G128A/Y217Q |
| 7 | Z096.01D/G097G/A098R/G100S/G128A/Y217Q |
| 8 | Z096.01D/G097G/A098R/S101D/G128A/Y217Q |
| 9 | G097A/X099.01S/G100S/G128A/Y217Q |
| 10 | G097A/Z099.01S/S101D/G128A/Y217Q |
| 11 | G097A/G100S/S101D/G128A/Y217Q |

(continued)

| Table 4.5: Combinatorial Libraries of BPN': G97A-G128A-Y217Q Parent | |
|---|---|
| Variant | Name |
| 12 | G097A/I122S/N123G/G128A/Y217Q |
| 13 | V093Z/K094S/V095C/L096S/G097A/I122S/N123G/G128A/Y217Q |
| 14 | Z096.01D/G097G/A098R/I122S/N123G/G128A/Y217Q |
| 15 | G097A/Z099.01S/I122S/N123G/G128A/Y217Q |
| 16 | G097A/G100S/I122S/N123G/G128A/Y217Q |
| 17 | G097A/S101D/I122S/N123G/G128A/Y217Q |
| 18 | Z096.01D/G097G/A098R/Z.099.01S/I122S/N123G/G128A/Y217Q |
| 19 | Z096.01D/G097G/A098R/G100S/I122S/N123G/G128A/Y217Q |
| 20 | Z096.01D/C097C/A098R/S101D/I122S/N123G/G128A/Y217Q |
| 21 | G097A/Z099.01S/G100S/I122S/N123G/G128A/Y217Q |
| 22 | G097A/Z099.01S/S101D/I122S/N123G/G128A/Y217Q |
| 23 | G097A/C100S/S101D/I122S/N123G/G128A/Y217Q |
| 24 | G097A/G128S/Y217Q |
| 25 | V093Z/K094S/V095C/L096S/G097A/G128S/Y217Q |
| 26 | Z096.01D/G097G/A097R/G128S/Y217Q |
| 27 | G097A/Z099.01S/G128S/Y217Q |
| 28 | G097A/G100S/G128S/Y217Q |
| 29 | G097A/S101D/G128S/Y217Q |
| 30 | Z096.01D/G097G/A098R/Z099.01S/G128S/Y217Q |
| 31 | Z096.01D/G097G/A098R/G100S/G128S/Y217Q |
| 32 | Z096.01D/G097G/A098R/S101D/G128S/Y217Q |
| 33 | G097A/Z099.01S/G100S/G128S/Y217Q |
| 34 | G097A/Z099.01S/S101D/G128S/Y217Q |
| 35 | G097A/G100S/S101D/G128S/Y217Q |
| 36 | G097A/G128A/P129V/Y217Q |
| 37 | V093Z/K094S/V095C/L096S/G097A/G128A/P129V/Y217Q |
| 38 | Z096.01D/G097G/A098R/G128A/P129V/Y217Q |
| 39 | G097A/Z099.01S/G128A/P129V/Y217Q |
| 40 | G097A/G100S/G128A/P129V/Y217Q |
| 41 | G097A/S101D/G128A/P129V/Y217Q |
| 42 | Z096.01D/G097G/A098R/Z099.01S/G128A/P129V/Y217Q |
| 43 | Z096.01D/G097G/A098R/G100S/G128A/P129V/Y217Q |
| 44 | Z096.01D/G097G/A098R7S101D/G128A/P129V/Y217Q |
| 45 | G097A/Z099.01S/G100S/G128A/P129V/Y217Q |
| 46 | G097A/Z099.01S/S101D/G128A/P129V/Y217Q |
| 47 | G097A/G100S/S101D/G128A/P129V/Y217Q |
| 48 | G097A/G128A/P129D/Y217Q |

(continued)

| Table 4.5: Combinatorial Libraries of BPN': G97A-G128A-Y217Q Parent | |
|---|---|
| **Variant** | **Name** |
| 49 | V093Z/K094S/V095C/L096S/G097A/G128A/P129D/Y217Q |
| 50 | Z096.01D/G097G/A098R/G128A/P129D/Y217Q |
| 51 | G097A/Z099.01S/G128A/P129D/Y217Q |
| 52 | G097A/G100S/G128A/P129D/Y217Q |
| 53 | G097A/S101D/G128A/P129D/Y217Q |
| 54 | Z096.01D/G097G/A098R/Z099.01S/G128A/P129D/Y217Q |
| 55 | Z096.01D/G097G/A098R/G100S/G128A/P129D/Y217Q |
| 56 | Z096.01D/G097G/A098R/S101D/G128A/P129D/Y217Q |
| 57 | G097A/Z099.01S/G100S/G128A/P129D/Y217Q |
| 58 | G097A/Z099.01S/S101D/G128A/P129D/Y217Q |
| 59 | G097A/G100S/S101D/G128A/P129D/Y217Q |
| 60 | G097A/G128A/P129Z/Y217Q |
| 61 | V093Z/K094S/V095C/L096S/G097A/G128A/P129Z/Y217Q |
| 62 | Z096.01D/G097G/A098R/G128A/P129Z/Y217Q |
| 63 | G097A/Z099.01S/G128A/P129Z/Y217Q |
| 64 | G097A/G100S/G128A/P129Z/Y217Q |
| 65 | G097A/S101D/G128A/P129Z/Y217Q |
| 66 | Z096.01D/G097G/A098R/Z099.01S/G128A/P129Z/Y217Q |
| 67 | Z096.01D/G097G/A098R/G100S/G128A/P129Z/Y217Q |
| 68 | Z096.01D/G097G/A098R/S101D/G128A/P129Z/Y217Q |
| 69 | G097A/Z099.01S/G100S/G128A/P129Z/Y217Q |
| 70 | G097AF/099.01S/S101D/G128A/P129Z/Y217Q |
| 71 | G097A/C100S/S101D/G128A/P1297/Y217Q |
| 72 | G097A/G128A/P129G/S130Z/Y217Q |
| 73 | V093Z/K094S/V095C/L096S/G097A/G128A/P129G/S130Z/Y217Q |
| 74 | Z096.01D/G097G/A098R/G128A/P129G/S130Z/Y217Q |
| 75 | G097A/Z099,01S/G128A/P129G/S130Z/Y217Q |
| 76 | G097A/G100S/G128A/P129G/S130Z/Y217Q |
| 77 | G097A/S101D/G128A/P129G/S130Z/Y217Q |
| 78 | Z096.01D/G097G/A098R/Z099.01S/G128A/P129G/S130Z/Y217Q |
| 79 | Z096.01D/G097G/A098R/G100S/G128A/P129G/S130Z/Y217Q |
| 80 | Z096.01D/G097G/A098R/S101D/G128A/P129G/S130Z/Y217Q |
| 81 | G097A/Z099.01S/G100S/G128A/P129G/S130Z/Y217Q |
| 82 | G097A/Z099.01S/S101D/G128A/P129G/S130Z/Y217Q |
| 83 | G097AG100S/S101D/G128A/P129G/S130Z/Y217Q |
| 84 | G097A/G128A/S130V/G131D/S1321/Y217Q |
| 85 | V093Z/K094S/V095C/L096S/G097A/G128A/S130V/G131D/S132I/Y217Q |

(continued)

| Table 4.5: Combinatorial Libraries of BPN': G97A-G128A-Y217Q Parent | |
|---|---|
| **Variant** | **Name** |
| 86 | Z096.01D/G097G/A098R/G128A/S130V/G131D/S132I/Y217Q |
| 87 | G097A/Z099.01S/G128A/S130V/G131D/S132I/Y217Q |
| 88 | G097A/G100S/G128A/S130V/G131D/S1321/Y217Q |
| 89 | G097A/S101D/G128A/S130V/G131D/S132I/Y217Q |
| 90 | Z096.01D/G097G/A098R/Z099.01S/G128A/S130V/G131D/S132I/Y217Q |
| 91 | Z096.01D/G097G/A098R/G100S/G128A/S130V/G131D/S132I/Y217Q |
| 92 | Z096.01D/G097G/A098R/S101D/G128A/S130V/G131D/S132I/Y217Q |
| 93 | G097A/Z099.01S/G100S/G1 28A/S 130V/G131D/S132I/Y217Q |
| 94 | G097A/Z099.01S/S101D/G128A/S130V/G131D/S132I/Y217Q |
| 95 | G097A/G100S/S101D/G128A/S130V/G131D/S132I/Y217Q |
| 96 | G097A/G128A/A134T/Y217Q |
| 97 | V093Z/K094S/V095C/L096S/G097A/G128A/A134T/Y217Q |
| 98 | Z096.01D/G097G/A098R/G128A/A134T/Y217Q |
| 99 | G097A/Z099.01S/G128A/A134T/Y217Q |
| 100 | G097A/G100S/G128A/A134T/Y217Q |
| 101 | G097A/S101D/G128A/A134T/Y217Q |
| 102 | Z096.01D/G097G/A098R/Z099.01S/G128A/A134T/Y217Q |
| 103 | Z096.01D/G097G/A098R/G100S/G128A/A134T/Y217Q |
| 104 | Z096.01D/G097G/A098R/S101D/G128A/A134T/Y217Q |
| 105 | G097A/Z099.01S/G100S/G128A/A134T/Y217Q |
| 106 | G097A/G099.01S/S101D/G128A/A134T/Y217Q |
| 107 | G097A/G100S/S101D/G128A/A134T/Y217Q |
| 108 | G097A/I122S/N123G/G128S/Y217Q |
| 109 | V093Z/K094S/V095C/L096S/G097A/I122S/N123G/G128S/Y217Q |
| 110 | Z096.01D/G097G/A098R/I122S/N123G/G128S/Y217Q |
| 111 | G097A/Z099.01S/I22S/N123G/G128S/Y217Q |
| 112 | G097A/G100S/I122S/N123G/G128S/Y217Q |
| 113 | G097A/S101D/I122S/N123G/G128S/Y217Q |
| 114 | Z096.01D/G097G/A098R/Z099.01S/I122S/N123G/G128S/Y217Q |
| 115 | Z096.01D/G097G/A098R/G100S/I122S/N123G/G128S/Y217Q |
| 116 | Z096.01D/G097G/A098R/S101D/I122S/N123G/G128S/Y217Q |
| 117 | G097A/Z099.01S/G100S/I122S/N123G/G128S/Y217Q |
| 118 | G097A/Z099.01SS101D/I122S/N123G/G128S/Y217Q |
| 119 | G097A/G100S/S101D/I122S/N123G/G128S/Y217Q |
| 120 | G097A/I122S/N123G/G128A/P129V/Y217Q |
| 121 | V093Z/K094S/V091G/L096S/G097A/I122S/N123G/G128A/P129V/Y217Q |
| 122 | Z096.01D/G097G/A098R/I122S/N123G/G128A/P129V/Y217Q |

(continued)

| Table 4.5: Combinatorial Libraries of BPN': G97A-G128A-Y217Q Parent | |
|---|---|
| Variant | Name |
| 123 | G097A/Z099.01S/I122S/N123G/G128A/P129V/Y217Q |
| 124 | G097A/G100S/I122S/N123G/G128A/P129V/Y217Q |
| 125 | G097A/S101D/I122S/N123G/G128A/P129V/Y217Q |
| 126 | Z096.01D/G097G/A098R/Z099.01S/I122S/N123G/G128A/P129V/Y217Q |
| 127 | Z096.01D/G097G/A098R/G100S/I122S/N123G/G128A/P129V/Y217Q |
| 128 | Z096.01D/G097G/A098R/S101D/I122S/N123G/G128A/P129V/Y217Q |
| 129 | G097A/Z099.01S/G100S/I122S/N123G/G128A/P129V/Y217Q |
| 130 | G097A/Z099.01S/S101D/I122S/N123G/G128A/P129V/Y217Q |
| 131 | G097A/G100S/S101D/I122S/N123G/G128A/P129V/Y217Q |
| 132 | G097A/I122S/N123G/G128A/P129D/Y217Q |
| 133 | V093Z/K094S/V095C/L096S/G097A/I122S/N123G/G128A/P129D/Y217Q |
| 134 | Z096.01D/G097G/A098R/I122S/N123G/G128A/P129D/Y217Q |
| 135 | G097A/Z099.01S/I122S/N123G/G128A/P129D/Y217Q |
| 136 | G097A/G100S/I122S/N123G/G128A/P129D/Y217Q |
| 137 | G097A/S101D/I122S/N123G/G128A/P129D/Y217Q |
| 138 | Z096.01D/G097G/A098R/Z099.01S/I122S/N123G/G128A/P129D/Y217Q |
| 139 | Z096.01D/G097G/A098R/G100S/I122S/N123G/G128A/P129D/Y217Q |
| 140 | Z096.01D/G097G/A098R/S101D/I122S/N123G/G128A/P129D/Y217Q |
| 141 | G097A/Z099.01S/G100S/I122S/N123G/I128A/P129D/Y217Q |
| 142 | G097A/Z099.01S/S101D/I122S/N123G/I128A/P129D/Y217Q |
| 143 | G097A/G100S/S101D/I122S/N123G/G128A/P129D/Y217Q |
| 144 | G097A/1122S/N123G/G128A/P129Z/Y217Q |
| 145 | V093Z/K094S/V095C/L096S/G097A/I122S/N123G/G128A/P129Z/Y217Q |
| 146 | Z096.01D/G097G/A098R/I122S/N123G/G128A/P129Z/Y217Q |
| 147 | G097A/Z099.01S/I122S/N123G/G128A/P129Z/Y217Q |
| 148 | G097A/G100S/I122S/N123G/G128A/P129Z/Y217Q |
| 149 | G097A/S101D/1122S/N123G/G128A/P129Z/Y217Q |
| 150 | Z096.01D/G097G/A098R/Z099.01S/1122S/N123G/G128A/P129Z/Y217Q |
| 151 | Z096.01D/G097G/A098R/G100S/I122S/N123G/G128A/P129Z/Y217Q |
| 152 | Z096.01D/G097G/A098R/S101D/I122S/N123G/G128A/P129Z/Y217Q |
| 153 | G097A/Z099.01S/G100S/I122S/N123G/G128A/P129Z/Y217Q |
| 154 | G097A/Z099.01S/S101D/I122S/N123G/G128A/P129Z/Y217Q |
| 155 | G097A/G100S/S101D/I122S/N123G/G128A/P129Z/Y217Q |
| 156 | G097A/G128S/P129V/Y217Q |
| 157 | V093Z/K094S/V095C/L096S/G097A/G128S/P129V/Y217Q |
| 158 | Z096.01D/G097G/A098R/G128S/P129V/Y217Q |
| 159 | G097A/Z099.01S/G128S/P129V/Y217Q |

(continued)

| Table 4.5: Combinatorial Libraries of BPN': G97A-G128A-Y217Q Parent | |
|---|---|
| Variant | Name |
| 160 | G097A/G100S/G128S/P129V/Y217Q |
| 161 | G097A/S101D/G128S/P129V/Y217Q |
| 162 | Z096.01D/G097G/A098R/Z099.01S/G128S/P129V/Y217Q |
| 163 | Z096.01D/G097G/A098R/G100S/G128S/P129V/Y217Q |
| 164 | Z096.01D/G097G/A098R/S101D/G128S/P129V/Y217Q |
| 165 | G097A/Z099.01S/G100S/G128S/P129V/Y217Q |
| 166 | G097A/Z099.01S/S101D/G128S/P129V/Y217Q |
| 167 | G097A/G100S/S101D/G128S/P129V/Y217Q |
| 168 | G097A/G128S/P129D/Y217Q |
| 169 | V093Z/K094S/V095C/L096S/G097A/G128S/P129D/Y217Q |
| 170 | Z096.01D/G097G/A098R/G128S/P129D/Y217Q |
| 171 | G097A/Z099.01S/G128S/P129D/Y217Q |
| 172 | G097A/G100S/G128S/P129D/Y217Q |
| 173 | G097A/S101D/G128S/P129D/Y217Q |
| 174 | Z096.01D/G097G/A098R/Z099.01S/G128S/P129D/Y217Q |
| 175 | Z096.01D/G097G/A098R/G100S/G128S/P129D/Y217Q |
| 176 | Z096.01D/G097G/A098R/S101D/G128S/P129D/Y217Q |
| 177 | G097A/Z099.01S/G100S/G128S/P129D/Y217Q |
| 178 | G097A/Z099.01S/S101D/G128S/P129D/Y217Q |
| 179 | G097A/G100S/S101D/G128S/P129D/Y217Q |
| 180 | G097A/G128S/P129Z/Y217Q |
| 181 | V093Z/K094S/V095C/L096S/G097A/G128S/P129Z/Y217Q |
| 182 | Z096.01D/G097G/A098R/G128S/P129Z/Y217Q |
| 183 | G097A/Z099.01S/G128S/P129Z/Y217Q |
| 184 | G097A/G100S/G128S/P129Z/Y217Q |
| 185 | G097A/S101D/G128S/P129Z/Y217Q |
| 186 | Z096.01D/G097G/A098R/Z099.01S/G128S/P129Z/Y217Q |
| 187 | Z096.01D/G097G/A098R/G100S/G128S/P1297/Y217Q |
| 188 | Z096.01D/G097G/A098R/S101D/G128S/P1297/Y217Q |
| 189 | G097A/Z099.01S/G100S/G128S/P129Z/Y217Q |
| 190 | G097A/Z099.01S/S100D/G128S/P129Z/Y217Q |
| 191 | G097AG100S/S101D/G128S/P129Z/Y217Q |

**EXAMPLE 5**

**Stain Removal Performance of Combinatorial Variants of BPN' 3**

The stain removal activity of combinatorial variants of BPN' 3 generated as described in Example 4 was tested as described in Example 3. As described throughout, functionality of BPN' variants, was quantified as a performance

index (Pi), which is the ratio of performance of a variant to parent protein BPN'3. BPN'3 variants showing a Pi value greater or equal than 0.5 for BMI stain removal performance and/or TCA precipitation showed improved cleaning benefits and/or expression. Performance indices less than or equal to 0.05 were fixed to 0.05 and indicated in bold italics in the table. Results are shown in Table 5-1.

**Table 5-1: Stain Removal Performance and Expression of Combinatorial Variants of BPN'3**

| Variants | TCA | BMI pH 8 /16°C |
|---|---|---|
| V072G/G097A/G128A/Y217Q | 0.93 | 1.08 |
| G097A/G128S/Y217Q | 1.16 | 1.06 |
| G128A/Y217Q | 1.07 | 1.05 |
| G097A/G128S/P129D/Y217Q | 0.85 | 1.03 |
| G097A/G128A/A134T/Y217Q | 0.56 | 1.03 |
| M124V/L126A/Y217Q | 1.05 | 1.03 |
| G097A/G128A/P129D/Y217Q/A232P | 0.46 | 1.01 |
| G097A/G128A/P129D/Y217Q | 0.57 | 1.00 |
| G097A/G128S/P129V/A134T/Y217Q | 0.29 | 0.95 |
| G097A/G128S/P129V/Y217Q | 0.31 | 0.95 |
| V068I/G097A/G128S/P129D/Y217Q | 0.41 | 0.85 |
| G097A/S101D/G128S/S204F/Y217Q/S236F/T254P | 0.41 | 0.79 |
| G097A/S101D/G128S/Y217Q | 0.43 | 0.76 |
| G097A/G128A/P129V/Y217Q | 0.30 | 0.70 |
| G097A/S101D/G128A/Y217Q | 0.50 | 0.64 |
| G097A/S101D/G128S/P129D/Y217Q | 0.35 | 0.63 |
| G097A/S101D/G128A/A134T/Y217Q | 0.40 | 0.58 |
| G097A/Z099.01S/A114S/G128S/Y217Q | 0.21 | 0.53 |
| G097A/S101D/G128A/P194L/Y217Q | 0.35 | 0.49 |
| G097A/Z099.01S/G128S/Y217Q | 0.39 | 0.38 |
| G097A/S101D/G128A/P129D/Y217Q | 0.36 | 0.36 |
| Q019R/G097A/Z099.01S/G128S/P129D/Y217Q | 0.33 | 0.33 |
| G097A/G100S/G128S/Y217Q | 0.46 | 0.25 |
| G097A/S101D/G128S/P129V/Y217Q | 0.39 | 0.22 |
| G097A/Z099.01S/G128A/A134T/Y217Q | 0.35 | 0.16 |
| G097A/G100S/I107V/G128S/P129D/Y217Q | 0.37 | 0.15 |
| G097A/G100S/G128S/Y217Q/A230V | 0.38 | 0.13 |
| G097A/G100S/G128A/Y217Q/K237N | 0.39 | 0.12 |
| G097A/Z099.01S/G128S/P129D/Y217Q | 0.37 | 0.12 |
| **Variants** | **TCA** | **BMI pH 8/16°C** |
| G097A/G100S/G128S/P129D/Y217Q | 0.37 | 0.07 |
| Z096.01D/G097A/A098R/G128A/Y217Q | 0.36 | ***0.05*** |
| G097A/Z099.01S/I122S/N123G/G128A/Y217Q | 0.37 | ***0.05*** |

(continued)

| Variants | TCA | BMI pH 8/16°C |
|---|---|---|
| G097A/Z099.01S/I122S/N123G/G128A/Y217Q/T220A/A232T | 0.38 | ***0.05*** |
| P005S/G097A/Z099.01S/S101D/G128A/Y217Q | 0.42 | ***0.05*** |
| G097A/S101D/G128A/P129V/Y217Q | 0.46 | ***0.05*** |
| V093Z/K094S/V095C/L096S/G097A/G128A/Y217Q | 0.50 | ***0.05*** |
| G097A/G100S/S101D/G128A/Y217Q/A232P | 0.51 | ***0.05*** |
| Z096.01D/G097A/A098R/S101D/G128A/Y217Q | 0.53 | ***0.05*** |
| G097A/Z099.01S/G100S/G128A/Y217Q | 0.53 | ***0.05*** |

## EXAMPLE 6

### Construction of Combinatorial Variants of BPN' 3 at Positions 128, 129, and 130

Combinatorial libraries focused around positions 128/129/130 in BPN'G97A-G128A-Y217Q (BPN'3) were created using site specific saturation mutagenesis and/or site specific insertion and/or deletion. The variants generated contain substitution, insertion, and deletion mutations. Libraries L1, L2 and L3 are based on WT position S130S and L4 is based on S130A. Four saturation libraries focused around positions 128/129 and 130 were constructed as shown in Table 6-1.

| Table 6-1: Construction of Saturation Library at Positions 128, 129 and 130 in BPN'3 | | | | | | |
|---|---|---|---|---|---|---|
| Library | Position 128 | Insertion | Position 129 | Position 130 | Short Name | Degenerate Primer Names |
| **L1** | NNS | | NNS | S | XX | 128/129 deg QC F/R |
| **L2** | deletion | | NNS | S | -X | 128/129 del QC F/R |
| **L3** | NNS | Λ | NNS | S | XAX | 128 A 129 QC F/R |
| **L4** | deletion | | NNS | A | -XΛ | 128/129 del A QC F/R |

Prior to library construction, two stop codons were introduced at positions 128 and 129 in the BPN3' sequence to inactivate the protease. This was done to ensure a low background of the parent plasmid in the libraries. The parent plasmid (pHPLT- BPN'3) was methylated using two micrograms of plasmid DNA and methylase (New England Biolabs), according to the NEB protocol. Methylated DNA was then purified with DNA Clean and Concentrator kit from Zymo Research. Mutagenesis was performed using methods known in the art (*See*, Amin et al., Biotechniques 35: 1134- 1140, |2003|) using a modified version of the QuikChange multi site- directed mutagenesis (QCMS) kit from Stratagene.

The mutagenesis reaction contained 60 ng template plasmid, 0.5 ul forward primer 128/129 Stop F (25 uM), 0.5 ul reverse primer 128/129 Stop R (25 uM), 1 ul dNTP's (QCMS kit), 2.5 ul 10x QCMS reaction buffer, 18.5 ul deionized water, and 1 ul of enzyme blend (QCMS kit), for a total volume of 25ul. For the mutagenesis reaction, the thermocycler program used was 1 cycle at 95° for 1 min., followed by 25 cycles of 95°C for 1 min, 55°C for 1 min., and 68°C for 11 minutes (MJ Research thermocycler). The template DNA was digested by DpnI (QCMS kit, Qiagen) and 1.5uL of the reaction was amplified by rolling circle amplification (RCA) using the Templiphi kit (Amersham) using manufacturer's protocol.

| Table 6-2. Primers | | |
|---|---|---|
| **SEQ ID NO:** | **Primer Name** | **Primer Sequence** |
| 189 | 128/129 Stop F | /5Phos/CATGAGCCTGGGATAATGAA GCGGCAGTGCGGCACTTAAAGCAG |

(continued)

| Table 6-2. Primers | | |
|---|---|---|
| SEQ ID NO: | Primer Name | Primer Sequence |
| 190 | 128/129 Stop R | /5Phos/GTGCCGCACTGCCGCTTCAT TATCCCAGGCTCATGTTGATTACATC |

One microliter of the amplified DNA was used to transform 100 μL of competent *Bacillus subtilis* cells (genotype: *ΔaprE, ΔnprE, ΔspoHE, amyE::xylRPxylAcomK-phleo*). Aliquots of 20 uL or 80 uL of the transformation mixture were plated on Luria Agar plates supplemented with 10 μg/ml neomycin + 1.6% skim milk (Teknova). After growth, 4 colonies not forming halos on the skim milk plates were picked to identify the plasmid with the correct sequence. The correct variant with the two stop codons at positions 128/129, called "pIIPLT-BPN' 128/129 Stop" was chosen as the parent plasmid for the construction of combinatorial libraries described in Table 6-1.

Plasmid pIIPLT-BPN' 128/129 Stop was methylated and used as template with the degenerate primer pairs shown in Table 6-3.

| Table 6-3: NNS Mutagenesis Primer Sequences | | |
|---|---|---|
| SEQ ID NO. | Primer Name | Sequence |
| 191 | 128/129 deg QC F | /5Phos/CAT GAG CCT GGG ANN SNN SAG CGG CAG TGC GGC ACT TAA AGC AG |
| 192 | 128/129 deg QC R | /5Phos/CCG CAC TGC CGC TSN NSN NTC CCA GGC TCA TGT TGA TTA CAT C |
| 193 | 128/129 del QC F | /5Phos/CAT GAG CCT GGG ANN SAG CGG CAG TGC GGC ACT TAA AGC AG |
| 194 | 128/129 del QC R | /5Phos/CCG CAC TGC CGC TSN NTC CCA GGC TCA TGT TGA TTA CAT C |
| 195 | 128 A 129 QC F | /5Phos/CAT GAG CCT GGG ANN SGC GNN SAG CGG CAG TGC GGC ACT TAA AGC AG |
| 196 | 128 A 129 QC R | /5Phos/CCG CAC TGC CGC TSN NCG CSN NTC CCA GGC TCA TGT TGA TTA CAT C |
| 197 | 128/129 del A QC F | /5Phos/CAT GAG CCT GGG ANN SGC AGG CAG TGC GGC ACT TAA AGC AG |
| 198 | 128/129 del A QC R | /5Phos/GTG CCG CAC TGC CTG CSN NTC CCA GGC TCA TGT TGA TTA CAT C |

Overlapping forward and reverse primers (PAGE- purified) with the NNS codon (s) in the middle and 17- 20 flanking bases were designed for each chosen library, and the sequences for each forward (F) and reverse (R) primer are shown in Table 6- 3. Quik- change mutagenesis and rolling circle amplification for the 4 reactions was performed as described above. Transformations were plated on Luria Agar plates supplemented with 10 μg/ml neomycin + 1.6% skim milk. All halo forming colonies were picked into two microtiter plates containing 125ul LB medium supplemented with 10ug/ml neomycin. The clones were sequenced by Quintara Biosciences. For protein expression, cultures were grown overnight in micro- filter plates (. 22um, Millipore) containing 180ul of a enriched semi- defined media based on MOPs buffer, with urea as major nitrogen source, glucose as the main carbon source, 1% soytone for robust cell growth, and supplemented with 2.5ug/ml neomycin. Cultures were grown for 64 hours at 37C, 250rpm, and 70% humidity. Protein concentration of culture supernatants was determined by TCA precipitation as described in Example 1.

**EXAMPLE 7**

**Stain Removal Performance of Combinatorial Variants of BPN' 3 at positions 128, 129, and 130**

The stain removal activity of combinatorial variants of BPN' 3 at positions 128, 129, and 130 generated as described in Example 6 was tested as described in Example 3. As described throughout, functionality of BPN' variants, was quantified as a performance index (*Pi*), which is the ratio of performance of a variant to parent protein BPN'3. BPN'3 variants showing a Pi value greater or equal than 0.5 for BMI stain removal performance and/or TCA precipitation showed improved cleaning benefits and/or expression. Results are shown in Table 7-1.

**Table 7-1: Stain Removal Performance and Expression of BPN' 3 Variants at Positions 128, 129, and 130**

| Variant | TCA | BMI |
|---|---|---|
| G097A/G128S/P129T/Y217Q | 0.77 | 1.04 |
| G097A/G128A/P129S/Y217Q | 0.70 | 1.02 |
| G097A/G128S/P129C/Y217Q | 0.61 | 1.00 |
| G097A/G128S/P129Q/Y217Q | 0.96 | 1.00 |
| G097A/G128S/P129F/Y217Q | 0.99 | 0.99 |
| G097A/P129S/Y217Q | 0.87 | 0.98 |
| G097A/G128S/P129A/Y217Q | 0.65 | 0.98 |
| G097A/G128S/P129K/Y217Q | 0.90 | 0.96 |
| G097A/P129N/Y217Q | 0.80 | 0.96 |
| G097A/P129F/Y217Q | 1.01 | 0.95 |
| G097A/G128S/P129K/G131S/Y217Q | 0.95 | 0.93 |
| G097A/G128S/P129R/Y217Q | 0.83 | 0.92 |
| G097A/G128S/P129V/Y217Q | 0.48 | 0.90 |
| G097A/G128S/P129Y/Y217Q | 0.41 | 0.89 |
| G097A/G128A/P129R/Y217Q | 0.69 | 0.88 |
| G097A/G128H/P129S/Y217Q | 0.59 | 0.85 |
| G097A/G128N/P129S/Y217Q/V270A | 0.51 | 0.84 |
| G097A/G128S/P129L/Y217Q | 0.45 | 0.81 |
| G097A/P129R/Y217Q | 0.70 | 0.80 |
| G097A/P129W/Y217Q | 0.51 | 0.79 |
| G097A/P1297/S130A/Y217Q | 0.51 | 0.78 |
| G097A/G128H/P129R/Y217Q | 0.58 | 0.76 |
| G097A/G128H/P129T/Y217Q | 0.57 | 0.73 |
| G097A/P129G/Y217Q | 0.55 | 0.70 |
| G097A/G128N/P129A/Y217Q | 0.42 | 0.70 |
| G097A/G128H/Y217Q | 0.58 | 0.68 |
| G097A/G128S/P129W/Y217Q | 0.36 | 0.63 |
| G097A/P1297/Y217Q | 0.47 | 0.60 |
| G097A/G128N/Z128.01A/P129S/Y217Q | 0.39 | 0.60 |
| G097A/G128N/Y217Q | 0.47 | 0.59 |
| G097A/G128A/P129I/Y217Q | 0.39 | 0.58 |

(continued)

| Table 7-1: Stain Removal Performance and Expression of BPN' 3 Variants at Positions 128, 129, and 130 | | |
|---|---|---|
| Variant | TCA | BMI |
| G097A/G128T/P129N/Y217Q | 0.39 | 0.56 |
| G097A/G128H/P129A/Y217Q | 0.50 | 0.53 |
| G097A/G128M/P129R/Y217Q | 0.53 | 0.53 |
| G097A/G128Q/P129K/Y217Q | 0.49 | 0.53 |
| G097A/G128T/P129Q/Y217Q | 0.42 | 0.47 |
| G097A/G128T/P129H/Y217Q | 0.41 | 0.46 |
| G097A/G128T/P129R/Y217Q | 0.40 | 0.44 |
| G097A/G128D/P129F/Y217Q | 0.46 | 0.43 |
| G097Λ/G128T/P129T/Y217Q | 0.37 | 0.42 |
| G097A/G128T/P129A/Y217Q | 0.39 | 0.38 |
| G097A/G128Z/P129Z/Y217Q | 0.45 | 0.36 |
| G097A/G128S/Z128.01A/P129S/Y217Q | 0.42 | 0.31 |
| G097A/G128N/P129M/Y217Q | 0.42 | 0.26 |
| G097A/G128N/P129V/Y217Q | 0.46 | 0.26 |
| G097A/G128F/P129K/Y217Q | 0.52 | 0.25 |
| G097A/G128N/Z128.01A/P129F/Y217Q | 0.30 | 0.22 |
| G097A/G128C/P129R/Y217Q | 0.77 | 0.21 |
| G097A/G128T/P129M/Y217Q | 0.34 | 0.20 |
| G097A/G128R/Z128.01A/P129S/Y217Q | 0.41 | 0.20 |
| G097A/G128S/Z128.01A/P129A/Y217Q | 0.33 | 0.15 |
| G097A/G128D/Y217Q | 0.41 | 0.15 |
| G097A/G128T/P129Y/Y217Q | 0.38 | 0.14 |
| G097A/G128R/P129S/Y217Q | 0.47 | 0.13 |
| G097A/G128R/Z128.01A/P129T/Y217Q | 0.42 | 0.12 |
| G097A/G128R/Z128.01A/P129A/Y217Q | 0.38 | 0.11 |
| G097A/G128T/P129W/Y217Q | 0.37 | 0.11 |
| G097Λ/G28Y/Z128.01A/P129R/Y217Q | 0.41 | 0.10 |
| G097A/G128C/P129D/Y217Q | 0.84 | 0.10 |
| V084I/G097A/P1297/Y217Q | 0.46 | 0.10 |
| G097A/G128R/P129D/Y217Q | 0.51 | 0.09 |
| G097A/G128A/Z128.01A/P129H/E195A/Y217Q | 0.35 | 0.09 |
| G097A/G128S/Z128.01A/P129Y/Y217Q | 0.40 | 0.09 |
| G097A/G128N/P129L/Y217Q | 0.51 | 0.08 |
| G097A/G128C/P129H/Y217Q | 0.71 | 0.07 |
| G097A/G128T/Z128.01A/P129T/Y217Q | 0.38 | 0.05 |
| G097A/G128C/Z128.01A/P129A/Y217Q | 0.39 | 0.05 |
| G097A/G128S/Z128.01A/P129I/Y217Q | 0.41 | 0.05 |

(continued)

| Table 7-1: Stain Removal Performance and Expression of BPN' 3 Variants at Positions 128, 129, and 130 | | |
|---|---|---|
| Variant | TCA | BMI |
| G097A/G128S/Z128.01A/P129E/Y217Q/Q271H | 0.41 | 0.05 |
| G097A/G128S/Z128.01A/P129I/Y217Q | 0.42 | 0.05 |
| G097A/G128Y/Z128.01A/P129S/Y217Q | 0.43 | 0.05 |
| G097A/G128S/Z128.01A/P129M/R186H/Y217Q | 0.44 | 0.05 |
| G097A/G128E/Z128.01A/P129D/Y217Q | 0.44 | 0.05 |
| G097A/G128T/P129I/Y217Q | 0.46 | 0.05 |
| G097A/G128I/P129D/Y217Q | 0.47 | 0.05 |
| G097A/G128V/Z128.01A/P129R/Y217Q | 0.47 | 0.05 |
| G097A/G128L/Z128.01A/P129S/Y217Q | 0.49 | 0.05 |
| G097A/G128Y/P129T/Y217Q | 0.51 | 0.05 |
| G097A/G128C/P129Y/Y217Q | 0.53 | 0.05 |
| G097A/G128C/P129T/Y217Q | 0.59 | 0.05 |
| Parent: G097A/G128A/Y217Q | 1.00 | 1.00 |

## EXAMPLE 8

### Construction of Combinatorial Variants of BPN'

Combinatorial variants of BPN' were created by extension (fusion) PCR using the following BPN' mutants as parent molecules-- BPN' G97A/G128A/Y217Q, BPN' M124V/L126A/Y217Q, BPN' G128A/Y217Q, and BPN' N123G/Y217Q to create insertions or substitutions at positions 96, 98, 129 and/or 222. For each combinatorial mutant created, the parent DNA molecule, mutations introduced and PCR primers used are listed in the Table 8-1. To create cach mutant, two fragments 1 and 2 were generated by the PCR primers (Table 8-2) in the 5' and 3' regions of the gene using the respective parent molecule listed in Table 8-1. These fragments were mixed and amplified by the flanking primers P4973 and P4950 (Table 8-2) to restore the full-length gene.

Each amplification reaction contained 30pmol of each PCR primer and 100 ng of the template DNA. Amplifications were carried out using Vent DNA polymerase (New England Biolabs). The PCR mix (20 ul) was initially heated at 95°C for 2.5 min followed by 30 cycles of denaturation at 94°C for 15s, annealing at 55°C for 15s and extension at 72°C for 40s. Following amplification, the 5' and 3' fragments were gel-purified by the QIAGEN gel-band purification kit, mixed and amplified by the flanking primers P4973 and P4950. The full-length DNA fragment produced was gel-purified by the QIAGEN gel-band purification kit, digested by the *Bam*HI and *Hind*III restriction enzymes and ligated with the pHPLT-BPN' vector containing the respective parent molecules, which were also cut with the same restriction enzymes. Ligation mixtures were amplified using rolling circle amplification according to manufacturer's recommendation (Epicentre). One microliter of the ligation mixture was mixed with 5 ul of the sample buffer, heated to 95°C for 3 min and cooled on ice. Next, 5 ul of the reaction buffer and 0.2 ul of the enzyme was added to each tube, followed by incubation at 30°C for 10 hours. Products of the rolling circle amplification were diluted 100 times and used to transform *Bacillus subtilis* cells (genotype: *ΔaprE, ΔnprE, ΔspoIIE, amyE::xylRPxylAcomK-phleo*) as described in Example 2. Transformations were plated on LB plates containing 1.6% skim milk and 10 ppm Neomycin and incubated overnight at 37°C. Only colonies with halos were picked and grown in microtiter plates for further analysis as described in

Example 2. Protein concentration of culture supernatants was determined by TCA precipitation as described in Example 1.

| Table 8-1: Parent DNA Molecule, Mutations Introduced and PCR Primers for the Generation of Combinatorial Variants in BPN' | | | | |
|---|---|---|---|---|
| | | | Primers Used for PCR Amplification | |
| Variant | Parent Molecule/ DNA Template | Mutations Introduced | Fragment 1 | Fragment 2 |
| 1 | G97A/G128A/Y217Q | Z098.001S | P4947, P4952 | P4951 , P4948 |
| 2 | G97A/G128A/Y217Q | Z096.001D/A098R | P4947, P4956 | P4955 , P4948 |
| 3 | G97A/G128A/Y217Q | P129V | P4947, P4960 | P4959 , P4948 |
| 4 | M124V/L126A/Y217Q | Z098.001S | P4947, P4954 | P4953 , P4948 |
| 5 | M124V/L126A/Y217Q | Z096.001D/A098R | P4947, P4958 | P4957 , P4948 |
| 6 | M124V/L126A/Y217Q | P129V | P4947, P4962 | P4961 , P4948 |
| 7 | G129A/Y217Q | Z098.001S | P4947, P4954 | P4953 , P4948 |
| 8 | G128A/Y217Q | Z096.001D/A098R | P4947, P4958 | P4957 , P4948 |
| 9 | G128A/Y217Q | P129V | P4947, P4966 | P4965 , P4948 |
| 10 | N123G/Y217Q | Z098.001S | P4947. P4954 | P4953 , P4948 |
| 11 | N123G/Y217Q | Z096.001D/A098R | P4947, P4958 | P4957 , P4948 |
| 12 | N1236/Y217Q | P129V | P4947,P4964 | P4963 , P4948 |
| 13 | G97A/G128A/Y217Q | M222Q | P4947, P4968 | P4967 , P4948 |

| Table 8-2: PCR primer Sequences Used for the Creation of Combinatorial Variants of BPN'. | | |
|---|---|---|
| SEQ ID NO: | Primer Name | Sequence |
| 199 | P4947 | CTTTAGTCTAGCAAAAGGAGAG |
| 200 | P4948 | GTGTTTTTCTTCCAATTGTGCTC |
| 201 | P4949 | CGACTCGAGCCATCCAGATC |
| 202 | P4950 | CTTGTCTCCAAGCTTAAAATAAAA |
| 203 | P4951 | AAAGTTCTGCAGCAAGTGACGGATCAGGCCAATACTCA |
| 204 | P4952 | GCCTGATCCCTCACTTGCTGCAAGAACTTTAACGGCGTAG |
| 205 | P4953 | TAAACTTCTTGGCGCAAGTGACGGATCAGGCCAATACTCA |
| 206 | P4954 | CCTCATCCGTCACTTGCGCCAAGAACTTTAACGGCGTA |
| 207 | P4955 | CTTAAAGTTCTTGATGCACGTGACGGATCAGGCCAATACTCA |
| 208 | P4956 | GATCCGTCACGTGCATCAAGAACTTTAACGGCGTAGAGCGA |
| 209 | P4957 | GTTAAAGTTCTTGATGGTCGTGACGGATCAGGCCAATACTCA |
| 210 | P4958 | GATCCCTCACCACCATCAAGAACTTTAACGGCGTAGAGCGAA |
| 211 | P4959 | TGAGCCTGGGAGCAGTAAGCGGCAGTGCGGCACTTAAA |
| 212 | P4960 | GCACTGCCGCTTACTGCTCCCAGGCTCATGTTGATTAC |
| 213 | P4961 | TTAGCGCAGGAGGAGTAAGCGGCAGTGCGGCACTTAAA |
| 214 | P4962 | GCACTGCCGCTTACTCCTCCTGCGGTAACGTTGATTA |

| Table 8-2: PCR primer Sequences Used for the Creation of Combinatorial Variants of BPN'. | | |
|---|---|---|
| **SEQ ID NO:** | **Primer Name** | **Sequence** |
| 215 | P4963 | GAGCCTGGGAGGAGTAAGCGGCAGTGCGGCACTTAAA |
| 216 | P4964 | TGCCGCACTGCCGCTTACTCCTCCCAGGCTCATGCCGATT |
| 217 | P4965 | GAGCCTGGGAGCAGTAAGCGGCAGTGGGCCACTTAAA |
| 218 | P4966 | GCACTGCCGCTTACTGCTCCCAGGCTCATGTTGATT |
| 219 | P4967 | AACGGGACTTCCCAAGCCTCGCCGCATGTAGCTG |
| 220 | P4968 | ACATGCGGCGAGGCTTGGGAAGTCCCGTTTTGCGCAC |
| 221 | P4973 | AAAGGATCCTAATCGGCGCTTTTC |

## EXAMPLE 9

### Stain Removal Performance of Combinatorial Variants of BPN'

The stain removal activity of combinatorial variants of BPN' generated as described in Example 8 was tested as described in Example 3. As described throughout, functionality of BPN' variants was quantified as a performance index (Pi), which is the ratio of performance of a variant to BPN'3 (BPN' G97A- G128A- Y217Q) . Variants showing a Pi value greater or equal than 0.5 for BMI stain removal performance and/or TCA precipitation showed improved cleaning benefits and/or expression. Performance indices less than or equal to 0.05 were fixed to 0.05 and indicated in bold italics in the table. Results are shown in Table 9- 1.

| Table 9-1: Stain Removal Performance and Expression of BPN' Variants | | |
|---|---|---|
| **Variants** | **TCA** | **BMI pH 8/16°C** |
| G097A/G128A/Y217Q/M222Q | 0.56 | 0.88 |
| M124V/L126A/P129V/Y217Q | 0.69 | 0.79 |
| G128A/P129V/Y217Q | 0.36 | 0.75 |
| G097A/G128A/P129V/Y217Q | 0.27 | 0.75 |
| N123G/P129V/Y217Q | 0.47 | 0.37 |
| V30L/Z096.01D/G097A/A098R/G128A/Y217Q | 0.54 | 0.19 |
| Z096.01D/A098R/G128A/Y217Q | 0.47 | 0.19 |
| Z096.01D/A098R/M124V/L126A/Y217Q | 0.49 | 0.17 |
| G097A/Z098.01S/G128A/Y217Q | 0.71 | 0.17 |
| Z096.01D/A098R/N123G/Y217Q | 0.54 | 0.16 |
| Z096.01D/G097A/A098R/G128A/Y217Q/S236F | 0.78 | 0.12 |
| 7098.01S/M124V/L126A/Y217Q | 0.45 | 0.11 |
| Z098.01S/G128A/Y217Q | 0.56 | 0.07 |
| Z096.01D/G097A/A098R/G128A/Y217Q | 0.55 | 0.07 |
| V093I/Z098.01S/M124V/L126A/Y217Q | 0.64 | 0.06 |
| A073V/Z098.01S/N123G/Y217Q | 0.52 | 0.05 |
| G065D/Z098.01S/G128A/Y217Q | 0.66 | *0.05* |
| I011L/Z098.01S/G128A/Y217Q | 0.53 | *0.05* |
| L082F/Z096.01D/A098R/N123G/Y217Q | 0.41 | *0.05* |
| Z098.01S/N123G/A138V/Y217Q | 0.44 | *0.05* |

(continued)

| Table 9-1: Stain Removal Performance and Expression of BPN' Variants | | |
|---|---|---|
| **Variants** | **TCA** | **BMI pH 8/16°C** |
| Z098.01S/N123G/Y217Q | 0.52 | *0.05* |

All patents and publications mentioned in the specification are indicative of the levels of those skilled in the art to which the invention pertains. Those of skill in the art readily appreciate that the present invention is well adapted to carry out the objects and obtain the ends and advantages mentioned, as well as those inherent therein. The compositions and methods described herein are representative of preferred embodiments, are exemplary, and are not intended as limitations on the scope of the invention. It is readily apparent to one skilled in the art that varying substitutions and modifications may be made to the invention disclosed herein without departing from the scope and spirit of the invention.

The invention illustratively described herein suitably may be practiced in the absence of any element or elements, limitation or limitations which is not specifically disclosed herein. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by herein.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not excised material is specifically recited herein.

The following numbered paragraphs (paras.) contain statements of broad combinations of the inventive technical features herein disclosed :-

1. A subtilisin variant comprising at least one modification, wherein said modification is selected from: G100C, S101D, S101H, Q103D, M124G, L126G, S132H, S161N, Q275Z, and/or at least set of modifications selected from A001T/L096V/G097S/A098D/D099I, A001V/G097S/A098R/D099G, V004M/A098D/D099G, V004M/D099R/G100S/Z100.01L, V004M/G127C/G128S/P129R, V004M/M119C/D120S, V008I/A098G/D099R, V008I/A098N, V008I/M119C/D120G, Q010L/G097R/A098C/D099S, K012N/A098R/D099V, K012R/G097D/A098H/D099G, A013T/S101R/Q103S, A013V/G097N/A098H/D099G/G160S, A013V/G097S/Z097.01R/A098R, A013V/G100R/S101G, A013V/N118G/M119I, A013V/P129Z/S130H/S132G, P014L/N062D/S101R/Q103S, P014S/A098S/D099G, A015T/V068N/A069G, L016M/G128R/P129R/Z129.01G, H017Q/G127R/P129G, H017Y/A098R/D099I, H017Y/A098R/D099Z/S101G, H017Y/P129R, H017Y/S130Y/G131S/S132L, Q019H/G128R/Z128.01R/P129D, Q019H/Z128.01G/Z128.02G, G020D/G097R/A098G/D099C, G020D/P129Z/S130G/G131R/S132C, N025Y/S101L/Q103N, A029V/G097R/A098S, S033I/T066G/H067R/V068D/A216E, S038F/A098R/D099S, V044I/G097C/A098D/D099G, V044I/G097R/A098R, V044I/G100S/S101G, A045V/G097C/A098R/D099S, A048V/G097L/A098G/D099S, A048V/G100R/S101G, A049V/G128S/P129L/Z129.01G, M050I/A098G, S053F/S130I/S132G, S053T/D099H/S101Z, E054D/A098R/D099G, T055I/S101R/Q103R, T055S/T066I/Z066.01G/Z066.02G, Q059H/D099S/S101G, Q059H/M119S/D120R, Q059K/M119V/V121C, Q059L/P129G, N061D/G128R/P129V/Z129.01G, S063T/G097D/A098R/D099S, G065R/Z101.01R/Z101.02G, T066S/H067S, V068C/A069G, V068C/A069G/H238Q, V068G/A069G, V068G/A069G/V150I, V068G/A069S, V068I/A069G/A144V, V068I/G097D/A098G/D0997Z/G100R, V068L/A069G, V068L/A069S, V068S/A069G, V068S/A069G/A116T, A069G/T071G, A069G/T07H, L075G/N076G/N077Z, L075R/N076G/N077Z, N076D/D099R/Z100.01G, I079F/A098G, V081A/S101R/Q103V, V081I/G097R/A098G/D099C, V084D/G097S/A098G/D099N, V084I/G127C/P129G, V084I/G127L, A085V/A098G, A085V/G097R/A098S/D099N, A085V/G128S/P129R/Z129.01G, A085V/N218I, A085V/S101G, A085V/S204P, A085V/Z099.01H/S101R, P086S/G131D/S132G, P086T/S101D/Q103S/V147I, A088S/S101R/Q103V, A088T/A098R/D099R, A088T/D099G/Z100.01G. A088T/G127N/P129R, A088T/G127S/P129R/Q206R/A273S, A088T/P129S/G146D, A088T/S101G, A088T/S130R/G131H/S132N, A088V/G100Z/S101C/Q103R, A088V/P129Z, A092G/S249R, A092G/V093C, A092G/V093I, A092G/V093L, A092L/V093G/K094Z, A092S/K094V, A092S/V093C, A092S/V093G, A092S/V093S, A092V/V093L, V093I1/G128D/P129R, V093I/P129Z, V093R/Z093.01G/K094R, K094E/V095D, K094S/V148I, L096I/N118R/M119C/D120G/A151V, Z096.01D/A098R, Z096.01H/A098G, Z096.01N/A098H,

Z096.01R/A098C, Z096.01R/A098G, Z096.01R/A098G/V192I, Z096.01R/A098R, Z096.01R/A098R/V192A, Z096.01R/A098S, Z096.01R/A098S/G157S, Z096.01R/Z096.02G, Z096.01R/Z096.02G/V147I, Z096.01S/A098G, Z096.01S/A098N, G097C/A098D/D099S, G097C/A098G/D099G, G097C/A098G/D099N, G097C/A098G/D099S, G097C/A098G/D099S/G100Z, G097C/A098G/D099V, G097C/A098N/D099S, G097C/A098R, G097C/A098R/D099G, G097C/A098R/D099H, G097C/A098R/D099H, G097C/A098R/D099N, G097C/A098R/D099R, G097C/A098/D099R/N109D/A114S, G097C/A098S/D099H/A151V, G097C/A098V/D099S, G097C/Z097.01G/A098H/T253I, G097C/Z097.01S/A098G, G097D/A098C, G097D/A098G/D099G, G097D/A098G/D099R, G097D/A098H/D099R, G097D/A098H/D099V, G097D/A098R/D099G, G097D/A098R/D099R, G097D/A098R/D099S, G097D/A098R/D099Z, G097D/G100C/S101R, G097D/G100R/S101G, G097H/A098C/D099G, G097H/A098D/D099N, G097H/A098G, G097H/A098G/D099G, G097H/A098G/D099R, G097H/A098G/D099Z, G097H/A098G/D099Z/A151V, G097H/A098G/D099Z/G100R, G097H/A098G/D099Z/G100V, G097H/A098G/D099Z/S101N/G166S, G097H/A098R, G097H/A098R/D099G, G09H/A098R/D099L, G097H/A098R/D099N, G097H/A098S/D099G, G097H/A098S/D099I, G097H/A098S/D099L, G097H/A098S/D099S/K237E, G097L/A098C/D099S, G097L/A098G/D099S, G097L/A098G/D099S/G100Z, G097L/A098G/D099Z, G097L/A098H, G097L/A098R/D099N, G097L/A098V, G097N/A098G/D099C, G097N/A098G/D099R, G097N/A098G/D099R/V270I, G097N/A098G/D099S, G097N/A098G/D099V, G097N/A098G/D099V/A151V, G097N/A098G/D099V/S101F, G097N/A098H/D099N, G097N/A098R/D099R, G097N/A098R/D099S, G097N/A098R/D099V, G097N/A098S/D099S, G097R/A098C, G097R/A098C/D099S, G097R/A098F/A200V, G097R/A098G, G097R/A098G/D099C, G097R/A098G/D099G, G097R/A098G/D099G/A273T, G097R/A098G/D099S, G097R/A098G/D099S/A133D, G097R/A098G/D099V, G097R/A098H/D099C/S182N, G097R/A098H/D099G, G097R/A098N/D099G, G097R/A098N/D099Z, G097R/A098R, G097R/A098R/D099C, G097R7A098R/D099G, G097R/A098R/D099S, G097R/A098S/D099C, G097R/A098S/D099G, G097R/A098S/D099L, G097R/A098S/D099R, G097R/A098S/D099S, G097R/A098S/D099V, G097R/A098V/D099G, G097R1Z097.01H/A098C, G097R/Z097.01R/A098G, G097R/Z097.01R/A098G/V147I, G097R/Z097.01R/A098S, G097R/Z097.01R, G097R/Z097.01S/A098G, G097R/Z097.01S/A098R, G097S/A098C, G097S/A098C/D099G, G097S/A098C/D099H, G097S/A098C/D099L, G097S/A098C/D099R, G097S/A098C/D099S, G097S/A098C/D099Z, G097S/A098G/D099F, G097S/A098G/D099G, G097S/A098G/D099G. G097S/A098G/D099S, G097S/A098G/D099Z, G097S/A098G/D099Z/G100R, G097S/A098H/D099C, G097S/A098H/D099G, G097S/A098N/D099G, G097S/A098R, G097S/A098R/D099C, G097S/A098R/D099G, G097S/A098R/D099L, G097S/A098R/D099V, G097S/A098R/D099Y, G097S/A098S/D099G, G097S/A098S/D099G/A133V/A153V/S236Y, G097S/A098S/D099G/A153V, G097S/A098S/D099Z, G097S/Z097.01D/A098G/A144T/Q271H, G097S/Z097.01G/A098V, G097S/Z097.01S/A098G, G097S/Z097.01S/A098G/A273T, G097S/Z097.01Y, G097V/A098R, G097V/A098R/D099G, G097V/A098S/D099S, G097Y/A098H/D099N, G097Z/A098S/D099H, Z097.01D/A098R/D099G, Z097.01D/A098S/D099L, Z097.01D/A098V/D099G, Z097.01G, Z097.01G/A098G, Z097.01G/A098G/D099S, Z097.01G/A098R, Z097.01G/A098R/D099C, Z097.01G/A098R/D099G, Z097.01G/A098R/D099S, A098C/D099G, A098C/D099C/A116T, A098C/D099H, A098C/D099L, A098C/D099L/K213R, A098C/D099L, A098C/D099N, A098C/D099R, A098C/D099S, A098C/D099S/P194L, A098D/D099C, A098D/D099G, A098D/D099G/G100L, A098D/D099H/G100S, A098D/D099H/H238Y, A098D/D099R, A098D/D099R/G100D, A098D/D099R/G160S, A098F/D099S/G100L, A098G/D099C A098G/D099G, A098G/D099G/A228T, A098G/D099G/G100H, A098G/D099G/N243D, A098G/D099G/S101P, A098G/D099G/T244S, A098G/D099G, A098G/D099H, A098G/D099L, A098G/D099R, A098G/D099R/A116T, A098G/D099R/G100D, A098G/D099R/V148I. A098G/D099S, A098G/D099S/L267M, A098G/D099V, A098G/D099Z, A098H/D099C, A098H/D099G, A098H/D099G/A216T, A098H/D099G/G100D, A098H/D099G/G100N/L267M, A098H/D099G/C100S, A098H/D099G/V143I, A098H/D099R, A098H/D099R/G100S, A098H/D099S/G100C, A098H/D099S/G100N, A098H/D099Y, A098I/D099G, A098I/D099S, A098L/D099G, A098L/D099S, A098N/D099G, A098N/D099L, A098N/D099S, A098R/A144T, A098R/A274V, A098R/R099C, A098R/D099G, A098R/D099G/G100C, A098R/D099G/G100D, A098R/D099G/G100L/S145T, A098R/D099G/G100N, A098R/D099G/G100R, A098R/D099G/G100S, A098R/D099G/V150I, A098R/D099H, A098R/D099I, A098R/D099L, A098R/D099N, A098R/D099N/G100L, A098R/D099R, A098R/D099R/G100D, A098R/D099R/S101Z, A098R/D099R/S130F, A098R/D099R, A098R/D099S, A098R/D099S/G100L/A153V, A098R/D099S, A098R/D099V, A098R/D099Z/S101G, A098R/G100C/A137T, A098R/G100S, A098S/D099C, A098S/D099G, A098S/D099G/G100C, A098S/D099G/G100D, A098S/D099G/G100R/L250I, A098S/D099G/G100S, A098S/D099G/Z099.01R, A098S/D099H, A098S/D099H/M124V, A098S/D099H/Z099.01R/G169A/V180A, A098S/D099R, A098S/D099R/G100L, A098S/D099R/G154S, A098S/D099R/G166S, A098S/D099R/Z100.01G, A098S/D0995, A098S/D099S/Z099.01R, A098S/D099V, A098T/P129Z/S130R/S132G, A098T/S101G, A098V/D099G,

A098V/D099G/G100D, A098V/D099G/G100D/A153V, A098V/D099G/G100S, A098Y/D099R/G100L, A098Y/D099R/G100Z/S101Z/G102D, A098Z/S101R, Z098.01N, Z098.01R, Z098.01R/G100R, D099C/K136N, D099C/S101D, D099C/S101R, D099C/S101Z, D099C/Z099.01S, D099C/Z100.01G, D099F/S101Z, D099G/G100C, D099G/G100D/S101R, D099G/G100H, D099G/G100R/S101V, D099G/G100S/S101R, D099G/S101G, D099G/S101H, D099C/S101V, D099G/S101Z, D099G/S101Z/N269D, D099G/Z099.01N, D099G/Z099.01R/G100S, D099G/Z099.01S, D099G/Z100.01G, D099G/Z100.01G/S183N/Q275K, D099H/S101Z, D099H/S101Z/V150I, D099H/Z100.01G/A273V, D099H/Z100.01G/Z100.02G, D099I/Z099.01S, D099L/N118D, D099L/S101G, D099L/S101V, D099L/Z100.01G, D099N/A116T, D099N/G100R/S101V, D099N/S101G, D099N/, S101H, D099N/S101Z, D099N/Z099.01S, D099N/Z100.01G, D099R/S101D, D099R/S101G, D099R/S101V, D099R/S101Z, D099R/S101Z/A133T, D099R/Z099.01N, D099R/099.01S, D099R/100.01G, D099R/Z100.01N, D099S/S101G, D099S/S101I, D099S/S101Z, D099S/Z099.01H, D099S/Z099.01L, D099S/Z099.01V, D099S/Z100.01G, D099S/Z100.01G/Z100.02G, D099V/S101D, D099V/S101V, D099V/S101Z, Z099.01H/S101L, Z099.01H/Z099.02R, Z099.01N/S101R, Z099.01S, Z099.01S/S101V, Z099.01S/Z099.02G/A228T, G100D/S101G, G100D/S101R, G100H/G102V, G100H/S101G, G100H/S101R, G100L/S101G, G100L/S101R, G100N/S101D, G100N/S101L, G100N/S101R, G100R/L267R/I268V, G100R/S101G, G100R/S101G/G160S/S182N, G100R/S101G/Q103Z/Y104Z/S161N, G100R/S101G/S163N, G100R/S101N, G100R/S101R, G100R/S101R/Q103Z/V270I, G100R/S101Z/G102C/Q103D, G100S/S101C, G100S/S101G, G100S/S101R, G100S/S101R, G100S/S101V, G100S/S101V/A231T, Z100.01G, Z100.01L, Z100.01N, Z100.01R, Z100.01R/S101G, S101C/Q103I, S101C/Q103S, S101D/Q103C, S101D/Q103D, S101D/Q103F, S101D/Q103H, S101D/Q103H, S101D/Q103R, S101D/Q103R/H238Y, S101D/Q103S, S101D/Q103V, S101F/Q103G, S101G/A151V, S101G/A179V, S101G/G102S, S101G/Q103G, S101G/Q103L, S101G/Q103N, S101G/Q103R, S101G/Q103R/A179T, S101G/Q103R/G157D, S101G/Q103S, S101G/Q103S/L233S, S101G/Q103S/L233S, S101G/Q245H, S101G/S249N, S101G/V139I, S101G/Z101.01R, S101G/Z101.01R/A200T, S101G/Z102.01G, S101H/G102S, S101H/Q103D, S101H/Q103G, S101H/Q103G/E251Q, S101H/Q103H, S101H/Q103I, S101H/Q103R, S101H/Q103R/A274Z/Q275Z, S101H/Q103S, S101H/Q103V, S101I/Q103R, S101L/G102S/Q103R, S101L/Q103D, S101L/Q103G, S101L/Q103R, S101L/Q103R/A138T, S101L/Q103R/V121I/K213E, S101L/Q103S,S101L/Q103V, S101L/Z101.01R, S101N/Q103R, S101N/Z102.01G, S101R/A151V, S101R/A273V, S101R/E156G, S101R/G102L/Q103G, S101R/G102S, S101R/G102S/Q103R, S101R/G102S/Q103V, S101R/G131D, S101R/Q103C, S101R/Q103D, S101R/Q103D/S248R, S101R/Q103G, S101R/Q103G/A116T,S101R/Q103G/V147I/A153V, S101R/Q103G, S101R/Q103L, S101R/Q103N, S101R/Q103R, S101R/Q103R/S161N, S101R/Q103R/V148I, S101R/Q103S, S101R/Q103S/L126F, S101R/Q103V, S101V/Q103G, S101V/Q103H, S101V/Q103N/Z275.01K, S101V/Q103R, S101V/Q103V, S101Y/G102S/Q103R, S101Y/Q103L, S101Y/Q103S, S101Y/Q103V, S101Z/G102I/Q103H/Y104G/L135F, S101Z/G102V/Q103R/Y104L, S101Z/Q103D, S101Z/Q103F, S101Z/Q103L, S101Z/Q103R, G102C/Q103L/Y104S, G102R/Q103C/Y 104C/V192I, G102R/Q103G/Y104R, G102R/Q103R/Z103.01G, G102Z/Q103S, Z102.01G/Q103R, Z102.01G/Q103R/L267V, Z102.01G/Z102.02R/Z102.03G, Q103H/Y104N/Q275Z, Q103N/156D, Q103R/I122I/N123S/M124V, Q103R/Y104F, Q103Y/S182N, S105G/P129S/S130H/G131H, W106C/A116D/N117S/N118C, N109S/M119C/D120G/V121L, A116G/N117Z/N118R/M119C, A116H/N117G/N118R, N117C/N118G/M119S, N117C/N118G/M119S/A216V, N117G/N118G/M119C, N117G/N118R/M119C, N117H/N118C/M119C, N117H/N118D/M119S, N117H/N118G/M119C, N117H/N118G/M119L, N117H/N118G/M119S, N117H/N118G/M119V, N117H/N118H/M119V, N117H/N118R/M119C, N117H/N118R/M119S, N117S/M118S/M119, N117H/N118S/M119S, .N117H/N118S/M119V, N117I/N118G/M119V, N117L/118G/M119V, N117R/N118G/M119C, N117R/N118G/M119I/V270I, N117R/N118G/M119S, N117R/N118H/M119V, N117R/N118R/M119C, N117R/N118R/M119I, N117R/N118R/M119S, N117R/N118R/M119V, N117R/N118S/M119I, N117R/N118S/M119L, N117S/N118G/M119C, N117S/N118G/M119S, N117S/N118H/M119V, N117S/N118R/M119V, N117V/N118G/M119C, N117V/N118H/M119C, N117Y/N118G/M119S, N117Y/N118G/M119V, Z117.01G/N118R/M119C, N118C/M119V/D120S, N118C/Z118.01G/M119C, N118D/M119C/D120L, N118D/M119S/D120R, N118G/M119C/D120G, N118G/M119C/D120L, N118G/M119I/D120R, N118G/M119N, N118G/M119S/D120G, N118G/M119S/D120R, N118G/M119V/D120G, N118G/M119V/D120S/S182N, N118H/M119C/D120R, N118H/M119I/D120G, N118H/M119N/V148I, N118H/M119S/D120G, N118H/M119V/D120G, N118R/M119C, N118R/M119C/D120G, N118R/M119C/D120R, N118R/M119S, N118R/M119S/D120G, N118R/M119S/D120R, N118R/M119S/D120S, N118R/M119V/D120C, N118R/M119V/D120G, N118R/M119V/D120/A232T, N118S/M119C/D120R, M119C/D120G, M119C/D120G/V121I, M119C/D120G/V121L, M119C/D120H, M119C/D120H/A187V, M119C/D120R, M119H/V121I. M119I/D120C, M119I/D120G, M119I/D120R, M119I/D120G, M119S/D120R M119S/D120S/V121I, M119S/V121I, M119V/D120C, M119V/D120G, M119V/D120R, M119V/D120S/V198L, D120G/122C, D120G/I122C/L217S, D120G/I122G, D120G/I122V, D120H/I122L, D120H/V121C/I122V,

D120N/V121L/I122V, D120R/A151V, D120R/I122V, D120R/V121I, D120R/V121I/I122C, D120R/V121I/I122C/A228T, D120S/I122C/A133S, V121C/I122C/N123C, V121C/I122G/N123V, V121C/I122S/N123V, V121C/I122V/N123S, V121I/I12C/N123C, V121I/I122C/V150I, V121I/I122G/N123C, V121I/I122S/N123C, V121I/I122S/N123I, V121I/I122V/N123S, V121L/I122S/N123V, V121L/N123C, I122C/N123C, I122C/N123S/M124L, I122D/N123V/M124L, I122G/N123C/M124L, I122G/N123G/Z123V.01C, I122H/N123V/M124LI122S/N123S, I122V/N123G/M124C/A138V, I122V/N123S/M124I, I122V/S183G, N123C/M124S, N123C/M124V, N123S/M124L, N123S/M124S, M124C/L126S, M124C/L126S/T244I, M124C/L126V, M124MI/L126H, L126F/P129Z, L126F/P129Z/S182N, L126I/P129Z, L126R/G127R, Z126.01S, G127C/G128S/P129R, G127C/P129G, G127C/P129G/A200T, G127C/P129R, G127C/P129R/A153T, G127D/P129G, G127D/P129S, G127H/P129G, G127H/P129R, G127L/P129R, G127N/P129C, G127N/P129G, G127N/P129G/A133T, G127N/P129G/A151V/K213N, G127N/P129R, G127N/P129S/A273V, G127N/P129V, G127R/A230V, G127R/P129D, G127R/P129D/L217S, G127R/P129G, G127R/P129R, G127R/P129S, G127S/P129D, G127S/P129F, G127S/P129G, G127S/P129G/G166D, G127S/P129G/I175T, G127S/P129R, G127S/P129S, G127S/P129V, G127V/P129G, G127V/P129R, Z127.01G/Z127.02H/P129D, Z127.01/GZ127.02H/P129G, Z127.01G/Z127.02H/P129R, Z127.01H/G128C/P129C/A230V, Z127.01H/P129G, Z127.01H/P129G/V148I, Z127.01H/P129R, Z127.01N/G128C/P129V, Z127.01N/G128S/P129S, Z127.01N/C128S/P129V, Z127.01N/P129R, Z127.01R/G128S/P129S, Z127.01R/P129G, Z127.01S/P129D, Z127.01S/P129G, Z127.01S/P129R, G128D/P129G, G128D/P129R/Z129.01R, G128D/P129S/G154S, G128H/P129C, G128H/P129H/Z129.01G, G128H/P129H/Z129.01R, G128H/P129R, G128H/P129R/Z129.01G, G128H/P129R/Z129.01R, G128H/P129S/Z129.01G, G128H/P129S/Z129.01R, G128H/P129S/Z129.01R/S248N, G128H/P1129Y, G128H/Z128.01R/P129D, G128H/Z128.01R/P129S, G128H/Z128.01R/P129S/A144T, G128H/Z129.01R, 128N/P129R/Z129.01G, G128N/P129R/Z129.01R, G128N/P129S/Z129.01D, G128N/Z128.01R/P129S, G128R/P129G, G128R/P129H/Z129.01G, G128R/P129L/Z130.01S, G128R/P129R, G128R/P129R/Z129.01G, G128R/P129R/Z129.01G/A216V, G128R/P129S, G128R/P129S/Z129.01G, 128R/P129Z/S130R/S132G, G128R/Z128.01R/P129D, G128R/Z128.01R/P129F, G128S/P129C, G128S/P129C/Z129.01R, C128S/P129D, G128S/P129D/S248R, G128S/P129D/T244N, G128S/P129G, G128S/P129G/A187V, G128S/P129H/Z129.01R, G128S/P129R/L209F, G128S/P129R/Z129.01G, G128S/P129S/Z129.01D, G128S/P129S/Z29.01R, G128S/P129V, G128Y/P129R/Z129.01G, Z128.01G, Z128.01G/P129D, Z128.01G/P129R, Z128.01G/P129S/A134T, Z128.01G/Z128.02G, Z128.01R/P129D, Z128.01R/Z128.02G/P129S, Z128.01R/Z128.02H/P129G/G160S, Z128.01R/Z128.02R/P129D, Z128.01Y/Z128.02H/P129R, P129C/S130C/S132Z, P129D/Z129.01G/Z129.02D/G160D, P129D/Z129.01G/Z129.02G, P129G/G131Z, P129G/S130G/Z131.01G, P129G/S130H/S132Z, P129G/S130R/S132Z, P129G/V2701, P129G/Z129.01G, P129G/Z129.01R/Z129.02G, P129H/G131Z, P129H/G131Z/S132C, P129H/G131Z/S132H, P129H/S132Z, P129H/Z130.01D/Z130.02S, P129R/G131Z/S132H, P129R/S130D/S132Z, P129R/S130N/G131Z, P129R/S132T, P129R/S132Z, P129R/Z129.01G, P129R/Z129.01G/Z129.02G, P129R/Z129.01G/Z129.02G/A144T, P129R/Z129.01G/Z129.02G/G146D, P129R/Z129.01G/Z129.02R, P129R/Z129.01G, P129R/Z129.01R/Z129.02G, P129S/G131ZP129S/S130H/S132Z, P129S/Z129.01C, P129S/Z129.01R/Z129.02S, P129S/Z130.01N/Z130.02S, P129V/S132Z, P129Z, P129Z/G131R/S132C, P129Z/S130G, P129Z/S130G/G131H/S132H, P129Z/S130G/G131V, P129Z/S130H, P129Z/S130H/S132G, P129Z/S130H/S132N, P129Z/S130L, P129Z/S130R, P129Z/S130R/G131C, P129Z/S130R/G131C, P129Z/S130R/G131D/S132C, P129Z/S130R/G131H/A144E, P1292/S130R/G131H/S132D, P129Z/S130R/G131R/S132D, P129Z/S130R/L267M, P129Z/S130R/S132C, P129Z/S130R/S132G, P129Z/S130V/S132G, P129Z/S130Z/G131N/S132G, S130D/G131C/S132D, S130D/G131R, S130D/G131R/S132D, S130D/G131Z, S130D/G131Z/S132H, S 130F/G131N/S132V, S130F/G131S/S132V, S130F/S132V, S130G/G131D/S132C, S130G/G131H/S132D, S130G/G131S/S132I, S130G/S132G, S130H/G131C/S132G, S130H/G131H/S132R, S130H/G131R/S132C, S130H/G131S, S130H/G131S/S132C, S130H/VS132Z, S130I/G131S/S132H, S130I/S132G, S130N/G131S/S132C, S130R/G131R/S132C, S130R/G131S/S132C/S161N, S130R/S132G, S130R/S132G/S183N, S130R/S132Z, S130V/G131D/S132I, S130V/G131H/S132C, S130V/S132N, S130V/S132Z, S130Y/G131H/S132C, S130Y/S132G, S130Z/G131H/S132H, G131H/S132L/W241R, G131N/S132C, G131S/S132G, G131Y/S132G, and A142V/G211V, wherein the positions correspond to the positions of BPN' subtilisin of SEQ ID NO:2.

2. A subtilisin variant comprising at least one modification, wherein said modification is selected from: V072G/G097A/G128A/Y217Q, G097A/G128S/Y217Q, G097A/G128S/P129D/Y217Q, G097A/G128A/A134T/Y217Q, M124V/L126A/Y217Q, G097A/G128A/P129D/Y217Q/A232P, G097A/G128A/P129D/Y217Q, G097A/G12S/P129V/A134T/Y217Q, G097A/G128S/P129V/Y217Q, V068I/G097A/G128S/P129D/Y217Q, G097A/S101D/G128S/S204F/Y217Q/S236F/T254P, G097A/S101D/G128S/Y217Q, G097A/G128A/P129V/Y217Q, G097A/S101D/G128A/Y217Q,

G097A/S101D/G128S/P129D/Y217Q, G097A/S101D/G128A/A134T/Y217Q, G097A/Z099.01S/A114S/G128S/Y217Q, G097A/S101D/G128A/P194L/Y217Q, G097A/Z099.01S/G128S/Y217Q, G097A/S101D/G128A/P129D/Y217Q, Q019R/G097A/Z099.01S/G128S/P129D/Y217Q, G097A/G100S/G128S/Y217Q, G097A/S101D/G128S/P129V/Y217Q, G097A/Z099.01S/G128A/A134T/Y217Q, G097A/G100S/I107V/G128S/P129D/Y217Q, G097A/G100S/G128S/Y217Q/A230V, G097A/G100S/G128A/Y217Q/K237N, G097A/Z099.01S/G128S/P129D/Y217Q, G097A/G100S/G128S/P129D/Y217Q, Z096.01D/G097A/A098R/G128A/Y217Q, G097A/Z099.01S/I122S/N123G/G128A/Y217Q, G097A/Z099.01S/I122S/N123G/G128A/Y217Q/T220A/A232T, P005S/G097A/Z099.01S/S101D/G128A/Y217Q, G097A/S101 D/G128A/P129V/Y217Q, V093Z/K094S/V095C/L096S/G097A/G128A/Y217Q, G097A/G100S/S101D/G128A/Y217Q/A232P, Z096.01D/G097A/A098R/S101D/G128A/Y217Q, and G097A/Z099.01S/G100S/G 128A/Y217Q, wherein the positions correspond to the positions of BPN' subtilisin of SEQ ID NO:2.

3. A subtilisin variant comprising at least two modifications, wherein said modifications are selected from: G097A/G128S/P129T/Y217Q, G097A/G128A/P129S/Y217Q, G097A/G128S/P129C/Y217Q, G097A/G128S/P129Q/Y217Q, G097A/G128S/P129E/Y217Q, G097A/P129S/Y217Q, G097A/G128S/P129A/Y217Q, G097A/G128S/P129K/Y217Q, G097A/P129N/Y217Q, G097A/P129E/Y217Q, G097A/G128S/P129K/G131S/Y217Q, G097A/G128S/P129R/Y217Q, G097A/G128S/P129V/Y217Q, G097A/G 128S/P129Y/Y217Q, G097A/G128A/P129R/Y217Q, G097A/G128H/P129S/Y217Q, G097A//G128N/P129S/Y217Q/V270A, G097A/G128S/P129L/Y217Q, G097A/P129R/Y217Q, G097A/P129W/Y217Q, G097A/P129Z/S130A/Y217Q, G097A/G128H/P129R/Y217Q, G097A/G128H/P129T/Y217Q, G097A/P129G/Y217Q, G097A/G128N/P129A/Y217Q, G097A/G128H/Y217Q, G097A/G128S/P129W/Y217Q, G097A/P129Z/Y217Q, G097A/G128N/Z128.01A/P129S/Y217Q, G097A/G128N/Y217Q, G097A/G128A/P129L/Y217Q, G097A/G128T/P129N/Y217Q, G097A/G128H/P129A/Y217Q, G097A/G128M/P129R/Y217Q, G097A/G128Q/P129K/Y217Q, G097A/G128T/P129Q/Y217Q, G097A/G128T/P129Q/Y217Q, G097A/G128T/P129R/Y217Q, G097A/G128D/P129E/Y217Q, G097A/G128T/P129T/Y217Q, G097A/G128T/P129A/Y217Q, G097A/G128Z/P129Z/217Q, G097A/G128S/Z128.01A/P129S/Y217Q, G097A/G128N/P129M/Y217Q, G097A/G128N/P129V/Y217Q, G097A/G128E/P129K/Y217Q, G097A/G128N/Z128.01A/P129F/Y217Q, G097A/G128C/P129R/Y217Q, G097A/G128T/P129M/Y217Q, G097A/G128R/Z128.01A/P129S/Y217Q, G097A/G128S/Z128.01A/P129A/Y217Q, G097A/G128D/Y217Q, G097A/G128T/P129Y/Y217Q, G097A/G128R/P129S/Y217Q, G097A/G128R/Z128.01A/P129T/Y217Q, G097A/G128R/Z128.01A/P129A/Y217Q, G097A/G128T/P129W/Y217Q, G097A/G128Y/Z128.01A/P129R/Y217Q, G097A/G128C/P129D/Y217Q, V084L/G097A/P129Z/Y217Q, G097A/G128R/P129D/Y217Q, G097A/G128A/Z128.01A/P129H/E195A/Y217Q, G097A/G128S/Z128.01A/P129Y/Y217Q, G097A/G128N/P129L/Y217Q, G097A/G128C/P129H/Y217Q, G097A/G128T/Z128.01A/P129T/Y217Q, G097A/G128C/Z128.01A/P129A/Y217Q, G097A/G128S/Z128-01A/P129I/Y217Q, G097A/G129S/Z128.01A/P129E/Y217Q/Q271H, G097A/G128S/Z128-01A/P129L/Y217Q, G097A/G128Y/Z128.01A/P129S/Y217Q, G097A/G128S/Z128.0A/P129M/R186H/Y217Q, G097A/G128E/Z128.01A/P129D/Y217Q, G097A/G128T/P129L/Y217Q, G097A/G128F/P129D/Y217Q, G097A/G128V/Z128.01A/P129R/Y217Q, G097A/G128L/Z128.01A/P129S/Y217Q, G097A/G128Y/P129T/Y217Q, G097A/G128C/P129Y/Y217Q, G097A/G128C/P129/Y217Q, wherein the positions correspond to the positions of BPN' subtilisin of SEQ ID NO:2.

4. A subtilisin variant comprising at least two modifications, wherein said modifications are selected From: G097A/G128A/Y217Q/M222Q, M124V/L126A/P129V/Y217Q, G128A/P129V/Y217Q G097A/G128A/P129V/Y217Q, N123G/P129V/Y217Q, V30I/Z096.01D/G097A/A098R/G128A/Y217Q, Z096-01D/A098R/G128A/Y217Q, Z096.01D/A098R/M124V/L126A/Y217Q, G097A/Z098-01S/G128A/Y217Q, Z096.01D/A098R/N123G/Y217Q, Z096.01D/G097A/A098R/G128A/Y217Q/S236F, Z098.01S/M124V/L126A/Y217Q, Z098-01S/G128A/Y217Q, Z096.01D/G097A/A098R/G128A/Y217Q, V093I/Z098.01S/M124V/L126A/Y217Q, A073V/Z098.01S/N123G/Y217Q, G065D/Z098.01S/G128A/Y217Q, I011L/Z098.01S/G128A/Y217Q, L082F/Z096.01D/A098R/N123G/Y217Q, Z098.01S/N123G/A138V/Y217Q, Z098.01S/N123G/Y217Q, wherein the positions correspond to the positions of BPN' subtilisin of SEQ ID NO:2.

5. A subtilisin variant comprising the substitution Y217L and further comprising at least one modification set forth in any of paras. 1-4, and wherein the positions correspond to the positions of BPN' subtilisin of SEQ ID NO:2.

6. A subtilisin variant comprising the substitutions G97A/G128A/Y217Q and further comprising at least one modification set forth in any of paras. 1-4, and wherein the positions correspond to the positions of BPN' subtilisin of SEQ

ID NO:2.

7. A cleaning composition comprising at least one subtilisin variant of any of paras. 1 to 6.

8. The cleaning composition of para. 7, wherein said cleaning composition is a laundry detergent.

9. The laundry detergent of para. 8, wherein said laundry detergent is a heavy duty liquid laundry detergent.

10. The cleaning composition of para. 7, wherein said cleaning composition is a dish detergent.

11. The cleaning composition of any of paras. 7-10, further comprising one or more additional enzymes or enzyme derivatives selected from the group consisting of hemicellulases, peroxidases, proteases, cellulases, xylanases, lipases, phospholipases, esterases, cutinases, pectinases, keratinases, reductases, oxidases, phenol oxidases, lipoxygenases, ligninases, pullulanases, tannases, pentosanases, malanases, ß-glucanases, arabinosidases, hyaluronidase, chondroitinase, laccase, and amylases, or mixtures thereof.

12. The cleaning composition of any of paras. 7-11, further comprising at least one stabilizing agent.

13. A cleaning composition comprising at least 0.0001 weight percent of at least one subtilisin variant of any of paras. 1-7, and optionally, at least one suitable adjunct ingredient.

14. A method of cleaning, said method comprising the steps of:

a) contacting a surface and/or an article comprising a fabric with the cleaning composition of any of paras. 7-13; and
b) optionally washing and/or rinsing said surface or article.

15. An animal feed comprising at least one subtilisin variant of any of paras. 1-7.

16. A food processing composition comprising at least one subtilisin variant of any of paras. 1-7.

## Claims

**1.** A cleaning composition comprising a subtilisin variant comprising at least one modification, wherein said modification is selected from: G100C, S101D, S101H, Q103D, M124G, L126G, S132H, S161N, Q275Z, and/or at least set of modifications selected from A001T/L096V/G097S/A098D/D099I, A001V/G097S/A098R/D099G, V004M/A098D/D099G, V004M/D099R/G100S/Z100.01L, V004M/G127C/G128S/P129R, V004M/M119C/D120S, V008I/A098G/D099R, V008I/A098N, V008I/M119C/D120G, Q010L/G097R/A098C/D099S, K012N/A098R/D099V, K012R/G097D/A098H/D099G, A013T/S101R/Q103S, A013V/G097N/A098H/D099G/G160S, A013V/G097S/Z097.01R/A098R, A013V/G100R/S101G, A013V/N118G/M119I, A013V/P129Z/S130H/S132G, P014L/N062D/S101R/Q103S, P014S/A098S/D099G, A015T/V068N/A069G, L016M/G128R/P129R/Z129.01G, H017Q/G127R/P129G, H017Y/A098R/D099I, H017Y/A098R/D099Z/S101G, H017Y/P129R, H017Y/S130Y/G131S/S132L, Q019H/G128R/Z128.01R/P129D, Q019H/Z128.01G/Z128.02G, G020D/G097R/A098G/D099C, G020D/P129Z/S130G/G131R/S132C, N025Y/S101L/Q103N, A029V/G097R/A098S, S033I/T066G/H067R/V068D/A216E, S038F/A098R/D099S, V0441/G097C/A098D/D099G, V044I/G097R/A098R, V044I/G100S/S101G, A045V/G097C/A098R/D099S, A048V/G097L/A098G/D099S, A048V/G100R/S101G, A048V/G128S/P129L/Z129.01G, M050I/A098G, S053F/S130I/S132G, S053T/D099H/S101Z, E054D/A098R/D099G, T055I/S101R/Q103R, T055S/T066I/Z066.01G/Z066.02G, Q059H/D099S/S101G, Q059H/M119S/D120R, Q059K/M119V/V121C, Q059L/P129G, N061D/G128R/P129V/Z129.01G, S063T/G097D/A098R/D099S, G065R/Z101.01R/Z101.02G, T066S/H067S, V068C/A069G, V068C/A069G/H238Q, V068G/A069G, V068G/A069G/V150I, V068G/A069S, V068I/A069G/A144V, V0681/G097D/A098G/D099Z/G100R, V068L/A069G, V068L/A069S, V068S/A069G, V068S/A069G/A116T, A069G/T071G, A069G/T071I, L075G/N076G/N077Z, L075R/N076G/N077Z, N076D/D099R/Z100.01G, I079F/A098G, V081A/S101R/Q103V, V0811/G097R/A098G/D099C, V084D/G097S/A098G/D099N, V084I/G127C/P129G, V084I/G127L, A085V/A098G, A085V/G097R/A098S/D099N, A085V/G128S/P129R/Z129.01G, A085V/N218I, A085V/S101G, A085V/S204P, A085V/Z099.01H/S101R, P086S/G131D/S132G, P086T/S101D/Q103S/V147I, A088S/S101R/Q103V, A088T/A098R/D099R, A088T/D099G/Z100.01G, A088T/G127N/P129R, A088T/G127S/P129R/Q206R/A273S, A088T/P129S/G146D, A088T/S101G, A088T/S130R/G131H/S132N, A088V/G100Z/S101C/Q103R, A088V/P129Z, A092G/S249R, A092G/V093C, A092G/V0931, A092G/V093L, A092L/V093G/K094Z, A092S/K094V, A092S/V093C, A092S/V093G, A092S/V093S, A092V/V093L, V093I/G128D/P129R, V093I/P129Z, V093R/Z093.01G/K094R, K094E/V095D, K094S/V148I, L096I/N118R/M119C/D120G/A151V, Z096.01D/A098R, Z096.01H/A098G, Z096.01N/A098H, Z096.01R/A098C, Z096.01R/A098G, Z096.01R/A098G/V192I, Z096.01R/A098R, Z096.01R/A098R/V192A, Z096.01R/A098S, Z096.01R/A098S/G157S, Z096.01R/Z096.02G, Z096.01R/Z096.02G/V147I, Z096.01S/A098G, Z096.01S/A098N, G097C/A098D/D099S, G097C/A098G/D099G, G097C/A098G/D099N, G097C/A098G/D099S, G097C/A098G/D099S/G100Z, G097C/A098G/D099V, G097C/A098N/D099S, G097C/A098R, G097C/A098R/D099G, G097C/A098R/D099H, G097C/A098R/D099H, G097C/A098R/D099N, G097C/A098R/D099R, G097C/A098R/D099R/N109D/A114S, G097C/A098S/D099H/A151V, G097C/A098V/D099S, G097C/Z097.01G/A098H/T253I, G097C/Z097.01S/A098G, G097D/A098C, G097D/A098G/D099G, G097D/A098G/D099R, G097D/A098H/D099R, G097D/A098H/D099V, G097D/A098R/D099G, G097D/A098R/D099R, G097D/A098R/D099S, G097D/A098R/D099Z, G097D/G100C/S101R, G097D/G100R/S101G, G097H/A098C/D099G, G097H/A098D/D099N, G097H/A098G, G097H/A098G/D099G, G097H/A098G/D099R, G097H/A098G/D099Z, G097H/A098G/D099Z/A151V,

G097H/A098G/D099Z/G100R, G097H/A098G/D099Z/G100V, G097H/A098G/D099Z/S101N/G166S, G097H/A098R, G097H/A098R/D099G, G097H/A098R/D099L, G097H/A098R/D099N, G097H/A098S/D099G, G097H/A098S/D099I, G097H/A098S/D099L, G097H/A098S/D099S/K237E, G097L/A098C/D099S, G097L/A098G/D099S, G097L/A098G/D099S/G100Z, G097L/A098G/D099Z, G097L/A098H, G097L/A098R/D099N, G097L/A098V, G097N/A098G/D099C, G097N/A098G/D099R, G097N/A098G/D099R/V270I, G097N/A098G/D099S, G097N/A098G/D099V, G097N/A098G/D099V/A151V, G097N/A098G/D099V/S101F, G097N/A098H/D099N, G097N/A098R/D099R, G097N/A098R/D099S, G097N/A098R/D099V, G097N/A098S/D099S, G097R/A098C, G097R/A098C/D099S, G097R/A098F/A200V, G097R/A098G, G097R/A098G/D099C, G097R/A098G/D099G, G097R/A098G/D099G/A273T, G097R/A098G/D099S, G097R/A098G/D099S/A133D, G097R/A098G/D099V, G097R/A098H/D099C/S182N, G097R/A098H/D099G, G097R/A098N/D099G, G097R/A098N/D099Z, G097R/A098R, G097R/A098R/D099C, G097R/A098R/D099G, G097R/A098R/D099S, G097R/A098S/D099C, G097R/A098S/D099G, G097R/A098S/D099L, G097R/A098S/D099R, G097R/A098S/D099S, G097R/A098S/D099V, G097R/A098V/D099G, G097R/Z097.01H/A098C, G097R/Z097.01R/A098G, G097R/Z097.01R/A098G/V147I, G097R/Z097.01R/A098S, G097R/Z097.01R, G097R/Z097.01S/A098G, G097R/Z097.01S/A098R, G097S/A098C, G097S/A098C/D099G, G097S/A098C/D099H, G097S/A098C/D099L, G097S/A098C/D099R, G097S/A098C/D099S, G097S/A098C/D099Z, G097S/A098G/D099F, G097S/A098G/D099G, G097S/A098G/D099G, G097S/A098G/D099S, G097S/A098G/D099Z, G097S/A098G/D099Z/G100R, G097S/A098H/D099C, G097S/A098H/D099G, G097S/A098N/D099G, G097S/A098R, G097S/A098R/D099C, G097S/A098R/D099G, G097S/A098R/D099L, G097S/A098R/D099V, G097S/A098R/D099Y, G097S/A098S/D099G, G097S/A098S/D099G/A133V/A153V/S236Y, G097S/A098S/D099G/A153V, G097S/A098S/D099Z, G097S/Z097.01D/A098G/A144T/Q271H, G097S/Z097.01G/A098V, G097S/Z097.01S/A098G, G097S/Z097.01S/A098G/A273T, G097S/Z097.01Y, G097V/A098R, G097V/A098R/D099G, G097V/A098S/D099S, G097Y/A098H/D099N, G097Z/A098S/D099H, Z097.01D/A098R/D099G, Z097.01D/A098S/D099L, Z097.01D/A098V/D099G, Z097.01G, Z097.01G/A098G, Z097.01G/A098G/D099S, Z097.01G/A098R, Z097.01G/A098R/D099C, Z097.01G/A098R/D099G, Z097.01G/A098R/D099S, A098C/D099G, A098C/D099G/A116T, A098C/D099H, A098C/D099L, A098C/D099L/K213R, A098C/D099L, A098C/D099N, A098C/D099R, A098C/D099S, A098C/D099S/P194L, A098D/D099C, A098D/D099G, A098D/D099G/G100L, A098D/D099H/G100S, A098D/D099H/H238Y, A098D/D099R, A098D/D099R/G100D, A098D/D099R/G160S, A098F/D099S/G100L, A098G/D099C A098G/D099G, A098G/D099G/A228T, A098G/D099G/G100H, A098G/D099G/N243D, A098G/D099G/S101P, A098G/D099G/T244S, A098G/D099G, A098G/D099H, A098G/D099L, A098G/D099R, A098G/D099R/A116T, A098G/D099R/G100D, A098G/D099R/V148I, A098G/D099S, A098G/D099S/L267M, A098G/D099V, A098G/D099Z, A098H/D099C, A098H/D099G, A098H/D099G/A216T, A098H/D099G/G100D, A098H/D099G/G100N/L267M, A098H/D099G/G100S, A098H/D099G/V143I, A098H/D099R, A098H/D099R/G100S, A098H/D099S/G100C, A098H/D099S/G100N, A098H/D099Y, A098I/D099G, A098I/D099S, A098L/D099G, A098L/D099S, A098N/D099G, A098N/D099L, A098N/D099S, A098R/A144T, A098R/A274V, A098R/D099C, A098R/D099G, A098R/D099G/G100C, A098R/D099G/G100D, A098R/D099G/G100L/S145T, A098R/D099G/G100N, A098R/D099G/G100R, A098R/D099G/G100S, A098R/D099G/V150I, A098R/D099H, A098R/D099I, A098R/D099L, A098R/D099N, A098R/D099N/G100L, A098R/D099R, A098R/D099R/G100D, A098R/D099R/S101Z, A098R/D099R/S130F, A098R/D099R, A098R/D099S, A098R/D099S/G100L/A153V, A098R/D099S, A098R/D099V, A098R/D099Z/S101G, A098R/G100C/A137T, A098R/G100S, A098S/D099C, A098S/D099G, A098S/D099G/G100C, A098S/D099G/G100D, A098S/D099G/G100R/L250I, A098S/D099G/G100S, A098S/D099G/Z099.01R, A098S/D099H, A098S/D099H/M124V, A098S/D099H/Z099.01R/G169A/V180A, A098S/D099R, A098S/D099R/G100L, A098S/D099R/G154S, A098S/D099R/G166S, A098S/D099R/Z100.01G, A098S/D099S, A098S/D099S/Z099.01R, A098S/D099V, A098T/P129Z/S130R/S132G, A098T/S101G, A098V/D099G, A098V/D099G/G100D, A098V/D099G/G100D/A153V, A098V/D099G/G100S, A098Y/D099R/G100L, A098Y/D099R/G100Z/S101Z/G102D, A098Z/S101R, Z098.01N, Z098.01R, Z098.01R/G100R, D099C/K136N, D099C/S101D, D099C/S101R, D099C/S101Z, D099C/Z099.01S, D099C/Z100.01G, D099F/S101Z, D099G/G100C, D099G/G100D/S101R, D099G/G100H, D099G/G100R/S101V, D099G/G100S/S101R, D099G/S101G, D099G/S101H, D099G/S101V, D099G/S101Z, D099G/S101Z/N269D, D099G/Z099.01N, D099G/Z099.01R/G100S, D099G/Z099.01S, D099G/Z100.01G, D099G/Z100.01G/S183N/Q275K, D099H/S101Z, D099H/S101Z/V150I, D099H/Z100.01G/A273V, D099H/Z100.01G/Z100.02G, D099I/Z099.01S, D099L/N118D, D099L/S101G, D099L/S101V, D099L/Z100.01G, D099N/A116T, D099N/G100R/S101V, D099N/S101G, D099N/S101H, D099N/S101Z, D099N/Z099.01S, D099N/Z100.01G, D099R/S101D, D099R/S101G, D099R/S101V, D099R/S101Z, D099R/S101Z/A133T, D099R/Z099.01N, D099R/Z099.01S, D099R/Z100.01G, D099R/Z100.01N, D099S/S101G, D099S/S101I, D099S/S101Z, D099S/Z099.01H, D099S/Z099.01L,

D099S/Z099.01V, D099S/Z100.01G, D099S/Z100.01G/Z100.02G, D099V/S101D, D099V/S101V, D099V/S101Z, Z099.01H/S101L, Z099.01H/Z099.02R, Z099.01N/S101R, Z099.01S, Z099.01S/S101V, Z099.01S/Z099.02G/A228T, G100D/S101G, G100D/S101R, G100H/G102V, G100H/S101G, G100H/S101R, G100L/S101G, G100L/S101R, G100N/S101D, G100N/S101L, G100N/S101R, G100R/L267R/I268V, G100R/S101G, G100R/S101G/G160S/S182N, G100R/S101G/Q103Z/Y104Z/S161N, G100R/S101G/S163N, G100R/S101N, G100R/S101R, G100R/S101R/Q103Z/V270I, G100R/S101Z/G102C/Q103D, G100S/S101C, G100S/S101G, G100S/S101R, G100S/S101R, G100S/S101V, G100S/S101V/A231T, Z100.01G, Z100.01L, Z100.01N, Z100.01R, Z100.01R/S101G, S101C/Q103I, S101C/Q103S, S101D/Q103C, S101D/Q103D, S101D/Q103F, S101D/Q103H, S101D/Q103H, S101D/Q103R, S101D/Q103R/H238Y, S101D/Q103S, S101D/Q103V, S101F/Q103G, S101G/A151V, S101G/A179V, S101G/G102S, S101G/Q103G, S101G/Q103L, S101G/Q103N, S101G/Q103R, S101G/Q103R/A179T, S101G/Q103R/G157D, S101G/Q103S, S101G/Q103S/L233S, S101G/Q103S/L233S, S101G/Q245H, S101G/S249N, S101G/N139I, S101G/Z101.01R, S101G/Z101.01R/A200T, S101G/Z102.01G, S101H/G102S, S101H/Q103D, S101H/Q103G, S101H/Q103G/E251Q, S101H/Q103H, S101H/Q103I, S101H/Q103R, S101H/Q103R/A274Z/Q275Z, S101H/Q103S, S101H/Q103V, S101I/Q103R, S101L/G102S/Q103R, S101L/Q103D, S101L/Q103G, S101L/Q103R, S101L/Q103R/A138T, S101L/Q103R/V121I/K213E, S101L/Q103S, S101L/Q103V, S101L/Z101.01R, S101N/Q103R, S101N/Z102.01G, S101R/A151V, S101R/A273V, S101R/E156G, S101R/G102L/Q103G, S101R/G102S, S101R/G102S/Q103R, S101R/G102S/Q103V, S101R/G131D, S101R/Q103C, S101R/Q103D, S101R/Q103D/S248R, S101R/Q103G, S101R/Q103G/A116T, S101R/Q103G/V147I/A153V, S101R/Q103G, S101R/Q103L, S101R/Q103N, S101R/Q103R, S101R/Q103R/S161N, S101R/Q103R/V148I, S101R/Q103S, S101R/Q103S/L126F, S101R/Q103V, S101V/Q103G, S101V/Q103H, S101V/Q103N/Z275.01K, S101V/Q103R, S101V/Q103V, S101Y/G102S/Q103R, S101Y/Q103L, S101Y/Q103S, S101Y/Q103V, S101Z/G102I/Q103H/Y104G/L135F, S101Z/G102V/Q103R/Y104L, S101Z/Q103D, S101Z/Q103F, S101Z/Q103L, S101Z/Q103R, G102C/Q103L/Y104S, G102R/Q103C/Y104C/V192I, G102R/Q103G/Y104R, G102R/Q103R/Z103.01G, G102Z/Q103S, Z102.01G/Q103R, Z102.01G/Q103R/L267V, Z102.01G/Z102.02R/Z102.03G, Q103H/Y104N/Q275Z, Q103N/E156D, Q103R/I122L/N123S/M124V, Q103R/Y104F, Q103Y/S182N, S105G/P129S/S130H/G131H, W106C/A116D/N117S/N118C, N109S/M119C/D120G/V121L, A116G/N117Z/N118R/M119C, A116H/N117G/N118R, N117C/N118G/M119S, N117C/N118G/M119S/A216V, N117G/N118G/M119C, N117G/N118R/M119C, N117H/N118C/M119C, N117H/N118D/M119S, N117H/N118G/M119C, N117H/N118G/M119L, N117H/N118G/M119S, N117H/N118G/M119V, N117H/N118H/M119V, N117H/N118R/M119C, N117H/N118R/M119S, N117H/N118S/M119C, N117H/N118S/M119S, N117H/N118S/M119V, N117I/N118G/M119V, N117L/N118G/M119V, N117R/N118G/M119C, N117R/N118G/M119I/V270I, N117R/N118G/M119S, N117R/N118H/M119V, N117R/N118R/M119C, N117R/N118R/M119I, N117R/N118R/M119S, N117R/N118R/M119V, N117R/N118S/M119I, N117R/N118S/M119L, N117S/N118G/M119C, N117S/N118G/M119S, N117S/N118H/M119V, N117S/N118R/M119V, N117V/N118G/M119C, N117V/N118H/M119C, N117Y/N118G/M119S, N117Y/N118G/M119V, Z117.01G/N118R/M119C, N118C/M119V/D120S, N118C/Z118.01G/M119C, N118D/M119C/D120L, N118D/M119S/D120R, N118G/M119C/D120G, N118G/M119C/D120L, N118G/M119I/D120R, N118G/M119N, N118G/M119S/D120G, N118G/M119S/D120R, N118G/M119V/D120G, N118G/M119V/D120S/S182N, N118H/M119C/D120R, N118H/M119I/D120G, N118H/M119N/V148I, N118H/M119S/D120G, N118H/M119V/D120G, N118R/M119C, N118R/M119C/D120G, N118R/M119C/D120R, N118R/M119S, N118R/M119S/D120G, N118R/M119S/D120R, N118R/M119S/D120S, N118R/M119V/D120C, N118R/M119V/D120G, N118R/M119V/D120G/A232T, N118S/M119C/D120R, M119C/D120G, M119C/D120G/V121I, M119C/D120G/V121L, M119C/D120H, M119C/D120H/A187V, M119C/D120R, M119H/V121I, M119I/D120C, M119I/D120G, M119I/D120R, M119L/D120G, M119S/D120R, M119S/D120S/V121I, M119S/V121I, M119V/D120C, M119V/D120G, M119V/D120R, M119V/D120S/V198L, D120G/I122C, D120G/I122C/L217S, D120G/I122G, D120G/I122V, D120H/I122L, D120H/V121C/I122V, D120N/V121L/I122V, D120R/A151V, D120R/I122V, D120R/V121I, D120R/V121I/I122C, D120R/V121I/I122C/A228T, D120S/I122C/A133S, V121C/I122C/N123C, V121C/I122G/N123V, V121C/I122S/N123V,V121C/I122V/N123S, V121I/I12C/N123C, V121I/I122C/V150I, V121I/I122G/N123C, V121I/I122S/N123C, V121I/I122S/N123I, V121I/I122V/N123S, V121L/I122S/N123V, V121L/N123C, I122C/N123C, I122C/N123S/M124L, I122D/N123V/M124L, I122G/N123C/M124L, I122G/N123G/Z123.01C, I122H/N123V/M124LI122S/N123S, I122V/N123G/M124C/A138V, I122V/N/23S/M124I, I122V/S183G, N123C/M124S, N123C/M124V, N123S/M124L, N123S/M124S, M124C/L126S, M124C/L126S/T244I, M124C/L126V, M124I/L126H, L126F/P129Z, L126F/P129Z/S182N, L126I/P129Z, L126R/G127R, Z126.01S, G127C/G128S/P129R, G127C/P129G, G127C/P129G/A200T, G127C/P129R, G127C/P129R/A153T, G127D/P129G, G127D/P129S, G127H/P129G, G127H/P129R, G127L/P129R, G127N/P129C, G127N/P129G, G127N/P129G/A133T,

G127N/P129G/A151V/K213N, G127N/P129R, G127N/P129S/A273V, G127N/P129V, G127R/A230V, G127R/P129D, G127R/P129D/L217S, G127R/P129G, G127R/P129R, G127R/P129S, G127S/P129D, G127S/P129F, G127S/P129G, G127S/P129G/G166D, G127S/P129G/I175T, G127S/P129R, G127S/P129S, G127S/P129V, G127V/P129G, G127V/P129R, Z127.01G/Z127.02H/P129D, Z127.01G/Z127.02H/P129G, Z127.01G/Z127.02H/P129R, Z127.01H/G128C/P129C/A230V, Z127.01H/P129G, Z127.01H/P129G/V148I, Z127.01H/P129R, Z127.01N/G128C/P129V, Z127.01N/G128S/P129S, Z127.01N/G128S/P129V, Z127.01N/P129R, Z127.01R/G128S/P129S, Z127.01R/P129G, Z127.01S/P129D, Z127.01S/P129G, Z127.01S/P129R, G128D/P129G, G128D/P129R/Z129.01R, G128D/P129S/G154S, G128H/P129C, G128H/P129H/Z129.01G, G128H/P129H/Z129.01R, G128H/P129R, G128H/P129R/Z129.01G, G128H/P129R/Z129.01R, G128H/P129S/Z129.01G, G128H/P129S/Z129.01R, G128H/P129S/Z129.01R/S248N, G128H/P129Y, G128H/Z128.01R/P129D, G128H/Z128.01R/P129S, G128H/Z128.01R/P129S/A144T, G128H/Z129.01R, 128N/P129R/Z129.01G, G128N/P129R/Z129.01R, G128N/P129S/Z129.01D, G128N/Z128.01R/P129S, G128R/P129G, G128R/P129H/Z129.01G, G128R/P129L/Z130.01S, G128R/P129R, G128R/P129R/Z129.01G, G128R/P129R/Z129.01G/A216V, G128R/P129S, G128R/P129S/Z129.01G, 128R/P129Z/S130R/S132G, G128R/Z128.01R/P129D, G128R/Z128.01R/P129F, G128S/P129C, G128S/P129C/Z129.01R, G128S/P129D, G128S/P129D/S248R, G128S/P129D/T244N, G128S/P129G, G128S/P129G/A187V, G128S/P129H/Z129.01R, G128S/P129R/L209F, G128S/P129R/Z129.01G, G128S/P129S/Z129.01D, G128S/P129S/Z129.01R, G128S/P129V, G128Y/P129R/Z129.01G, Z128.01G, Z128.01G/P129D, Z128.01G/P129R, Z128.01G/P129S/A134T, Z128.01G/Z128.02G, Z128.01R/P129D, Z128.01R/Z128.02G/P129S, Z128.01R/Z128.02H/P129G/G160S, Z128.01R/Z128.02R/P129D, Z128.01Y/Z128.02H/P129R, P129C/S130C/S132Z, P129D/Z129.01G/Z129.02D/G160D, P129D/Z129.01G/Z129.02G, P129G/G131Z, P129G/S130G/Z131.01G, P129G/S130H/S132Z, P129G/S130R/S132Z, P129G/V270I, P129G/Z129.01G, P129G/Z129.01R/Z129.02G, P129H/G131Z, P129H/G131Z/S132C, P129H/G131Z/S132H, P129H/S132Z, P129H/Z130.01D/Z130.02S, P129R/G131Z/S132H, P129R/S130D/S132Z, P129R/S130N/G131Z, P129R/S132T, P129R/S132Z, P129R/Z129.01G, P129R/Z129.01G/Z129.02G, P129R/Z129.01G/Z129.02G/A144T, P129R/Z129.01G/Z129.02G/G146D, P129R/Z129.01G/Z129.02R, P129R/Z129.01G, P129R/Z129.01R/Z129.02G, P129S/G131ZP129S/S130H/S132Z, P129S/Z129.01C, P129S/Z129.01R/Z129.02S, P129S/Z130.01N/Z130.02S, P129V/S132Z, P129Z, P129Z/G131R/S132C, P129Z/S130G, P129Z/S130G/G131H/S132H, P129Z/S130G/G131V, P129Z/S130H, P129Z/S130H/S132G, P129Z/S130H/S132N, P129Z/S130L, P129Z/S130R, P129Z/S130R/G131C, P129Z/S130R/G131C, P129Z/S130R/G131D/S132C, P129Z/S130R/G131H/A144E, P129Z/S130R/G131H/S132D, P129Z/S130R/G131R/S132D, P129Z/S130R/L267M, P129Z/S130R/S132C, P129Z/S130R/S132G, P129Z/S130V/S132G, P129Z/S130Z/G131N/S132G, S130D/G131C/S132D, S130D/G131R, S130D/G131R/S132D, S130D/G131Z, S130D/G131Z/S132H, S130F/G131N/S132V, S130F/G131S/S132V, S130F/S132V, S130G/G131D/S132C, S130G/G131H/S132D, S130G/G131S/S132I, S130G/S132G, S130H/G131C/S132G, S130H/G131H/S132R, S130H/G131R/S132C, S130H/G131S, S130H/G131S/S132C, S130H/S132Z, S130I/G131S/S132H, S130I/S132G, S130N/G131S/S132C, S130R/G131R/S132C, S130R/G131S/S132C/S161N, S130R/S132G, S130R/S132G/S183N, S130R/S132Z, S130V/G131D/S132I, S130V/G131H/S132C, S130V/S132N, S130V/S132Z, S130Y/G131H/S132C, S130Y/S132G, S130Z/G131H/S132H, G131H/S132L/W241R, G131N/S132C, G131S/S132G, G131Y/S132G, and A142V/G211V, wherein the positions correspond to the positions of BPN' subtilisin of SEQ ID NO:2.

**2.** A cleaning composition comprising a subtilisin variant comprising at least one modification, wherein said modification is selected from: V072G/G097A/G128A/Y217Q, G097A/G128S/Y217Q, G097A/G128S/P129D/Y217Q, G097A/G128A/A134T/Y217Q, M124V/L126A/Y217Q, G097A/G128A/P129D/Y217Q/A232P, G097A/G128A/P129D/Y217Q, G097A/G128S/P129V/A134T/Y217Q, G097A/G128S/P129V/Y217Q, V068I/G097A/G128S/P129D/Y217Q, G097A/S101D/G128S/S204F/Y217Q/S236F/T254P, G097A/S101D/G128S/Y217Q, G097A/G128A/P129V/Y217Q, G097A/S101D/G128A/Y217Q, G097A/S101D/G128S/P129D/Y217Q, G097A/S101D/G128A/A134T/Y217Q, G097A/Z099.01S/A114S/G128S/Y217Q, G097A/S101D/G128A/P194L/Y217Q, G097A/Z099.01S/G128S/Y217Q, G097A/S101D/G128A/P129D/Y217Q, Q019R/G097A/Z099.01S/G128S/P129D/Y217Q, G097A/G100S/G128S/Y217Q, G097A/S101D/G128S/P129V/Y217Q, G097A/Z099.01S/G128A/A134T/Y217Q, G097A/G100S/I107V/G128S/P129D/Y217Q, G097A/G100S/G128S/Y217Q/A230V, G097A/G100S/G128A/Y217Q/K237N, G097A/Z099.01S/G128S/P129D/Y217Q, G097A/G100S/G128S/P129D/Y217Q, Z096.01D/G097A/A098R/G128A/Y217Q, G097A/Z099.01S/I122S/N123G/G128A/Y217Q, G097A/Z099.01S/I122S/N123G/G128A/Y217Q/T220A/A232T, P005S/G097A/Z099.01S/S101D/G128A/Y217Q, G097A/S101D/G128A/P129V/Y217Q, V093Z/K094S/V095C/L096S/G097A/G128A/Y217Q, G097A/G100S/S101D/G128A/Y217Q/A232P,

Z096.01D/G097A/A098R/S101D/G128A/Y217Q, and G097A/Z099.01S/G100S/G128A/Y217Q, wherein the positions correspond to the positions of BPN' subtilisin of SEQ ID NO:2.

**3.** A cleaning composition comprising a subtilisin variant comprising at least two modifications, wherein said modifications are selected from: G097A/G128S/P129T/Y217Q, G097A/G128A/P129S/Y217Q, G097A/G128S/P129C/Y217Q, G097A/G128S/P129Q/Y217Q, G097A/G128S/P129E/Y217Q, G097A/P129S/Y217Q, G097A/G128S/P129A/Y217Q, G097A/G128S/P129K/Y217Q, G097A/P129N/Y217Q, G097A/P129E/Y217Q, G097A/G128S/P129K/G131S/Y217Q, G097A/G128S/P129R/Y217Q, G097A/G128S/P129V/Y217Q, G097A/G128S/P129Y/Y217Q, G097A/G128A/P129R/Y217Q, G097A/G128H/P129S/Y217Q, G097A/G128N/P129S/Y217Q/V270A, G097A/G128S/P129L/Y217Q, G097A/P129R/Y217Q, G097A/P129W/Y217Q, G097A/P129Z/S130A/Y217Q, G097A/G128H/P129R/Y217Q, G097A/G128H/P129T/Y217Q, G097A/P129G/Y217Q, G097A/G128N/P129A/Y217Q, G097A/G128H/Y217Q, G097A/G128S/P129W/Y217Q, G097A/P129Z/Y217Q, G097A/G128N/Z128.01A/P129S/Y217Q, G097A/G128N/Y217Q, G097A/G128A/P129L/Y217Q, G097A/G128T/P129N/Y217Q, G097A/G128H/P129A/Y217Q, G097A/G128M/P129R/Y217Q, G097A/G128Q/P129K/Y217Q, G097A/G128T/P129Q/Y217Q, G097A/G128T/P129H/Y217Q, G097A/G128T/P129R/Y217Q, G097A/G128D/P129E/Y217Q, G097A/G128T/P129T/Y217Q, G097A/G128T/P129A/Y217Q, G097A/G128Z/P129Z/Y217Q, G097A/G128S/Z128.01A/P129S/Y217Q, G097A/G128N/P129M/Y217Q, G097A/G128N/P129V/Y217Q, G097A/G128E/P129K/Y217Q, G097A/G128N/Z128.01A/P129F/Y217Q, G097A/G128C/P129R/Y217Q, G097A/G128T/P129M/Y217Q, G097A/G128R/Z128.01A/P129S/Y217Q, G097A/G128S/Z128.01A/P129A/Y217Q, G097A/G128D/Y217Q, G097A/G128T/P129Y/Y217Q, G097A/G128R/P129S/Y217Q, G097A/G128R/Z128.01A/P129T/Y217Q, G097A/G128R/Z128.01A/P129A/Y217Q, G097A/G128T/P129W/Y217Q, G097A/G128Y/Z128.01A/P129R/Y217Q, G097A/G128C/P129D/Y217Q, V084L/G097A/P129Z/Y217Q, G097A/G128R/P129D/Y217Q, G097A/G128A/Z128.01A/P129H/E195A/Y217Q, G097A/G128S/Z128.01A/P129Y/Y217Q, G097A/G128N/P129L/Y217Q, G097A/G128C/P129H/Y217Q, G097A/G128T/Z128.01A/P129T/Y217Q, G097A/G128C/Z128.01A/P129A/Y217Q, G097A/G128S/Z128.01A/P129I/Y217Q, G097A/G128S/Z128.01A/P129E/Y217Q/Q271H, G097A/G128S/Z128.01A/P129L/Y217Q, G097A/G128Y/Z128.01A/P129S/Y217Q, G097A/G128S/Z128.01A/P129M/R186H/Y217Q, G097A/G128E/Z128.01A/P129D/Y217Q, G097A/G128T/P129L/Y217Q, G097A/G128F/P129D/Y217Q, G097A/G128V/Z128.01A/P129R/Y217Q, G097A/G128L/Z128.01A/P129S/Y217Q, G097A/G128Y/P129T/Y217Q, G097A/G128C/P129Y/Y217Q, G097A/G128C/P129T/Y217Q, wherein the positions correspond to the positions of BPN' subtilisin of SEQ ID NO:2.

**4.** A cleaning composition comprising a subtilisin variant comprising at least two modifications, wherein said modifications are selected from: G097A/G128A/Y217Q/M222Q, M124V/L126A/P129V/Y217Q, G128A/P129V/Y217Q G097A/G128A/P129V/Y217Q, N123G/P129V/Y217Q, V30I/Z096.01D/G097A/A098R/G128A/Y217Q, Z096.01D/A098R/G128A/Y217Q, Z096.01D/A098R/M124V/L126A/Y217Q, G097A/Z098.01S/G128A/Y217Q, Z096.01D/A098R/N123G/Y217Q, Z096.01D/G097A/A098R/G128A/Y217Q/S236F, Z098.01S/M124V/L126A/Y217Q, Z098.01S/G128A/Y217Q, Z096.01D/G097A/A098R/G128A/Y217Q, V093I/Z098.01S/M124V/L126A/Y217Q, A073V/Z098.01S/N123G/Y217Q, G065D/Z098.01S/G128A/Y217Q, I011L/Z098.01S/G128A/Y217Q, L082F/Z096.01D/A098R/N123GN217Q, Z098.01S/N123G/A138V/Y217Q, Z098.01S/N123G/Y217Q, wherein the positions correspond to the positions of BPN' subtilisin of SEQ ID NO:2.

**5.** A cleaning composition comprising a subtilisin variant comprising the substitution Y217L and further comprising at least one modification set forth in any of Claims 1-4, and wherein the positions correspond to the positions of BPN' subtilisin of SEQ ID NO:2.

**6.** A cleaning composition comprising a subtilisin variant comprising the substitutions G97A/G128A/Y217Q and further comprising at least one modification set forth in any of Claims 1-4, and wherein the positions correspond to the positions of BPN' subtilisin of SEQ ID NO:2.

**7.** The cleaning composition of any of claims 1-6, wherein said cleaning composition is a laundry detergent.

**8.** The laundry detergent of Claim 7, wherein said laundry detergent is a heavy duty liquid laundry detergent.

**9.** The cleaning composition of any of claims 1-6, wherein said cleaning composition is a dish detergent.

**10.** The cleaning composition of any of Claims 1-9, further comprising one or more additional enzymes or enzyme

derivatives selected from the group consisting of hemicellulases, peroxidases, proteases, cellulases, xylanases, lipases, phospholipases, esterases, cutinases, pectinases, keratinases, reductases, oxidases, phenol oxidases, lipoxygenases, ligninases, pullulanases, tannases, pentosanases, malanases, ß-glucanases, arabinosidases, hyaluronidase, chondroitinase, laccase, and amylases, or mixtures thereof.

**11.** The cleaning composition of any of Claims 1-10, further comprising at least one stabilizing agent.

**12.** The cleaning composition of any of claims 1-11 comprising at least 0.0001 weight percent of the subtilisin variant of any of Claims 1-6, and optionally, at least one suitable adjunct ingredient.

**13.** A method of cleaning, said method comprising the steps of:

a) contacting a surface and/or an article comprising a fabric with the cleaning composition of any of Claims 1-12; and
b) optionally washing and/or rinsing said surface or article.

**14.** A PCR primer selected from the list provided in Table 2-1 (SEQ ID NOs: 8-121).

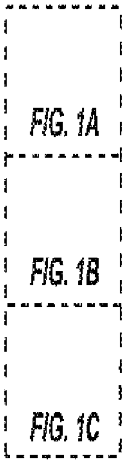
FIG. 1A
FIG. 1B
FIG. 1C

FIG. 1

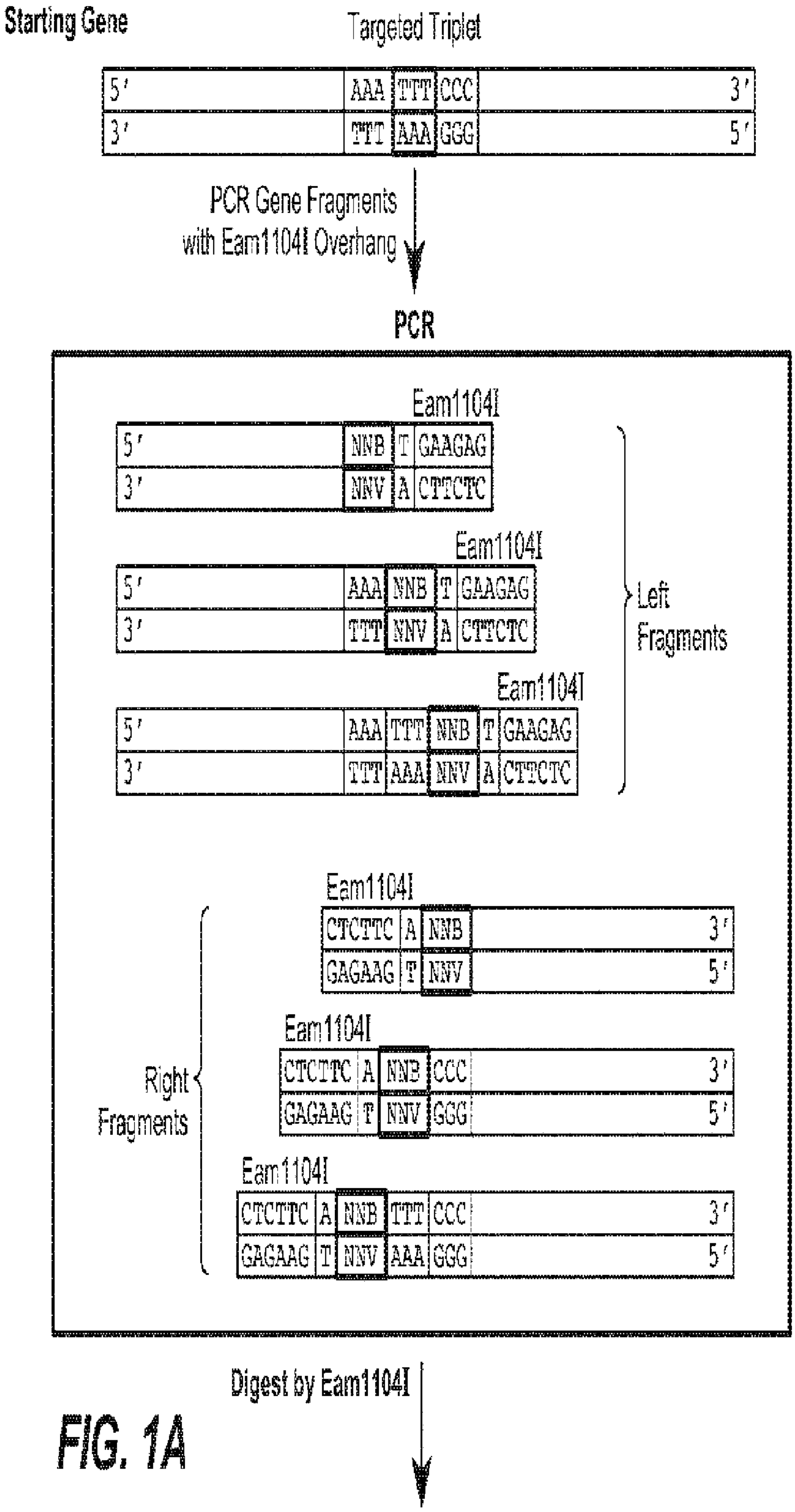
Starting Gene
Targeted Triplet
5′ AAA TTT CCC 3′
3′ TTT AAA GGG 5′
PCR Gene Fragments with Eam1104I Overhang
PCR
Eam1104I
5′ NNB T GAAGAG
3′ NNV A CTTCTC
Eam1104I
5′ AAA NNB T GAAGAG
3′ TTT NNV A CTTCTC
Eam1104I
5′ AAA TTT NNB T GAAGAG
3′ TTT AAA NNV A CTTCTC
Left Fragments
Eam1104I
CTCTTC A NNB 3′
GAGAAG T NNV 5′
Eam1104I
CTCTTC A NNB CCC 3′
GAGAAG T NNV GGG 5′
Eam1104I
CTCTTC A NNB TTT CCC 3′
GAGAAG T NNV AAA GGG 5′
Right Fragments
Digest by Eam1104I

FIG. 1A

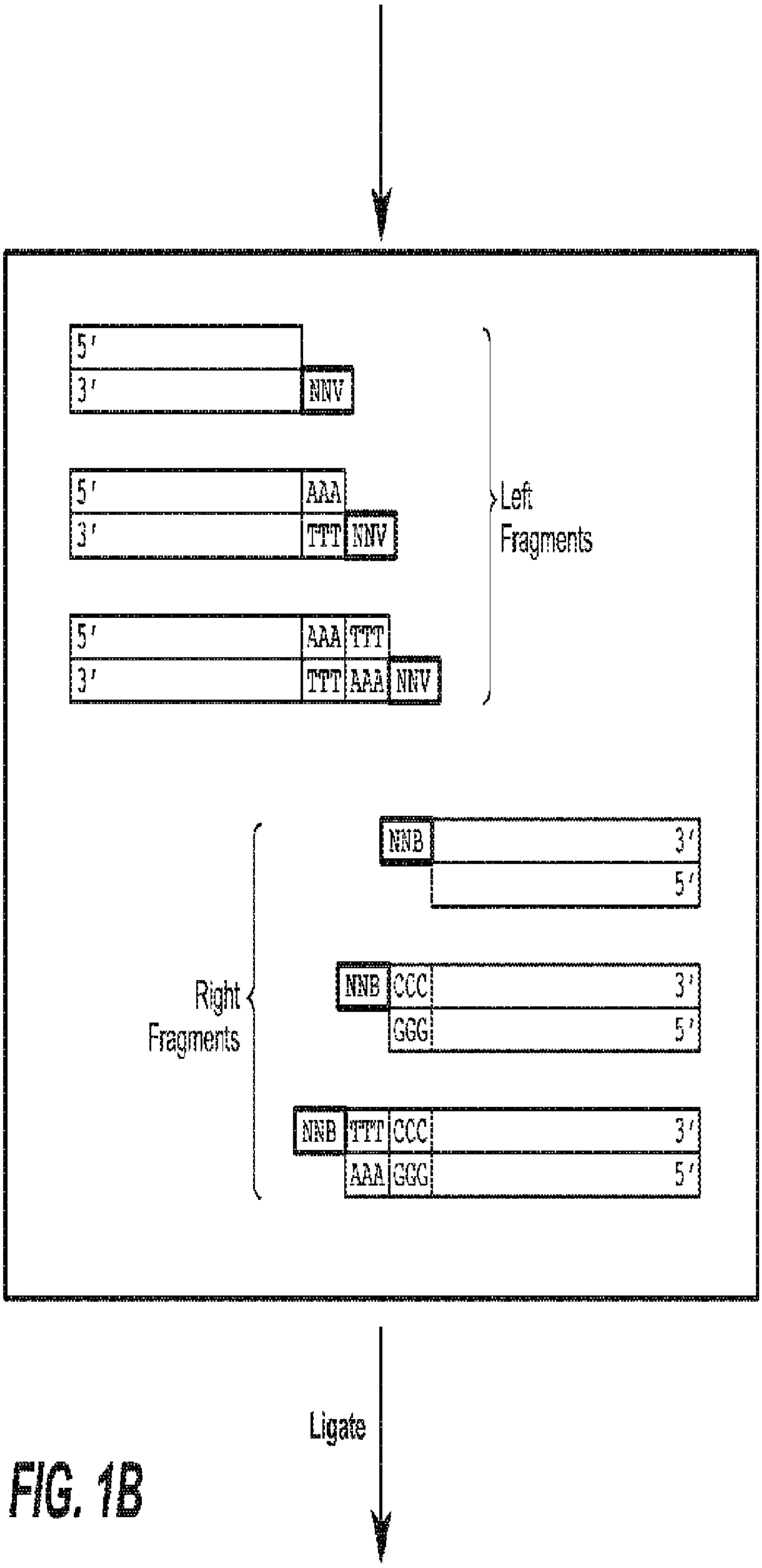
5′
3′
NNV
5′
AAA
3′
TTT
NNV
5′
AAA
TTT
3′
TTT
AAA
NNV
Left
Fragments
NNB
3′
5′
Right
Fragments
NNB
CCC
3′
GGG
5′
NNB
TTT
CCC
3′
AAA
GGG
5′
Ligate

FIG. 1B

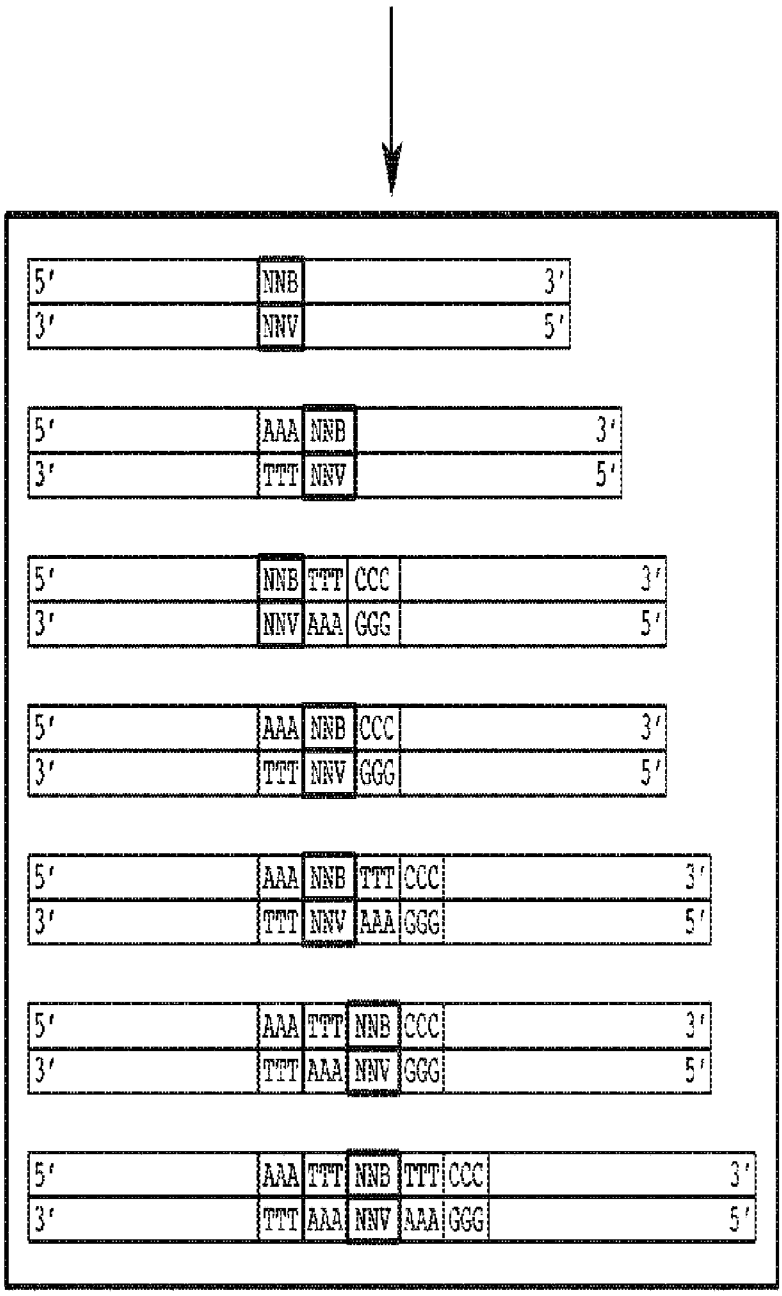


Examples of the Resulting Mutants

*FIG. 1C*

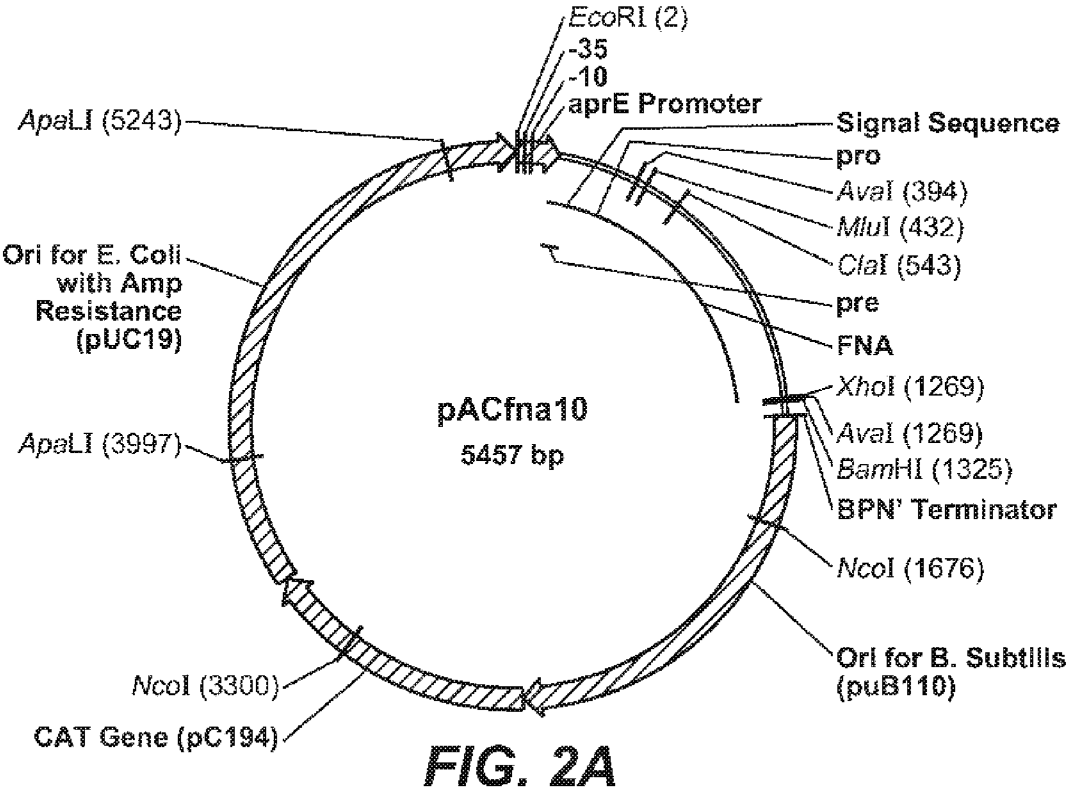
EcoRI (2)
-35
-10
aprE Promoter
ApaLI (5243)
Signal Sequence
pro
AvaI (394)
MluI (432)
ClaI (543)
pre
FNA
Ori for E. Coli with Amp Resistance (pUC19)
pACfna10
5457 bp
XhoI (1269)
AvaI (1269)
BamHI (1325)
BPN' Terminator
ApaLI (3997)
NcoI (1676)
Ori for B. Subtilis (puB110)
NcoI (3300)
CAT Gene (pC194)

FIG. 2A

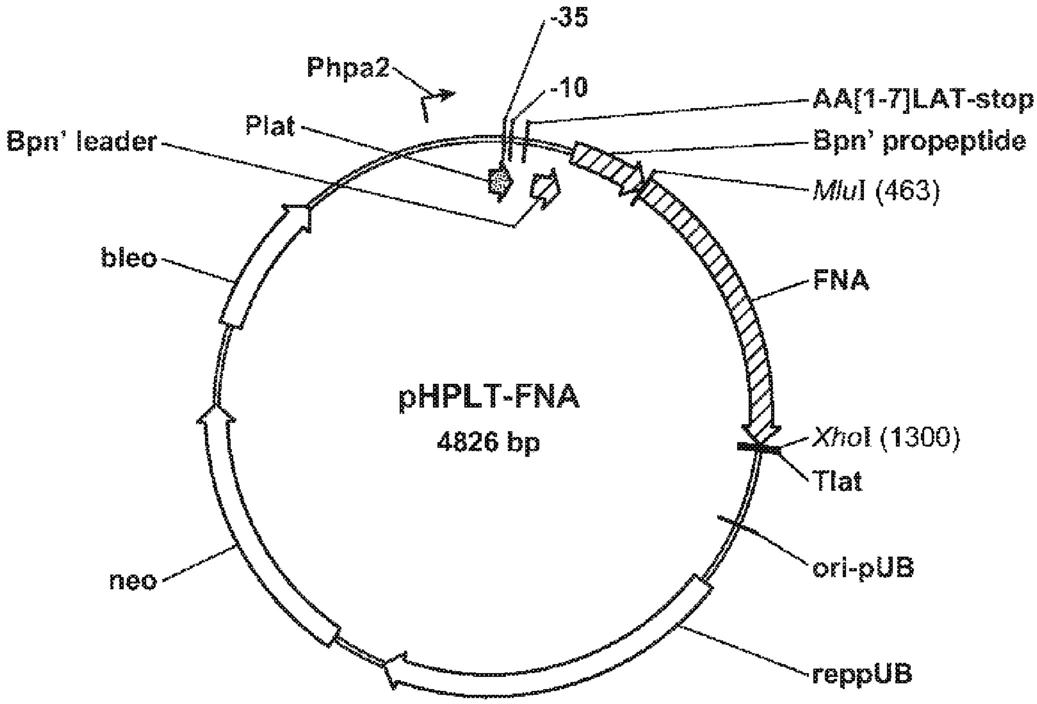
-35
-10
Phpa2
Plat
AA[1-7]LAT-stop
Bpn' leader
Bpn' propeptide
MluI (463)
bleo
FNA
pHPLT-FNA
4826 bp
XhoI (1300)
Tlat
ori-pUB
neo
reppUB

FIG. 2B

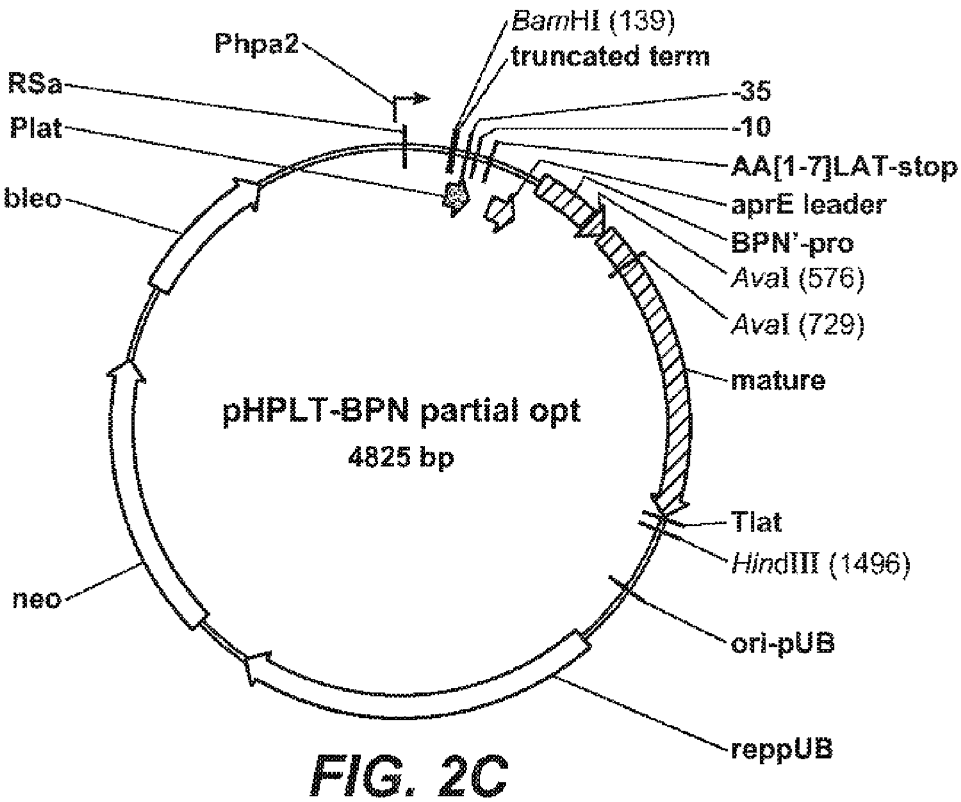
BamHI (139)
truncated term
Phpa2
RSa
-35
-10
Plat
AA[1-7]LAT-stop
bleo
aprE leader
BPN'-pro
AvaI (576)
AvaI (729)
mature
pHPLT-BPN partial opt
4825 bp
Tlat
HindIII (1496)
neo
ori-pUB
reppUB

*FIG. 2C*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9711151 A
- EP 0922499 A
- US 4977252 A
- US 5354559 A
- US 5935826 A
- US RE34606 E
- US 5955340 A
- US 5700676 A
- US 6312936 B
- US 6482628 B
- WO 8906270 A
- WO 9523221 A
- WO 9221760 A
- US 5801039 A
- US 5340735 A
- US 5500364 A
- US 5855625 A
- EP 258068 A
- EP 305216 A
- EP 238023 A
- EP 214761 A
- EP 218272 A
- EP 331376 A
- GB 1372034 A
- JP 64744992 B
- WO 9116422 A
- WO 8809367 A
- WO 9009446 A
- GB 1296839 A
- US 4435307 A
- EP 0495257 A
- US 5874276 A
- US 6566114 B
- US 6602842 B
- US 6440991 B
- WO 9412621 A
- WO 9501426 A
- WO 05056782 A
- US 6610642 B
- US 6605458 B
- US 5705464 A
- US 5710115 A
- US 5698504 A
- US 5695679 A
- US 5686014 A
- US 5646101 A
- US 6376450 B
- US 5879584 A
- US 5691297 A
- US 5574005 A
- US 5569645 A
- US 5565422 A
- US 5516448 A
- US 5489392 A
- US 5486303 A
- US 5576282 A
- US 6306812 B
- US 6326348 B
- US 4430243 A
- US 5597936 A
- US 5595967 A
- WO 00332601 A
- US 6225464 B
- WO 9934011 A
- US 4683195 A
- US 4683202 A
- US 4965188 A
- US 5322770 A
- US 69925000 A
- US 6312936 A
- US 6482628 A
- WO 9707770 A


### Non-patent literature cited in the description


- **MCKENZIE et al.** *Plasmid,* 1986, vol. 15, 93-103
- **BOLIVAR et al.** *Gene,* 1977, vol. 2, 95-113
- **HORINOUCHI ; WEISBLUM.** *J. Bacteriol,* 1982, vol. 150, 815-825
- **STEMMER.** *Proc Natl Acad Sci U S A.,* 1994, vol. 25, 10747-51
- **OSTERMEIER et al.** *Bioorg Med Chem.,* 1999, vol. 7, 2139-44
- **LUTZ et al.** *Proc Natl Acad Sci USA.,* 2001, vol. 98, 11248-53
- **SIEBER et al.** *Nat Biotechnol.,* 2001, vol. 19, 456-60
- **BITTKER et al.** *Nat Biotechnol.,* 2001, vol. 20, 1024-9
- **BITTKER et al.** *Proc Natl Acad Sci. USA.,* 2004, vol. 101, 7011-6

- **NESS et al.** *Nat Biotechnol.,* 2002, vol. 20, 1251-5
- **COCO et al.** *Nat Biotechnol.,* 2002, vol. 20, 1246-50
- **ZHA et al.** *Chembiochem,* 2003, vol. 3, 34-9
- **GLASER et al.** *J Immunol.,* 1992, vol. 149, 3903-13
- **SONDEK ; SHORTLE.** *Proc Natl Acad Sci USA,* 1992, vol. 89, 3581-5
- **YÁÑEZ et al.** *Nucleic Acids Res.,* 2004, vol. 32, e158
- **OSUNA et al.** *Nucleic Acids Res.,* 2004, vol. 32, e136
- **GAYTÁN et al.** *Nucleic Acids Res.,* 2001, vol. 29, E9
- **GAYTÁN et al.** *Nucleic Acids Res.,* 2002, vol. 30, e84
- **PISARCHIK et al.** *Prot. Eng. Des. Select.,* 2007, vol. 20, 257-265
- **DARTOIS et al.** *Biochem. Biophys. Acta,* 1993, vol. 1131, 253-260
- **YAMAGUCHI et al.** *Gene,* 1991, vol. 103, 61-67
- **SCHIMADA et al.** *J. Biochem.,* 1989, vol. 106, 383-388
- **HASS et al.** *Gene,* 1991, vol. 109, 117-113
- **KUGIMIYA et al.** *Biosci. Biotech. Biochem.,* 1992, vol. 56, 716-719
- **SAMBROOK et al.** Molecular Cloning: A Laboratory Manual. Cold Spring Harbor, 1989
- **AUSUBEL et al.** Current Protocols in Molecular Biology. 1987
- microbial Proteinases. **KALISZ.** Advances in Biochemical Engincering/Biotechnology. 1988
- **DEL MAR et al.** *Anal. Biochem.,* 1979, vol. 99, 316-320
- Genetics,. **FERRARI et al.** Bacillus. Plenum Publishing Corp, 1989, 57-72
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *Proc. Natl. Acad. Sci. USA,* 1988, vol. 85, 2444
- **DEVEREUX et al.** *Nucl. Acid Res.,* 1984, vol. 12, 387-395
- **FENG ; DOOLITTLE.** *J. Mol. Evol.,* 1987, vol. 35, 351-360
- **HIGGINS ; SHARP.** *CABIOS,* 1989, vol. 5, 151-153
- **ALTSCHUL et al.** *J. Mol. Biol.,* 1990, vol. 215, 403-410
- **KARLIN et al.** *Proc. Natl. Acad. Sci. USA,* 1993, vol. 90, 5873-5787
- **ALTSCHUL et al.** *Meth. Enzymol.,* 1996, vol. 266, 460-480
- **DYNAN ; TIJAN.** *Nature,* 1985, vol. 316, 774-78
- **HAHN et al.** *Mol Microbiol,* 1996, vol. 21, 713-775
- **AMIN et al.** *Biotechniques,* 2003, vol. 35, 1134-1140

SEQUENCE LISTING

<110> DANISCO US INC.
925 Page Mill Road
Palo Alto
California 94304-1013
USA

<120> COMPOSITIONS AND METHODS COMPRISING VARIANT MICROBIAL PROTEASES

<130> GRF/FP6905103

<140>
<141>

<150> 12183921.1
<151> 2009-06-03

<150> Divisional application of 09759304.0
<151> 2009-06-03

<150> EP 09759304.0
<151> 2009-06-03

<150> PCT/US09/46067
<151> 2009-06-03

<150> US 61/059,695
<151> 2008-06-06

<160> 221

<170> PatentIn version 3.5

<210> 1
<211> 275
<212> PRT
<213> B. amyloliquefaciens

<220>
<221> misc_feature
<223> Mature BPN' sequence

<400> 1

```
Ala Gln Ser Val Pro Tyr Gly Val Ser Gln Ile Lys Ala Pro Ala Leu
1               5                   10                  15

His Ser Gln Gly Tyr Thr Gly Ser Asn Val Lys Val Ala Val Ile Asp
            20                  25                  30

Ser Gly Ile Asp Ser Ser His Pro Asp Leu Lys Val Ala Gly Gly Ala
        35                  40                  45

Ser Met Val Pro Ser Glu Thr Asn Pro Phe Gln Asp Asn Asn Ser His
    50                  55                  60

Gly Thr His Val Ala Gly Thr Val Ala Ala Leu Asn Asn Ser Ile Gly
65                  70                  75                  80
```

```
Val Leu Gly Val Ala Pro Ser Ala Ser Leu Tyr Ala Val Lys Val Leu
                85                  90                  95

Gly Ala Asp Gly Ser Gly Gln Tyr Ser Trp Ile Ile Asn Gly Ile Glu
            100                 105                 110

Trp Ala Ile Ala Asn Asn Met Asp Val Ile Asn Met Ser Leu Gly Gly
        115                 120                 125

Pro Ser Gly Ser Ala Ala Leu Lys Ala Ala Val Asp Lys Ala Val Ala
    130                 135                 140

Ser Gly Val Val Val Val Ala Ala Ala Gly Asn Glu Gly Thr Ser Gly
145                 150                 155                 160

Ser Ser Ser Thr Val Gly Tyr Pro Gly Lys Tyr Pro Ser Val Ile Ala
                165                 170                 175

Val Gly Ala Val Asp Ser Ser Asn Gln Arg Ala Ser Phe Ser Ser Val
            180                 185                 190

Gly Pro Glu Leu Asp Val Met Ala Pro Gly Val Ser Ile Gln Ser Thr
        195                 200                 205

Leu Pro Gly Asn Lys Tyr Gly Ala Tyr Asn Gly Thr Ser Met Ala Ser
    210                 215                 220

Pro His Val Ala Gly Ala Ala Ala Leu Ile Leu Ser Lys His Pro Asn
225                 230                 235                 240

Trp Thr Asn Thr Gln Val Arg Ser Ser Leu Glu Asn Thr Thr Thr Lys
                245                 250                 255

Leu Gly Asp Ser Phe Tyr Tyr Gly Lys Gly Leu Ile Asn Val Gln Ala
            260                 265                 270

Ala Ala Gln
        275
```

<210>  2
<211>  275
<212>  PRT
<213>  B. amyloliquefaciens

<220>
<221>  misc_feature
<223>  Mature form of the BPN’ subtilisin protease

<400> 2

Ala Gln Ser Val Pro Tyr Gly Val Ser Gln Ile Lys Ala Pro Ala Leu
1 5 10 15

His Ser Gln Gly Tyr Thr Gly Ser Asn Val Lys Val Ala Val Ile Asp
20 25 30

Ser Gly Ile Asp Ser Ser His Pro Asp Leu Lys Val Ala Gly Gly Ala
35 40 45

Ser Met Val Pro Ser Glu Thr Asn Pro Phe Gln Asp Asn Asn Ser His
50 55 60

Gly Thr His Val Ala Gly Thr Val Ala Ala Leu Asn Asn Ser Ile Gly
65 70 75 80

Val Leu Gly Val Ala Pro Ser Ala Ser Leu Tyr Ala Val Lys Val Leu
85 90 95

Gly Ala Asp Gly Ser Gly Gln Tyr Ser Trp Ile Ile Asn Gly Ile Glu
100 105 110

Trp Ala Ile Ala Asn Asn Met Asp Val Ile Asn Met Ser Leu Gly Gly
115 120 125

Pro Ser Gly Ser Ala Ala Leu Lys Ala Ala Val Asp Lys Ala Val Ala
130 135 140

Ser Gly Val Val Val Val Ala Ala Ala Gly Asn Glu Gly Thr Ser Gly
145 150 155 160

Ser Ser Ser Thr Val Gly Tyr Pro Gly Lys Tyr Pro Ser Val Ile Ala
165 170 175

Val Gly Ala Val Asp Ser Ser Asn Gln Arg Ala Ser Phe Ser Ser Val
180 185 190

Gly Pro Glu Leu Asp Val Met Ala Pro Gly Val Ser Ile Gln Ser Thr
195 200 205

Leu Pro Gly Asn Lys Tyr Gly Ala Tyr Asn Gly Thr Ser Met Ala Ser
210 215 220

Pro His Val Ala Gly Ala Ala Ala Leu Ile Leu Ser Lys His Pro Asn
225 230 235 240

Trp Thr Asn Thr Gln Val Arg Ser Ser Leu Glu Asn Thr Thr Thr Lys

245 250 255

Leu Gly Asp Ser Phe Tyr Tyr Gly Lys Gly Leu Ile Asn Val Gln Ala
260 265 270

Ala Ala Gln
275

<210> 3
<211> 1326
<212> DNA
<213> B. amyloliquefaciens

<220>
<221> misc_feature
<223> Coding region of the BPN'-Y217L mature protease

<400> 3
gaattcatct caaaaaaatg ggtctactaa aatattattc catctattat aataaattca 60

cagaatagtc ttttaagtaa gtctactctg aatttttta aaaggagagg gtaaagagtg 120

agaagcaaaa aattgtgaga agcaaaaaat tgtggatcag tttgctgttt gctttagcgt 180

taatctttac gatggcgttc ggcagcacat ccagcgcgca ggctgcaggg aaatcaaacg 240

gggaaaagaa atatattgtc gggtttaaac agacaatgag cacgatgagc gccgctaaga 300

agaaagacgt catttctgaa aaaggcggga aagtgcaaaa gcaattcaaa tatgtagacg 360

cagctagcgc tacattaaac gaaaaagctg taaaagaatt gaaaaaagac ccgagcgtcg 420

cttacgttga agaagatcac gtagcacacg cgtacgcgca gtccgtgcca tatggcgtat 480

cacaaattaa agcccctgct ctgcactctc aaggctacac cggttcaaat gttaaagtag 540

cggttatcga cagcggtatc gattcttctc atccagatct taaagtagca ggcggagcca 600

gcatggttcc ttctgaaaca aatcctttcc aagacaacaa ctctcacgga acacacgttg 660

ctggtaccgt tgcggctctt aataactcaa tcggtgtatt aggcgttgcg ccaagcgcat 720

cactttacgc tgtaaaagtt ctcggcgccg acggttccgg ccaatacagc tggatcatta 780

acggaatcga gtgggcgatc gcaaacaata tggacgttat taacatgagc ctcggcggac 840

cgtccggttc tgctgcttta aaagcggcag ttgataaagc cgttgcatcc ggcgtcgtag 900

tcgttgcggc agccggcaac gaaggcactt ccggcagctc aagcacagtg ggctaccctg 960

gtaaataccc ttctgtcatt gcagtaggcg ctgtcgacag cagcaaccaa agagcatctt 1020

tctcaagcgt aggacctgag ctcgatgtca tggcacctgg cgtatctatc caaagcacgc 1080

ttcctggaaa caaatacggc gcgttgaacg gtacatcaat ggcatctccg cacgttgccg 1140

gagccgcggc tttgattctt tctaagcacc cgaactggac aaacactcaa gtccgcagct 1200

ctctagaaaa caccactaca aaacttggtg attctttcta ctatggaaaa gggctgatca 1260

atgtacaggc ggcagctcag taaaactcga gataaaaaac cggccttggc cccgccggtt 1320

ttttat 1326

<210> 4
<211> 382
<212> PRT
<213> B. amyloliquefaciens

<220>
<221> misc_feature
<223> Amino acid sequence of the BPN'-Y217L precursor protein

<400> 4

Met Arg Ser Lys Lys Leu Trp Ile Ser Leu Leu Phe Ala Leu Ala Leu
1 5 10 15

Ile Phe Thr Met Ala Phe Gly Ser Thr Ser Ser Ala Gln Ala Ala Gly
20 25 30

Lys Ser Asn Gly Glu Lys Lys Tyr Ile Val Gly Phe Lys Gln Thr Met
35 40 45

Ser Thr Met Ser Ala Ala Lys Lys Lys Asp Val Ile Ser Glu Lys Gly
50 55 60

Gly Lys Val Gln Lys Gln Phe Lys Tyr Val Asp Ala Ala Ser Ala Thr
65 70 75 80

Leu Asn Glu Lys Ala Val Lys Glu Leu Lys Lys Asp Pro Ser Val Ala
85 90 95

Tyr Val Glu Glu Asp His Val Ala His Ala Tyr Ala Gln Ser Val Pro
100 105 110

Tyr Gly Val Ser Gln Ile Lys Ala Pro Ala Leu His Ser Gln Gly Tyr
115 120 125

Thr Gly Ser Asn Val Lys Val Ala Val Ile Asp Ser Gly Ile Asp Ser
130 135 140

Ser His Pro Asp Leu Lys Val Ala Gly Gly Ala Ser Met Val Pro Ser
145 150 155 160

Glu Thr Asn Pro Phe Gln Asp Asn Asn Ser His Gly Thr His Val Ala
165 170 175

Gly Thr Val Ala Ala Leu Asn Asn Ser Ile Gly Val Leu Gly Val Ala
180 185 190

```
Pro Ser Ala Ser Leu Tyr Ala Val Lys Val Leu Gly Ala Asp Gly Ser
        195                 200                 205

Gly Gln Tyr Ser Trp Ile Ile Asn Gly Ile Glu Trp Ala Ile Ala Asn
    210                 215                 220

Asn Met Asp Val Ile Asn Met Ser Leu Gly Gly Pro Ser Gly Ser Ala
225                 230                 235                 240

Ala Leu Lys Ala Ala Val Asp Lys Ala Val Ala Ser Gly Val Val Val
                245                 250                 255

Val Ala Ala Ala Gly Asn Glu Gly Thr Ser Gly Ser Ser Ser Thr Val
            260                 265                 270

Gly Tyr Pro Gly Lys Tyr Pro Ser Val Ile Ala Val Gly Ala Val Asp
        275                 280                 285

Ser Ser Asn Gln Arg Ala Ser Phe Ser Ser Val Gly Pro Glu Leu Asp
    290                 295                 300

Val Met Ala Pro Gly Val Ser Ile Gln Ser Thr Leu Pro Gly Asn Lys
305                 310                 315                 320

Tyr Gly Ala Leu Asn Gly Thr Ser Met Ala Ser Pro His Val Ala Gly
                325                 330                 335

Ala Ala Ala Leu Ile Leu Ser Lys His Pro Asn Trp Thr Asn Thr Gln
            340                 345                 350

Val Arg Ser Ser Leu Glu Asn Thr Thr Thr Lys Leu Gly Asp Ser Phe
        355                 360                 365

Tyr Tyr Gly Lys Gly Leu Ile Asn Val Gln Ala Ala Ala Gln
    370                 375                 380
```

<210> 5
<211> 275
<212> PRT
<213> B. amyloliquefaciens

<220>
<221> misc_feature
<223> Amino acid sequence of the mature BPN'-Y217L protease

<400> 5

```
Ala Gln Ser Val Pro Tyr Gly Val Ser Gln Ile Lys Ala Pro Ala Leu
1               5                   10                  15
```

```
His Ser Gln Gly Tyr Thr Gly Ser Asn Val Lys Val Ala Val Ile Asp
            20                  25                  30

Ser Gly Ile Asp Ser Ser His Pro Asp Leu Lys Val Ala Gly Gly Ala
        35                  40                  45

Ser Met Val Pro Ser Glu Thr Asn Pro Phe Gln Asp Asn Asn Ser His
    50                  55                  60

Gly Thr His Val Ala Gly Thr Val Ala Ala Leu Asn Asn Ser Ile Gly
65                  70                  75                  80

Val Leu Gly Val Ala Pro Ser Ala Ser Leu Tyr Ala Val Lys Val Leu
                85                  90                  95

Gly Ala Asp Gly Ser Gly Gln Tyr Ser Trp Ile Ile Asn Gly Ile Glu
            100                 105                 110

Trp Ala Ile Ala Asn Asn Met Asp Val Ile Asn Met Ser Leu Gly Gly
        115                 120                 125

Pro Ser Gly Ser Ala Ala Leu Lys Ala Ala Val Asp Lys Ala Val Ala
    130                 135                 140

Ser Gly Val Val Val Val Ala Ala Ala Gly Asn Glu Gly Thr Ser Gly
145                 150                 155                 160

Ser Ser Ser Thr Val Gly Tyr Pro Gly Lys Tyr Pro Ser Val Ile Ala
                165                 170                 175

Val Gly Ala Val Asp Ser Ser Asn Gln Arg Ala Ser Phe Ser Ser Val
            180                 185                 190

Gly Pro Glu Leu Asp Val Met Ala Pro Gly Val Ser Ile Gln Ser Thr
        195                 200                 205

Leu Pro Gly Asn Lys Tyr Gly Ala Leu Asn Gly Thr Ser Met Ala Ser
    210                 215                 220

Pro His Val Ala Gly Ala Ala Ala Leu Ile Leu Ser Lys His Pro Asn
225                 230                 235                 240

Trp Thr Asn Thr Gln Val Arg Ser Ser Leu Glu Asn Thr Thr Thr Lys
                245                 250                 255

Leu Gly Asp Ser Phe Tyr Tyr Gly Lys Gly Leu Ile Asn Val Gln Ala
            260                 265                 270
```

```
Ala Ala Gln
        275


<210>  6
<211>  21
<212>  DNA
<213>  Artificial Sequence

<220>
<223>  Synthetic primer:   P3203

<400>  6
tggatcagtt tgctgtttgc t                                                 21


<210>  7
<211>  25
<212>  DNA
<213>  Artificial Sequence

<220>
<223>  Synthetic primer:   P3435

<400>  7
atgtatcaag ataagaaaga acaag                                             25


<210>  8
<211>  40
<212>  DNA
<213>  Artificial Sequence

<220>
<223>  Synthetic primer:   P4328


<220>
<221>  misc_feature
<222>  (11)..(11)
<223>  n is a, c, g, or t

<220>
<221>  misc_feature
<222>  (14)..(14)
<223>  n is a, c, g, or t

<400>  8
aaactcttca ndtndtcacg gaactcacgt tgccggcaca                             40


<210>  9
<211>  40
<212>  DNA
<213>  Artificial Sequence

<220>
<223>  Synthetic primer:   P4329


<220>
```

<221> misc_feature
<222> (11)..(11)
<223> n is a, c, g, or t

<220>
<221> misc_feature
<222> (14)..(14)
<223> n is a, c, g, or t

<400> 9
aaactcttca ndtndtggaa ctcacgttgc cggcacagtt 40

<210> 10
<211> 38
<212> DNA
<213> Artificial Sequence

<220>
<223> Synthetic primer: P4330

<220>
<221> misc_feature
<222> (11)..(11)
<223> n is a, c, g, or t

<220>
<221> misc_feature
<222> (14)..(14)
<223> n is a, c, g, or t

<400> 10
aaactcttca ndtndtactc acgttgccgg cacagttg 38

<210> 11
<211> 40
<212> DNA
<213> Artificial Sequence

<220>
<223> Synthetic primer: P4331

<220>
<221> misc_feature
<222> (11)..(11)
<223> n is a, c, g, or t

<220>
<221> misc_feature
<222> (14)..(14)
<223> n is a, c, g, or t

<400> 11
aaactcttca ndtndtcacg ttgccggcac agttgcggct 40

<210> 12
<211> 40
<212> DNA
<213> Artificial Sequence

```
<220>
<223>  Synthetic primer:   P4332


<220>
<221>  misc_feature
<222>  (11)..(11)
<223>  n is a, c, g, or t

<220>
<221>  misc_feature
<222>  (14)..(14)
<223>  n is a, c, g, or t

<400>  12
aaactcttca ndtndtgttg ccggcacagt tgcggctctt                              40


<210>  13
<211>  40
<212>  DNA
<213>  Artificial Sequence

<220>
<223>  Synthetic primer:   P4333


<220>
<221>  misc_feature
<222>  (11)..(11)
<223>  n is a, c, g, or t

<220>
<221>  misc_feature
<222>  (14)..(14)
<223>  n is a, c, g, or t

<400>  13
aaactcttca ndtndtgccg gcacagttgc ggctcttaat                              40


<210>  14
<211>  41
<212>  DNA
<213>  Artificial Sequence

<220>
<223>  Synthetic primer:   P4334


<220>
<221>  misc_feature
<222>  (11)..(11)
<223>  n is a, c, g, or t

<220>
<221>  misc_feature
<222>  (14)..(14)
<223>  n is a, c, g, or t

<400>  14
aaactcttca ndtndtcggc acagttgcgg ctcttaataa c                            41
```

<210> 15
<211> 40
<212> DNA
<213> Artificial Sequence

<220>
<223> Synthetic primer: P4335

<220>
<221> misc_feature
<222> (11)..(11)
<223> n is a, c, g, or t

<220>
<221> misc_feature
<222> (14)..(14)
<223> n is a, c, g, or t

<400> 15
aaactcttca ndtndtacag ttgcggctct taataactca 40

<210> 16
<211> 40
<212> DNA
<213> Artificial Sequence

<220>
<223> Synthetic primer: P4336

<220>
<221> misc_feature
<222> (11)..(11)
<223> n is a, c, g, or t

<220>
<221> misc_feature
<222> (14)..(14)
<223> n is a, c, g, or t

<400> 16
aaactcttca ndtndtgttg cggctcttaa taactcaatc 40

<210> 17
<211> 40
<212> DNA
<213> Artificial Sequence

<220>
<223> Synthetic primer: P4337

<220>
<221> misc_feature
<222> (11)..(11)
<223> n is a, c, g, or t

<220>

<221> misc_feature
<222> (14)..(14)
<223> n is a, c, g, or t

<400> 17
aaactcttca ndtndtgcgg ctcttaataa ctcaatcggt 40

<210> 18
<211> 40
<212> DNA
<213> Artificial Sequence

<220>
<223> Synthetic primer: P4338

<220>
<221> misc_feature
<222> (11)..(11)
<223> n is a, c, g, or t

<220>
<221> misc_feature
<222> (14)..(14)
<223> n is a, c, g, or t

<400> 18
aaactcttca ndtndtgctc ttaataactc aatcggtgta 40

<210> 19
<211> 40
<212> DNA
<213> Artificial Sequence

<220>
<223> Synthetic primer: P4339

<220>
<221> misc_feature
<222> (11)..(11)
<223> n is a, c, g, or t

<220>
<221> misc_feature
<222> (14)..(14)
<223> n is a, c, g, or t

<400> 19
aaactcttca ndtndtctta ataactcaat cggtgtatta 40

<210> 20
<211> 38
<212> DNA
<213> Artificial Sequence

<220>
<223> Synthetic primer: P4340

<220>
<221> misc_feature
<222> (11)..(11)
<223> n is a, c, g, or t

<220>
<221> misc_feature
<222> (14)..(14)
<223> n is a, c, g, or t

<400> 20
aaactcttca ndtndtaata actcaatcgg tgtattag 38

<210> 21
<211> 40
<212> DNA
<213> Artificial Sequence

<220>
<223> Synthetic primer: P4341

<220>
<221> misc_feature
<222> (11)..(11)
<223> n is a, c, g, or t

<220>
<221> misc_feature
<222> (14)..(14)
<223> n is a, c, g, or t

<400> 21
aaactcttca ndtndtaact caatcggtgt attaggcgtt 40

<210> 22
<211> 38
<212> DNA
<213> Artificial Sequence

<220>
<223> Synthetic primer: P4342

<220>
<221> misc_feature
<222> (11)..(11)
<223> n is a, c, g, or t

<220>
<221> misc_feature
<222> (14)..(14)
<223> n is a, c, g, or t

<400> 22
aaactcttca ndtndttcaa tcggtgtatt aggcgttg 38

<210> 23
<211> 35
<212> DNA

<213> Artificial Sequence

<220>
<223> Synthetic primer: P4343

<220>
<221> misc_feature
<222> (11)..(11)
<223> n is a, c, g, or t

<400> 23
aaactcttca ndttcaatcg gtgtattagg cgttg 35

<210> 24
<211> 39
<212> DNA
<213> Artificial Sequence

<220>
<223> Synthetic primer: P4344

<220>
<221> misc_feature
<222> (11)..(11)
<223> n is a, c, g, or t

<220>
<221> misc_feature
<222> (14)..(14)
<223> n is a, c, g, or t

<400> 24
aaactcttca ndtndtaaag ttctcggtgc tgacggttc 39

<210> 25
<211> 37
<212> DNA
<213> Artificial Sequence

<220>
<223> Synthetic primer: P4345

<220>
<221> misc_feature
<222> (11)..(11)
<223> n is a, c, g, or t

<220>
<221> misc_feature
<222> (14)..(14)
<223> n is a, c, g, or t

<400> 25
aaactcttca ndtndtgttc tcggtgctga cggttcc 37

<210> 26
<211> 40

<212> DNA
<213> Artificial Sequence

<220>
<223> Synthetic primer: P4346

<220>
<221> misc_feature
<222> (11)..(11)
<223> n is a, c, g, or t

<220>
<221> misc_feature
<222> (14)..(14)
<223> n is a, c, g, or t

<400> 26
aaactcttca ndtndtctcg gtgctgacgg ttccggccaa 40

<210> 27
<211> 40
<212> DNA
<213> Artificial Sequence

<220>
<223> Synthetic primer: P4347

<220>
<221> misc_feature
<222> (11)..(11)
<223> n is a, c, g, or t

<220>
<221> misc_feature
<222> (14)..(14)
<223> n is a, c, g, or t

<400> 27
aaactcttca ndtndtggtg ctgacggttc cggccaatac 40

<210> 28
<211> 38
<212> DNA
<213> Artificial Sequence

<220>
<223> Synthetic primer: P4348

<220>
<221> misc_feature
<222> (11)..(11)
<223> n is a, c, g, or t

<220>
<221> misc_feature
<222> (14)..(14)
<223> n is a, c, g, or t

<400> 28
aaactcttca ndtndtgctg acggttccgg ccaataca 38

<210> 29
<211> 39
<212> DNA
<213> Artificial Sequence

<220>
<223> Synthetic primer: P4349

<220>
<221> misc_feature
<222> (11)..(11)
<223> n is a, c, g, or t

<220>
<221> misc_feature
<222> (14)..(14)
<223> n is a, c, g, or t

<400> 29
aaactcttca ndtndtgacg gttccggcca atacagctg 39

<210> 30
<211> 40
<212> DNA
<213> Artificial Sequence

<220>
<223> Synthetic primer: P4350

<220>
<221> misc_feature
<222> (11)..(11)
<223> n is a, c, g, or t

<220>
<221> misc_feature
<222> (14)..(14)
<223> n is a, c, g, or t

<400> 30
aaactcttca ndtndtggtt ccggccaata cagctggatc 40

<210> 31
<211> 40
<212> DNA
<213> Artificial Sequence

<220>
<223> Synthetic primer: P4351

<220>
<221> misc_feature
<222> (11)..(11)
<223> n is a, c, g, or t

<220>
<221> misc_feature
<222> (14)..(14)
<223> n is a, c, g, or t

<400> 31
aaactcttca ndtndttccg gccaatacag ctggatcatt 40

<210> 32
<211> 40
<212> DNA
<213> Artificial Sequence

<220>
<223> Synthetic primer: P4352

<220>
<221> misc_feature
<222> (11)..(11)
<223> n is a, c, g, or t

<220>
<221> misc_feature
<222> (14)..(14)
<223> n is a, c, g, or t

<400> 32
aaactcttca ndtndtggcc aatacagctg gatcattaac 40

<210> 33
<211> 40
<212> DNA
<213> Artificial Sequence

<220>
<223> Synthetic primer: P4353

<220>
<221> misc_feature
<222> (11)..(11)
<223> n is a, c, g, or t

<220>
<221> misc_feature
<222> (14)..(14)
<223> n is a, c, g, or t

<400> 33
aaactcttca ndtndtcaat acagctggat cattaacgga 40

<210> 34
<211> 40
<212> DNA
<213> Artificial Sequence

<220>
<223> Synthetic primer: P4354

<220>
<221> misc_feature
<222> (11)..(11)
<223> n is a, c, g, or t

<220>
<221> misc_feature
<222> (14)..(14)
<223> n is a, c, g, or t

<400> 34
aaactcttca ndtndttaca gctggatcat taacggaatc 40

<210> 35
<211> 40
<212> DNA
<213> Artificial Sequence

<220>
<223> Synthetic primer: P4355

<220>
<221> misc_feature
<222> (11)..(11)
<223> n is a, c, g, or t

<220>
<221> misc_feature
<222> (14)..(14)
<223> n is a, c, g, or t

<400> 35
aaactcttca ndtndtagct ggatcattaa cggaatcgag 40

<210> 36
<211> 39
<212> DNA
<213> Artificial Sequence

<220>
<223> Synthetic primer: P4356

<220>
<221> misc_feature
<222> (11)..(11)
<223> n is a, c, g, or t

<220>
<221> misc_feature
<222> (14)..(14)
<223> n is a, c, g, or t

<400> 36
aaactcttca ndtndttgga tcattaacgg aatcgagtg 39

<210> 37

<211> 36
<212> DNA
<213> Artificial Sequence

<220>
<223> Synthetic primer: P4357

<220>
<221> misc_feature
<222> (11)..(11)
<223> n is a, c, g, or t

<220>
<221> misc_feature
<222> (14)..(14)
<223> n is a, c, g, or t

<400> 37
aaactcttca ndtndtatca ttaacggaat cgagtg 36

<210> 38
<211> 40
<212> DNA
<213> Artificial Sequence

<220>
<223> Synthetic primer: P4358

<220>
<221> misc_feature
<222> (11)..(11)
<223> n is a, c, g, or t

<220>
<221> misc_feature
<222> (14)..(14)
<223> n is a, c, g, or t

<400> 38
aaactcttca ndtndtatta acggaatcga gtgggcgatc 40

<210> 39
<211> 40
<212> DNA
<213> Artificial Sequence

<220>
<223> Synthetic primer: P4359

<220>
<221> misc_feature
<222> (11)..(11)
<223> n is a, c, g, or t

<220>
<221> misc_feature
<222> (14)..(14)
<223> n is a, c, g, or t

<400> 39
aaactcttca ndtndtaacg gaatcgagtg ggcgatcgca 40

<210> 40
<211> 40
<212> DNA
<213> Artificial Sequence

<220>
<223> Synthetic primer: P4360

<220>
<221> misc_feature
<222> (11)..(11)
<223> n is a, c, g, or t

<220>
<221> misc_feature
<222> (14)..(14)
<223> n is a, c, g, or t

<400> 40
aaactcttca ndtndtggaa tcgagtgggc gatcgcaaac 40

<210> 41
<211> 40
<212> DNA
<213> Artificial Sequence

<220>
<223> Synthetic primer: P4361

<220>
<221> misc_feature
<222> (11)..(11)
<223> n is a, c, g, or t

<220>
<221> misc_feature
<222> (14)..(14)
<223> n is a, c, g, or t

<400> 41
aaactcttca ndtndtatcg agtgggcgat cgcaaacaat 40

<210> 42
<211> 40
<212> DNA
<213> Artificial Sequence

<220>
<223> Synthetic primer: P4362

<220>
<221> misc_feature
<222> (11)..(11)

<223> n is a, c, g, or t

<220>
<221> misc_feature
<222> (14)..(14)
<223> n is a, c, g, or t

<400> 42
aaactcttca ndtndtgagt gggcgatcgc aaacaatatg 40

<210> 43
<211> 40
<212> DNA
<213> Artificial Sequence

<220>
<223> Synthetic primer: P4363

<220>
<221> misc_feature
<222> (11)..(11)
<223> n is a, c, g, or t

<220>
<221> misc_feature
<222> (14)..(14)
<223> n is a, c, g, or t

<400> 43
aaactcttca ndtndttggg cgatcgcaaa caatatggac 40

<210> 44
<211> 40
<212> DNA
<213> Artificial Sequence

<220>
<223> Synthetic primer: P4364

<220>
<221> misc_feature
<222> (11)..(11)
<223> n is a, c, g, or t

<220>
<221> misc_feature
<222> (14)..(14)
<223> n is a, c, g, or t

<400> 44
aaactcttca ndtndtgcga tcgcaaacaa tatggacgtt 40

<210> 45
<211> 40
<212> DNA
<213> Artificial Sequence

<220>

<223> Synthetic primer: P4365

<220>
<221> misc_feature
<222> (11)..(11)
<223> n is a, c, g, or t

<220>
<221> misc_feature
<222> (14)..(14)
<223> n is a, c, g, or t

<400> 45
aaactcttca ndtndtatcg caaacaatat ggacgttatt 40

<210> 46
<211> 40
<212> DNA
<213> Artificial Sequence

<220>
<223> Synthetic primer: P4366

<220>
<221> misc_feature
<222> (11)..(11)
<223> n is a, c, g, or t

<220>
<221> misc_feature
<222> (14)..(14)
<223> n is a, c, g, or t

<400> 46
aaactcttca ndtndtgcaa acaatatgga cgttattaac 40

<210> 47
<211> 40
<212> DNA
<213> Artificial Sequence

<220>
<223> Synthetic primer: P4367

<220>
<221> misc_feature
<222> (11)..(11)
<223> n is a, c, g, or t

<220>
<221> misc_feature
<222> (14)..(14)
<223> n is a, c, g, or t

<400> 47
aaactcttca ndtndtaaca atatggacgt tattaacatg 40

<210> 48
<211> 39
<212> DNA
<213> Artificial Sequence

<220>
<223> Synthetic primer: P4368

<220>
<221> misc_feature
<222> (11)..(11)
<223> n is a, c, g, or t

<220>
<221> misc_feature
<222> (14)..(14)
<223> n is a, c, g, or t

<400> 48
aaactcttca ndtndtaata tggacgttat taacatgag 39

<210> 49
<211> 40
<212> DNA
<213> Artificial Sequence

<220>
<223> Synthetic primer: P4369

<220>
<221> misc_feature
<222> (11)..(11)
<223> n is a, c, g, or t

<220>
<221> misc_feature
<222> (14)..(14)
<223> n is a, c, g, or t

<400> 49
aaactcttca ndtndtatgg acgttattaa catgagcctc 40

<210> 50
<211> 37
<212> DNA
<213> Artificial Sequence

<220>
<223> Synthetic primer: P4370

<220>
<221> misc_feature
<222> (11)..(11)
<223> n is a, c, g, or t

<220>
<221> misc_feature
<222> (14)..(14)

<223>  n is a, c, g, or t

<400>  50
aaactcttca ndtndtgacg ttattaacat gagcctc 37

<210>  51
<211>  40
<212>  DNA
<213>  Artificial Sequence

<220>
<223>  Synthetic primer:   P4371

<220>
<221>  misc_feature
<222>  (11)..(11)
<223>  n is a, c, g, or t

<220>
<221>  misc_feature
<222>  (14)..(14)
<223>  n is a, c, g, or t

<400>  51
aaactcttca ndtndtgtta ttaacatgag cctcggcgga 40

<210>  52
<211>  40
<212>  DNA
<213>  Artificial Sequence

<220>
<223>  Synthetic primer:   P4372

<220>
<221>  misc_feature
<222>  (11)..(11)
<223>  n is a, c, g, or t

<220>
<221>  misc_feature
<222>  (14)..(14)
<223>  n is a, c, g, or t

<400>  52
aaactcttca ndtndtatta acatgagcct cggcggacct 40

<210>  53
<211>  40
<212>  DNA
<213>  Artificial Sequence

<220>
<223>  Synthetic primer:   P4373

<220>
<221>  misc_feature

<222> (11)..(11)
<223> n is a, c, g, or t

<220>
<221> misc_feature
<222> (14)..(14)
<223> n is a, c, g, or t

<400> 53
aaactcttca ndtndtaaca tgagcctcgg cggaccttct 40

<210> 54
<211> 40
<212> DNA
<213> Artificial Sequence

<220>
<223> Synthetic primer: P4374

<220>
<221> misc_feature
<222> (11)..(11)
<223> n is a, c, g, or t

<220>
<221> misc_feature
<222> (14)..(14)
<223> n is a, c, g, or t

<400> 54
aaactcttca ndtndtatga gcctcggcgg accttctggt 40

<210> 55
<211> 40
<212> DNA
<213> Artificial Sequence

<220>
<223> Synthetic primer: P4375

<220>
<221> misc_feature
<222> (11)..(11)
<223> n is a, c, g, or t

<220>
<221> misc_feature
<222> (14)..(14)
<223> n is a, c, g, or t

<400> 55
aaactcttca ndtndtagcc tcggcggacc ttctggttct 40

<210> 56
<211> 40
<212> DNA
<213> Artificial Sequence

<220>
<223> Synthetic primer: P4376

<220>
<221> misc_feature
<222> (11)..(11)
<223> n is a, c, g, or t

<220>
<221> misc_feature
<222> (14)..(14)
<223> n is a, c, g, or t

<400> 56
aaactcttca ndtndtctcg gcggaccttc tggttctgct 40

<210> 57
<211> 40
<212> DNA
<213> Artificial Sequence

<220>
<223> Synthetic primer: P4377

<220>
<221> misc_feature
<222> (11)..(11)
<223> n is a, c, g, or t

<220>
<221> misc_feature
<222> (14)..(14)
<223> n is a, c, g, or t

<400> 57
aaactcttca ndtndtggcg gaccttctgg ttctgctgct 40

<210> 58
<211> 40
<212> DNA
<213> Artificial Sequence

<220>
<223> Synthetic primer: P4378

<220>
<221> misc_feature
<222> (11)..(11)
<223> n is a, c, g, or t

<220>
<221> misc_feature
<222> (14)..(14)
<223> n is a, c, g, or t

<400> 58
aaactcttca ndtndtggac cttctggttc tgctgcttta 40

<210> 59
<211> 40
<212> DNA
<213> Artificial Sequence

<220>
<223> Synthetic primer: P4379

<220>
<221> misc_feature
<222> (11)..(11)
<223> n is a, c, g, or t

<220>
<221> misc_feature
<222> (14)..(14)
<223> n is a, c, g, or t

<400> 59
aaactcttca ndtndtcctt ctggttctgc tgctttaaaa 40

<210> 60
<211> 38
<212> DNA
<213> Artificial Sequence

<220>
<223> Synthetic primer: P4380

<220>
<221> misc_feature
<222> (11)..(11)
<223> n is a, c, g, or t

<220>
<221> misc_feature
<222> (14)..(14)
<223> n is a, c, g, or t

<400> 60
aaactcttca ndtndttctg gttctgctgc tttaaaag 38

<210> 61
<211> 40
<212> DNA
<213> Artificial Sequence

<220>
<223> Synthetic primer: P4381

<220>
<221> misc_feature
<222> (11)..(11)
<223> n is a, c, g, or t

<220>
<221> misc_feature

<222> (14)..(14)
<223> n is a, c, g, or t

<400> 61
aaactcttca ndtndtggtt ctgctgcttt aaaagcggca 40

<210> 62
<211> 40
<212> DNA
<213> Artificial Sequence

<220>
<223> Synthetic primer: P4382

<220>
<221> misc_feature
<222> (11)..(11)
<223> n is a, c, g, or t

<220>
<221> misc_feature
<222> (14)..(14)
<223> n is a, c, g, or t

<400> 62
aaactcttca ndtndttctg ctgctttaaa agcggcagtt 40

<210> 63
<211> 40
<212> DNA
<213> Artificial Sequence

<220>
<223> Synthetic primer: P4383

<220>
<221> misc_feature
<222> (11)..(11)
<223> n is a, c, g, or t

<220>
<221> misc_feature
<222> (14)..(14)
<223> n is a, c, g, or t

<400> 63
aaactcttca ndtndtgctg ctttaaaagc ggcagttgat 40

<210> 64
<211> 37
<212> DNA
<213> Artificial Sequence

<220>
<223> Synthetic primer: P4384

<220>

<221> misc_feature
<222> (11)..(11)
<223> n is a, c, g, or t

<400> 64
aaactcttca ndtgctgctt taaaagcggc agttgat 37

<210> 65
<211> 37
<212> DNA
<213> Artificial Sequence

<220>
<223> Synthetic primer: P4385

<220>
<221> misc_feature
<222> (13)..(13)
<223> n is a, c, g, or t

<400> 65
aaactcttca ahngttgtct tggaaaggat ttgtttc 37

<210> 66
<211> 40
<212> DNA
<213> Artificial Sequence

<220>
<223> Synthetic primer: P4386

<220>
<221> misc_feature
<222> (13)..(13)
<223> n is a, c, g, or t

<220>
<221> misc_feature
<222> (16)..(16)
<223> n is a, c, g, or t

<400> 66
aaactcttca ahnahngttg tcttggaaag gatttgtttc 40

<210> 67
<211> 40
<212> DNA
<213> Artificial Sequence

<220>
<223> Synthetic primer: P4387

<220>
<221> misc_feature
<222> (13)..(13)
<223> n is a, c, g, or t

<220>
<221> misc_feature
<222> (16)..(16)
<223> n is a, c, g, or t

<400> 67
aaactcttca ahnahngttg ttgtcttgga aaggatttgt 40

<210> 68
<211> 40
<212> DNA
<213> Artificial Sequence

<220>
<223> Synthetic primer: P4388

<220>
<221> misc_feature
<222> (13)..(13)
<223> n is a, c, g, or t

<220>
<221> misc_feature
<222> (16)..(16)
<223> n is a, c, g, or t

<400> 68
aaactcttca ahnahnagag ttgttgtctt ggaaaggatt 40

<210> 69
<211> 40
<212> DNA
<213> Artificial Sequence

<220>
<223> Synthetic primer: P4389

<220>
<221> misc_feature
<222> (13)..(13)
<223> n is a, c, g, or t

<220>
<221> misc_feature
<222> (16)..(16)
<223> n is a, c, g, or t

<400> 69
aaactcttca ahnahngtga gagttgttgt cttggaaagg 40

<210> 70
<211> 40
<212> DNA
<213> Artificial Sequence

<220>
<223> Synthetic primer: P4390

<220>
<221> misc_feature
<222> (13)..(13)
<223> n is a, c, g, or t

<220>
<221> misc_feature
<222> (16)..(16)
<223> n is a, c, g, or t

<400> 70
aaactcttca ahnahntccg tgagagttgt tgtcttggaa 40

<210> 71
<211> 40
<212> DNA
<213> Artificial Sequence

<220>
<223> Synthetic primer: P4391

<220>
<221> misc_feature
<222> (13)..(13)
<223> n is a, c, g, or t

<220>
<221> misc_feature
<222> (16)..(16)
<223> n is a, c, g, or t

<400> 71
aaactcttca ahnahnagtt ccgtgagagt tgttgtcttg 40

<210> 72
<211> 40
<212> DNA
<213> Artificial Sequence

<220>
<223> Synthetic primer: P4392

<220>
<221> misc_feature
<222> (13)..(13)
<223> n is a, c, g, or t

<220>
<221> misc_feature
<222> (16)..(16)
<223> n is a, c, g, or t

<400> 72
aaactcttca ahnahngtga gttccgtgag agttgttgtc 40

<210> 73
<211> 40

<212> DNA
<213> Artificial Sequence

<220>
<223> Synthetic primer: P4393

<220>
<221> misc_feature
<222> (13)..(13)
<223> n is a, c, g, or t

<220>
<221> misc_feature
<222> (16)..(16)
<223> n is a, c, g, or t

<400> 73
aaactcttca ahnahnaacg tgagttccgt gagagttgtt 40

<210> 74
<211> 40
<212> DNA
<213> Artificial Sequence

<220>
<223> Synthetic primer: P4394

<220>
<221> misc_feature
<222> (13)..(13)
<223> n is a, c, g, or t

<220>
<221> misc_feature
<222> (16)..(16)
<223> n is a, c, g, or t

<400> 74
aaactcttca ahnahnggca acgtgagttc cgtgagagtt 40

<210> 75
<211> 40
<212> DNA
<213> Artificial Sequence

<220>
<223> Synthetic primer: P4395

<220>
<221> misc_feature
<222> (13)..(13)
<223> n is a, c, g, or t

<220>
<221> misc_feature
<222> (16)..(16)
<223> n is a, c, g, or t

<400> 75
aaactcttca ahnahngccg gcaacgtgag ttccgtgaga 40

<210> 76
<211> 40
<212> DNA
<213> Artificial Sequence

<220>
<223> Synthetic primer: P4396

<220>
<221> misc_feature
<222> (13)..(13)
<223> n is a, c, g, or t

<220>
<221> misc_feature
<222> (16)..(16)
<223> n is a, c, g, or t

<400> 76
aaactcttca ahnahntgtg ccggcaacgt gagttccgtg 40

<210> 77
<211> 40
<212> DNA
<213> Artificial Sequence

<220>
<223> Synthetic primer: P4397

<220>
<221> misc_feature
<222> (13)..(13)
<223> n is a, c, g, or t

<220>
<221> misc_feature
<222> (16)..(16)
<223> n is a, c, g, or t

<400> 77
aaactcttca ahnahnaact gtgccggcaa cgtgagttcc 40

<210> 78
<211> 40
<212> DNA
<213> Artificial Sequence

<220>
<223> Synthetic primer: P4398

<220>
<221> misc_feature
<222> (13)..(13)
<223> n is a, c, g, or t

<220>
<221> misc_feature
<222> (16)..(16)
<223> n is a, c, g, or t

<400> 78
aaactcttca ahnahncgca actgtgccgg caacgtgagt 40

<210> 79
<211> 40
<212> DNA
<213> Artificial Sequence

<220>
<223> Synthetic primer: P4399

<220>
<221> misc_feature
<222> (13)..(13)
<223> n is a, c, g, or t

<220>
<221> misc_feature
<222> (16)..(16)
<223> n is a, c, g, or t

<400> 79
aaactcttca ahnahnagcc gcaactgtgc cggcaacgtg 40

<210> 80
<211> 40
<212> DNA
<213> Artificial Sequence

<220>
<223> Synthetic primer: P4400

<220>
<221> misc_feature
<222> (13)..(13)
<223> n is a, c, g, or t

<220>
<221> misc_feature
<222> (16)..(16)
<223> n is a, c, g, or t

<400> 80
aaactcttca ahnahnaaga gccgcaactg tgccggcaac 40

<210> 81
<211> 37
<212> DNA
<213> Artificial Sequence

<220>
<223> Synthetic primer: P4401

```
<220>
<221>  misc_feature
<222>  (13)..(13)
<223>  n is a, c, g, or t

<400>  81
aaactcttca ahngtaaagt gatgcgcttg gcgcaac                              37


<210>  82
<211>  40
<212>  DNA
<213>  Artificial Sequence

<220>
<223>  Synthetic primer:   P4402


<220>
<221>  misc_feature
<222>  (13)..(13)
<223>  n is a, c, g, or t

<220>
<221>  misc_feature
<222>  (16)..(16)
<223>  n is a, c, g, or t

<400>  82
aaactcttca ahnahngtaa agtgatgcgc ttggcgcaac                           40


<210>  83
<211>  36
<212>  DNA
<213>  Artificial Sequence

<220>
<223>  Synthetic primer:   P4403


<220>
<221>  misc_feature
<222>  (13)..(13)
<223>  n is a, c, g, or t

<220>
<221>  misc_feature
<222>  (16)..(16)
<223>  n is a, c, g, or t

<400>  83
aaactcttca ahnahnagcg taaagtgatg cgcttg                               36


<210>  84
<211>  39
<212>  DNA
<213>  Artificial Sequence

<220>
```

<223> Synthetic primer: P4404

<220>
<221> misc_feature
<222> (13)..(13)
<223> n is a, c, g, or t

<220>
<221> misc_feature
<222> (16)..(16)
<223> n is a, c, g, or t

<400> 84
aaactcttca ahnahntaca gcgtaaagtg atgcgcttg 39

<210> 85
<211> 40
<212> DNA
<213> Artificial Sequence

<220>
<223> Synthetic primer: P4405

<220>
<221> misc_feature
<222> (13)..(13)
<223> n is a, c, g, or t

<220>
<221> misc_feature
<222> (16)..(16)
<223> n is a, c, g, or t

<400> 85
aaactcttca ahnahntttt acagcgtaaa gtgatgcgct 40

<210> 86
<211> 39
<212> DNA
<213> Artificial Sequence

<220>
<223> Synthetic primer: P4406

<220>
<221> misc_feature
<222> (13)..(13)
<223> n is a, c, g, or t

<220>
<221> misc_feature
<222> (16)..(16)
<223> n is a, c, g, or t

<400> 86
aaactcttca ahnahnaact tttacagcgt aaagtgatg 39

<210> 87
<211> 40
<212> DNA
<213> Artificial Sequence

<220>
<223> Synthetic primer: P4407

<220>
<221> misc_feature
<222> (13)..(13)
<223> n is a, c, g, or t

<220>
<221> misc_feature
<222> (16)..(16)
<223> n is a, c, g, or t

<400> 87
aaactcttca ahnahngaga acttttacag cgtaaagtga 40

<210> 88
<211> 40
<212> DNA
<213> Artificial Sequence

<220>
<223> Synthetic primer: P4408

<220>
<221> misc_feature
<222> (13)..(13)
<223> n is a, c, g, or t

<220>
<221> misc_feature
<222> (16)..(16)
<223> n is a, c, g, or t

<400> 88
aaactcttca ahnahnaccg agaactttta cagcgtaaag 40

<210> 89
<211> 40
<212> DNA
<213> Artificial Sequence

<220>
<223> Synthetic primer: P4409

<220>
<221> misc_feature
<222> (13)..(13)
<223> n is a, c, g, or t

<220>
<221> misc_feature
<222> (16)..(16)

<223> n is a, c, g, or t

<400> 89
aaactcttca ahnahnagca ccgagaactt ttacagcgta 40

<210> 90
<211> 39
<212> DNA
<213> Artificial Sequence

<220>
<223> Synthetic primer: P4410

<220>
<221> misc_feature
<222> (13)..(13)
<223> n is a, c, g, or t

<220>
<221> misc_feature
<222> (16)..(16)
<223> n is a, c, g, or t

<400> 90
aaactcttca ahnahngtca gcaccgagaa cttttacag 39

<210> 91
<211> 40
<212> DNA
<213> Artificial Sequence

<220>
<223> Synthetic primer: P4411

<220>
<221> misc_feature
<222> (13)..(13)
<223> n is a, c, g, or t

<220>
<221> misc_feature
<222> (16)..(16)
<223> n is a, c, g, or t

<400> 91
aaactcttca ahnahnaccg tcagcaccga gaacttttac 40

<210> 92
<211> 40
<212> DNA
<213> Artificial Sequence

<220>
<223> Synthetic primer: P4412

<220>
<221> misc_feature

<222> (13)..(13)
<223> n is a, c, g, or t

<220>
<221> misc_feature
<222> (16)..(16)
<223> n is a, c, g, or t

<400> 92
aaactcttca ahnahnggaa ccgtcagcac cgagaacttt 40

<210> 93
<211> 40
<212> DNA
<213> Artificial Sequence

<220>
<223> Synthetic primer: P4413

<220>
<221> misc_feature
<222> (13)..(13)
<223> n is a, c, g, or t

<220>
<221> misc_feature
<222> (16)..(16)
<223> n is a, c, g, or t

<400> 93
aaactcttca ahnahngccg gaaccgtcag caccgagaac 40

<210> 94
<211> 40
<212> DNA
<213> Artificial Sequence

<220>
<223> Synthetic primer: P4414

<220>
<221> misc_feature
<222> (13)..(13)
<223> n is a, c, g, or t

<220>
<221> misc_feature
<222> (16)..(16)
<223> n is a, c, g, or t

<400> 94
aaactcttca ahnahnttgg ccggaaccgt cagcaccgag 40

<210> 95
<211> 39
<212> DNA
<213> Artificial Sequence

<220>
<223> Synthetic primer: P4415

<220>
<221> misc_feature
<222> (13)..(13)
<223> n is a, c, g, or t

<220>
<221> misc_feature
<222> (16)..(16)
<223> n is a, c, g, or t

<400> 95
aaactcttca ahnahngtat tggccggaac cgtcagcac 39

<210> 96
<211> 39
<212> DNA
<213> Artificial Sequence

<220>
<223> Synthetic primer: P4416

<220>
<221> misc_feature
<222> (13)..(13)
<223> n is a, c, g, or t

<220>
<221> misc_feature
<222> (16)..(16)
<223> n is a, c, g, or t

<400> 96
aaactcttca ahnahngctg tattggccgg aaccgtcag 39

<210> 97
<211> 40
<212> DNA
<213> Artificial Sequence

<220>
<223> Synthetic primer: P4417

<220>
<221> misc_feature
<222> (13)..(13)
<223> n is a, c, g, or t

<220>
<221> misc_feature
<222> (16)..(16)
<223> n is a, c, g, or t

<400> 97
aaactcttca ahnahnccag ctgtattggc cggaaccgtc 40

<210> 98
<211> 39
<212> DNA
<213> Artificial Sequence

<220>
<223> Synthetic primer: P4418

<220>
<221> misc_feature
<222> (13)..(13)
<223> n is a, c, g, or t

<220>
<221> misc_feature
<222> (16)..(16)
<223> n is a, c, g, or t

<400> 98
aaactcttca ahnahngatc cagctgtatt ggccggaac 39

<210> 99
<211> 40
<212> DNA
<213> Artificial Sequence

<220>
<223> Synthetic primer: P4419

<220>
<221> misc_feature
<222> (13)..(13)
<223> n is a, c, g, or t

<220>
<221> misc_feature
<222> (16)..(16)
<223> n is a, c, g, or t

<400> 99
aaactcttca ahnahnaatg atccagctgt attggccgga 40

<210> 100
<211> 37
<212> DNA
<213> Artificial Sequence

<220>
<223> Synthetic primer: P4420

<220>
<221> misc_feature
<222> (13)..(13)
<223> n is a, c, g, or t

<220>
<221> misc_feature

<222> (16)..(16)
<223> n is a, c, g, or t

<400> 100
aaactcttca ahnahngtta atgatccagc tgtattg 37

<210> 101
<211> 40
<212> DNA
<213> Artificial Sequence

<220>
<223> Synthetic primer: P4421

<220>
<221> misc_feature
<222> (13)..(13)
<223> n is a, c, g, or t

<220>
<221> misc_feature
<222> (16)..(16)
<223> n is a, c, g, or t

<400> 101
aaactcttca ahnahntccg ttaatgatcc agctgtattg 40

<210> 102
<211> 40
<212> DNA
<213> Artificial Sequence

<220>
<223> Synthetic primer: P4422

<220>
<221> misc_feature
<222> (13)..(13)
<223> n is a, c, g, or t

<220>
<221> misc_feature
<222> (16)..(16)
<223> n is a, c, g, or t

<400> 102
aaactcttca ahnahngatt ccgttaatga tccagctgta 40

<210> 103
<211> 40
<212> DNA
<213> Artificial Sequence

<220>
<223> Synthetic primer: P4423

<220>

<221> misc_feature
<222> (13)..(13)
<223> n is a, c, g, or t

<220>
<221> misc_feature
<222> (16)..(16)
<223> n is a, c, g, or t

<400> 103
aaactcttca ahnahnctcg attccgttaa tgatccagct 40

<210> 104
<211> 40
<212> DNA
<213> Artificial Sequence

<220>
<223> Synthetic primer: P4424

<220>
<221> misc_feature
<222> (13)..(13)
<223> n is a, c, g, or t

<220>
<221> misc_feature
<222> (16)..(16)
<223> n is a, c, g, or t

<400> 104
aaactcttca ahnahnccac tcgattccgt taatgatcca 40

<210> 105
<211> 40
<212> DNA
<213> Artificial Sequence

<220>
<223> Synthetic primer: P4425

<220>
<221> misc_feature
<222> (13)..(13)
<223> n is a, c, g, or t

<220>
<221> misc_feature
<222> (16)..(16)
<223> n is a, c, g, or t

<400> 105
aaactcttca ahnahncgcc cactcgattc cgttaatgat 40

<210> 106
<211> 40
<212> DNA
<213> Artificial Sequence

<220>
<223> Synthetic primer: P4426

<220>
<221> misc_feature
<222> (13)..(13)
<223> n is a, c, g, or t

<220>
<221> misc_feature
<222> (16)..(16)
<223> n is a, c, g, or t

<400> 106
aaactcttca ahnahngatc gcccactcga ttccgttaat 40

<210> 107
<211> 40
<212> DNA
<213> Artificial Sequence

<220>
<223> Synthetic primer: P4427

<220>
<221> misc_feature
<222> (13)..(13)
<223> n is a, c, g, or t

<220>
<221> misc_feature
<222> (16)..(16)
<223> n is a, c, g, or t

<400> 107
aaactcttca ahnahntgcg atcgcccact cgattccgtt 40

<210> 108
<211> 40
<212> DNA
<213> Artificial Sequence

<220>
<223> Synthetic primer: P4428

<220>
<221> misc_feature
<222> (13)..(13)
<223> n is a, c, g, or t

<220>
<221> misc_feature
<222> (16)..(16)
<223> n is a, c, g, or t

<400> 108
aaactcttca ahnahngttt gcgatcgccc actcgattcc 40

<210> 109
<211> 40
<212> DNA
<213> Artificial Sequence

<220>
<223> Synthetic primer: P4429

<220>
<221> misc_feature
<222> (13)..(13)
<223> n is a, c, g, or t

<220>
<221> misc_feature
<222> (16)..(16)
<223> n is a, c, g, or t

<400> 109
aaactcttca ahnahnattg tttgcgatcg cccactcgat 40

<210> 110
<211> 40
<212> DNA
<213> Artificial Sequence

<220>
<223> Synthetic primer: P4430

<220>
<221> misc_feature
<222> (13)..(13)
<223> n is a, c, g, or t

<220>
<221> misc_feature
<222> (16)..(16)
<223> n is a, c, g, or t

<400> 110
aaactcttca ahnahncata ttgtttgcga tcgcccactc 40

<210> 111
<211> 40
<212> DNA
<213> Artificial Sequence

<220>
<223> Synthetic primer: P4431

<220>
<221> misc_feature
<222> (13)..(13)
<223> n is a, c, g, or t

<220>

<221> misc_feature
<222> (16)..(16)
<223> n is a, c, g, or t

<400> 111
aaactcttca ahnahngtcc atattgtttg cgatcgccca 40

<210> 112
<211> 40
<212> DNA
<213> Artificial Sequence

<220>
<223> Synthetic primer: P4432

<220>
<221> misc_feature
<222> (13)..(13)
<223> n is a, c, g, or t

<220>
<221> misc_feature
<222> (16)..(16)
<223> n is a, c, g, or t

<400> 112
aaactcttca ahnahnaacg tccatattgt ttgcgatcgc 40

<210> 113
<211> 40
<212> DNA
<213> Artificial Sequence

<220>
<223> Synthetic primer: P4433

<220>
<221> misc_feature
<222> (13)..(13)
<223> n is a, c, g, or t

<220>
<221> misc_feature
<222> (16)..(16)
<223> n is a, c, g, or t

<400> 113
aaactcttca ahnahnaata acgtccatat tgtttgcgat 40

<210> 114
<211> 40
<212> DNA
<213> Artificial Sequence

<220>
<223> Synthetic primer: P4434

<220>
<221> misc_feature
<222> (13)..(13)
<223> n is a, c, g, or t

<220>
<221> misc_feature
<222> (16)..(16)
<223> n is a, c, g, or t

<400> 114
aaactcttca ahnahngtta ataacgtcca tattgtttgc 40

<210> 115
<211> 40
<212> DNA
<213> Artificial Sequence

<220>
<223> Synthetic primer: P4435

<220>
<221> misc_feature
<222> (13)..(13)
<223> n is a, c, g, or t

<220>
<221> misc_feature
<222> (16)..(16)
<223> n is a, c, g, or t

<400> 115
aaactcttca ahnahncatg ttaataacgt ccatattgtt 40

<210> 116
<211> 40
<212> DNA
<213> Artificial Sequence

<220>
<223> Synthetic primer: P4436

<220>
<221> misc_feature
<222> (13)..(13)
<223> n is a, c, g, or t

<220>
<221> misc_feature
<222> (16)..(16)
<223> n is a, c, g, or t

<400> 116
aaactcttca ahnahngctc atgttaataa cgtccatatt 40

<210> 117
<211> 40
<212> DNA

<213> Artificial Sequence

<220>
<223> Synthetic primer: P4437

<220>
<221> misc_feature
<222> (13)..(13)
<223> n is a, c, g, or t

<220>
<221> misc_feature
<222> (16)..(16)
<223> n is a, c, g, or t

<400> 117
aaactcttca ahnahngagg ctcatgttaa taacgtccat 40

<210> 118
<211> 40
<212> DNA
<213> Artificial Sequence

<220>
<223> Synthetic primer: P4438

<220>
<221> misc_feature
<222> (13)..(13)
<223> n is a, c, g, or t

<220>
<221> misc_feature
<222> (16)..(16)
<223> n is a, c, g, or t

<400> 118
aaactcttca ahnahngccg aggctcatgt taataacgtc 40

<210> 119
<211> 40
<212> DNA
<213> Artificial Sequence

<220>
<223> Synthetic primer: P4439

<220>
<221> misc_feature
<222> (13)..(13)
<223> n is a, c, g, or t

<220>
<221> misc_feature
<222> (16)..(16)
<223> n is a, c, g, or t

<400> 119

aaactcttca ahnahntccg ccgaggctca tgttaataac 40

<210> 120
<211> 40
<212> DNA
<213> Artificial Sequence

<220>
<223> Synthetic primer: P4440

<220>
<221> misc_feature
<222> (13)..(13)
<223> n is a, c, g, or t

<220>
<221> misc_feature
<222> (16)..(16)
<223> n is a, c, g, or t

<400> 120
aaactcttca ahnahnaggt ccgccgaggc tcatgttaat 40

<210> 121
<211> 40
<212> DNA
<213> Artificial Sequence

<220>
<223> Synthetic primer: P4441

<220>
<221> misc_feature
<222> (13)..(13)
<223> n is a, c, g, or t

<220>
<221> misc_feature
<222> (16)..(16)
<223> n is a, c, g, or t

<400> 121
aaactcttca ahnahnagaa ggtccgccga ggctcatgtt 40

<210> 122
<211> 22
<212> DNA
<213> Artificial Sequence

<220>
<223> Synthetic primer: P4299

<400> 122
cgttgaagaa gatcacgtag ca 22

<210> 123
<211> 26

<212> DNA
<213> Artificial Sequence

<220>
<223> Synthetic primer: P3246

<400> 123
tttattttat aaactcattc cctgat 26

<210> 124
<211> 1351
<212> DNA
<213> Artificial Sequence

<220>
<223> Polynucleotide sequence of the expression cassette from the pHPLT vector (Plat promoter-pre-pro-BPN'-Y217L-terminator)

<400> 124
gcttttcttt tggaagaaaa tatagggaaa atggtacttg ttaaaaattc ggaatattta 60

tacaatatca tatgtttcac attgaaaggg gaggaaaatc gtgaaacaac aaaaacggct 120

ttagtctagc aaaaggagag ggtaaagagt gagaagcaaa aaattgtgga tcagtttgct 180

gtttgcttta gcgttaatct ttacgatggc gttcggcagc acatcctctg cccaggcggc 240

agggaaatca aacggggaaa agaaatatat tgtcgggttt aaacagacaa tgagcacgat 300

gagcgccgct aagaagaaag atgtcatttc tgaaaaaggc gggaaagtgc aaaagcaatt 360

caaatatgta gacgcagctt cagctacatt aaacgaaaaa gctgtaaaag aattgaaaaa 420

agacccgagc gtcgcttacg ttgaagaaga tcacgtagca cacgcgtacg cgcagtccgt 480

gccttacggc gtatcacaaa ttaaagcccc tgctctgcac tctcaaggct acactggatc 540

aaatgttaaa gtagcggtta tcgacagcgg tatcgattct tctcatcctg atttaaaggt 600

agcaggcgga gccagcatgg ttccttctga aacaaatcct ttccaagaca acaactctca 660

cggaactcac gttgccggca cagttgcggc tcttaataac tcaatcggtg tattaggcgt 720

tgcgccaagc gcatcacttt acgctgtaaa agttctcggt gctgacggtt ccggccaata 780

cagctggatc attaacggaa tcgagtgggc gatcgcaaac aatatggacg ttattaacat 840

gagcctcggc ggaccttctg gttctgctgc tttaaaagcg gcagttgata aagccgttgc 900

atccggcgtc gtagtcgttg cggcagccgg taacgaaggc acttccggca gctcaagcac 960

agtgggctac cctggtaaat acccttctgt cattgcagta ggcgctgttg acagcagcaa 1020

ccaaagagca tctttctcaa gcgtaggacc tgagcttgat gtcatggcac ctggcgtatc 1080

tatccaaagc acgcttcctg gaaacaaata cggcgcgttg aacggtacat caatggcatc 1140

tccgcacgtt gccggagcgg ctgctttgat tctttctaag cacccgaact ggacaaacac 1200

tcaagtccgc agcagtttag aaaacaccac tacaaaactt ggtgattctt tctactatgg 1260

aaaagggctg atcaacgtac aggcggcagc tcagtaaact cgagagagga cggatttcct 1320

gaaggaaatc cgttttttta ttttaagctt g 1351

<210> 125
<211> 23
<212> DNA
<213> Artificial Sequence

<220>
<223> Synthetic primer: P4948

<400> 125
gtgtttttct tggaattgtg ctg 23

<210> 126
<211> 21
<212> DNA
<213> Artificial Sequence

<220>
<223> Synthetic primer: P4974

<400> 126
gcctcacatt tgtgccacct a 21

<210> 127
<211> 23
<212> DNA
<213> Artificial Sequence

<220>
<223> Synthetic primer: P4975

<400> 127
catatgagtt atgcagtttg tag 23

<210> 128
<211> 22
<212> DNA
<213> Artificial Sequence

<220>
<223> Synthetic primer: P4976

<400> 128
cctctcggtt atgagttagt tc 22

<210> 129
<211> 23
<212> DNA
<213> Artificial Sequence

<220>
<223> Synthetic primer: P4977

<400> 129
gtagagcgaa gcagacgggg cta 23

<210> 130
<211> 24
<212> DNA
<213> Artificial Sequence

<220>
<223> Synthetic primer: P4978

<400> 130
cttaaagcag cagttgataa agct 24

<210> 131
<211> 22
<212> DNA
<213> Artificial Sequence

<220>
<223> Synthetic primer: P4979

<400> 131
cttggtgtag ccccgtctgc tt 22

<210> 132
<211> 26
<212> DNA
<213> Artificial Sequence

<220>
<223> Synthetic primer: P4980

<400> 132
atccatgtta ttcgcgatgg cccatt 26

<210> 133
<211> 80
<212> DNA
<213> Artificial Sequence

<220>
<223> Synthetic primer: P4981

<400> 133
cttggtgtag ccccgtctgc ttcgctctac gcctcttgct ctgcagcaga cggatcaggc 60

caatactcat ggattatcaa 80

<210> 134
<211> 80
<212> DNA
<213> Artificial Sequence

<220>
<223> Synthetic primer: P4982

<400> 134
atccatgtta ttcgcgatgg cccattcgat gccgttgata atccatgagt attggcctga 60

tccgtctgct gcagagcaag 80

<210> 135
<211> 80
<212> DNA
<213> Artificial Sequence

<220>
<223> Synthetic primer: P4983

<400> 135
cttggtgtag ccccgtctgc ttcgctctac gccgttaaag ttcttgatgc acgtgacgga 60

tcaggccaat actcatggat 80

<210> 136
<211> 80
<212> DNA
<213> Artificial Sequence

<220>
<223> Synthetic primer: P4984

<400> 136
atccatgtta ttcgcgatgg cccattcgat gccgttgata atccatgagt attggcctga 60

tccgtcacgt gcatcaagaa 80

<210> 137
<211> 80
<212> DNA
<213> Artificial Sequence

<220>
<223> Synthetic primer: P4985

<400> 137
cttggtgtag ccccgtctgc ttcgctctac gccgttaaag ttcttgcagc agactctgga 60

tcaggccaat actcatggat 80

<210> 138
<211> 80
<212> DNA
<213> Artificial Sequence

<220>
<223> Synthetic primer: P4986

<400> 138
atccatgtta ttcgcgatgg cccattcgat gccgttgata atccatgagt attggcctga 60

tccagagtct gctgcaagaa 80

<210> 139
<211> 80
<212> DNA
<213> Artificial Sequence

<220>
<223>  Synthetic primer:   P4987

<400>  139
cttggtgtag ccccgtctgc ttcgctctac gccgttaaag ttcttgcagc agactcttca        60

ggccaatact catggattat                                                    80

<210>  140
<211>  80
<212>  DNA
<213>  Artificial Sequence

<220>
<223>  Synthetic primer:   P4988

<400>  140
atccatgtta ttcgcgatgg cccattcgat gccgttgata atccatgagt attggcctga        60

agagtctgct gcaagaactt                                                    80

<210>  141
<211>  80
<212>  DNA
<213>  Artificial Sequence

<220>
<223>  Synthetic primer:   P4989

<400>  141
cttggtgtag ccccgtctgc ttcgctctac gccgttaaag ttcttgcagc agacggagat        60

ggccaatact catggattat                                                    80

<210>  142
<211>  80
<212>  DNA
<213>  Artificial Sequence

<220>
<223>  Synthetic primer:   P4990

<400>  142
atccatgtta ttcgcgatgg cccattcgat gccgttgata atccatgagt attggccatc        60

tccgtctgct gcaagaactt                                                    80

<210>  143
<211>  80
<212>  DNA
<213>  Artificial Sequence

<220>
<223>  Synthetic primer:   P4991

<400>  143
cttggtgtag ccccgtctgc ttcgctctac gccgttaaag ttcttgatgc acgtgactct        60

ggatcaggcc aatactcatg                                                    80

<210> 144
<211> 80
<212> DNA
<213> Artificial Sequence

<220>
<223> Synthetic primer: P4992

<400> 144
atccatgtta ttcgcgatgg cccattcgat gccgttgata atccatgagt attggcctga 60

tccagagtca cgtgcatcaa 80

<210> 145
<211> 80
<212> DNA
<213> Artificial Sequence

<220>
<223> Synthetic primer: P4993

<400> 145
cttggtgtag ccccgtctgc ttcgctctac gccgttaaag ttcttgatgc acgtgactct 60

tcaggccaat actcatggat 80

<210> 146
<211> 80
<212> DNA
<213> Artificial Sequence

<220>
<223> Synthetic primer: P4994

<400> 146
atccatgtta ttcgcgatgg cccattcgat gccgttgata atccatgagt attggcctga 60

agagtcacgt gcatcaagaa 80

<210> 147
<211> 80
<212> DNA
<213> Artificial Sequence

<220>
<223> Synthetic primer: P4995

<400> 147
cttggtgtag ccccgtctgc ttcgctctac gccgttaaag ttcttgatgc acgtgacgga 60

gatggccaat actcatggat 80

<210> 148
<211> 80
<212> DNA
<213> Artificial Sequence

<220>
<223>  Synthetic primer:   P4996

<400>  148
atccatgtta ttcgcgatgg cccattcgat gccgttgata atccatgagt attggccatc        60

tccgtcacgt gcatcaagaa                                                    80

<210>  149
<211>  80
<212>  DNA
<213>  Artificial Sequence

<220>
<223>  Synthetic primer:   P4997

<400>  149
cttggtgtag ccccgtctgc ttcgctctac gccgttaaag ttcttgcagc agactcttct        60

tcaggccaat actcatggat                                                    80

<210>  150
<211>  80
<212>  DNA
<213>  Artificial Sequence

<220>
<223>  Synthetic primer:   P4998

<400>  150
atccatgtta ttcgcgatgg cccattcgat gccgttgata atccatgagt attggcctga        60

agaagagtct gctgcaagaa                                                    80

<210>  151
<211>  80
<212>  DNA
<213>  Artificial Sequence

<220>
<223>  Synthetic primer:   P4999

<400>  151
cttggtgtag ccccgtctgc ttcgctctac gccgttaaag ttcttgcagc agactctgga        60

gatggccaat actcatggat                                                    80

<210>  152
<211>  80
<212>  DNA
<213>  Artificial Sequence

<220>
<223>  Synthetic primer:   P5000

<400>  152
atccatgtta ttcgcgatgg cccattcgat gccgttgata atccatgagt attggccatc        60

tccagagtct gctgcaagaa                                                    80

<210> 153
<211> 80
<212> DNA
<213> Artificial Sequence

<220>
<223> Synthetic primer: P5001

<400> 153
cttggtgtag ccccgtctgc ttcgctctac gccgttaaag ttcttgcagc agactctgat 60

ggccaatact catggattat 80

<210> 154
<211> 80
<212> DNA
<213> Artificial Sequence

<220>
<223> Synthetic primer: P5002

<400> 154
atccatgtta ttcgcgatgg cccattcgat gccgttgata atccatgagt attggccatc 60

agagtctgct gcaagaactt 80

<210> 155
<211> 21
<212> DNA
<213> Artificial Sequence

<220>
<223> Synthetic primer: P5003

<400> 155
atcgaatggg ccatcgcgaa t 21

<210> 156
<211> 22
<212> DNA
<213> Artificial Sequence

<220>
<223> Synthetic primer: P5004

<400> 156
tgcaacagct ttatcaactg ct 22

<210> 157
<211> 66
<212> DNA
<213> Artificial Sequence

<220>
<223> Synthetic primer: P5005

<400> 157

atcgaatggg ccatcgcgaa taacatggat gtatctggaa tgagcctggg agcaccaagc 60

ggcagt 66

<210> 158
<211> 70
<212> DNA
<213> Artificial Sequence

<220>
<223> Synthetic primer: P5006

<400> 158
tgcaacagct ttatcaactg ctgctttaag tgccgcactg ccgcttggtg ctcccaggct 60

cattccagat 70

<210> 159
<211> 66
<212> DNA
<213> Artificial Sequence

<220>
<223> Synthetic primer: P5007

<400> 159
atcgaatggg ccatcgcgaa taacatggat gtaatcaaca tgagcctggg atctccaagc 60

ggcagt 66

<210> 160
<211> 70
<212> DNA
<213> Artificial Sequence

<220>
<223> Synthetic primer: P5008

<400> 160
tgcaacagct ttatcaactg ctgctttaag tgccgcactg ccgcttggag atcccaggct 60

catgttgatt 70

<210> 161
<211> 66
<212> DNA
<213> Artificial Sequence

<220>
<223> Synthetic primer: P5009

<400> 161
atcgaatggg ccatcgcgaa taacatggat gtaatcaaca tgagcctggg agcagttagc 60

ggcagt 66

<210> 162
<211> 70

<212> DNA
<213> Artificial Sequence

<220>
<223> Synthetic primer: P5010

<400> 162
tgcaacagct ttatcaactg ctgctttaag tgccgcactg ccgctaactg ctcccaggct 60

catgttgatt 70

<210> 163
<211> 66
<212> DNA
<213> Artificial Sequence

<220>
<223> Synthetic primer: P5011

<400> 163
atcgaatggg ccatcgcgaa taacatggat gtaatcaaca tgagcctggg agcagatagc 60

ggcagt 66

<210> 164
<211> 70
<212> DNA
<213> Artificial Sequence

<220>
<223> Synthetic primer: P5012

<400> 164
tgcaacagct ttatcaactg ctgctttaag tgccgcactg ccgctatctg ctcccaggct 60

catgttgatt 70

<210> 165
<211> 69
<212> DNA
<213> Artificial Sequence

<220>
<223> Synthetic primer: P5013

<400> 165
atcgaatggg ccatcgcgaa taacatggat gtaatcaaca tgagcctggg agcaagcggc 60

agtgcggca 69

<210> 166
<211> 70
<212> DNA
<213> Artificial Sequence

<220>
<223> Synthetic primer: P5014

<400> 166

tgcaacagct ttatcaactg ctgctttaag tgccgcactg ccgcttgctc ccaggctcat 60

gttgattaca 70

<210> 167
<211> 69
<212> DNA
<213> Artificial Sequence

<220>
<223> Synthetic primer: P5015

<400> 167
atcgaatggg ccatcgcgaa taacatggat gtaatcaaca tgagcctggg agcaggaggc 60

agtgcggca 69

<210> 168
<211> 70
<212> DNA
<213> Artificial Sequence

<220>
<223> Synthetic primer: P5016

<400> 168
tgcaacagct ttatcaactg ctgctttaag tgccgcactg cctcctgctc ccaggctcat 60

gttgattaca 70

<210> 169
<211> 65
<212> DNA
<213> Artificial Sequence

<220>
<223> Synthetic primer: P5017

<400> 169
atcgaatggg ccatcgcgaa taacatggat gtaatcaaca tgagcctggg agcaccagtt 60

gatat 65

<210> 170
<211> 70
<212> DNA
<213> Artificial Sequence

<220>
<223> Synthetic primer: P5018

<400> 170
tgcaacagct ttatcaactg ctgctttaag tgccgcgata tcaactggtg ctcccaggct 60

catgttgatt 70

<210> 171
<211> 66

<212> DNA
<213> Artificial Sequence

<220>
<223> Synthetic primer: P5019

<400> 171
atcgaatggg ccatcgcgaa taacatggat gtaatcaaca tgagcctggg agcaccaagc 60

ggcagt 66

<210> 172
<211> 70
<212> DNA
<213> Artificial Sequence

<220>
<223> Synthetic primer: P5020

<400> 172
tgcaacagct ttatcaactg ctgctttaag tgtcgcactg ccgcttggtg ctcccaggct 60

catgttgatt 70

<210> 173
<211> 66
<212> DNA
<213> Artificial Sequence

<220>
<223> Synthetic primer: P5021

<400> 173
atcgaatggg ccatcgcgaa taacatggat gtatctggaa tgagcctggg atctccaagc 60

ggcagt 66

<210> 174
<211> 70
<212> DNA
<213> Artificial Sequence

<220>
<223> Synthetic primer: P5022

<400> 174
tgcaacagct ttatcaactg ctgctttaag tgccgcactg ccgcttggag atcccaggct 60

cattccagat 70

<210> 175
<211> 66
<212> DNA
<213> Artificial Sequence

<220>
<223> Synthetic primer: P5023

<400> 175

atcgaatggg ccatcgcgaa taacatggat gtatctggaa tgagcctggg agcagtaagc 60

ggcagt 66

<210> 176
<211> 70
<212> DNA
<213> Artificial Sequence

<220>
<223> Synthetic primer: P5024

<400> 176
tgcaacagct ttatcaactg ctgctttaag tgccgcactg ccgcttactg ctcccaggct 60

cattccagat 70

<210> 177
<211> 66
<212> DNA
<213> Artificial Sequence

<220>
<223> Synthetic primer: P5025

<400> 177
atcgaatggg ccatcgcgaa taacatggat gtatctggaa tgagcctggg agcagatagc 60

ggcagt 66

<210> 178
<211> 70
<212> DNA
<213> Artificial Sequence

<220>
<223> Synthetic primer: P5026

<400> 178
tgcaacagct ttatcaactg ctgctttaag tgccgcactg ccgctatctg ctcccaggct 60

cattccagat 70

<210> 179
<211> 69
<212> DNA
<213> Artificial Sequence

<220>
<223> Synthetic primer: P5027

<400> 179
atcgaatggg ccatcgcgaa taacatggat gtatctggaa tgagcctggg agcaagcggc 60

agtgcggca 69

<210> 180
<211> 78

<212> DNA
<213> Artificial Sequence

<220>
<223> Synthetic primer: P5028

<400> 180
tgcaacagct ttatcaactg ctgctttaag tgccgcactg ccgcttgctc ccaggctcat 60

tccagataca tccatgtt 78

<210> 181
<211> 66
<212> DNA
<213> Artificial Sequence

<220>
<223> Synthetic primer: P5029

<400> 181
atcgaatggg ccatcgcgaa taacatggat gtaatcaaca tgagcctggg atctgttagc 60

ggcagt 66

<210> 182
<211> 70
<212> DNA
<213> Artificial Sequence

<220>
<223> Synthetic primer: P5030

<400> 182
tgcaacagct ttatcaactg ctgctttaag tgccgcactg ccgctaacag atcccaggct 60

catgttgatt 70

<210> 183
<211> 66
<212> DNA
<213> Artificial Sequence

<220>
<223> Synthetic primer: P5031

<400> 183
atcgaatggg ccatcgcgaa taacatggat gtaatcaaca tgagcctggg atctgatagc 60

ggcagt 66

<210> 184
<211> 70
<212> DNA
<213> Artificial Sequence

<220>
<223> Synthetic primer: P5032

<400> 184

tgcaacagct ttatcaactg ctgctttaag tgccgcactg ccgctatcag atcccaggct 60

catgttgatt 70

<210> 185
<211> 69
<212> DNA
<213> Artificial Sequence

<220>
<223> Synthetic primer: P5033

<400> 185
atcgaatggg ccatcgcgaa taacatggat gtaatcaaca tgagcctggg atctagcggc 60

agtgcggca 69

<210> 186
<211> 67
<212> DNA
<213> Artificial Sequence

<220>
<223> Synthetic primer: P5034

<400> 186
tgcaacagct ttatcaactg ctgctttaag tgccgcactg ccgctagatc ccaggctcat 60

gttgatt 67

<210> 187
<211> 32
<212> DNA
<213> Artificial Sequence

<220>
<223> Synthetic primer: P5035

<400> 187
tgatccgtct gagcaggggc tttaatttgt ga 32

<210> 188
<211> 37
<212> DNA
<213> Artificial Sequence

<220>
<223> Synthetic primer: P5036

<400> 188
attaaagccc ctgctcagac ggatcaggcc aatactc 37

<210> 189
<211> 44
<212> DNA
<213> Artificial Sequence

<220>

<223> Synthetic primer: 128/129 Stop F

<400> 189
catgagcctg ggataatgaa gcggcagtgc ggcacttaaa gcag 44

<210> 190
<211> 46
<212> DNA
<213> Artificial Sequence

<220>
<223> Synthetic primer: 128/129 Stop R

<400> 190
gtgccgcact gccgcttcat tatcccaggc tcatgttgat tacatc 46

<210> 191
<211> 44
<212> DNA
<213> Artificial Sequence

<220>
<223> Synthetic primer: 128/129 deg QC F

<220>
<221> misc_feature
<222> (14)..(15)
<223> n is a, c, g, or t

<220>
<221> misc_feature
<222> (17)..(18)
<223> n is a, c, g, or t

<400> 191
catgagcctg ggannsnnsa gcggcagtgc ggcacttaaa gcag 44

<210> 192
<211> 43
<212> DNA
<213> Artificial Sequence

<220>
<223> Synthetic primer: 128/129 deg QC R

<220>
<221> misc_feature
<222> (15)..(16)
<223> n is a, c, g, or t

<220>
<221> misc_feature
<222> (18)..(19)
<223> n is a, c, g, or t

<400> 192
ccgcactgcc gctsnnsnnt cccaggctca tgttgattac atc 43

<210> 193
<211> 41
<212> DNA
<213> Artificial Sequence

<220>
<223> Synthetic primer: 128/129 del QC F

<220>
<221> misc_feature
<222> (14)..(15)
<223> n is a, c, g, or t

<400> 193
catgagcctg ggannsagcg gcagtgcggc acttaaagca g 41

<210> 194
<211> 40
<212> DNA
<213> Artificial Sequence

<220>
<223> Synthetic primer: 128/129 del QC R

<220>
<221> misc_feature
<222> (15)..(16)
<223> n is a, c, g, or t

<400> 194
ccgcactgcc gctsnntccc aggctcatgt tgattacatc 40

<210> 195
<211> 47
<212> DNA
<213> Artificial Sequence

<220>
<223> Synthetic primer: 128 A 129 QC F

<220>
<221> misc_feature
<222> (14)..(15)
<223> n is a, c, g, or t

<220>
<221> misc_feature
<222> (20)..(21)
<223> n is a, c, g, or t

<400> 195
catgagcctg ggannsgcgn nsagcggcag tgcggcactt aaagcag 47

<210> 196
<211> 46
<212> DNA

<213> Artificial Sequence

<220>
<223> Synthetic primer: 128 A 129 QC R

<220>
<221> misc_feature
<222> (15)..(16)
<223> n is a, c, g, or t

<220>
<221> misc_feature
<222> (21)..(22)
<223> n is a, c, g, or t

<400> 196
ccgcactgcc gctsnncgcs nntcccaggc tcatgttgat tacatc 46

<210> 197
<211> 41
<212> DNA
<213> Artificial Sequence

<220>
<223> Synthetic primer: 128/129 del A QC F

<220>
<221> misc_feature
<222> (14)..(15)
<223> n is a, c, g, or t

<400> 197
catgagcctg ggannsgcag gcagtgcggc acttaaagca g 41

<210> 198
<211> 43
<212> DNA
<213> Artificial Sequence

<220>
<223> Synthetic primer: 128/129 del A QC R

<220>
<221> misc_feature
<222> (18)..(19)
<223> n is a, c, g, or t

<400> 198
gtgccgcact gcctgcsnnt cccaggctca tgttgattac atc 43

<210> 199
<211> 22
<212> DNA
<213> Artificial Sequence

<220>
<223> Synthetic primer: P4947

<400> 199
ctttagtcta gcaaaaggag ag 22

<210> 200
<211> 23
<212> DNA
<213> Artificial Sequence

<220>
<223> Synthetic primer: P4948

<400> 200
gtgtttttct tggaattgtg ctg 23

<210> 201
<211> 20
<212> DNA
<213> Artificial Sequence

<220>
<223> Synthetic primer: P4949

<400> 201
cgactcgagc catccagatc 20

<210> 202
<211> 24
<212> DNA
<213> Artificial Sequence

<220>
<223> Synthetic primer: P4950

<400> 202
cttgtctcca agcttaaaat aaaa 24

<210> 203
<211> 39
<212> DNA
<213> Artificial Sequence

<220>
<223> Synthetic primer: P4951

<400> 203
aaagttcttg cagcaagtga cggatcaggc caatactca 39

<210> 204
<211> 40
<212> DNA
<213> Artificial Sequence

<220>
<223> Synthetic primer: P4952

<400> 204
gcctgatccg tcacttgctg caagaacttt aacggcgtag 40

<210> 205
<211> 40
<212> DNA
<213> Artificial Sequence

<220>
<223> Synthetic primer: P4953

<400> 205
taaagttctt ggcgcaagtg acggatcagg ccaatactca 40

<210> 206
<211> 38
<212> DNA
<213> Artificial Sequence

<220>
<223> Synthetic primer: P4954

<400> 206
cctgatccgt cacttgcgcc aagaacttta acggcgta 38

<210> 207
<211> 42
<212> DNA
<213> Artificial Sequence

<220>
<223> Synthetic primer: P4955

<400> 207
gttaaagttc ttgatgcacg tgacggatca ggccaatact ca 42

<210> 208
<211> 41
<212> DNA
<213> Artificial Sequence

<220>
<223> Synthetic primer: P4956

<400> 208
gatccgtcac gtgcatcaag aactttaacg gcgtagagcg a 41

<210> 209
<211> 42
<212> DNA
<213> Artificial Sequence

<220>
<223> Synthetic primer: P4957

<400> 209
gttaaagttc ttgatggtcg tgacggatca ggccaatact ca 42

<210> 210

<211> 42
<212> DNA
<213> Artificial Sequence

<220>
<223> Synthetic primer: P4958

<400> 210
gatccgtcac gaccatcaag aactttaacg gcgtagagcg aa 42

<210> 211
<211> 38
<212> DNA
<213> Artificial Sequence

<220>
<223> Synthetic primer: P4959

<400> 211
tgagcctggg agcagtaagc ggcagtgcgg cacttaaa 38

<210> 212
<211> 38
<212> DNA
<213> Artificial Sequence

<220>
<223> Synthetic primer: P4960

<400> 212
gcactgccgc ttactgctcc caggctcatg ttgattac 38

<210> 213
<211> 38
<212> DNA
<213> Artificial Sequence

<220>
<223> Synthetic primer: P4961

<400> 213
ttagcgcagg aggagtaagc ggcagtgcgg cacttaaa 38

<210> 214
<211> 37
<212> DNA
<213> Artificial Sequence

<220>
<223> Synthetic primer: P4962

<400> 214
gcactgccgc ttactcctcc tgcgctaacg ttgatta 37

<210> 215
<211> 37
<212> DNA
<213> Artificial Sequence

<220>
<223> Synthetic primer: P4963

<400> 215
gagcctggga ggagtaagcg gcagtgcggc acttaaa 37

<210> 216
<211> 40
<212> DNA
<213> Artificial Sequence

<220>
<223> Synthetic primer: P4964

<400> 216
tgccgcactg ccgcttactc ctcccaggct catgccgatt 40

<210> 217
<211> 37
<212> DNA
<213> Artificial Sequence

<220>
<223> Synthetic primer: P4965

<400> 217
gagcctggga gcagtaagcg gcagtgcggc acttaaa 37

<210> 218
<211> 36
<212> DNA
<213> Artificial Sequence

<220>
<223> Synthetic primer: P4966

<400> 218
gcactgccgc ttactgctcc caggctcatg ttgatt 36

<210> 219
<211> 34
<212> DNA
<213> Artificial Sequence

<220>
<223> Synthetic primer: P4967

<400> 219
aacgggactt cccaagcctc gccgcatgta gctg 34

<210> 220
<211> 37
<212> DNA
<213> Artificial Sequence

<220>
<223> Synthetic primer: P4968

<400> 220
acatgcggcg aggcttggga agtcccgttt tgcgcac 37

<210> 221
<211> 24
<212> DNA
<213> Artificial Sequence

<220>
<223> Synthetic primer: P4973

<400> 221
aaaggatcct aatcggcgct tttc 24